# EUROPEAN PATENT SPECIFICATION

(11) **EP 2 633 040 B1**
(45) Date of publication and mention of the grant of the patent: **10.07.2019**
(21) Application number: 11782501.8
(22) Date of filing: 27.10.2011
(51) Int. Cl.: C12N 9/22

(54) **METHOD FOR INCREASING THE EFFICIENCY OF DOUBLE-STRAND BREAK-INDUCED MUTAGENESIS**
VERFAHREN ZUR ERHÖHUNG DER WIRKUNG VON DOPPELSTRÄNGIGER BRUCHINDUZIERTER MUTAGENESE
PROCÉDÉ PERMETTANT D'AUGMENTER L'EFFICACITÉ D'UNE MUTAGENÈSE INDUITE PAR DES CASSURES DOUBLE BRIN

(30) Priority: 27.10.2010 US 407339 P; 08.07.2011 US 201161505783 P; 05.04.2011 US 201161472072 P
(43) Date of publication of application: 04.09.2013
(73) Proprietor: CELLECTIS, 75013 Paris (FR)
(72) Inventor: DUCHATEAU, Philippe, 91210 Draveil (FR); JUILLERAT, Alexandre, 10028 New York, NY (US); SILVA, George, H., F-94420 Le Plessis Trevise (FR); EPINAT, Jean-Charles, F-93260 Les Lilas (FR)
(74) Representative: Santarelli
(86) International application number: PCT/US2011/058133
(87) International publication number: WO 2012/058458

(56) References cited:
- WO-A1-2009/095742
- WO-A2-2012/118717
- ROTH TIMOTHY J ET AL: "Human-yeast chimeric repair protein protects mammalian cells against alkylating agents: enhancement of MGMT protection.", CANCER GENE THERAPY AUG 2003 LNKD- PUBMED:12872142, vol. 10, no. 8, August 2003 (2003-08), pages 603-610, XP002680707, ISSN: 0929-1903
- GOLAN G ET AL: "Coupling of the nucleotide incision and 3' to 5' exonuclease activities in Escherichia coli endonuclease IV: Structural and genetic evidences", MUTATION RESEARCH, ELSEVIER, AMSTERDAM, NL, vol. 685, no. 1-2, 1 March 2010 (2010-03-01), pages 70-79, XP026889400, ISSN: 0027-5107, DOI: 10.1016/J.MRFMMM.2009.08.017 [retrieved on 2009-09-12]
- MENG XIANGDONG ET AL: "Targeted gene inactivation in zebrafish using engineered zinc-finger nucleases.", NATURE BIOTECHNOLOGY JUN 2008 LNKD- PUBMED:18500337, vol. 26, no. 6, June 2008 (2008-06), pages 695-701, XP002678014, ISSN: 1546-1696 cited in the application
- BENNARDO NICOLE ET AL: "Limiting the persistence of a chromosome break diminishes its mutagenic potential.", PLOS GENETICS OCT 2009 LNKD- PUBMED:19834534, vol. 5, no. 10, October 2009 (2009-10), page E1000683, XP002680708, ISSN: 1553-7404
- BENNARDO NICOLE ET AL: "Alternative-NHEJ is a mechanistically distinct pathway of mammalian chromosome break repair.", PLOS GENETICS JUN 2008 LNKD- PUBMED:18584027, vol. 4, no. 6, 27 June 2008 (2008-06-27), page E1000110, XP002680904, ISSN: 1553-7404
- DAVIS BRYAN J ET AL: "End-bridging is required for pol mu to efficiently promote repair of noncomplementary ends by nonhomologous end joining.", NUCLEIC ACIDS RESEARCH MAY 2008 LNKD- PUBMED:18397950, vol. 36, no. 9, May 2008 (2008-05), pages 3085-3094, XP002680905, ISSN: 1362-4962
- SENEJANI ALIREZA G ET AL: "Structural stability and endonuclease activity of a PI-SceI GFP-fusion protein.", INTERNATIONAL JOURNAL OF BIOLOGICAL SCIENCES 2007 LNKD- PUBMED:17389927, vol. 3, no. 4, 2007, pages 205-211, XP002686167, ISSN: 1449-2288
- HUANG Y J ET AL: "Role of exonucleolytic degradation in group I intron homing in phage T4.", GENETICS DEC 1999 LNKD- PUBMED:10581261, vol. 153, no. 4, December 1999 (1999-12), pages 1501-1512, XP002686168, ISSN: 0016-6731
- LI HUI ET AL: "Generation of single-chain LAGLIDADG homing endonucleases from native homodimeric precursor proteins", 1 April 2009 (2009-04-01), NUCLEIC ACIDS RESEARCH, OXFORD UNIVERSITY PRESS, GB, PAGE(S) 1650 - 1662, XP002685900, ISSN: 1362-4962 abstract page 1652, left-hand column, paragraph 2

## Description

### Field of the Invention

The present invention relates to a method for increasing double-strand break-induced mutagenesis at a genomic locus of interest in a cell, thereby providing new tools for genome engineering, including therapeutic applications and cell line engineering. More specifically, the present invention concerns a method for increasing double-strand break-induced mutagenesis at a genomic locus of interest, leading to a loss of genetic information and preventing any scarless re-ligation of said genomic locus of interest by NHEJ (non-homologous end joining). The present invention also relates to chimeric rare-cutting endonucleases, vectors, compositions and kits used to implement this method.

### Background of the Invention

Mammalian genomes constantly suffer from various types of damage of which double-strand breaks (DSB) are considered the most dangerous (Haber 2000). For example, DSBs can arise when the replication fork encounters a nick or when ionizing radiation particles create clusters of reactive oxygen species along their path. These reactive oxygen species may in turn themselves cause DSBs. For cultured mammalian cells that are dividing, 5-10% appear to have at least one chromosomal break (or chromatid gap) at any one time (Lieber and Karanjawala 2004). Hence, the need to repair DSBs arises commonly (Li, Vogel et al. 2007) and is critical for cell survival (Haber 2000). Failure to correct or incorrect repair can result in deleterious genomic rearrangements, cell cycle arrest, and/or cell death.

Repair of DSBs can occur through diverse mechanisms that can depend on cellular context. Repair via homologous recombination, the most accurate process, is able to restore the original sequence at the break. Because of its strict dependence on extensive sequence homology, this mechanism is suggested to be active mainly during the S and G2 phases of the cell cycle where the sister chromatids are in close proximity (Sonoda, Hochegger et al. 2006). Single-strand annealing is another homology-dependent process that can repair a DSB between direct repeats and thereby promotes deletions (Paques and Haber 1999). Finally, non-homologous end joining (NHEJ) of DNA is a major pathway for the repair of DSBs because it can function throughout the cell cycle and because it does not require a homologous chromosome (Moore and Haber 1996).

NHEJ comprises at least two different processes (Feldmann, Schmiemann et al. 2000). The main and best characterized mechanism involves rejoining of what remains of the two DNA ends through direct re-ligation (Critchlow and Jackson 1998) or via the so-called microhomology-mediated end joining (MMEJ) (Ma, Kim et al. 2003). Although perfect re-ligation of the broken ends is probably the most frequent event, it could be accompanied by the loss or gain of several nucleotides.

Like most DNA repair processes, there are three enzymatic activities required for repair of DSBs by the NHEJ pathway: (*i*) nucleases to remove damaged DNA, (*ii*) polymerases to aid in the repair, and (*iii*) a ligase to restore the phosphodiester backbone. Depending on the nature of the DNA ends, DNA can be simply re-ligated or terminal nucleotides can be modified or removed by inherent enzymatic activities, such as phosphokinases and exo-nucleases. Missing nucleotides can also be added by polymerase µ or □. In addition, an alternative or so-called back-up pathway has been described that does not depend on ligase IV and Ku components and has been involved in class switching and V(D)J recombination (Ma, Kim et al. 2003). Overall, NHEJ can be viewed as a flexible pathway for which the unique goal is to restore the chromosomal integrity, even at the cost of excision or insertion of nucleotide(s).

DNA repair can be triggered by both physical and chemical means. Several chemicals are known to cause DNA lesions and are used routinely. Radiomimetic agents, for example, work through free-radical attack on the sugar moieties of DNA (Povirk 1996). A second group of drugs that induce DNA damage includes inhibitors of topoisomerase I (Topol) and II (TopoII) (Burden and N. 1998; Teicher 2008). Other classes of chemicals bind covalently to the DNA and form bulky adducts that are repaired by the nucleotide excision repair (NER) system (Nouspikel 2009). Chemicals inducing DNA damage have a diverse range of applications, however, although certain agents are more commonly applied in studying a particular repair pathway (*e.g.,* cross-linking agents are favored for NER studies), most drugs simultaneously provoke a variety of lesions (Nagy and Soutoglou 2009). Furthermore, the overall yield of induced mutations using these classical strategies is quite low, and the DNA damage leading to mutagenesis cannot be targeted to a precise genomic DNA sequence.

The most widely used site-directed mutagenesis strategy is gene targeting (GT) via homologous recombination (HR). Efficient GT procedures in yeast and mouse have been available for more than 20 years (Capecchi 1989; Rothstein 1991). Successful GT has also been achieved in *Arabidopsis* and rice plants (Hanin, Volrath et al. 2001) (Terada, Urawa et al. 2002; Endo, Osakabe et al. 2006; Endo, Osakabe et al. 2007). Typically, GT events occur in a fairly small population of treated mammalian cells and is extremely low in higher plant cells, ranging between 0.01-0.1% of the total number of random integration events (Terada, Johzuka-Hisatomi et al. 2007). The low GT frequencies reported in various organisms are thought to result from competition between HR and NHEJ for repair of DSBs. As a consequence, the ends of a donor molecule are likely to be joined by NHEJ rather than participating in HR, thus reducing GT frequency. There are extensive data indicating that DSB repair by NHEJ is error-prone due to end-joining processes that generate insertions and/or deletions (Britt 1999). Thus, these NHEJ-based strategies might be more effective than HR-based strategies for targeted mutagenesis into cells.

Expression of I-SceI, a rare cutting endonuclease, has been shown to introduce mutations at I-SceI cleavage sites in *Arabidopsis* and tobacco (Kirik, Salomon et al. 2000). However, the use of endonucleases is limited to rarely occurring natural recognition sites or to artificially introduced target sites. To overcome this problem, meganucleases with engineered specificity towards a chosen sequence have been developed. Meganucleases show high specificity to their DNA target. These proteins being able to cleave a unique chromosomal sequence and therefore do not affect global genome integrity. Natural meganucleases are essentially represented by homing endonucleases, a widespread class of proteins found in eukaryotes, bacteria and archae (Chevalier and Stoddard 2001). Early studies of the I-SceI and HO homing endonucleases illustrated how the cleavage activity of these proteins can be used to initiate HR events in living cells and demonstrated the recombinogenic properties of chromosomal DSBs (Dujon, Colleaux et al. 1986; Haber 1995). Since then, meganuclease-induced HR has been successfully used for genome engineering purposes in bacteria (Posfai, Kolisnychenko et al 1999), mammalian cells (Sargent, Brenneman et al. 1997; Cohen-Tannoudji, Robine et al. 1998; Donoho, Jasin et al. 1998), mice (Gouble, Smith et al. 2006) and plants (Puchta, Dujon et al. 1996; Siebert and Puchta 2002). Meganucleases have emerged as scaffolds of choice for deriving genome engineering tools cutting a desired target sequence (Paques and Duchateau 2007).

Combinatorial assembly processes allowing for the engineering of meganucleases with modified specificities have been described by Arnould *et al.* (Arnould, Chames et al. 2006; Arnould, Perez et al. 2007); Smith et al. (Smith, Grizot et al. 2006), Grizot et al. (Grizot, Smith et al. 2009). Briefly, these processes rely on the identification of locally engineered variants with a substrate specificity that differs from that of the wild-type meganuclease by only a few nucleotides. Another type of specific nucleases are the so-called Zinc-finger nucleases (ZFNs). ZFNs are chimeric proteins composed of a synthetic zinc-finger-based DNA binding domain fused to a DNA cleavage domain. By modification of the zinc-finger DNA binding domain, ZFNs can be specifically designed to cleave virtually any long stretch of dsDNA sequence (Kim, Cha et al. 1996; Cathomen and Joung 2008). A NHEJ-based targeted mutagenesis strategy was recently developed for several organisms by using synthetic ZFNs to generate DSBs at specific genomic sites (Lloyd, Plaisier et al. 2005; Beumer, Trautman et al. 2008; Doyon, McCammon et al. 2008; Meng, Noyes et al. 2008). Subsequent repair of the DSBs by NHEJ frequently produces deletions and/or insertions at the joining site. For example, in zebrafish embryos the injection of mRNA coding for engineered ZFNs led to animals carrying the desired heritable mutations (Doyon, McCammon et al. 2008). In plants, similar NHEJ-based targeted mutagenesis has also been successfully applied (Lloyd, Plaisier et al. 2005). Although these powerful tools are available, there is still a need to further improve double-strand break-induced mutagenesis.

Bennardo N et al. (2009), PLoS Genetics, Vol. 5, Issue 10, page e1000683 discloses co-expression of the exonuclease Trex2 and the rare-cutting endonuclease I-SceI and report a reduction in the frequency of repair pathways that lead to significant genetic loss.

Roth Timothy J et al, CANCER GENE THERAPY AUG 2003, vol. 10, no. 8, pages 603 - 610, disclose a fusion protein composed of the human O⁶-Methylguanine-DNA Methyltransferase (MGMT) and the yeast Apn1 proteins which retains both MGMT and AP endonuclease activity.

Golan G et al, MUTATION RESEARCH, ELSEVIER, vol. 685, no. 1-2, doi:10.1016/J.MRFMMM.2009.08.017, pages 70-79, report on the coupling of the nucleotide incision and 3' - 5' exonuclease activities in *Escherichia coli* endonuclease IV.

WO 2009/095742 (CELLECTIS) relates to a single chain I-CreI meganuclease (scI-CreI) comprising two domains (N-terminal and C-terminal), joined by a peptide linker.

Meng Xiangdong et al, NATURE BIOTECHNOLOGY JUN 2008 vol. 26, no. 6, pages 695- 701, disclose engineering of zinc finger nucleases that recognize sequences in the zebrafish ortholog of the VEGF-2 receptor, kdra, and use of these ZFNs to bring about targeted gene inactivation in zebrafish embryos that were transmitted through the germ line.

Bennardo Nicole et al, PLOS GENETICS JUN 2008 vol. 4, no. 6, ISSN 1553-7404, page E1000110 report on the comparison of the role of individual factors in the three different pathways of mammalian DSB repair : alternative-nonhomologous end joining (alt-NHEJ), single-strand annealing (SSA) and homology-directed repair (HDR/GC).

Senejani Alireza G et al, INTERNATIONAL JOURNAL OF BIOLOGICAL SCIENCES 2007, vol. 3, no. 4, pages 205 - 211 report on the structural stability and endonuclease activity of a fusion protein comprising Green Fluorescent Protein (GFP) and the homing endonuclease PI-SceI.

WO 2012/118717 (SEATTLE CHILDREN'S RESEARCH INSTITUTE) relates to methods of increasing mutagenic activity of an endonuclease by providing the endonuclease with an end-processing enzyme.

The inventors have developed a new approach to increase the efficiency of targeted DSB-induced mutagenesis and have created a new type of meganucleases comprising several catalytic domains to implement this new approach. These novel enzymes allow a DNA cleavage that will lead to the loss of genetic information and any NHEJ pathway will produce targeted mutagenesis.

### Brief summary of the invention

The present specification describes a method for increasing double-strand break-induced mutagenesis at a genomic locus of interest in a cell, thereby giving new tools for genome engineering, including therapeutic applications and cell line engineering. More specifically, the present specification describes a method for increasing double-strand break-induced mutagenesis at a genomic locus of interest, leading to a loss of genetic information and preventing any scarless re-ligation of said genomic locus of interest by NHEJ.

A first aspect of the present invention concerns a method for increasing double-strand break induced mutagenesis at a genomic locus of interest in a cell comprising the steps of:
(i) identifying at said genomic locus of interest at least one DNA target sequence cleavable by one rare-cutting endonuclease;
(ii) engineering said at least one rare-cutting endonuclease to provide a chimeric rare-cutting endonuclease able to generate one double-strand break in the genomic locus of interest and a loss of genetic information within the genomic locus of interest by another enzymatic activity, said another enzymatic activity being an exonuclease activity; and
(iii) contacting said DNA target sequence with said at least one chimeric rare-cutting endonuclease to generate one DNA double-strand break in the genomic locus of interest, and a loss of genetic information around said DNA target sequence within the genomic locus of interest;
wherein said chimeric rare-cutting endonuclease is a rare-cutting endonuclease fused to an exonuclease domain,
thereby obtaining a cell in which double-strand break induced mutagenesis at said genomic locus of interest is increased.

In a second aspect, the present invention relates to engineered enzymes which are chimeric rare-cutting endonucleases able to target a DNA sequence within a genomic locus of interest to generate at least one DNA double-strand break and a loss of genetic information around said DNA sequence thus preventing any scarless re-ligation of said genomic locus of interest by NHEJ. These chimeric rare-cutting endonuclease for increasing double-strand break induced mutagenesis into a genomic locus of interest comprise:
i) a rare-cutting endonuclease;
ii) an exonuclease domain.

A third aspect of the present disclosure concerns a method for the generation of at least two-nearby DNA double-strand breaks at a genomic locus of interest to prevent any scarless re-ligation of said genomic locus of interest by NHEJ.

A fourth aspect of the present disclosure, relates to engineered rare-cutting chimeric endonucleases able to target a DNA sequence within a genomic locus of interest to generate at said locus of interest at least two-nearby DNA double-strand breaks leading to at least the removal of a DNA fragment and thus preventing any scarless re-ligation of said genomic locus of interest by NHEJ.

The present invention also relates to specific vectors, compositions and kits used to implement the method of the invention.

Additional objects, aspects and embodiments of the invention are found in the following detailed description of the invention.

### Brief description of the figures

In addition to the preceding features, the invention further comprises other features which will emerge from the description which follows, as well as to the appended drawings. A more complete appreciation of the invention and many of the attendant advantages thereof will be readily obtained as the same becomes better understood by reference to the following Figures in conjunction with the detailed description below.

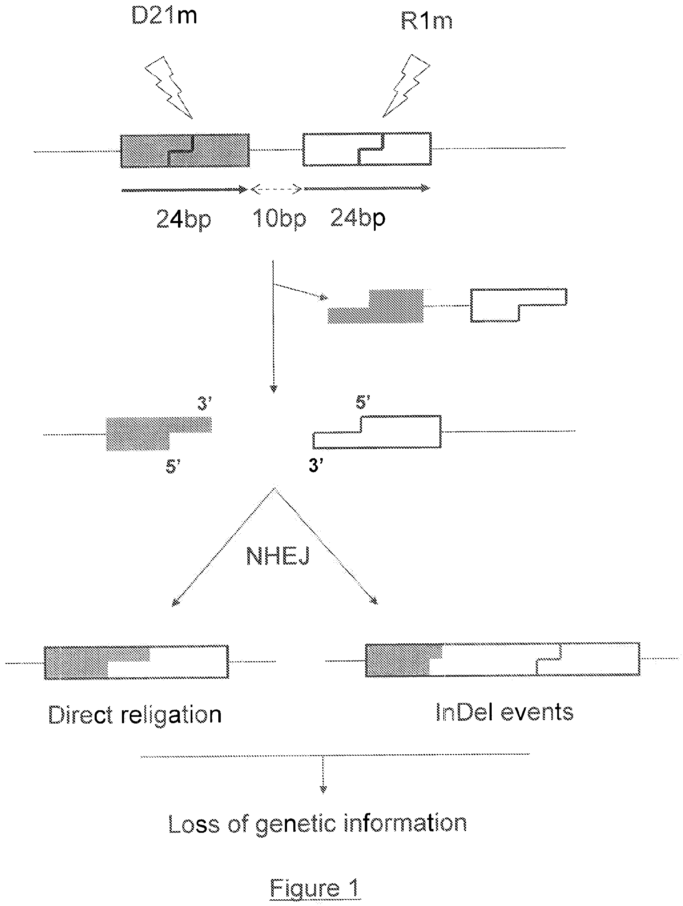

**Figure 1** **: Elimination of an intervening sequence enhances DSB-induced mutagenesis.** The 22bp DNA sequences recognized by D21m (or D21) and R1m (or R21), respectively, are introduced into a plasmid. A 10-bp intervening sequence is cloned between the two recognition sequences to avoid steric hindrance upon meganuclease binding. Introduction of the target plasmid within a cell, together with plasmids expressing the meganucleases D21m and R1m, results in the simultaneous cleavage of the two target sites. The intervening fragment comprising the 10-bp sequence surrounded by half of each target site is excised. Subsequent NHEJ, either via re-ligation of compatible or incompatible DNA ends, leads to mutagenic events since genetic information was lost.

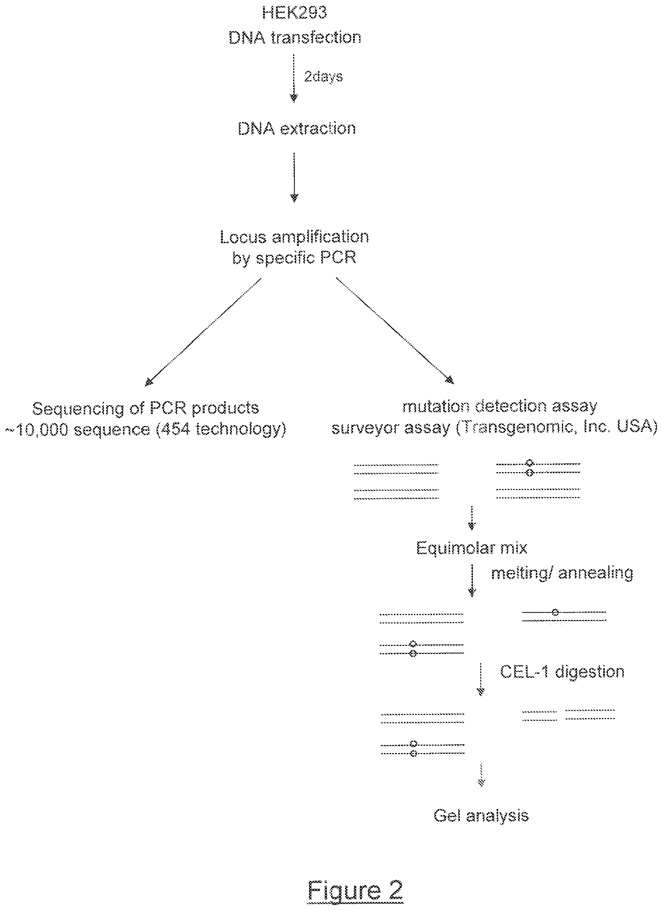

**Figure 2****: Schematic representation of the analyses performed to detect DSB-induced mutations.** HEK293 cells are simultaneously transfected with target plasmid and either one or two different meganuclease expressing plasmids. DNA is extracted two days post transfection and specific PCR is performed. PCR products are analyzed using deep sequencing technology (454, Roche). Alternatively, a mutation detection assay (Transgenomic, Inc. USA) is performed. PCR product from untreated cells is mixed (equimolar) with PCR products treated with the meganucleases. The melting/annealing step generates heteroduplex DNA, recognized and cleaved by the CEL-1 enzyme. After digestion, DNA bands are resolved on an analytic gel and each band is quantified by densitometry.

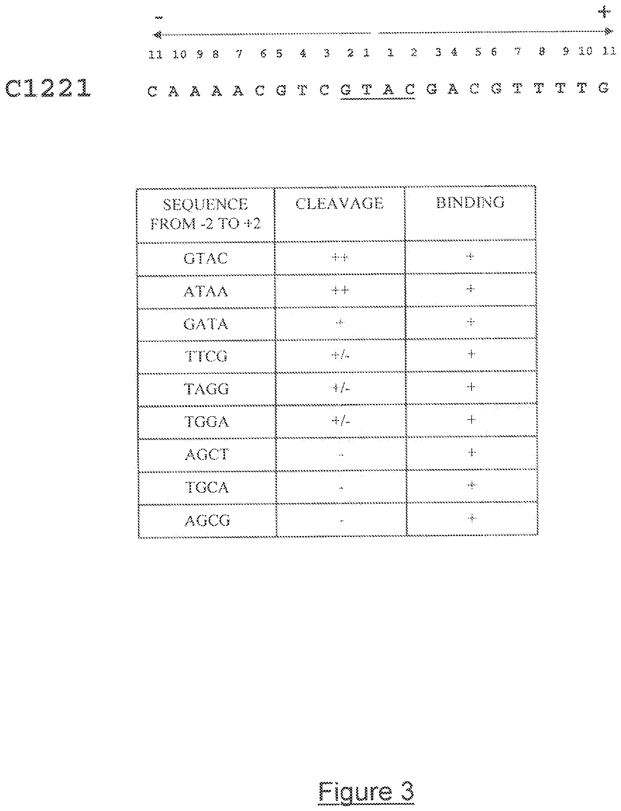

**Figure 3****: Sequence of the target DNA recognized by I-CreI.** C1221 represents a palindromic DNA sequence recognized and cleaved by the I-CreI meganuclease. Nucleotides are numbered outward (-/+) from the center of the target. Nucleotides at positions -2 to +2 do not directly contact the protein but rather interfere with the cleavage activity of the protein. The table represents a subset of the tested targets with nucleotide substitution at positions -2 to +2. The binding and cleavage activity of I-CreI on the target is indicated (++, strong, +, good, +/-, weak; -, no activity). Activities were determined in vitro.

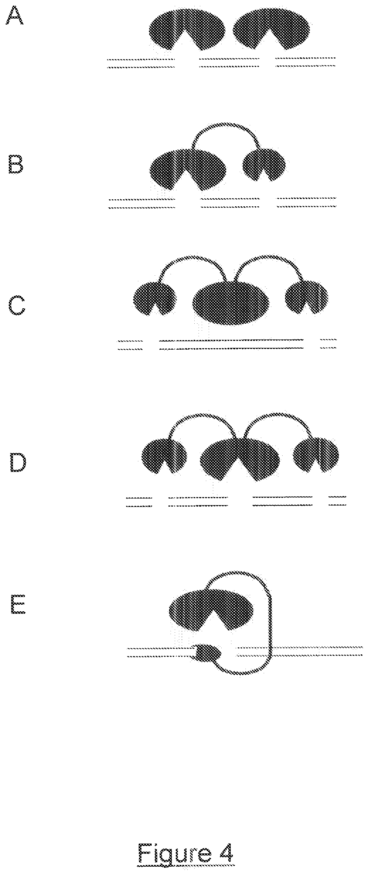

**Figure 4** **: Strategies to enhance DSB-induced mutagenesis.** Loss of genetic information can be obtained by one or any variations of the following described strategies as illustrating examples (slight vertical lanes indicate specific DNA recognition domains): - simultaneous DSBs generated by two different specific rare-cutting endonucleases (A); - chimeric rare-cutting endonucleases with two endonucleases catalytic domains (bi-functional) (B); - chimeric rare-cutting endonucleases with one DNA-binding domain and two endonucleases catalytic domains (bi-functional) (C); - fusion protein between a rare-cutting endonuclease, a endonuclease catalytic domain and a frequent-cutting endonuclease (multi-functional) (D); - chimeric rare-cutting endonucleases with one exonuclease catalytic domains capable to process DNA ends (bi-functional) (E).

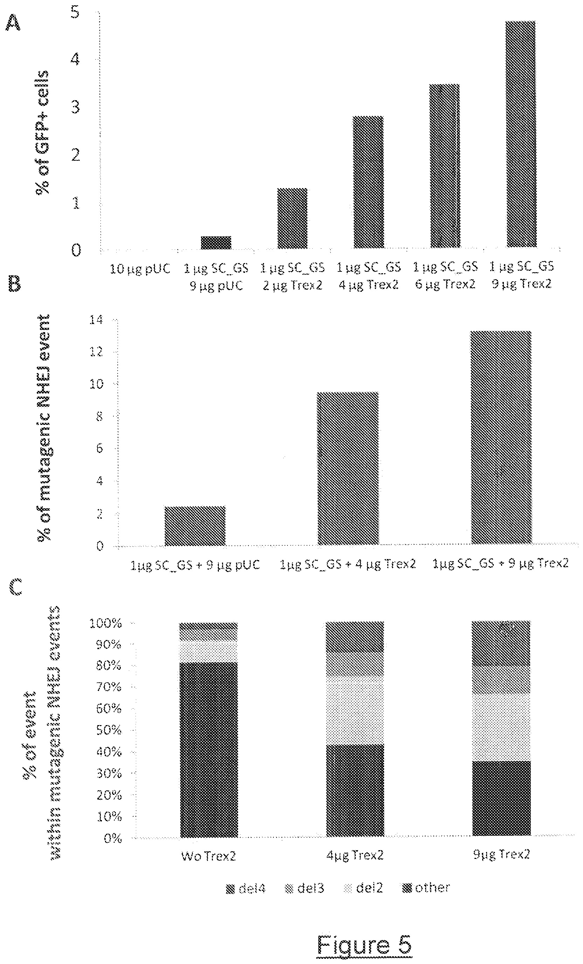

**Figure 5****: Effect of Trex2 expression on SC_GS-induced mutagenic DSB repair. A:** Percentage of GFP+ cells induced on NHEJ model after transfection of SC_GS (SEQ ID NO: 153) with empty vector (SEQ ID NO: 175) or with increasing amount of Trex2 expression vector (SEQ ID NO: 154). **B:** Percentage of mutagenesis (insertions and deletions) detected in the vicinity of the GS_CHO1 target present on the NHEJ model induced by either SC_GS (SEQ ID NO: 153) with empty vector (SEQ ID NO: 175) or with two different doses of Trex2 encoding vector (SEQ ID NO: 154). C: Percentage of events corresponding to a deletion of 2 (del2), 3 (del3) or 4 (del4) nucleotides at the end of double strand break generated by SC_GS (corresponding to the lost of the 3' overhang), other correspond to any other mutagenic NHEJ events detected.

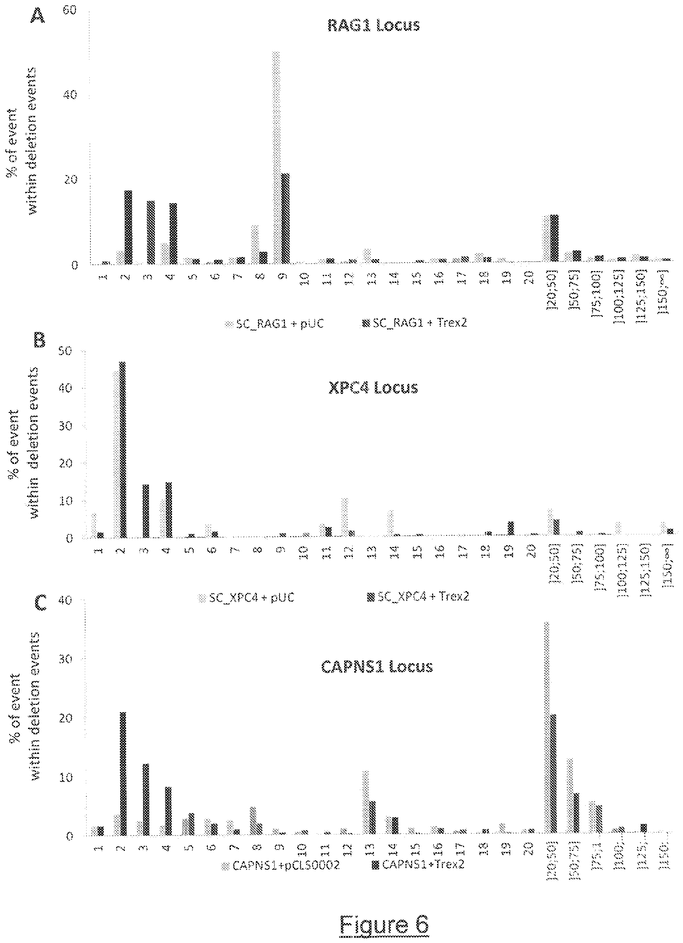

**Figure 6****: Effect of Trex2 expression on the nature of deletions induced by different engineered meganucleases.**
   Size of deletion events were analyzed and the frequency of indicated deletion among all deletion events were calculated after treatment with meganucleases SC_RAG1 (SEQ ID NO: 58 encoded by plasmid pCLS2222, SEQ ID NO: 156), SC_XPC4 (SEQ ID NO: 190 encoded by pCLS2510, SEQ ID NO: 157) and SC_CAPNS1 (SEQ ID NO: 192 encoded by pCLS6163, SEQ ID NO: 158) only (grey histogram) or with Trex2 (SEQ ID NO: 194 encoded by pCLS7673, SEQ ID NO: 154) (black histogram).
**Figure 7****: plasmid for SC_GS and SC_GS and Trex2 fusion expression**
   All fusion constructs were cloned in pCLS1853 (SEQ ID NO: 175), driving their expression by a CMV promoter.
**Figure 8****: SSA activity of SC_GS and SC_GS-fused to Trex2.**
   CHO-K1 cells were co-transfected with the plasmid measuring SSA activity containing the GS_CHO1.1 target and an increasing amounts of SC_GS (pCLS2690, SEQ ID NO: 153), SC_GS-5-Trex2 (pCLS8082, SEQ ID NO: 186), SC_GS-10-Trex2 (pCLS8052, SEQ ID NO: 187), Trex2-5-SC_GS (pCLS8053, SEQ ID NO: 188) or Trex2-10-SC_GS (pCLS8054, SEQ ID NO: 153). Beta-galactosidase activity was detected 72h after transfection using ONPG and 420 nm optical density detection. The entire process was performed on an automated Velocity11 BioCel platform.

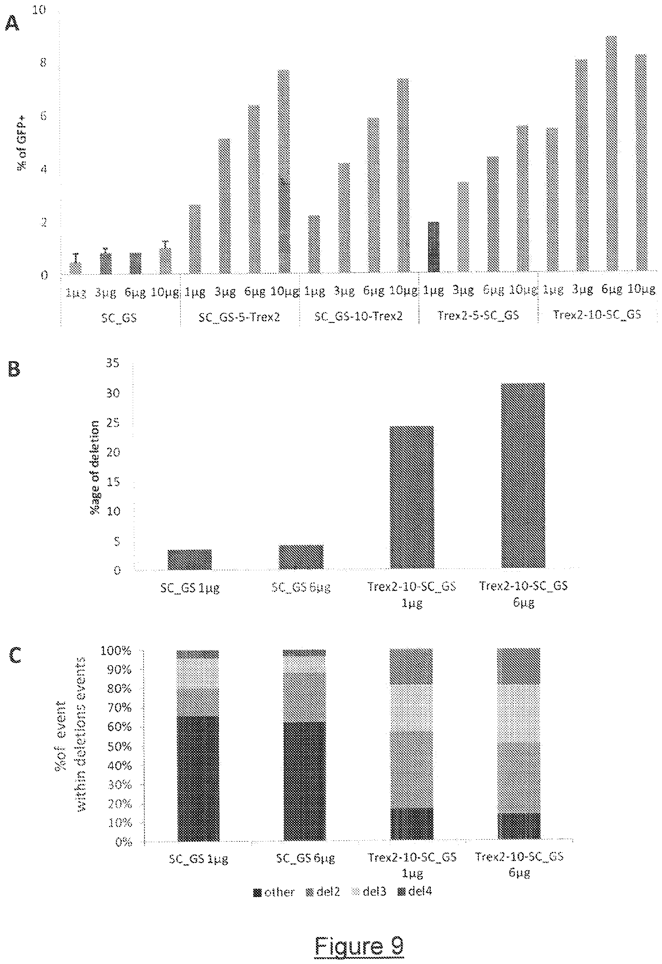

**Figure 9****: Effect of SC_GS fused to Trex2 on mutagenic DSB repair**
   **A:** Percentage of GFP+ cells induced on NHEJ model 3 or 4 days after transfection with increasing dose of either SC_GS (pCLS2690, SEQ ID NO: 153), SC_GS-5-Trex2 (pCLS8082, SEQ ID NO: 186), SC_GS-10-Trex2 (pCLS8052, SEQ ID NO: 187), Trex2-5-SC_GS (pCLS8053, SEQ ID NO: 188) or Trex2-10-SC_GS (pCLS8054, SEQ ID NO: 189). **B:** Deep-sequencing analysis of deletion events induced by 1 or 6 µg of SC_GS (pCLS2690, SEQ ID NO: 153) or Trex2-10-SC_GS (pCLS8054, SEQ ID NO: 189). **C:** Percentage of deletion events corresponding to a deletion of 2 (del2), 3 (del3) or 4 (del4) nucleotides at the end of double strand break generated by 1 or 6 µg of SC_GS (pCLS2690, SEQ ID NO: 153) or Trex2-10-SC_GS (pCLS8054, SEQ ID NO: 189), other correspond to any other deletions events detected.

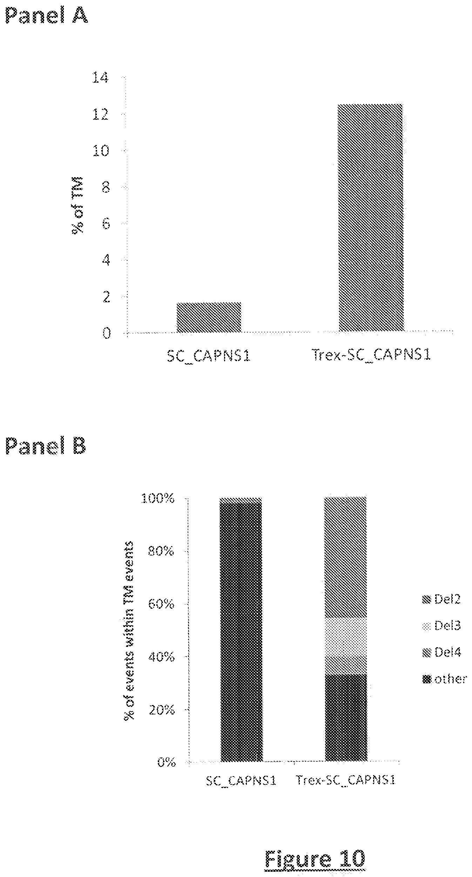

**Figure 10****: Effect of Trex-SC_CAPNS1 (SEQ ID NO: 197) fusion on targeted mutagenesis in 293H cell line**
   Panel A: Percentage of Targeted Mutagenesis [TM] obtained in 293H cell line transfected with SC_CAPNS1 (SEQ ID NO: 192) or Trex-SC_CAPNS1 (SEQ ID NO: 197).
   Panel B: Nature of Targeted Mutagenesis obtained in 293H cell line transfected with SC_CAPNS1 (SEQ ID NO: 192) or Trex-SC_CAPNS1 (SEQ ID NO: 197). Del2, Del3 and Del4 correspond to 2, 3 and 4 base pairs deletion events at the cleavage site of CAPNS1. "Other" represents all other TM events.

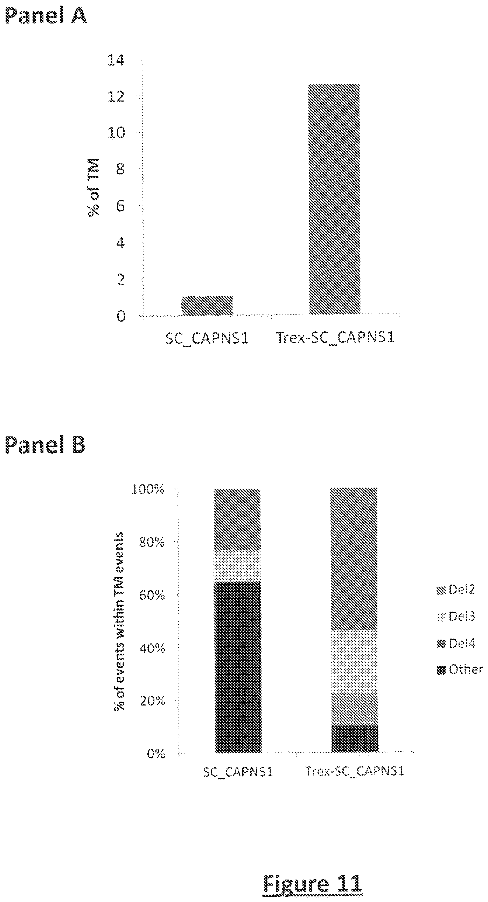

**Figure 11****: Effect of Trex-SC_CAPNS1 (SEQ ID NO: 197) fusion on targeted mutagenesis in 293H cell line**
   Panel A: Percentage of Targeted Mutagenesis obtained in Detroit551 cell line transfected with SC_CAPNS1 (SEQ ID NO: 192) or Trex-SC_CAPNS1 (SEQ ID NO: 197).
   Panel B: Nature of Targeted Mutagenesis obtained in Detroit551 cell line transfected with SC_CAPNS1 (SEQ ID NO: 192) or Trex-SC_CAPNS1 (SEQ ID NO: 197). Del2, Del3 and Del4 correspond to 2, 3 and 4 base pairs deletion events at the cleavage site of CAPNS1. "Other" represents all other TM events.

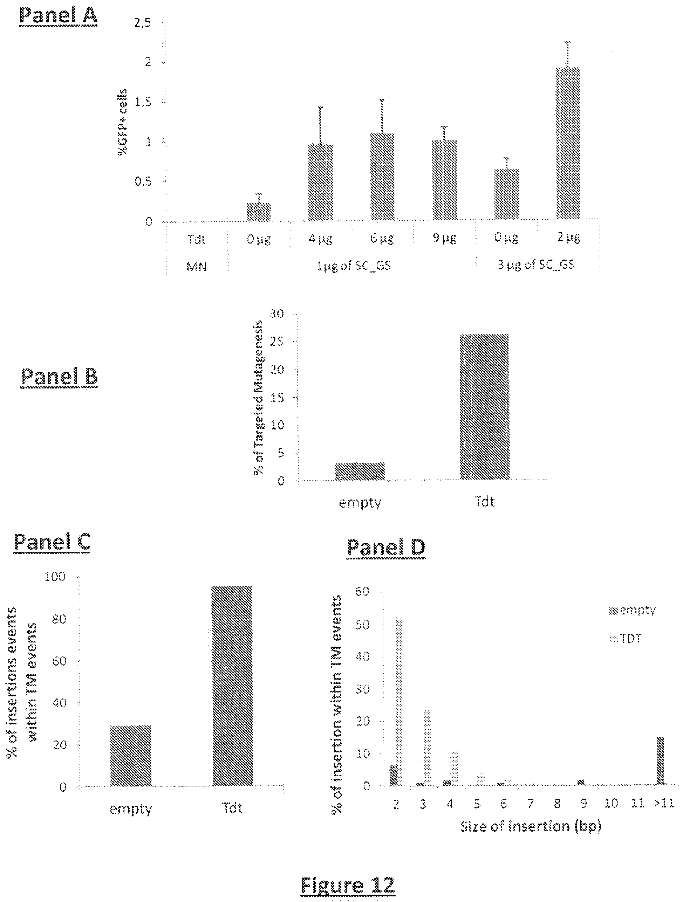

**Figure 12****: Effect of Tdt expression on targeted mutagenesis in cell line monitoring NHEJ.**
   Panel A: Percentage of GFP+ cells induced on NHEJ model after co-transfection of 1 µg or 3µg of SC_GS expressing plasmid (SEQ ID NO: 153) and with either an increasing amount of Tdt expression vector (SEQ ID NO: 153) or with 2µg of Tdt expressing plasmid (SEQ ID NO: 153), respectively.
   Panel B: Percentage of targeted mutagenesis detected by deep sequencing in the vicinity of the GS_CHO1 DNA target present on the NHEJ model, induced by either SC_GS with empty vector or with 2 µg of Tdt encoding vector.
   Panel C: Percentage of insertion events within targeted mutagenesis events after co-transfection of the NHEJ model by 3µg of SC_GS expressing vector with 2 µg of an empty vector or with 2 µg of Tdt encoding plasmid.
   Panel D: Percentage of insertion events in function of their size in presence (TDT) or absence (empty) of Tdt.

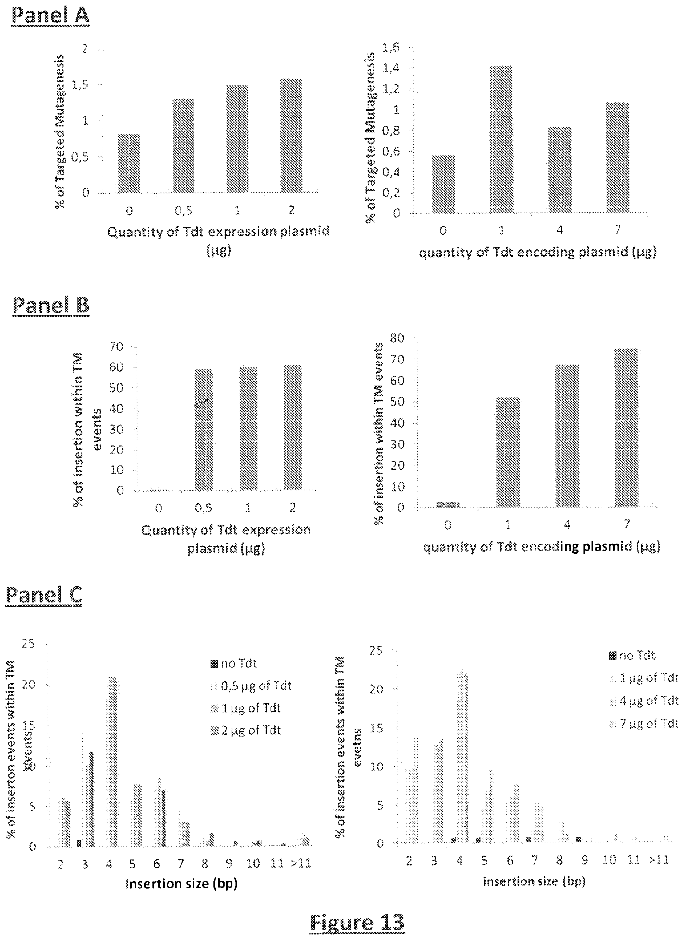

**Figure 13****: Effect of Tdt expression on targeted mutagenesis induced by SC_RAG1 (SEQ ID NO: 58) at endogenous RAG1 locus**
   Panel A: Percentage of targeted mutagenesis detected by deep sequencing in the vicinity of the SC_RAG1 target induced by co-transfection of 3 µg of SC_RAG1 encoding vector (SEQ ID NO: 156) with different amount of Tdt encoding vector (SEQ ID NO: 202) in 5 µg of total DNA (left part) or in 10 µg of total DNA (right part).
   Panel B: Percentage of insertion events within targeted mutagenesis events after co-transfection of 3 µg of SC_RAG1 encoding vector (SEQ ID NO: 156) with different amount of Tdt encoding vector (SEQ ID NO: 202) in 5 µg of total DNA (left part) or in 10 µg of total DNA (right part).
   Panel C: Percentage of insertion events in function of their size at endogenous RAG1 locus after co-transfection of 3 µg of SC_RAG1 encoding vector (SEQ ID NO: 156) with different amounts of Tdt encoding vector (SEQ ID NO: 202) in 5 µg of total DNA (left part) or in 10 µg of total DNA (right part).

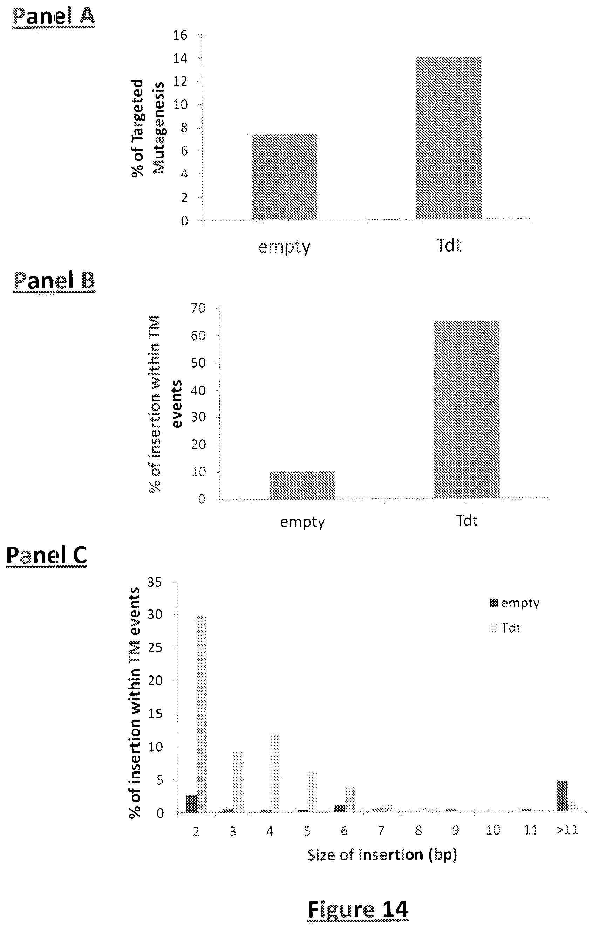

**Figure 14****: Effect of Tdt expression on targeted mutagenesis induced by SC_CAPNS1 (SEQ ID NO: 192) at endogenous CAPNS1 locus**
   Panel A: Percentage of targeted mutagenesis detected by deep sequencing in the vicinity of the SC_CAPNS1 target induced by co-transfection of 1 µg of SC_CAPNS1 expressing vector (SEQ ID NO: 158) with 2µg of Tdt encoding plasmid (SEQ ID NO: 202).
   Panel B: Percentage of insertion events within targeted mutagenesis events after co-transfection of 3µg of SC_CAPNS1 expressing vector (SEQ ID NO: 158) with 2µg of Tdt encoding plasmid (SEQ ID NO: 202).
   Panel C: Percentage of insertion events in function of their size at CAPNS1 locus after co-transfection of 3µg of SC_CAPNS1 expressing vector (SEQ ID NO: 158) with 2µg of Tdt encoding plasmid (SEQ ID NO: 202).

### Detailed description of the invention

Unless specifically defined herein below, all technical and scientific terms used herein have the same meaning as commonly understood by a skilled artisan in the fields of gene therapy, biochemistry, genetics, and molecular biology.

All methods and materials similar or equivalent to those described herein can be used in the practice or testing of the present invention, with suitable methods and materials being described herein. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

A first aspect of the present invention is a method for increasing double-strand break induced mutagenesis at a genomic locus of interest in a cell comprising the steps of:
(i) identifying at said genomic locus of interest at least one DNA target sequence cleavable by one rare-cutting endonuclease;
(ii) engineering said at least one rare-cutting endonuclease to provide a chimeric rare-cutting endonuclease able to generate one double-strand break in the genomic locus of interest and a loss of genetic information within the genomic locus of interest by another enzymatic activity, said another enzymatic activity being an exonuclease activity ;
(iii) contacting said DNA target sequence with said at least one chimeric rare-cutting endonuclease to generate one double-strand break in the genomic locus of interest and a loss of genetic information around said DNA target sequence within the genomic locus of interest;
wherein said chimeric rare-cutting endonuclease is a rare-cutting endonuclease fused to an exonuclease domain,
thereby obtaining a cell in which double-strand break induced mutagenesis at said genomic locus of interest is increased.

According to the invention, said rare-cutting endonuclease is able to generate one DNA double-strand break in the genomic locus of interest and a loss of genetic information by another enzymatic activity. Said another enzymatic activity is an exonuclease activity. According to the invention, said chimeric rare-cutting endonuclease generates one DNA double-strand break leading to DNA ends, thus processed by an exonuclease activity, allowing the loss of genetic information and preventing any scarless re-ligation of said genomic locus of interest.

Disclosed herein are chimeric rare-cutting endonucleases comprising a catalytic domain given in Table 2 (SEQ ID NO: 38-57) and Table 3 (SEQ ID NO: 96-152). In a preferred embodiment of the invention, said chimeric rare-cutting endonuclease comprises a catalytic domain of Trex (SEQ ID NO: 145-149).

In another preferred embodiment, said chimeric rare-cutting endonuclease comprises a catalytic domain of SEQ ID NO: 194. In another preferred embodiment, said chimeric rare-cutting endonuclease is fused to a protein of SEQ ID NO: 194. In another preferred embodiment, said chimeric rare-cutting endonuclease is a fusion protein comprising a single chain meganuclease and a protein of SEQ ID NO: 194. In another preferred embodiment, said chimeric rare-cutting endonuclease is selected from the group consisting of SEQ ID NO: 171-174 and SEQ ID NO: 197.

In a second aspect, the present invention also relates to engineered enzymes which are chimeric rare-cutting endonucleases for increasing double-strand break induced mutagenesis into a genomic locus of interest comprising:
i) a rare-cutting endonuclease;
ii) an exonuclease domain.
Such chimeric rare-cutting endonucleases are able to target a DNA sequence within a genomic locus of interest in order to generate at least one DNA double-strand break and a loss of genetic information by another enzymatic activity around said DNA sequence, thus preventing any scarless re-ligation of said genomic locus of interest by NHEJ. Said chimeric rare-cutting endonuclease of the present invention is a fusion protein between a rare-cutting endonuclease which generates one DNA double-strand break at a targeted sequence within the genomic locus of interest, leading to DNA ends and an exonuclease domain that is able to process said DNA ends in order to generate a loss of information at the genomic locus of interest.

Rare-cutting endonucleases disclosed herein include chimeric rare-cutting endonucleases comprising a catalytic domain given in Table 2 and Table 3. In a preferred embodiment of the invention, said chimeric rare-cutting endonuclease comprises a catalytic domain of Trex (SEQ ID NO: 145-149).

In another preferred embodiment, said chimeric rare-cutting endonuclease comprises a catalytic domain of SEQ ID NO: 194. In another preferred embodiment, said chimeric rare-cutting endonuclease is fused to a protein of SEQ ID NO: 194. In another preferred embodiment, said chimeric rare-cutting endonuclease is a fusion protein comprising a single chain meganuclease and a protein of SEQ ID NO: 194. In another preferred embodiment, said chimeric rare-cutting endonuclease is selected from the group consisting of SEQ ID NO: 171-174 and SEQ ID NO: 197.

In a third aspect, the present disclosure concerns a method for the generation of at least two-nearby DNA double-strand breaks at a genomic locus of interest to prevent any scarless re-ligation of said genomic locus of interest by NHEJ. In other words, said method comprises the generation of two nearby DNA double-strand breaks into said genomic locus of interest by the introduction of at least one double-strand break creating agent able to generate at least two nearby double-strand breaks such that said at least two nearby DNA double-strand breaks allow the removal of an intervening sequence, as a non limiting example, to prevent any scarless re-ligation of said genomic locus of interest (as illustrated in Figure 4A to 4C).

According to this third aspect, the present disclosure concerns a method comprising the steps of:
(i) identifying at said genomic locus of interest one DNA target sequence cleavable by one rare-cutting endonuclease;
(ii) engineering said at least one rare-cutting endonuclease such that said rare-cutting endonuclease is able to generate at least two nearby DNA double-strand breaks in the genomic locus of interest;
(iii) contacting said DNA target sequence with said at least one rare-cutting endonuclease;
thereby obtaining a cell in which double-strand break induced mutagenesis at said genomic locus of interest is increased.

According to this third aspect of the present disclosure, said rare-cutting endonuclease is engineered to provide one chimeric rare-cutting endonuclease that is able to generate two nearby DNA double-strand breaks in the genomic locus of interest (as illustrated in Figure 4B and 4C). Alternatively, said rare-cutting endonuclease is engineered to provide one chimeric rare-cutting endonuclease that is able to generate more than two nearby DNA double-strand breaks in the genomic locus of interest; in particular said one chimeric rare-cutting endonuclease may generate three nearby DNA double-strand breaks in the genomic locus of interest.

According to a variant of this third aspect, the present disclosure may also involve two engineered rare-cutting endonucleases and comprises the steps of:
(i) identifying at said genomic locus of interest two nearby DNA target sequences respectively cleavable by one rare-cutting endonuclease;
(ii) engineering a first rare-cutting endonuclease able to generate a first DNA double-strand break in the genomic locus of interest;
(iii) engineering a second rare-cutting endonuclease able to generate a second DNA double-strand break in the genomic locus of interest;
(iv) contacting said DNA target sequence with said two rare-cutting endonucleases;
thereby obtaining a cell in which double-strand break induced mutagenesis at said genomic locus of interest is increased.

In this variant of the third aspect, said two engineered rare-cutting endonucleases which respectively target a DNA sequence at a genomic locus of interest are chimeric rare-cutting endonucleases, or alternatively, only one of said two engineered rare-cutting endonucleases, which respectively target a DNA sequence at a genomic locus of interest, is a chimeric rare-cutting endonuclease.

A fourth aspect of the present disclosure relates to chimeric rare-cutting endonucleases, able to target a DNA sequence within a genomic locus of interest in order to generate at said locus of interest at least two-nearby DNA double-strand breaks leading to at least the removal of a DNA fragment and thus preventing any scarless re-ligation of said genomic locus of interest by NHEJ (as illustrated in Figure 4A, 4C and 4E). Said chimeric rare-cutting endonucleases may comprise at least two catalytic domains, for example two nuclease domains. This fourth aspect of the present disclosure relates to a chimeric rare-cutting endonuclease to generate at least two nearby DNA double-strand breaks into a genomic locus of interest comprising:
i) a rare-cutting endonuclease;
ii) a peptidic linker;
iii) a nuclease catalytic domain.

Preferably, said rare-cutting endonuclease part of said chimeric rare-cutting endonuclease is a meganuclease; said rare-cutting endonuclease part of said chimeric rare-cutting endonuclease may be a I-CreI derived meganuclease. Said rare-cutting endonuclease part of said chimeric rare-cutting endonuclease may be a single chain meganuclease derived from I-CreI meganuclease.

According to the invention, said chimeric rare-cutting endonuclease is a fusion protein between a meganuclease and at least one nuclease catalytic domain wherein said nuclease catalytic domain has an exonuclease activity.

In a preferred embodiment, said chimeric rare-cutting endonuclease is a fusion protein comprising a meganuclease and a protein of SEQ ID NO: 145-149,

In another preferred embodiment, said chimeric rare-cutting endonuclease comprises a catalytic domain of SEQ ID NO: 194. In another preferred embodiment, said chimeric rare-cutting endonuclease is fused to a protein of SEQ ID NO: 194. In another preferred embodiment, said rare-cutting endonuclease is a fusion protein comprising a single-chain meganuclease and a protein of SEQ ID NO: 194. In another preferred embodiment, said chimeric rare-cutting endonuclease is selected from the group consisting of SEQ ID NO: 171-174 and SEQ ID NO: 197.

Said chimeric rare-cutting endonuclease, as defined according to the invention, may further comprises a second peptidic linker and a supplementary catalytic domain. The present disclosure thus also relates to a chimeric rare-cutting endonuclease able to generate at least two nearby DNA double-strand breaks into a genomic locus of interest comprising:
i) a rare-cutting endonuclease;
ii) a peptidic linker;
iii) a nuclease catalytic domain.
iv) a second peptidic linker
v) a supplementary catalytic domain.
wherein said nuclease catalytic domain has an exonuclease activity. If said supplementary catalytic domain is a nuclease domain; said chimeric rare-cutting endonuclease is a fusion protein between a rare-cutting endonuclease and two nuclease catalytic domains. Preferably, said chimeric rare-cutting endonuclease is a fusion protein between a meganuclease and two nuclease catalytic domains. Alternatively, said chimeric rare-cutting endonuclease is a fusion protein between a meganuclease, one nuclease catalytic domain and one other catalytic domain.

Also encompassed within the scope of the present invention is a chimeric rare-cutting endonuclease able to generate two-nearby double-strand breaks and composed of the DNA-binding domain of a rare-cutting endonuclease and two other nuclease catalytic domains as defined above.

The present invention also relates to polynucleotides encoding the chimeric rare-cutting endonuclease proteins of the invention, specific vectors (polynucleotidic or not) encoding and/or vectorizing them, compositions and/or kits comprising them, all of them being used or part of a whole to implement methods of the present invention for increasing double-strand break-induced mutagenesis at a genomic locus of interest in a cell. Such kits may contain instructions for use in increasing double-strand break-induced mutagenesis in a cell, packaging materials, one or more containers for the ingredients, and other components used for increasing double-strand break-induced mutagenesis

### Definitions

- Amino acid residues in a polypeptide sequence are designated herein according to the one-letter code, in which, for example, Q means Gln or Glutamine residue, R means Arg or Arginine residue and D means Asp or Aspartic acid residue.
- Amino acid substitution means the replacement of one amino acid residue with another, for instance the replacement of an Arginine residue with a Glutamine residue in a peptide sequence is an amino acid substitution.
- Altered/enhanced/increased/improved cleavage activity, refers to an increase in the detected level of meganuclease cleavage activity, see below, against a target DNA sequence by a second meganuclease in comparison to the activity of a first meganuclease against the target DNA sequence. Normally the second meganuclease is a variant of the first and comprise one or more substituted amino acid residues in comparison to the first meganuclease.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "meganuclease", is intended an endonuclease having a double-stranded DNA target sequence of 12 to 45 bp. Said meganuclease is either a dimeric enzyme, wherein each domain is on a monomer or a monomeric enzyme comprising the two domains on a single polypeptide.
- by "meganuclease domain" is intended the region which interacts with one half of the DNA target of a meganuclease and is able to associate with the other domain of the same meganuclease which interacts with the other half of the DNA target to form a functional meganuclease able to cleave said DNA target.
- by "meganuclease variant" or "variant" it is intended a meganuclease obtained by replacement of at least one residue in the amino acid sequence of the parent meganuclease with a different amino acid. Variants include those with substitutions of 1, 2, 3, 4, 5, 6, 7, 8, 9, 10, 11, 12, 13, 14, 15, 16, 17, 18, 19, 20 or more amino acid residues. Such variants may have 75, 80, 85, 90, 95, 97.5, 98, 99, 99.5% or more homology or identity (or any intermediate value within this range) to a base or parental meganuclease sequence.
- by "peptide linker", "peptidic linker" or "peptide spacer" it is intended to mean a peptide sequence which allows the connection of different monomers in a fusion protein and the adoption of the correct conformation for said fusion protein activity and which does not alter the specificity of either of the monomers for their targets. Peptide linkers can be of various sizes, from 3 amino acids to 50 amino acids as a non limiting indicative range. Non-limiting examples of such peptidic linkers are given in Table 1.
- by "related to", particularly in the expression "one cell type related to the chosen cell type or organism", is intended a cell type or an organism sharing characteristics with said chosen cell type or said chosen organism; this cell type or organism related to the chosen cell type or organism, can be derived from said chosen cell type or organism or not.
- by "subdomain" it is intended the region of a LAGLIDADG homing endonuclease core domain which interacts with a distinct part of a homing endonuclease DNA target half-site.
- by "targeting DNA construct/minimal repair matrix/repair matrix" it is intended to mean a DNA construct comprising a first and second portions which are homologous to regions 5' and 3' of the DNA target *in situ.* The DNA construct also comprises a third portion positioned between the first and second portion which comprise some homology with the corresponding DNA sequence *in situ* or alternatively comprise no homology with the regions 5' and 3' of the DNA target *in situ.* Following cleavage of the DNA target, a homologous recombination event is stimulated between the genome containing the targeted gene comprised in the locus of interest and the repair matrix, wherein the genomic sequence containing the DNA target is replaced by the third portion of the repair matrix and a variable part of the first and second portions of the repair matrix.
- by "functional variant" is intended a variant which is able to cleave a DNA target sequence, preferably said target is a new target which is not cleaved by the parent meganuclease. For example, such variants have amino acid variation at positions contacting the DNA target sequence or interacting directly or indirectly with said DNA target.
- by "selection or selecting" it is intended to mean the isolation of one or more meganuclease variants based upon an observed specified phenotype, for instance altered cleavage activity. This selection can be of the variant in a peptide form upon which the observation is made or alternatively the selection can be of a nucleotide coding for selected meganuclease variant.
- by "screening" it is intended to mean the sequential or simultaneous selection of one or more meganuclease variant (s) which exhibits a specified phenotype such as altered cleavage activity.
- by "derived from" it is intended to mean a meganuclease variant which is created from a parent meganuclease and hence the peptide sequence of the meganuclease variant is related to (primary sequence level) but derived from (mutations) the sequence peptide sequence of the parent meganuclease.
- by "I-*Cre*I" is intended the wild-type I*-Cre*I having the sequence of pdb accession code 1g9y, corresponding to the sequence SEQ ID NO: 1 in the sequence listing.
- by "I*-Cre*I variant with novel specificity" is intended a variant having a pattern of cleaved targets different from that of the parent meganuclease. The terms "novel specificity", "modified specificity", "novel cleavage specificity", "novel substrate specificity" which are equivalent and used indifferently, refer to the specificity of the variant towards the nucleotides of the DNA target sequence. In the present Patent Application all the I-*Cre*I variants described comprise an additional Alanine after the first Methionine of the wild type I-*Cre*I sequence as shown in SEQ ID NO: 195. These variants also comprise two additional Alanine residues and an Aspartic Acid residue after the final Proline of the wild type I*-Cre*I sequence. These additional residues do not affect the properties of the enzyme and to avoid confusion these additional residues do not affect the numeration of the residues in I*-Cre*I or a variant referred in the present Patent Application, as these references exclusively refer to residues of the wild type I*-Cre*I enzyme (SEQ ID NO: 1) as present in the variant, so for instance residue 2 of I*-Cre*I is in fact residue 3 of a variant which comprises an additional Alanine after the first Methionine.
- by "I*-Cre*I site" is intended a 22 to 24 bp double-stranded DNA sequence which is cleaved by I-*Cre*I. I-*Cre*I sites include the wild-type non-palindromic I-*Cre*I homing site and the derived palindromic sequences such as the sequence 5'- t₋₁₂c₋₁₁a₋₁₀a₋₉a₋₈a₋₇c₋₆g₋₅t₋₄c₋₃g₋₂t₋₁a₊₁c₊₂g₊₃a₊₄c₊₅g₊₆t₊₇t₊₈t₊₉t₊₁₀g₊₁₁a₊₁₂ (SEQ ID NO: 2), also called C1221.
- by "domain" or "core domain" is intended the "LAGLIDADG homing endonuclease core domain" which is the characteristic αββαββα fold of the homing endonucleases of the LAGLIDADG family, corresponding to a sequence of about one hundred amino acid residues. Said domain comprises four beta-strands (β₁β₂β₃β₄) folded in an anti-parallel beta-sheet which interacts with one half of the DNA target. This domain is able to associate with another LAGLIDADG homing endonuclease core domain which interacts with the other half of the DNA target to form a functional endonuclease able to cleave said DNA target. For example, in the case of the dimeric homing endonuclease I*-Cre*I (163 amino acids), the LAGLIDADG homing endonuclease core domain corresponds to the residues 6 to 94.
- by "subdomain" is intended the region of a LAGLIDADG homing endonuclease core domain which interacts with a distinct part of a homing endonuclease DNA target half-site.
- by "chimeric DNA target" or "hybrid DNA target" it is intended the fusion of a different half of two parent meganuclease target sequences. In addition at least one half of said target may comprise the combination of nucleotides which are bound by at least two separate subdomains (combined DNA target). Is also encompassed in this definition a DNA target sequence, comprising a rare-cutting endonuclease target sequence (20-24 bp) and a frequent-cutting endonuclease target sequence (4-8 bp), recognized by a chimeric rare-cutting endonuclease according to the present invention.
- by "beta-hairpin" is intended two consecutive beta-strands of the antiparallel beta-sheet of a LAGLIDADG homing endonuclease core domain (β₁β₂ or β₃β₄) which are connected by a loop or a turn,
- by "single-chain meganuclease", "single-chain chimeric meganuclease", "single-chain meganuclease derivative", "single-chain chimeric meganuclease derivative" or "single-chain derivative" is intended a meganuclease comprising two LAGLIDADG homing endonuclease domains or core domains linked by a peptidic spacer as described in WO2009095793. The single-chain meganuclease is able to cleave a chimeric DNA target sequence comprising one different half of each parent meganuclease target sequence.
- by "DNA target", "DNA target sequence", "target sequence" , "target-site", "target" , "site", "site of interest", "recognition site", "polynucleotide recognition site", "recognition sequence", "homing recognition site", "homing site", "cleavage site" is intended a 20 to 24 bp double-stranded palindromic, partially palindromic (pseudo-palindromic) or non-palindromic polynucleotide sequence that is recognized and cleaved by a LAGLIDADG homing endonuclease such as I*-Cre*I, or a variant, or a single-chain chimeric meganuclease derived from I*-Cre*I. Said DNA target sequence is qualified of "cleavable" by an endonuclease, when recognized within a genomic sequence and known to correspond to the DNA target sequence of a given endonuclease or a variant of such endonuclease. These terms refer to a distinct DNA location, preferably a genomic location, at which a double stranded break (cleavage) is to be induced by the meganuclease. The DNA target is defined by the 5' to 3' sequence of one strand of the double-stranded polynucleotide, as indicate above for C1221. Cleavage of the DNA target occurs at the nucleotides at positions +2 and -2, respectively for the sense and the antisense strand. Unless otherwise indicated, the position at which cleavage of the DNA target by an I-*Cre* I meganuclease variant occurs, corresponds to the cleavage site on the sense strand of the DNA target.
- by "DNA target half-site", "half cleavage site" or half-site" is intended the portion of the DNA target which is bound by each LAGLIDADG homing endonuclease core domain.
- by "chimeric DNA target" or "hybrid DNA target" is intended the fusion of different halves of two parent meganuclease target sequences. In addition at least one half of said target may comprise the combination of nucleotides which are bound by at least two separate subdomains (combined DNA target).
- The term "endonuclease" refers to any wild-type or variant enzyme capable of catalyzing the hydrolysis (cleavage) of bonds between nucleic acids within of a DNA or RNA molecule, preferably a DNA molecule. Endonucleases do not cleave the DNA or RNA molecule irrespective of its sequence, but recognize and cleave the DNA or RNA molecule at specific polynucleotide sequences, further referred to as "target sequences" or "target sites". Endonucleases can be classified as rare-cutting endonucleases when having typically a polynucleotide recognition site of about 12-45 base pairs (bp) in length, more preferably of 14-45 bp. Rare-cutting endonucleases significantly increase HR by inducing DNA double-strand breaks (DSBs) at a defined locus (Rouet, Smih et al. 1994; Rouet, Smih et al. 1994; Choulika, Perrin et al. 1995; Pingoud and Silva 2007). Rare-cutting endonucleases can for example be a homing endonuclease (Paques and Duchateau 2007), a chimeric Zinc-Finger nuclease (ZFN) resulting from the fusion of engineered zinc-finger domains with the catalytic domain of a restriction enzyme such as FokI (Porteus and Carroll 2005) or a chemical endonuclease (Eisenschmidt, Lanio et al. 2005 ; Arimondo, Thomas et al. 2006; Simon, Cannata et al. 2008). In chemical endonucleases, a chemical or peptidic cleaver is conjugated either to a polymer of nucleic acids or to another DNA recognizing a specific target sequence, thereby targeting the cleavage activity to a specific sequence. Chemical endonucleases also encompass synthetic nucleases like conjugates of orthophenanthroline, a DNA cleaving molecule, and triplex-forming oligonucleotides (TFOs), known to bind specific DNA sequences (Kalish and Glazer 2005). Such chemical endonucleases are comprised in the term "endonuclease" according to the present invention.

Rare-cutting endonucleases can also be for example TALENs, a new class of chimeric nucleases using a FokI catalytic domain and a DNA binding domain derived from Transcription Activator Like Effector (TALE), a family of proteins used in the infection process by plant pathogens of the Xanthomonas genus (Boch, Scholze et al. 2009; Moscou and Bogdanove 2009; Christian, Cermak et al. 2010; Li, Huang et al. 2010). The functional layout of a FokI-based TALE-nuclease (TALEN) is essentially that of a ZFN, with the Zinc-finger DNA binding domain being replaced by the TALE domain. As such, DNA cleavage by a TALEN requires two DNA recognition regions flanking an unspecific central region. Rare-cutting endonucleases encompassed in the present invention can also be derived from TALENs.

Rare-cutting endonuclease can be a homing endonuclease, also known under the name of meganuclease. Such homing endonucleases are well-known to the art (Stoddard 2005). Homing endonucleases recognize a DNA target sequence and generate a single- or double-strand break. Homing endonucleases are highly specific, recognizing DNA target sites ranging from 12 to 45 base pairs (bp) in length, usually ranging from 14 to 40 bp in length. The homing endonuclease according to the invention may for example correspond to a LAGLIDADG endonuclease, to a HNH endonuclease, or to a GIY-YIG endonuclease. An expression such as "double-strand break creating agent" can be used to qualify a rare-cutting endonuclease according to the present invention.

In the wild, meganucleases are essentially represented by homing endonucleases. Homing Endonucleases (HEs) are a widespread family of natural meganucleases including hundreds of proteins families (Chevalier and Stoddard 2001). These proteins are encoded by mobile genetic elements which propagate by a process called "homing": the endonuclease cleaves a cognate allele from which the mobile element is absent, thereby stimulating a homologous recombination event that duplicates the mobile DNA into the recipient locus. Given their exceptional cleavage properties in terms of efficacy and specificity, they could represent ideal scaffolds to derive novel, highly specific endonucleases.

HEs belong to four major families. The LAGLIDADG family, named after a conserved peptidic motif involved in the catalytic center, is the most widespread and the best characterized group. Seven structures are now available. Whereas most proteins from this family are monomeric and display two LAGLIDADG motifs, a few have only one motif, and thus dimerize to cleave palindromic or pseudo-palindromic target sequences.

Although the LAGLIDADG peptide is the only conserved region among members of the family, these proteins share a very similar architecture. The catalytic core is flanked by two DNA-binding domains with a perfect two-fold symmetry for homodimers such as I-*Cre*I (Chevalier, Monnat et al. 2001), I-*Mso*I (Chevalier, Turmel et al. 2003) and I*-Ceu*I (Spiegel, Chevalier et al. 2006) and with a pseudo symmetry for monomers such as I-*Sce*I (Moure, Gimble et al. 2003), I-*Dmo*I (Silva, Dalgaard et al. 1999) or I*-Ani*I (Bolduc, Spiegel et al. 2003). Both monomers and both domains (for monomeric proteins) contribute to the catalytic core, organized around divalent cations. Just above the catalytic core, the two LAGLIDADG peptides also play an essential role in the dimerization interface. DNA binding depends on two typical saddle-shaped αββαββα folds, sitting on the DNA major groove. Other domains can be found, for example in inteins such as PI*-Pfu*I (Ichiyanagi, Ishino et al. 2000) and PI-*Sce*I (Moure, Gimble et al. 2002), whose protein splicing domain is also involved in DNA binding.

The making of functional chimeric meganucleases, by fusing the N-terminal I*-Dmo*I domain with an I*-Cre*I monomer (Chevalier, Kortemme et al. 2002; Epinat, Arnould et al. 2003); International PCT Application WO 03/078619 (Cellectis) and WO 2004/031346 (Fred Hutchinson Cancer Research Center, Stoddard et al)) have demonstrated the plasticity of LAGLIDADG proteins.

Different groups have also used a semi-rational approach to locally alter the specificity of the I*-Cre*I (Seligman, Stephens et al. 1997; Sussman, Chadsey et al. 2004); International PCT Applications WO 2006/097784, WO 2006/097853, WO 2007/060495 and WO 2007/049156 (Cellectis); (Arnould, Chames et al. 2006; Rosen, Morrison et al. 2006; Smith, Grizot et al. 2006), I*-Sce*I (Doyon, Pattanayak et al. 2006), PI-*Sce*I (Gimble, Moure et al. 2003) and I-*Mso*I (Ashworth, Havranek et al. 2006).

In addition, hundreds of I-*Cre*I derivatives with locally altered specificity were engineered by combining the semi-rational approach and High Throughput Screening:
- Residues Q44, R68 and R70 or Q44, R68, D75 and 177 of I*-Cre*I were mutagenized and a collection of variants with altered specificity at positions ± 3 to 5 of the DNA target (5NNN DNA target) were identified by screening (International PCT Applications WO 2006/097784 and WO 2006/097853 (Cellectis); (Arnould, Chames et al. 2006; Smith, Grizot et al. 2006).
- Residues K28, N30 and Q38 or N30, Y33 and Q38 or K28, Y33, Q38 and S40 of I-*Cre*I were mutagenized and a collection of variants with altered specificity at positions ± 8 to 10 of the DNA target (10NNN DNA target) were identified by screening (Arnould, Chames et al. 2006; Smith, Grizot et al. 2006); International PCT Applications WO 2007/060495 and WO 2007/049156 (Cellectis)).

Two different variants were combined and assembled in a functional heterodimeric endonuclease able to cleave a chimeric target resulting from the fusion of two different halves of each variant DNA target sequence ((Arnould, Chames et al. 2006; Smith, Grizot et al. 2006); International PCT Applications WO 2006/097854 and WO 2007/034262).

Furthermore, residues 28 to 40 and 44 to 77 of I*-Cre*I were shown to form two partially separable functional subdomains, able to bind distinct parts of a homing endonuclease target half-site (Smith, Grizot et al. 2006); International PCT Applications WO 2007/049095 and WO 2007/057781 (Cellectis)).

The combination of mutations from the two subdomains of I-*Cre*I within the same monomer allowed the design of novel chimeric molecules (homodimers) able to cleave a palindromic combined DNA target sequence comprising the nucleotides at positions ± 3 to 5 and ± 8 to 10 which are bound by each subdomain ((Smith, Grizot et al. 2006); International PCT Applications WO 2007/049095 and WO 2007/057781 (Cellectis)).

The method for producing meganuclease variants and the assays based on cleavage-induced recombination in mammal or yeast cells, which are used for screening variants with altered specificity are described in the International PCT Application WO 2004/067736; (Epinat, Arnould et al. 2003; Chames, Epinat et al. 2005; Arnould, Chames et al. 2006). These assays result in a functional LacZ reporter gene which can be monitored by standard methods.

The combination of the two former steps allows a larger combinatorial approach, involving four different subdomains. The different subdomains can be modified separately and combined to obtain an entirely redesigned meganuclease variant (heterodimer or single-chain molecule) with chosen specificity. In a first step, couples of novel meganucleases are combined in new molecules ("half-meganucleases") cleaving palindromic targets derived from the target one wants to cleave. Then, the combination of such "half-meganucleases" can result in a heterodimeric species cleaving the target of interest. The assembly of four sets of mutations into heterodimeric endonucleases cleaving a model target sequence or a sequence from different genes has been described in the following Cellectis International patent applications: XPC gene (WO2007/093918), RAG gene (WO2008/010093), HPRT gene (WO2008/059382), beta-2 microglobulin gene (WO2008/102274), Rosa26 gene (WO2008/152523), Human hemoglobin beta gene (WO2009/13622) and Human interleukin-2 receptor gamma chain gene (WO2009019614).

These variants can be used to cleave genuine chromosomal sequences and have paved the way for novel perspectives in several fields, including gene therapy.

Examples of such endonuclease include *I-Sce I, I-Chu I, I-Cre I, I-Csm I, PI-Sce I, PI-Tli I, PI-Mtu I, I-Ceu I, I-Sce II, I-Sce III, HO, PI-Civ I, PI-Ctr I, PI-Aae I, PI-Bsu I, PI-Dha I, PI-Dra I, PI-Mav I, PI-Mch I, PI-Mfu I, PI-Mfl I, PI-Mga I, PI-Mgo I, PI-Min I, PI-Mka I, PI-Mle I, PI-Mma I, PI-Msh I, PI-Msm I, PI-Mth I, PI-Mtu I, PI-Mxe I, PI-Npu I, PI-Pfu I, PI-Rma I, PI-Spb I, PI-Ssp I, PI-Fac I, PI-Mja I, PI-Pho I, PI-Tag I, PI-Thy I, PI-Tko I, PI-Tsp I, I-MsoI.*

A homing endonuclease can be a LAGLIDADG endonuclease such as I*-SceI,* I*-CreI,* I*-CeuI,* I*-MsoI,* and I*-DmoI.*

Said LAGLIDADG endonuclease can be I-Sce I, a member of the family that contains two LAGLIDADG motifs and functions as a monomer, its molecular mass being approximately twice the mass of other family members like I-Crel which contains only one LAGLIDADG motif and functions as homodimers.

Endonucleases mentioned in the present application encompass both wild-type (naturally-occurring) and variant endonucleases. Endonucleases according to the invention can be a "variant" endonuclease, i.e. an endonuclease that does not naturally exist in nature and that is obtained by genetic engineering or by random mutagenesis, i.e. an engineered endonuclease. This variant endonuclease can for example be obtained by substitution of at least one residue in the amino acid sequence of a wild-type, naturally-occurring, endonuclease with a different amino acid. Said substitution(s) can for example be introduced by site-directed mutagenesis and/or by random mutagenesis. In the frame of the present invention, such variant endonucleases remain functional, i.e. they retain the capacity of recognizing and specifically cleaving a target sequence to initiate gene targeting process.

The variant endonuclease according to the invention cleaves a target sequence that is different from the target sequence of the corresponding wild-type endonuclease. Methods for obtaining such variant endonucleases with novel specificities are well-known in the art.

Endonucleases variants may be homodimers (meganuclease comprising two identical monomers) or heterodimers (meganuclease comprising two non-identical monomers).

Endonucleases with novel specificities can be used in the method according to the present invention for gene targeting and thereby integrating a transgene of interest into a genome at a predetermined location.
- by "parent meganuclease" it is intended to mean a wild type meganuclease or a variant of such a wild type meganuclease with identical properties or alternatively a meganuclease with some altered characteristic in comparison to a wild type version of the same meganuclease. In the present invention the parent meganuclease can refer to the initial meganuclease from which the first series of variants are derived in step (a) or the meganuclease from which the second series of variants are derived in step (b), or the meganuclease from which the third series of variants are derived in step (k).
- By " delivery vector" or " delivery vectors" is intended any delivery vector which can be used in the present invention to put into cell contact or deliver inside cells or subcellular compartments agents/chemicals and molecules (proteins or nucleic acids) needed in the present invention. It includes, but is not limited to liposomal delivery vectors, viral delivery vectors, drug delivery vectors, chemical carriers, polymeric carriers, lipoplexes, polyplexes, dendrimers, microbubbles (ultrasound contrast agents), nanoparticles, emulsions or other appropriate transfer vectors. These delivery vectors allow delivery of molecules, chemicals, macromolecules (genes, proteins), or other vectors such as plasmids, peptides developed by Diatos. In these cases, delivery vectors are molecule carriers. By "delivery vector" or "delivery vectors" is also intended delivery methods to perform transfection
- The terms "vector" or "vectors" refer to a nucleic acid molecule capable of transporting another nucleic acid to which it has been linked. A "vector" in the present invention includes, but is not limited to, a viral vector, a plasmid, a RNA vector or a linear or circular DNA or RNA molecule which may consists of a chromosomal, non chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adenoassociated viruses), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).
- By "lentiviral vector" is meant HIV-Based lentiviral vectors that are very promising for gene delivery because of their relatively large packaging capacity, reduced immunogenicity and their ability to stably transduce with high efficiency a large range of different cell types. Lentiviral vectors are usually generated following transient transfection of three (packaging, envelope and transfer) or more plasmids into producer cells. Like HIV, lentiviral vectors enter the target cell through the interaction of viral surface glycoproteins with receptors on the cell surface. On entry, the viral RNA undergoes reverse transcription, which is mediated by the viral reverse transcriptase complex. The product of reverse transcription is a double-stranded linear viral DNA, which is the substrate for viral integration in the DNA of infected cells.
- By "integrative lentiviral vectors (or LV)", is meant such vectors as non limiting example, that are able to integrate the genome of a target cell.
- At the opposite by "non integrative lentiviral vectors (or NILV)" is meant efficient gene delivery vectors that do not integrate the genome of a target cell through the action of the virus integrase.

One type of preferred vector is an episome, *i.e.,* a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors. A vector according to the present invention comprises, but is not limited to, a YAC (yeast artificial chromosome), a BAC (bacterial artificial), a baculovirus vector, a phage, a phagemid, a cosmid, a viral vector, a plasmid, a RNA vector or a linear or circular DNA or RNA molecule which may consist of chromosomal, non chromosomal, semi-synthetic or synthetic DNA. In general, expression vectors of utility in recombinant DNA techniques are often in the form of "plasmids" which refer generally to circular double stranded DNA loops which, in their vector form are not bound to the chromosome. Large numbers of suitable vectors are known to those of skill in the art. Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; TRP1 for *S*. *cerevisiae;* tetracyclin, rifampicin or ampicillin resistance in *E. coli.* Preferably said vectors are expression vectors, wherein a sequence encoding a polypeptide of interest is placed under control of appropriate transcriptional and translational control elements to permit production or synthesis of said polypeptide. Therefore, said polynucleotide is comprised in an expression cassette. More particularly, the vector comprises a replication origin, a promoter operatively linked to said encoding polynucleotide, a ribosome binding site, a RNA-splicing site (when genomic DNA is used), a polyadenylation site and a transcription termination site. It also can comprise an enhancer or silencer elements. Selection of the promoter will depend upon the cell in which the polypeptide is expressed. Suitable promoters include tissue specific and/or inducible promoters. Examples of inducible promoters are: eukaryotic metallothionine promoter which is induced by increased levels of heavy metals, prokaryotic lacZ promoter which is induced in response to isopropyl-D-D-thiogalacto-pyranoside (IPTG) and eukaryotic heat shock promoter which is induced by increased temperature. Examples of tissue specific promoters are skeletal muscle creatine kinase, prostate-specific antigen (PSA), α-antitrypsin protease, human surfactant (SP) A and B proteins, β-casein and acidic whey protein genes.
- Inducible promoters may be induced by pathogens or stress, more preferably by stress like cold, heat, UV light, or high ionic concentrations (reviewed in Potenza C et al. 2004, In vitro Cell Dev Biol 40:1-22). Inducible promoter may be induced by chemicals (reviewed in (Moore, Samalova et al. 2006); (Padidam 2003); (Wang, Zhou et al. 2003); (Zuo and Chua 2000).

Delivery vectors and vectors can be associated or combined with any cellular permeabilization techniques such as sonoporation or electroporation or derivatives of these techniques.
- By cell or cells is intended any prokaryotic or eukaryotic living cells, cell lines derived from these organisms for *in vitro* cultures, primary cells from animal or plant origin.
- By "primary cell" or "primary cells" are intended cells taken directly from living tissue (i.e. biopsy material) and established for growth in vitro, that have undergone very few population doublings and are therefore more representative of the main functional components and characteristics of tissues from which they are derived from, in comparison to continuous tumorigenic or artificially immortalized cell lines. These cells thus represent a more valuable model to the in vivo state they refer to.
- In the frame of the present invention, "eukaryotic cells" refer to a fungal, plant or animal cell or a cell line derived from the organisms listed below and established for in vitro culture. More preferably, the fungus is of the genus Aspergillus, Penicillium, Acremonium, Trichoderma, Chrysoporium, Mortierella, Kluyveromyces or Pichia; More preferably, the fungus is of the species Aspergillus niger, Aspergillus nidulans, Aspergillus oryzae, Aspergillus terreus, Penicillium chrysogenum, Penicillium citrinum, Acremonium Chrysogenum, Trichoderma reesei, Mortierella alpine, Chrysosporium lucknowense, Kluyveromyces lactis, Pichia pastoris or Pichia ciferrii.

More preferably the plant is of the genus Arabidospis, Nicotiana, Solanum, lactuca, Brassica, Oryza, Asparagus, Pisum, Medicago, Zea, Hordeum, Secale, Triticum, Capsicum, Cucumis, Cucurbita, Citrullis, Citrus, Sorghum; More preferably, the plant is of the species Arabidospis thaliana, Nicotiana tabaccum, Solanum lycopersicum, Solanum tuberosum, Solanum melongena, Solanum esculentum, Lactuca saliva, Brassica napus, Brassica oleracea, Brassica rapa, Oryza glaberrima, Oryza sativa, Asparagus officinalis, Pisum sativum, Medicago sativa, zea mays, Hordeum vulgare, Secale cereal, Triticum aestivum, Triticum durum, Capsicum sativus, Cucurbita pepo, Citrullus lanatus, Cucumis melo, Citrus aurantifolia, Citrus maxima, Citrus medica, Citrus reticulata.

More preferably the animal cell is of the genus Homo, Rattus, Mus, Sus, Bos, Danio, Canis, Felis, Equus, Salmo, Oncorhynchus, Gallus, Meleagris, Drosophila, Caenorhabditis; more preferably, the animal cell is of the species Homo sapiens, Rattus norvegicus, Mus musculus, Sus scrofa, Bos taurus, Danio rerio, Canis lupus, Felis catus, Equus caballus, Salmo salar, Oncorhynchus mykiss, Gallus gallus, Meleagris gallopavo, Drosophila melanogaster, Caenorhabditis elegans.
- by "homologous" is intended a sequence with enough identity to another one to lead to homologous recombination between sequences, more particularly having at least 95 % identity, preferably 97 % identity and more preferably 99 %.
- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting.
- by "mutation" is intended the substitution, deletion, insertion of one, two, three, four, five, six, seven, eight, nine, ten, eleven, twelve, thirteen, fourteen, fifteen, sixteen, seventeen, eighteen, nineteen, twenty or more nucleotides/amino acids in a polynucleotide (cDNA, gene) or a polypeptide sequence. Said mutation can affect the coding sequence of a gene or its regulatory sequence. It may also affect the structure of the genomic sequence or the structure/stability of the encoded mRNA.
- In the frame of the present invention, the expression "double-strand break-induced mutagenesis" (DSB-induced mutagenesis) refers to a mutagenesis event consecutive to an NHEJ event following an endonuclease-induced DSB, leading to insertion/deletion at the cleavage site of an endonuclease.
- By "gene" is meant the basic unit of heredity, consisting of a segment of DNA arranged in a linear manner along a chromosome, which codes for a specific protein or segment of protein. A gene typically includes a promoter, a 5' untranslated region, one or more coding sequences (exons), optionally introns, a 3' untranslated region. The gene may further comprise a terminator, enhancers and/or silencers.
- As used herein, the term "transgene" refers to a sequence encoding a polypeptide. Preferably, the polypeptide encoded by the transgene is either not expressed, or expressed but not biologically active, in the cell, tissue or individual in which the transgene is inserted. Most preferably, the transgene encodes a therapeutic polypeptide useful for the treatment of an individual.
- The term "gene of interest" or "GOI" refers to any nucleotide sequence encoding a known or putative gene product.
- As used herein, the term "locus" is the specific physical location of a DNA sequence (e.g. of a gene) on a chromosome. The term "locus" usually refers to the specific physical location of an endonuclease's target sequence on a chromosome. Such a locus, which comprises a target sequence that is recognized and cleaved by an endonuclease according to the invention, is referred to as "locus according to the invention". Also, the expression "genomic locus of interest" is used to qualify a nucleic acid sequence in a genome that can be a putative target for a double-strand break according to the invention. By "endogenous genomic locus of interest" is intended a native nucleic acid sequence in a genome, *i.e.,* a sequence or allelic variations of this sequence that is naturally present at this genomic locus. It is understood that the considered genomic locus of interest of the present invention can be between two overlapping genes the considered endonuclease's target sequences are located in two different genes. It is understood that the considered genomic locus of interest of the present invention can not only qualify a nucleic acid sequence that exists in the main body of genetic material (*i.e.,* in a chromosome) of a cell but also a portion of genetic material that can exist independently to said main body of genetic material such as plasmids, episomes, virus, transposons or in organelles such as mitochondria or chloroplasts as non-limiting examples.
- By the expression "loss of genetic information" is understood the elimination or addition of at least one given DNA fragment (at least one nucleotide) or sequence, bordering the recognition sites of the endonucleases of the present invention and leading to a change of the original sequence around said endonuclease-cutting sites, within the genomic locus of interest. This loss of genetic information can be, as a non-limiting example, the elimination of an intervening sequence between two endonuclease-cutting sites; it can also be, in another non-limiting example, the result of an exonuclease DNA-ends processing activity after a unique endonuclease DNA double-strand break. In this last case, loss of genetic information within the genomic locus of interest is generated "around said DNA target sequence", i.e. around the endonuclease-cutting site (DSB), taken as reference. It can also be, in other non-limiting examples, the result of DNA-ends processing activities by other enzymes, after a unique endonuclease DNA double-strand break, such as polymerase activity (TdT...), dephosphatase activity...

- By the expression "two nearby DNA double strand breaks" within the genomic locus of interest, is meant two endonucleases cutting sites distant at between 12 bp and 1000 bp.
- By "scarless re-ligation" is intended the perfect re-ligation event, without loss of genetic information (no insertion/deletion events) of the DNA broken ends through NHEJ process after the creation of a double-strand break event. The present invention relates to a method to increase double-strand break mediated mutagenesis by avoiding any such "scarless re-ligation" process.
- By "fusion protein" is intended the result of a well-known process in the art consisting in the joining of two or more genes which originally encode for separate proteins, the translation of said "fusion gene" resulting in a single polypeptide with functional properties derived from each of the original proteins.
- By "chimeric rare-cutting endonuclease" is meant any fusion protein comprising a rare-cutting endonuclease fused to a catalytic domain having exonuclease activity. Said rare-cutting endonuclease might be at the N- terminus part of said chimeric rare-cutting endonuclease; at the opposite, said rare-cutting endonuclease might be at the C- terminus part of said chimeric rare-cutting endonuclease. A "chimeric rare-cutting endonuclease" according to the present invention which comprises two catalytic domains can be described as "bi-functional" or as "bi-functional meganuclease". A "chimeric rare-cutting endonuclease" according to the present invention which comprises more than two catalytic domains can be described as "multi-functional" or as "multi-functional meganuclease". As non-limiting examples, chimeric rare-cutting endonucleases according to the present invention can be a fusion protein between a rare-cutting endonuclease and one catalytic domain; chimeric rare-cutting endonucleases according to the present invention can also be a fusion protein between a rare-cutting endonuclease and two catalytic domains. As mentioned previously, the rare-cutting endonuclease part of chimeric rare-cutting endonucleases according to the present invention can be a meganuclease comprising either two identical monomers, either two non identical monomers, or a single chain meganuclease. The rare-cutting endonuclease part of chimeric rare-cutting endonucleases according to the present invention can also be the DNA-binding domain of a rare-cutting endonuclease. In other non-limiting examples, chimeric rare-cutting endonucleases according to the present invention can be derived from a TALE-derived from a Transcription Activator Like Effector (TALE) and one or two catalytic domains.
- By "frequent-cutting endonuclease" is intended an endonuclease typically having a polynucleotide recognition site of about 4-8 base pairs (bp) in length, more preferably of 4-6 bp.
- By a "TALE-nuclease" (TALEN) is intended a fusion protein consisting of a DNA-binding domain derived from a Transcription Activator Like Effector (TALE) and one FokI catalytic domain, that need to dimerize to form an active entity able to cleave a DNA target sequence.
- By "catalytic domain" is intended the protein domain or module of an enzyme containing the active site of said enzyme; by active site is intended the part of said enzyme at which catalysis of the substrate occurs. Enzymes, but also their catalytic domains, are classified and named according to the reaction they catalyze. The Enzyme Commission number (EC number) is a numerical classification scheme for enzymes, based on the chemical reactions they catalyze (http://www.chem.qmul.ac.uk/iubmb/enzyme/). In the scope of the present invention, any catalytic domain can be fused to a rare-cutting endonuclease to generate a chimeric rare-cutting endonuclease. Non-limiting examples of such catalytic domains are given in table 2 and in table 3 with a GenBank or NCBI or UniProtKB/Swiss-Prot number as a reference.
- By "nuclease catalytic domain" is intended the protein domain comprising the active site of an endonuclease or an exonuclease enzyme. Non-limiting examples of such catalytic domains are given in table 2 and in table 3 with a GenBank or NCBI or UniProtKB/Swiss-Prot number as a reference.

The above written description of the invention provides a manner and process of making and using it such that any person skilled in this art is enabled to make and use the same, this enablement being provided in particular for the subject matter of the appended claims, which make up a part of the original description.

As used above, the phrases "selected from the group consisting of," "chosen from," and the like include mixtures of the specified materials.

Where a numerical limit or range is stated herein, the endpoints are included. Also, all values and subranges within a numerical limit or range are specifically included as if explicitly written out.

Having generally described this invention, a further understanding can be obtained by reference to certain specific examples, which are provided herein for purposes of illustration only, and are not intended to be limiting unless otherwise specified.

### Examples

### Example 1 (Comparative)

Two engineered single-chain meganucleases called R1 or R1m (SEQ ID NO: 58) and D21 or D21m (SEQ ID NO: 59) are produced using the methods disclosed in International PCT Applications WO2003078619, WO2004/067736, WO2006/097784, WO2006/097853, WO2007/060495, WO 2007/049156, WO 2006/097854, WO2007/034262, WO 2007/049095, WO2007/057781 and WO2009095793 (Cellectis) and in (Chames, Epinat et al. 2005; Arnould, Chames et al. 2006; Smith, Grizot et al. 2006). These meganucleases, derived from I-CreI, are designed to recognize two different DNA sequences, neither of which are recognized by wild-type I-CreI. (recognition sequences, respectively, tgttctcaggtacctcagccag SEQ ID NO: 3 and aaacctcaagtaccaaatgtaa SEQ ID NO: 4). Expression of these two meganucleases is driven by a CMV promoter and a polyA signal sequence. The two corresponding recognition sites are cloned in close proximity to generate the target plasmid. For this example, the recognition sites are separated by 10 bp (Figure 1). DNA cleavage by a meganuclease generates characteristic 4-nt 3'-OH overhangs. The simultaneous cleavage of both sites is expected to eliminate the intervening sequence and therefore abolish "scarless" re-ligation by NHEJ (Figure 1).

Human HEK293 cells are transiently co-transfected with two plasmids carrying the expression cassette for R1 (SEQ ID NO: 58) and D21 (SEQ ID NO: 59), as well as the target plasmid. For comparison, HEK293 cells are transiently co-transfected with the target plasmid and only one meganuclease-expressing plasmid. DNA is extracted 2 days post-transfection and targeted mutagenesis is assessed by a mutation detection assay as depicted in figure 2 (surveyor assay from Transgenomic, Inc. USA). High-fidelity PCR amplification of the DNA encompassing the two recognition sites is performed using appropriate specific primers. The same PCR amplification is performed on genomic DNA extracted from cells transfected with the target plasmid alone. After quantification and purification, equimolar amounts of PCR products are mixed in an annealing buffer and a fraction of this mixture is subjected to a melting/annealing step, resulting in the formation of distorted duplex DNA through random re-annealing of mutant and wild-type DNA. CEL-1 enzyme (surveyor assay from Transgenomic, Inc. USA) is added to specifically cleave the DNA duplexes at the sites of mismatches. The CEL-1 cleaved samples are resolved on analytical gel, stained with ethidium bromide and the DNA bands are quantified using densitometry. The frequency of mutagenesis can then be calculated essentially as described in Miller et al (2007).

PCR products from cells transfected with the target plasmid and (a) an empty plasmid; (b) one meganuclease expressing plasmid or; (c) two plasmids expressing respectively R1 (SEQ ID NO: 58) and D21 (SEQ ID NO: 59), are also analyzed by high-throughput sequencing (Figure 2). In this case, PCR amplification is performed with appropriate primers to obtain a fragment flanked by specific adaptor sequences (adaptor A: 5'-CCATCTCATCCCTGCGTGTCTCCGAC-**NNNN**-3', SEQ ID NO: 5 and adaptor B, 5'-CCTATCCCCTGTGTGCCTTGGCAGTCTCAG-3', SEQ ID NO: 6) provided by the company (GATC Biotech AG, Germany) offering sequencing service on a 454 sequencing system (454 Life Sciences). Approximately 10,000 exploitable sequences are obtained per PCR pool and then analyzed for the presence of site-specific insertion or deletion events.
The inventers are able to show that deletion of the intervening sequence or microsequence following the creation of two DNA DSBs greatly enhances the site-specific NHEJ-driven mutation rate. This deletion is observed only when both meganucleases are introduced into the cells.

### Example 2

In this example, an engineered single-chain meganuclease derived from I-CreI (described in International PCT Applications WO2003078619, WO 2004/067736, WO 2006/097784, WO 2006/097853, WO 2007/060495, WO 2007/049156, WO 2006/097854, WO 2007/034262, WO 2007/049095, WO 2007/057781, WO 2008/010093 and WO2009095793 (Cellectis) and in (Chames, Epinat et al. 2005; Arnould, Chames et al. 2006; Smith, Grizot et al. 2006)) is fused to various nuclease domains to create a bi-functional meganuclease. To obtain maximal activity, 31 different linkers are tested ranging in size from 3 to 26 amino acids (Table 1). Fusions are made using 18 different catalytic domains (Table 2) that are chosen based on their having essentially non-specific nuclease activity. Altogether, a library of 1116 different constructs are created via fusion to the N- or C-terminus of the engineered single-chain I-CreI-derived meganuclease, generating a collection of potential bi-functional meganucleases. Expression of these chimeric meganucleases are driven by a CMV promoter and a polyA signal sequence. The activity of each chimeric protein is assessed using our yeast assay previously described in International PCT Applications WO 2004/067736 and in (Epinat, Arnould et al. 2003; Chames, Epinat et al. 2005; Arnould, Chames et al. 2006; Smith, Grizot et al. 2006). To monitor DNA cleavage activity resulting from the addition of the new catalytic domain, an I-CreI DNA target sequence is selected that can be bound but not cleaved by the wild-type meganuclease. This target contains 4-nucleotide substitutions at positions -2 to +2 (Figure 3). Enzymes exhibiting cleavage activity are then tested following protocols described in example 1, except that the target plasmid carried only one cleavage site for the I-Crel meganuclease.

To further validate the high rate of site-specific mutagenesis induced by a bi-functional chimeric endonuclease, the same strategy is applied to an engineered single-chain meganuclease designed to cleave the human RAG1 gene as described in International PCT Applications WO2003078619, WO 2008/010093 and WO2009095793. The bi-functional meganuclease is tested for its ability to induced NHEJ-driven mutation at its endogenous cognate recognition site. The mutagenesis activity is quantified by high-throughput sequencing of PCR products as described in example 1. PCR amplification is performed on genomic DNA extracted from meganuclease-transfected cells using appropriate primers.

The bi-functional meganucleases displayed an increased mutation rate since the intervening sequence is deleted, thereby preventing "scarless" re-ligation of DNA ends through NHEJ.

**Table 1: sequence of linkers used to fuse the catalytic domains to meganucleases**

| **Name (PDB)** | **Amino Acids** | **Length** | **Size (Da)** | **Sequence** | **SEQ ID NO** |
|---|---|---|---|---|---|
| 1a8h_1 | 285 - 287 | 3 | 6,636 | NVG | 7 |
| 1dnpA_1 | 130 - 133 | 4 | 7,422 | DSVI | 8 |
| 1d8cA_2 | 260 - 263 | 4 | 8,782 | IVEA | 9 |
| 1ckqA_3 | 169 - 172 | 4 | 9,91 | LEGS | 10 |
| 1sbp_1 | 93 - 96 | 4 | 10,718 | YTST | 11 |
| 1ev7A_1 | 169 - 173 | 5 | 11,461 | LQENL | 12 |
| 1alo_3 | 360 - 364 | 5 | 12,051 | VGRQP | 13 |
| 1amf_1 | 81 - 85 | 5 | 13,501 | LGNSL | 14 |
| 1adjA_3 | 323 -328 | 6 | 14,835 | LPEEKG | 15 |
| 1 fcdC_1 | 76 - 81 | 6 | 14,887 | QTYQPA | 16 |
| 1a13_2 | 265 - 270 | 6 | 15,485 | FSHSTT | 17 |
| 1g3p_1 | 99 - 105 | 7 | 17,903 | GYTYINP | 18 |
| 1acc_3 | 216 - 222 | 7 | 19,729 | LTKYKSS | 19 |
| 1ahjB_1 | 106 - 113 | 8 | 17,435 | SRPSESEG | 20 |
| 1acc_1 | 154 - 161 | 8 | 18,776 | PELKQKSS | 21 |
| 1af7_1 | 89 - 96 | 8 | 22,502 | LTTNLTAF | 22 |
| 1heiA_1 | 322 - 330 | 9 | 13,534 | TATPPGSVT | 23 |
| 1bia_2 | 268 - 276 | 9 | 16,089 | LDNFINRPV | 24 |
| 1igtb_1 | 111 - 119 | 9 | 19,737 | VSSAKTTAP | 25 |
| 1nfkA_1 | 239 - 248 | 10 | 13,228 | DSKAPNASNL | 26 |
| 1au7A_1 | 103 - 112 | 10 | 20,486 | KRRTTISIAA | 27 |
| 1bpob_1 | 138 - 148 | 11 | 21,645 | PVKMFDRHSSL | 28 |
| 1b0pA_2 | 625 - 635 | 11 | 26,462 | APAETKAEPMT | 29 |
| 1c05A_2 | 135 - 148 | 14 | 23,819 | YTRLPERSELPAEI | 30 |

| **Name (PDB)** | **Amino Acids** | **Length** | **Size (Da)** | **Sequence** | **SEQ ID NO** |
|---|---|---|---|---|---|
| Igcb_1 | 57 - 70 | 14 | 27,39 | VSTDSTPVTNQKSS | 31 |
| 1bt3A_1 | 38 - 51 | 14 | 28,818 | YKLPAVTTMKVRPA | 32 |
| 1b3oB_2 | 222 - 236 | 15 | 20,054 | IARTDLKKNRDYPLA | 33 |
| 16vpA_6 | 312 - 332 | 21 | 23,713 | TEEPGAPLTTPPTLHGNQARA | 34 |
| 1dhx_1 | 81 - 101 | 21 | 42,703 | ARFTLAVGDNRVLDMASTYFD | 35 |
| 1b8aA_1 | 95 - 120 | 26 | 31,305 | IVVLNRAETPLPLDPTGKVKAELDTR | 36 |
| 1qu6A_1 | 79 - 106 | 28 | 51,301 | ILNKEKKAVSPLLLTTTNSSEGLSMGNY | 37 |
| NFS1 | - | 20 | - | GSDITKSKISEKMKGQGPSG | 78 |
| NFS2 | - | 23 | - | GSDITKSKISEKMKGLGPDGRKA | 79 |
| CFS1 | - | 10 | - | SLTKSKISGS | 80 |
| RM2 | - | 32 | - | AAGGSALTAGALSLTAGALSLTAGALSGGGGS | 94 |
| BQY | - | 25 | - | AAGASSVSASGHIAPLSLPSSPPSVGS | 95 |
| QGPSG | - | 5 | - | QGPSG | 67 |
| LGPDGRKA | - | 8 | - | LGPDGRKA | 68 |

**Table 2 : sequences of the catalytic domains fused to meganucleases.**

| **DATABASE reference** | **Name** | **SEQ ID NO** | **SEQUENCE** |
|---|---|---|---|
| GenBank: ACC85607.1 | **MmeI** | 38 | |
| GenBank: EAJ03172.1 | **EsaSSII** | 39 | |
| NCBI Reference Sequence: NP_862240.1 | **CstMI** | 40 | |
| | | | |
| GenBank: CAA45962.1 | **NucA** | 41 | |
| P25736 (END1_ECOLI), UniProtKB/Swiss-Prot | **EndA Escherichia coli** | 42 | |
| P37994 (NUCM_DICD3), UniProtKB/Swiss-Prot | **NucM** | 43 | |
| P0A3S3 (NUCE_STRPN), UniProtKB/Swiss-Prot | **EndA Streptococcus pneumonia** | 44 | |
| P00644 (NUC_STAAU), UniProtKB/Swiss-Prot | **SNase Staphylococcus aureus** | 45 | |
| P43270 (NUC_STAHY), UniProtKB/Swiss-Prot | **SNase Staphylococcus hyicus** | 46 | |
| P29769 (NUC_SHIFL), UniProtKB/Swiss-Prot | **SNase Shigella flexneri** | 47 | |
| P94492 (YNCB_BACSU), UniProtKB/Swiss-Prot | **Bacillus subtilis yncB** | 48 | |
| P00641 (ENRN_BPT7), UniProtKB/Swiss-Prot | **Endodeoxyribonucle ase 1 Enterobacteria phage T7** | 49 | |
| P38447 (NUCG_BOVIN), UniProtKB/Swiss-Prot | **EndoG bovine** | 50 | |
| Q56239 (MUTS_THET8), UniProtKB/Swiss-Prot | **ttSmr DNA mismatch repair protein mutS** | 51 | |
| Q53H47(SETMR HUM AN), UniProtKB/Swiss-Prot | **Cleavage domain of Metnase** | 52 | |
| GenBank: AAF19759.1 | **Vvn** | 53 | |
| Q47112 (CEA7_ECOLX), UniProtKB/Swiss-Prot | **ColE7 nuclease domain** | 54 | |
| P34081 (HMUI_BPSPI), UniProtKB/Swiss-Prot | **I-HmuI** | 55 | |
| P13299 (TEV1_ BPT4), UniProtKB/Swiss-Prot | **I-TevI** | 56 | |
| Q38419 (TEV3_BPR03), UniProtKB/Swiss-Prot | **I-TevHIII** | 57 | |

**Table 3 : sequences of the catalytic domains fused to meganucleases.**

| **GENB ANK/S WISS-PROT ID** | **NAME** | **SEQ ID NO** | **FASTA SEQUENCE** |
|---|---|---|---|
| ACC85 607.1 | **Mmel** | 96 | >gi\|186469979\|gb\|ACC85607.1\| MmeI [Methylophilus methylotrophus] |
| | | | |
| Q47112 .2 | **Colicin-E7 (CEA7_ECO LX)** | 97 | >gi\|12644448\|sp\|Q47112.2\|CEA7_ ECOLX RecName: Full=Colicin-E7 |
| | | | |
| CAA38 134.1 | **EndA** | 98 | >g1\|47374\|emb\|CAA38134.1\| EndA [Streptococcus pneumoniae] |
| | | | |
| P25736 .1 | **Endo I (END1_ECO LI)** | 99 | >gi\|119325\|sp\|P25736.1\|END1_ECOLI RecName: Full=Endonuclease-1; AltName: Full=Endonuclease I; Short=Endo I; Flags: Precursor |
| | | | |
| Q14249 .4 | **Human Endo G (NUCG_HU MAN)** | 100 | >gi\|317373579\|sp\|Q14249.4\|NUCG_ HUMAN RecName: Full=Endonuclease G, mitochondrial; Short=Endo G; Flags: Precursor |
| | | | |
| P38447 .1 | **Bovine Endo G (NUCG_BO VIN)** | 101 | >gi\|585596\|sp\|P38447.1\|NUCG_BOVIN RecName: Full=Endonuclease G, mitochondrial; Short=Endo G; Flags: Precursor |
| | | | |
| AAW3 3811.1 | **R.HinP1I** | 102 | >gi\|57116674\|gb\|AAW33811.1\| R.HinPlI restriction endonuclease [Haemophilus influenzae] |
| | | | |
| AAO93 095.1 | **I-BasI** | 103 | >gi\|29838473\|gb\|AA093095.1\| I-BasI [Bacillus phage Bastille] |
| | | | |
| AAK09 365.1 | **I-BmoI** | 104 | >gi\|12958590\|gb\|AAK09365.1\|AF321518_2 intron encoded I-BmoI [Bacillus mojavensis] |
| | | | |
| P34081 .1 | **I-HmuI** | 105 | >gi\|465641\|sp\|P34081.1\|HMUI_BPSP1 RecName: Full=DNA endonuclease I-HmuI; AltName: Full=HNH homing endonuclease I-HmuI |
| | | | |
| P13299 .2 | **I-TevI** | 106 | >gi\|6094464\|sp\|P13299.2\|TEV1_BPT4 RecName: Full=Intron-associated endonuclease 1; AltName: Full=I-TevI; AltName: Full=IRF protein |
| | | | |
| P07072 .2 | **I-TevII** | 107 | >gi\|20141823\|sp\|PO7O72.2\|TEV2_BPT4 RecName: Full=Intron-associated endonuclease 2; AltName: Full=I-TevII |
| | | | |
| Q38419 .1 | **I-TevIII** | 108 | >gi\|11387192\|sp\|Q38419.1\|TEV3_BPR03 RecName: Full=Intron-associated endonuclease 3; AltName: Full=I-TevIII |
| | | | |
| AAM0 0817.1 | **I-TwoI** | 109 | >gi\|19881200\|gb\|AAM00817.1\|AF485080_2 HNH endonuclease I-TwoI [Staphylococcus phage Twort] |
| | | | |
| P11405 .1 | **R.MspI** | 110 | >gi\|135239\|sp\|P11405.1\|T2M1_MORSP RecName: Full=Type-2 restriction enzyme MspI; Short=R.MspI; AltName: Full=Endonuclease MspI; AltName: Full=Type II restriction enzyme MspI |
| | | | |
| R.MvaI | **R.MvaI** | 111 | >gi\|119392963\|gb\|AAM03024.2\|AF472612_1 R.MvaI [Kocuria varians] |
| | | | |
| CAA45 962.1 | **NucA** | 112 | >gi\|39041\|emb\|CAA45962.1\| NucA [Nostoc sp. PCC 7120] |
| | | | |
| P37994 .2 | **NucM** | 113 | >gi\|313104150\|sp\|P37994.2\|NUCM_DICD3 RecName: Full=Nuclease nucM; Flags: Precursor |
| | | | |
| AAF19 759.1 | **Vvn** | 114 | >gi\|6635279\|gb\|AAF19759.1\|AF063303_1 nuclease precursor Vvn [Vibrio vulnificus] |
| | | | |
| AAF19 759.1 (referen ce) | **Vvn_CLS** | 115 | >Vvn_CLS (variant of AAF19759.1) |
| | | | |
| P00644 .1 | **Staphylococe al nuclease (NUC_STAA U)** | 116 | >gi\|128852\|sp\|P00644.1\|NUC_STAAU RecName: Full=Thermonuclease; Short=TNase; AltName: Full=Micrococcal nuclease; AltName: Full=Staphylococcal nuclease; Contains: RecName: Full=Nuclease B; Contains: RecName: Full=Nuclease A; Flags: Precursor |
| | | | |
| | | | |
| P43270 .1 | **Staphylococe al nuclease (NUC_STAH Y)** | 117 | >gi\|1171859\|sp\|P43270.1\|NUC_STAHY RecName: Full=Thermonuclease; Short=TNase; AltName: Full=Micrococcal nuclease; AltName: Full=Staphylococcal nuclease; Flags: Precursor |
| | | | |
| P29769 .1 | **Micrococcal nuclease (NUC_SHIF L)** | 118 | >gi\|266681\|sp\|P29769.1\|NUC_SHIFL RecName: Full=Micrococcal nuclease; Flags: Precursor |
| | | | |
| P94492 .1 | **Endonuclease yncB** | 119 | >gi\|81345826\|sp\|\|YNCB_BACSU RecName: Full=Endonuclease yncB; Flags: Precursor |
| | | | |
| 1300641 .1 | **Endodeoxyri bonuclease I (ENRN_BPT 7)** | 120 | >gi\|119370\|sp\|P00641.1\|ENRN_BPT7 RecName: Full=Endodeoxyribonuclease 1; AltName: Full=Endodeoxyribonuclease I; Short=Endonuclease |
| | | | |
| Q53H4 7.1 | **Metnase** | 121 | >gi\|74740552\|sp\|Q53H47.1\|SETMR_HUMAN RecName: Full=Histone-lysine N-methyltransferase SETMAR; AltName: Full=SET domain and mariner transposase fusion gene-containing protein; Short=HsMar1; Short=Metnase; Includes: RecName: Full=Histone-lysine N-methyltransferase; Includes: RecName: Full=Mariner transposase Hsmarl |
| | | | |
| ABD15 132.1 | **Nb.BsrDI** | 122 | >gi\|86757493\|gb\|ABD15132.1\| Nb.BsrDI [Geobacillus stearothermophilus] |
| | | | |
| ABD15 133.1 | **BsrDI A** | 123 | >gi\|86757494\|gb\|ABD15133.1\| BsrDI A [Geobacillus stearothermophilus] |
| | | | |
| ABN42 182.1 | **Nt.BspD6I (R.BspD6I large subunit)** | 124 | >gi\|125396996\|gb\|ABN42182.1\| heterodimeric restriction endonuclease R.BspD6I large subunit [Bacillus sp. D6] |
| | | | |
| ABN42 183.1 | **ss.BspD6I (R.BspD6I small subunit)** | 125 | >gi\|125396997\|gb\|ABN42183.1\| heterodimeric restriction endonuclease R.BspD6I small subunit [Bacillus sp. D6] |
| | | | |
| AAK27 215.1 | **R.PleI** | 126 | >gi\|13448813\|gb\|AAK27215.1\|AF355461_2 restriction endonuclease R.PleI [Paucimonas lemoignei] |
| | | | |
| AAK39 546.1 | **MlyI** | 127 | >gi\|13786046\|gb\|AAK39546.1\|AF355462_2 MlyIR [Micrococcus lylae] |
| | | | |
| YP_004 134094. 1 | **AlwI** | 128 | >gi\|319768594\|ref\|YP_004134094.1\| restriction endonuclease, type II, AlwI [Geobacillus sp. Y412MC52] |
| | | | |
| AAY97 906.1 | **Mva1269I** | 129 | >gi\|68480350\|gb\|AAY97906.1\| Mva1269I restriction endonuclease [Kocuria varians] |
| | | | |
| ADR72 996.1 | **BsrI** | 130 | >gi\|313667100\|gb\|ADR72996.1\| BsrI [Geobacillus stearothermophilus] |
| | | | |
| AAL86 024.1 | **BsmI** | 131 | >gi\|19347662\|gb\|AAL86024.1\| BsmI [Geobacillus stearothermophilus] |
| | | | |
| | | | |
| ADI242 25.1 | **Nb.BtsCI** | 132 | >gi\|297185870\|gb\|ADI24225.1\| BtsCI bottom-strand nicking enzyme variant [synthetic construct] |
| | | | |
| ADI242 24.1 | **Nt.BtsCI** | 133 | >gi\|297185868\|gb\|ADI24224.1\| BtsCI top-strand nicking enzyme variant [synthetic construct] |
| | | | |
| | | | >gi\|85720924\|gb\|ABC75874.1\| R1.BtsI [Geobacillus thermoglucosidasius] |
| | | | |
| ABC75 874.1 | **R1.BtsI** | 134 | >gi\|85720924\|gb\|ABC75874.1\| R1.BtsI [Geobacillus thermoglucosidasius] |
| | | | |
| ABC75 876.1 | **R2.BtsI** | 135 | >gi\|85720926\|gb\|ABC75876.1\| R2.BtsI [Geobacillus thermoglucosidasius] |
| | | | |
| | | | |
| AAX14 652.1 | **BbvCI subunit 1** | 136 | >gi\|60202520\|gb\|AAX14652.1\| BbvCI endonuclease subunit 1 [Brevibacillus brevis] |
| | | | |
| AAX14 653.1 | **BbvCI subunit 2** | 137 | >gi\|60202521\|gb\|AAX14653.1\| BbvCI endonuclease subunit 2 [Brevibacillus brevis] |
| | | | |
| CAA74 998.1 | **Bpu10I alpha subunit** | 138 | >gi\|2894388\|emb\|CAA74998.1\| BpulOI restriction endonuclease alpha subunit [Bacillus pumilus] |
| | | | |
| CAA74 999.1 | **Bpu10I beta subunit** | 139 | >gi\|2894389\|emb\|CAA74999.1\| Bpu10I restriction endonuclease beta subunit [Bacillus pumilus] |
| | | | |
| ABM69 266.1 | **BmrI** | 140 | >gi\|123187377\|gb\|ABM69266.1\| BmrI [Bacillus megaterium] |
| | | | |
| CAC12 783.1 | **BfiI** | 141 | >gi\|10798463\|emb\|CAC12783.1\| restriction endonuclease BfiI [Bacillus firmus] |
| | | | |
| | | | |
| Q9UQ8 4.2 | **hExoI (EXO1_HU MAN)** | 142 | >gi\|85700954\|sp\|Q9UQ84.2\|EXO1_ HUMAN RecName: Full=Exonuclease 1; Short=hExo1; AltName: Full=Exonuclease I; Short=hExoI |
| | | | |
| P39875 .2 | **Yeast Exol (EXO1_YEA ST)** | 143 | >gi\|1706421\|sp\|P39875.2\|EXO1_YEAST RecName: Full=Exodeoxyribonuclease 1; AltName: Full=Exodeoxyribonuclease I; Short=EXO I; Short=Exonuclease I; AltName: Full=Protein DHS1 |
| | | | |
| BAJ438 03.1 | **E.coli Exol** | 144 | >gi\|315136644\|dbj\|BAJ43803.1\|exonuclease I [Escherichia Coli DH1] |
| | | | |
| Q9BQ5 0.1 | **Human TREX2** | 145 | >gi\|47606206\|sp\|Q9BQ50.1\|TREX2_HUMAN RecName: Full=Three prime repair exonuclease 2; AltName: Full=3'-5' exonuclease TREX2 |
| | | | |
| | | | |
| Q91XB 0.2 | **Mouse TREX1** | 146 | >gi\|47606196\|sp\|Q91XB0.2\|TREX1_MOUSE RecName: Full=Three prime repair exonuclease 1; AltName: Full=3'-5' exonuclease TREX1 |
| | | | |
| Q9NSU 2.1 | **Human TREX1** | 147 | >gi\|47606216\|sp\|Q9NSU2.1\|TREX1_ HUMAN RecName: Full=Three prime repair exonuclease 1; AltName: Full=3'-5' exonuclease TREX1; AltName: Full=DNase III |
| | | | |
| Q9BG9 9.1 | **Bovine TREX1** | 148 | >gi\|47606205\|sp\|Q9BG99.1\|TREX1_BOVIN RecName: Full=Three prime repair exonuclease 1; AltName: Full=3'-5' exonuclease TREX1 |
| | | | |
| AAH91 242.1 | **Rat TREX1** | 149 | >gi\|60688197\|gb\|AAH91242.1\| Trex1 protein [Rattus norvegicus] |
| | | | |
| AAH63 664.1 | **Human DNA2** | 150 | >gi\|39793966\|gb\|AAH63664.1\| **DNA2 protein** [Homo sapiens] |
| | | | |
| | | | |
| P38859 .1 | **Yeast DNA2 (DNA2_ YEA ST)** | 151 | >gi\|731738\|sp\|P38859.1\|DNA2_**YEAST** RecName: Full=DNA replication ATP-dependent helicase **DNA2** |
| | | | |
| AAA45 863.1 | **VP16** | 152 | >gi\|330318\|gb\|AAA45863.1\| **VP16** [Human herpesvirus 2] |
| | | | |

### Example 3 (Comparative)

I-CreI meganuclease (SEQ ID NO: 76) was chosen as the parent scaffold on which to fuse the catalytic domain of I-TevI (SEQ ID NO: 60). Wild-type I-TevI functions as a monomeric cleavase of the GIY-YIG family to generate a staggered double-strand break in its target DNA. Guided by biochemical and structural data, variable length constructs were designed from the N-terminal region of I-TevI that encompass the entire catalytic domain and deletion-intolerant region of its linker (SEQ ID NOs: 61 to 66). In all but one case, fragments were fused to the N-terminus of I-CreI with an intervening 5-residue polypeptide linker (-QGPSG- = SEQ ID NO: 67). The linker-less fusion construct naturally contained residues (-LGPDGRKA- = SEQ ID NO: 68) similar to those in the artificial linker. As I-CreI is a homodimer, all fusion constructs contain three catalytic centers (Figure 4D): the natural I-CreI active site at the interface of the dimer and one I-TevI active site per monomer.

The activity of each "tri-functional" meganuclease was assessed using yeast assay previously described in International PCT Applications WO 2004/067736 and in (Epinat, Arnould et al. 2003; Chames, Epinat et al. 2005; Arnould, Chames et al. 2006; Smith, Grizot et al. 2006). All constructs were able to cleave the C1221 target DNA with an activity comparable to that of wild-type I-Crel (Table 4).

To validate the activity of the I-TevI catalytic domain independent of the I-Crel catalytic core, D20N point mutants were made to inactivate the I-CreI scaffold (SEQ ID NOs: 69 to 75). Tests in yeast assays showed no visible activity from the inactivated I-Crel (D20N) mutant protein alone (Table 4). However, cleavage activity could be observed for fusions having the I-TevI catalytic domain (Table 4).

**Table 4**

| **Protein Construct** | **Relative Activity in Yeast Assay (37°C)** | |
|---|---|---|
| | **C1221 Target** | **I-TevI Target** |
| I-CreI | ++++ | - |
| I-TevI | - | ++++ |
| I-CreI_N20 | - | - |
| hTevCre_D01 | ++++ | - |
| hTevCre_D02 | ++++ | - |
| hTevCre_D03 | ++++ | - |
| hTevCre_D04 | ++++ | - |
| hTevCre_D05 | ++++ | - |
| hTevCre_D06 | ++++ | - |
| hTevCre_D01_N20 | ++ | - |
| hTevCre_D02_N20 | ++ | - |
| hTevCre_D03_N20 | ++ | - |
| hTevCre_D04_N20 | ++ | - |
| hTevCre_D05_N20 | - | - |
| hTevCre_D06_N20 | - | - |

**Table 4: Activity in Yeast assay for I-TevI/I-CreI fusions.** The relative activity of wild-type and fusion proteins on the two parent protein targets (C1221 for I-CreI and Tev for I-TevI) is shown. Maximal activity (++++) is seen with each given protein on its native DNA target. I-CreI_N20 is an inactive variant of the wild-type I-CreI scaffold. In all other cases, activity is only detected on the C1221 target since DNA recognition is driven by the I-CreI scaffold. The "N20" fusion variants illustrate cleavage activity due to the I-TevI catalytic domain.

Relative activity is scaled as: -, no activity detectable; +, <25% activity; ++, 25% to <50% activity; +++, 50% to <75% activity; ++++, 75% to 100% activity.

### Example 4 (Comparative)

Protein-fusion scaffolds were designed based on a truncated form of I-CreI (SEQ ID NO: 76, 1-CreI_X: SEQ ID NO: 77) and three different linker polypeptides (SEQ ID NOs: 78 to 80) fused to either the N- or C-terminus of the protein. Structure models were generated in all cases, with the goal of designing a "baseline" fusion linker that would traverse the I-CreI parent scaffold surface with little to no effect on its DNA binding or cleavage activities. For the two N-terminal fusion scaffolds, the polypeptide spanning residues 2 to 153 of I-CreI was used, with a K82A mutation to allow for linker placement. The C-terminal fusion scaffold contains residues 2 to 155 of wild-type I-CreI. For both fusion scaffold types, the "free" end of the linker (i.e. onto which a polypeptide can be linked) is designed to be proximal to the DNA, as determined from models built using the I-CreI/DNA complex structures as a starting point (PDB id: 1g9z). The two I-CreI N-terminal fusion scaffolds (I-CreI_NFS1 = SEQ ID NO: 81 and I-CreI_NFS2 = SEQ ID NO: 82) and the single C-terminal fusion scaffold (I-CreI_CFS1: SEQ ID NO: 83) were tested in our yeast assay (see Example 3) and found to have activity similar to that of wild-type I-CreI (Table 5).

Colicin E7 is a non-specific nuclease of the HNH family able to process single- and double-stranded DNA. Guided by biochemical and structural data, the region of ColE7 that encompasses the entire catalytic domain (SEQ ID NO: 84) was selected. This ColE7 domain was fused to the N-terminus of either I-CreI_NFS1 (SEQ ID NO: 81) or I-CreI_NFS2 (SEQ ID NO: 83) to create hColE7Cre_D0101 (SEQ ID: 85) or hColE7Cre_D0102 (SEQ ID NO: 86), respectively. In addition, a C-terminal fusion construct, hCreColE7_D0101 (SEQ ID: 87), was generated using I-CreI_CFS1 (SEQ ID NO: 83). As I-CreI is a homodimer, all fusion constructs contain three catalytic centers (Figure 4D): the natural I-CreI active site at the interface of the dimer and one ColE7 active site per monomer.

The activity of each "tri-functional" meganuclease was assessed using yeast assay as previously mentioned (see Example 3). All constructs were able to cleave the C1221 target DNA with an activity comparable to that of wild-type I-CreI (Table 4). To validate the activity of the ColE7 catalytic domain independent of the I-Crel catalytic core, D20N point mutants were made to inactivate the I-CreI scaffold (SEQ ID NOs: 88-93). Tests in our yeast assays showed no visible activity from the inactivated I-CreI (D20N) mutant proteins alone (Table 5). However, cleavage activity could be observed for fusions having the ColE7 catalytic domain (Table 5).

**Table 5**

| **Protein Construct** | **Relative Activity in Yeast Assay (37°C)** |
|---|---|
| | **C1221 Target** |
| I-CreI | ++++ |
| I-CreI_X | ++++ |
| I-CreI_NFS1 | ++++ |
| I-CreI_NFS2 | ++++ |
| I-CreI_CFS1 | ++++ |
| I-CreI_NFS1_N20 | - |
| I-CreI_NFS2_N20 | - |
| I-CreI-CFS1_N20 | - |
| hColE7Cre_D0101 | ++++ |
| hColE7Cre_D0102 | ++++ |
| hCreColE7_D0101 | ++++ |
| hColE7Cre_D0101_N20 | +++ |
| hColE7Cre_D0102_N20 | +++ |
| hCreColE7_D0101_N20 | ++ |

**Table 5: Activity in Yeast assay for ColE7/I-CreI fusions.** The relative activity of wild-type and fusion proteins on theC1221 target is shown. I-CreI_X represents a truncated version of I-CreI based on the crystal structure and was used as the foundation for the fusion scaffolds (I-CreI_NFS1, I-CreI_NFS2 and I-CreI_CFS1). "N20" constructs are inactive variants of the respective I-CreI-based scaffolds. Activity is detected in all cases wherein the I-CreI scaffold is active or when DNA catalysis is provided by the ColE7 domain.

Relative activity is scaled as: -, no activity detectable; +, <25% activity; ++, 25% to <50% activity; +++, 50% to <75% activity; ++++, 75% to 100% activity.

### Example 5: Effect of Trex2 or TREX2 (SEQ ID NO: 145) on meganuclease-induced mutagenesis

Human Trex2 protein (SEQ ID NO: 145) is known to exhibit a 3' to 5' exonuclease activity (Mazur and Perrino, 2001). A 236 amino acid functional version of Trex2 (SEQ ID NO: 194) has been fused to single-chain meganucleases (SC-MN) for measuring improvements on meganuclease-induced targeted mutagenesis of such chimeric rare-cutting endonucleases. Levels of mutagenesis induced by SC-MN-Trex2 have been compared to levels of mutagenesis induced by co-transfecting vectors independently expressing SC-MN and Trex2 protein in a dedicated cellular model and at endogenous loci in 293H cells.

### Example 5A: Co-transfection of Trex2 (SEQ ID NO: 145) with meganucleases

A vector encoding meganuclease SC_GS (pCLS2690, SEQ ID NO: 153) was co-transfected into a cell line for monitoring mutagenic events in the presence or absence of a vector encoding Trex2 (pCLS7673, SEQID NO: 154). The SC_GS meganuclease is a single chain protein (SEQ ID NO: 193) derived from the fusion of two I-CreI variants. It recognizes a 22bp DNA sequence (5'-TGCCCCAGGGTGAGAAAGTCCA-3': GS_CHO.1 target, SEQ ID NO: 155) located in the first exon of the *Cricetulus griseus* glutamine synthetase gene. Different meganucleases such as SC_RAG1 (pCLS2222, SEQID NO: 156 i.e. the expression vector encoding SC_RAG1, SEQ ID NO: 58), SC_XPC4 (pCLS2510, SEQID NO: 157 i.e. the expression vector encoding SC_XPC4, SEQ ID NO: 190) and SC_CAPNS1 (pCLS6163, SEQID NO: 158 i.e. the expression vector encoding SC_CAPNS1, SEQ ID NO: 192) were co-transfected with or without a Trex2 expression vector (pCLS7673, SEQID NO: 154) to analyze the effect on meganuclease-induced mutagenesis at endogenous loci .

### Material and Methods

### a) Cellular model to monitor meganuclease-induced mutagenesis

The plasmid pCLS6810 (SEQID NO: 159) was designed to quantify the NHEJ repair frequency induced by the SC_GS meganuclease (pCLS2690, SEQ ID NO: 153). The sequence used to measure SC_GS-induced mutagenesis is made of an ATG start codon followed by (i) 2 codons for alanine; (ii) an HA-tag sequence; (iii) the SC_GS recognition site; (iv) a stretch of glycine-serine di-residues; (v) an additional 2 codons for alanine as in (i) and finally; (vi) a GFP reporter gene lacking its ATG start codon. The GFP reporter gene is inactive due to a frame-shift introduced by the GS recognition site. The creation of a DNA double-strand break (DSB) by the SC_GS meganuclease followed by error-prone NHEJ events can lead to restoration of the GFP gene expression in frame with the ATG start codon. The final construct was introduced at the RAG1 locus in 293H cell line using the hsRAG1 Integration Matrix CMV Neo from cGPS® Custom Human Full Kit DD (Cellectis Bioresearch) following the provider's instructions. Using this kit, a stable cell line containing a single copy of the transgene at the RAG1 locus was obtained. Thus, after transfection of this cell line by SC_GS meganuclease expressing plasmid with or without a plasmid encoding Trex2 (pCLS7673, SEQ ID NO: 154), the percentage of GFP positive cells is directly correlated to the mutagenic NHEJ repair frequency induced by the transfected molecular entity/ies.

### b) Transfection in a cellular model monitoring meganuclease-induced mutagenesis

One million of cells were seeded one day prior to transfection. Cells were co-transfected with 1µg of SC_GS encoding vector (pCLS2690, SEQID NO: 153) and with 0, 2, 4, 6 or 9 µg of plasmid encoding Trex2 (pCLS7673 SEQID NO: 154) in 10 µg of total DNA by complementation with a pUC vector (pCLS0002, SEQID NO: 191) using 25 µl of lipofectamine (Invitrogen) according to the manufacturer's instructions. Four days following transfection, cells were harvested for flow cytometry analysis using Guava instrumentation. Genomic DNA was extracted from cell populations transfected with 1 µg of SC_GS expressing plasmid and 0, 4 and 9 µg of Trex2 encoding plasmid. Locus specific PCR were performed using the following primers: 5'-CCATCTCATCCCTGCGTGTCTCCGACTCAG (forward adaptor sequence)-10N-(sequences needed for PCR product identification)-GCTCTCTGGCTAACTAGAGAACCC (transgenic locus specific forward sequence) -3' (SEQ ID NO: 160) and 5'-CCTATCCCCTGTGTGCCTTGGCAGTCTCAG-(reverse adaptor sequence)-TCGATCAGCACGGGCACGATGCC (transgenic locus specific reverse sequence) (SEQ ID NO: 161), and PCR products were sequenced by a 454 sequencing system (454 Life Sciences). Approximately 10,000 sequences were obtained per PCR product and then analyzed for the presence of site-specific insertion or deletion events.

### c) Transfection on 293H cells to monitor meganuclease-induced mutagenesis at endogenous loci

One million of cells were seeded one day prior to transfection. Cells were co-transfected with 3µg of plasmid expressing SC_RAG1 or SC_XPC4 or SC_CAPNS1 (pCLS2222, SEQID NO: 156; pCLS2510, SEQID NO: 157 and pCLS6163, SEQID NO: 158 respectively) and with 0 or 2 µg of plasmid encoding Trex2 (pCSL7673 SEQID NO: 154) in 5 µg of total DNA by complementation with a pUC vector (pCLS0002 SEQID NO: 191) using 25 µl of lipofectamine (Invitrogen) according to the manufacturer's instructions. Locus specific PCR were performed using the following primers: 5'-CCATCTCATCCCTGCGTGTCTCCGACTCAG-(forward adaptor sequence)-10N-(sequences needed for PCR product identification)-locus specific forward sequence for RAG1 : GGCAAAGATGAATCAAAGATTCTGTCC-3' (SEQ ID NO: 162), for XPC4: - AAGAGGCAAGAAAATGTGCAGC-3' (SEQ ID NO: 163) and for CAPNS1 - CGAGTCAGGGCGGGATTAAG-3' (SEQ ID NO: 164) and the reverse primer 5'-CCTATCCCCTGTGTGCCTTGGCAGTCTCAG-(reverse adaptor sequence)-(endogenous locus specific reverse sequence for RAG1:- GATCTCACCCGGAACAGCTTAAATTTC-3' (SEQ ID NO: 165), for XPC4: -GCTGGGCATATATAAGGTGCTCAA-3' (SEQ ID NO: 166) and for CAPNS1: -CGAGACTTCACGGTTTCGCC-3' (SEQ ID NO: 167). PCR products were sequenced by a 454 sequencing system (454 Life Sciences). Approximately 10,000 sequences were obtained per PCR product and then analyzed for the presence of site-specific insertion or deletion events.

### Results

### 1- On cellular model measuring meganuclease-induced mutagenesis

The percentage of GFP+ cells, monitoring mutagenesis events induced by SC_GS meganuclease in a dedicated cellular model, was analyzed 96h after a transfection with SC_GS expressing plasmid (pCLS2690 SEQID NO: 153) alone or with an increasing dose of Trex2 encoding vector (pCLS7673 SEQID NO: 154). The percentage of GFP+ cells increased with the amount of Trex2 expressing plasmid transfected. In absence of Trex2, SC_GS expression led to 0.3% of GFP+ cells whereas 2, 4, 6 and 9 µg of Trex2 encoding plasmid led to 1.3, 2.8, 3.4 and 4.8% of GFP+ respectively (**Figure 5A**). This phenotypic stimulation of GFP+ cells was confirmed at a molecular level. SC_GS led to 2.4% of targeted mutagenesis whereas co-transfection of SC_GS expressing plasmid with 4 and 9 µg of Trex2 encoding vector stimulate this mutagenic DSB repair to 9.4 and 13.1% respectively (**Figure 5B**). Moreover the nature of the mutagenic events was analyzed. In presence of Trex2, up to 65% of the mutagenic events correspond to the complete or partial loss of the 3' overhang (deletion2, deletion3 and deletion4) generated by SC_GS meganuclease. In contrast, in absence of Trex2 activity, such mutagenic events are found in 20% of the total mutagenic events (**Figure 5C**).

### 2- At endogenous loci

Trex2 effect on mutagenesis induced by engineered meganucleases was measured at RAG1, XPC4 and CAPNS1 endogenous loci by co-transfecting plasmids expressing SC_RAG1 or SC_XPC4 or SC_CAPNS1 with or without Trex2 encoding plasmid. Transfections of 3 µg of meganuclease expressing vector with 2 µg of Trex2 (3/2 ratio) encoding plasmid were performed.The mutagenesis induced by the different meganucleases was quantified and analyzed three days post transfection. In these conditions, Trex2 stimulates mutagenesis at all loci studied with a stimulating factor varying from 1.4 up to 5 depending on the locus (**Table 6**). The nature of mutagenic events was also analyzed. It showed a modification of the pattern of the deletions induced by the meganucleases. As showed in **Figure 6****,** particularly at RAG1 (panelA) and CAPNS1 loci (panelC), the frequency of small deletions corresponding to degradation of 3' overhangs is significantly increased in the presence of Trex2.

**Table 6: Specific meganuclease-induced NHEJ quantification at endogenous loci with or without Trex2 and corresponding stimulation factors**

| | 2µg pUC | 2µg Trex2 | Stimulation by Trex2 |
|---|---|---|---|
| XPC4 | 0,69 | 3,41 | 4,94 |
| RAG1 | 1,88 | 5,18 | 2,75 |
| CAPNS1 | 11,28 | 16,24 | 1,44 |

### Example 5B: Fusion of the human Trex2 protein to the N- or C-terminus of an engineered meganuclease.

Expressing Trex2 within a cell can lead to exonuclease activity at loci not targeted by the meganuclease. Moreover, for obvious reasons, co-tranfection of two expressing vectors makes difficult to control the optimum expression of both proteins. In order to bypass those difficulties and to target Trex2 activity to the DSB induced by the meganuclease, the human Trex2 protein was fused to the N- or C-terminus of the SC_GS engineered meganuclease (SEQ ID NO: 153). Four SC_GS/Trex2 fusion proteins were made and tested for their ability to cleave their target (GS_CHO.1 target). The level of mutagenesis induced by each construct was measured using the cellular model described in example 5A.

### Material and Methods

### a) Making of SC_GS/Trex2 fusion proteins

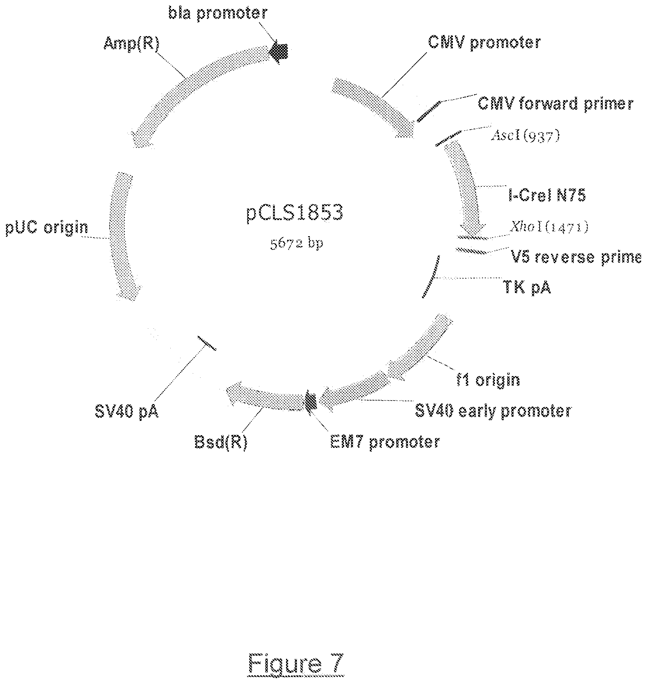

The Trex2 protein was fused to the SC_GS meganuclease either to its C-terminus or to its N-terminus using a five amino acids glycin stretch (sequence GGGGS) (SEQ ID NO: 169) or a ten amino acids glycin stretch (GGGGS)₂ (SEQ ID NO: 170) as linkers. This yielded to four protein constructs named respectively SC_GS-5-Trex, SC_GS-10-Trex, Trex-5-SC_GS, Trex-10-SC_GS (SEQ ID NO: 171 to 174). Both SC_GS and Trex2 were initially cloned into the AscI / XhoI restriction sites of the pCLS1853 (**Figure 7**, SEQ ID NO: 175), a derivative of the pcDNA3.1 (Invitrogen), which drives the expression of a gene of interest under the control of the CMV promoter. The four fusion protein constructs were obtained by amplifying separately the two ORFs using a specific primer and the primer CMVfor (5'-CGCAAATGGGCGGTAGGCGT-3'; SEQ ID NO: 176) or V5reverse (5'-CGTAGAATCGAGACCGAGGAGAGG-3'; SEQ ID NO: 177), which are located on the plasmid backbone. Then, after a gel purification of the two PCR fragments, a PCR assembly was realized using the CMVfor / V5reverse oligonucleotides. The final PCR product was then digested by AscI and Xhol and ligated into the pCLS1853 digested with these same enzymes. The following table gives the oligonucleotides that were used to create the different constructs.

**Table 7: Oligonucleotides used to create the different SC_GS/Trex2 constructs**

| **Construct** | **Amplified ORF** | **Forward primer** | **SEQ ID NO:** | **Reverse primer** | **SEQ ID NO:** |
|---|---|---|---|---|---|
| SC_GS-5-Trex | SC_GS | CMVfor | 176 | Link5GSRev | 179 |
| | Trex2 | Link5TrexFor | 178 | V5reverse | 177 |
| SC_GS-10-Trex | SC_GS | CMVfor | 176 | Link10GSRev | 181 |
| | Trex2 | Link10TrexFor | 180 | V5reverse | 177 |
| Trex-5-SC_GS | Trex2 | CMVfor | 176 | Link5TrexRev | 183 |
| | SC_GS | Link5GSFor | 182 | V5reverse | 177 |
| Trex-10-SC_GS | Trex2 | CMVfor | 176 | LinklOTrexRev | 185 |
| | SC_GS | Link10GSFor | 184 | V5reverse | 177 |

### b) Extrachromosomal SSA activity

CHO-K1 cells were transfected with the expression vector for the protein of interest and the reporter plasmid in the presence of Polyfect transfection reagent in accordance with the manufacturer's protocol (Qiagen). Culture medium was removed 72 hours after transfection and lysis/detection buffer was added for the β-galactosidase liquid assay. One liter of lysis/detection buffer contains: 100 ml of lysis buffer (10 mM Tris-HCl pH 7.5, 150 mM NaCl, 0.1% Triton X100, 0.1 mg/ml BSA, protease inhibitors), 10 ml of 100X Mg buffer (100 mM MgCl2, 35% 2-mercaptoethanol), 110ml of a 8 mg/ml solution of ONPG and 780 ml of 0.1M sodium phosphate pH 7.5. The OD₄₂₀ is measured after incubation at 37°C for 2 hours. The entire process was performed using a 96-well plate format on an automated Velocity 11 BioCel platform (Grizot, Epinat et al. 2009).

### c) Meganuclease-induced mutageneis

One million of cells were seeded one day prior transfection. Cells were transfected with an increasing amount (from 1µg up to 9µg) of plasmid encoding SC_GS (pCLS2690 , SEQ ID NO: 153) or SC_GS-5-Trex (pCLS8082 SEQ ID NO: 186), SC_GS-10-Trex (pCLS8052 SEQ ID NO: 187), Trex-5-SC_GS (pCLS8053 SEQ ID NO: 188) and Trex-10-SC_GS (pCLS8054 SEQ ID NO: 189) in 10 µg of total DNA by complementation with a pUC vector (SEQ ID NO: 191) using 25 µl of lipofectamine (Invitrogen) according to the manufacturer's instructions. Three to four days following transfection, cells were harvested for flow cytometry analysis using Guava instrumentation. Cells transfected with 1 µg and 6 µg of SC_GS or SC_GS-10-Trex2 expressing plasmid were harvested for genomic DNA extraction. Locus specific PCR were performed using the following primers: 5'-CCATCTCATCCCTGCGTGTCTCCGACTCAG (forward adaptor sequence)- 10N-(sequences needed for PCR product identification)-GCTCTCTGGCTAACTAGAGAACCC (transgenic locus specific forward sequence) -3' (SEQ ID NO: 160) and 5'-CCTATCCCCTGTGTGCCTTGGCAGTCTCAG-(reverse adaptor sequence)-TCGATCAGCACGGGCACGATGCC (transgenic locus specific reverse sequence) (SEQ ID NO: 161). PCR products were sequenced by a 454 sequencing system (454 Life Sciences). Approximately 10,000 sequences were obtained per PCR product and then analyzed for the presence of site-specific insertion or deletion events.

### Results

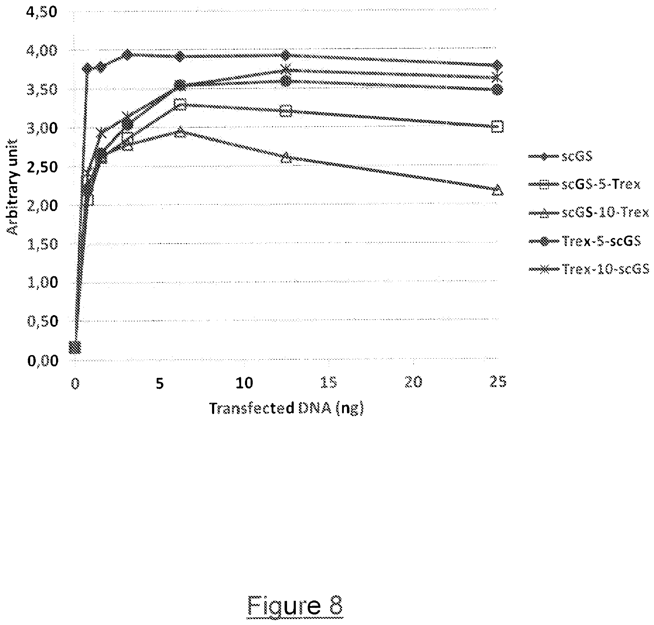

The activity of the four fusion proteins was first monitored using an extrachromosomal assay in CHO-K1 cells (Grizot, Epinat et al. 2009). The fusion of Trex2 to the SC_GS could indeed impair its folding and/or its activity. **Figure 8** shows that the four fusion proteins (SC_GS-5-Trex, SC_GS-10-Trex, Trex-5-SC_GS and Trex-10-SC_GS, SEQ ID NO: 171 to 174 encoded in plasmids of SEQ ID NO: 186 to 189) are active in that assay.

### 1- On cellular model measuring meganuclease-induced mutagenesis

The cell line described in example 5A was transfected with plasmids expressing either SC_GS or the 4 different fusion proteins. Quantification of the percentage of GFP+ cells was determined by flow cytometry 4 days post transfection. SC_GS induced 0.5 to 1% of GFP+ cells whereas the all four fusion constructs enhance the percentage of GFP+ cells in dose dependent manner from 2 up to 9% (**Figure 9A**). This strategy appears to be more efficient than the co-transfection strategy as the highest frequency of 4.5% of GFP+ cells was obtained using 9 µg of Trex2 expressing vector (**Figure 5A**) whereas this frequency can be obtained using only 3µg of any fusion expressing vector (**Figure 9A**). The targeted locus was analyzed by PCR amplification followed by sequencing, after cellular transfection of 1ug and 6µg of SC_GS or Trex2-10-SC_GS expressing plasmid. The deletions events were greatly enhanced with the fusion construct compared to the native meganuclease. 1 µg or 6µg of Trex2-10-SC_GS expressing plasmid led to 24% and 31% of mutagenic events all corresponding to deletions. These NHEJ frequencies were higher than the ones obtained using 4 or 9 µg of Trex2 expressing vector in co-transfection experiments (9% and 13% respectively), (**Figures 5B** **and** **9B**). Finally molecular analysis showed that the complete or partial loss of the 3' overhang (deletion2, deletion3 and deletion4) generated by SC_GS or the fusion Trex2-10-SC_GS were 35% and 80% respectively. Altogether these results demonstrate that the fusion protein Trex2-SC_GS is highly active as a targeted mutagenic reagent (frequency of GFP+ cells obtained, frequency of mutagenic events analyzed by deep-sequencing and the frequency of the signature of Trex2 nuclease activity).

### Example 5C: Effect of Trex2 fused with an engineered meganuclease on mutagenesis at an endogenous locus in immortalized or primary cell line

Trex2 fused to SC_GS was shown to stimulate Targeted Mutagenesis [TM] at a transgenic locus in immortalized cell line. In order to apply the fusion to other engineered meganucleases and to stimulate TM in primary cell line Trex2 was fused to SC_CAPNS1 and TM was monitored at an endogenous locus in immortalized cell line as well as in primary cell line.

### Material and Methods

### d) Making of Trex2-SC_CAPNS 1 fusion protein

The Trex2 protein (SEQ ID NO: 194) was fused to the SC_CAPNS1 meganuclease (SEQ ID NO: 192) at its N-terminus using a (GGGGS)₂ ten amino acids linker (SEQ ID NO: 170). Cloning strategy was the same as used for the fusion Trex-SC_GS. Both SC_CAPNS1 and Trex2 were initially cloned into the AscI / XhoI restriction sites of the pCLS1853 (figure 7, SEQID NO: 175), a derivative of the pcDNA3.1 (Invitrogen), which drives the expression of a gene of interest under the control of the CMV promoter. The fusion protein construct was obtained by amplifying separately the two ORFs using specific primers: for CAPNS1 Link10GSFor
5'-GGAGGTTCTGGAGGTGGAGGTTCCAATACCAAATATAACGAAGAGTTC-3' (SEQ ID NO: 184)
was used with V5 reverse primer 5'-CGTAGAATCGAGACCGAGGAGAGG-3' (SEQ ID NO: 177); Trex ORF was amplified using CMVfor primer 5'-CGCAAATGGGCGGTAGGCGT-3' (SEQ ID NO: 176) and Link10TrexRev primer

Then, after a gel purification of the two PCR fragments, a PCR assembly was realized using the CMVfor / V5reverse oligonucleotides. The final PCR product was then digested by AscI and Xhol and ligated into the pCLS1853 plasmid digested with these same enzymes leading to Trex-SC_CAPNS1 encoding vector (pCLS8518 of SEQID NO: 196 encoding Trex-SC_CAPNS 1 protein of SEQ ID NO: 197).

### e) Transfection on 293H cells to monitor Trex2-Meganuclease fusion on mutagenesis at an endogenous locus

One million of cells were seeded one day prior to transfection. Cells were transfected with 100ng of either SC_CAPNS1 or Trex-SC_CAPNS1 encoding vector (respectively, protein sequence of SEQ ID NO: 192 encoded by pCLS6163 of SEQ ID NO: 158 and protein sequence of SEQ ID NO: 197 encoded by pCLS8518 of SEQID NO: 196) in 5 µg of total DNA by complementation with a pUC vector (pCLS0002 SEQID NO: 191) using 25 µl of lipofectamine (Invitrogen) according to the manufacturer's instructions. Three days following transfection, cells were harvested for genomic DNA extraction.

### f) Transfection on Detroit cells to monitor Trex2-Meganuclease fusion on mutagenesis at an endogenous locus

One million of cells were seeded one day prior to transfection. Cells were co-transfected with 6µg of either SC_CAPNS1 or Trex-SC_CAPNS1 encoding vector (respectively pCLS6163 of SEQID NO: 158 and pCLS8518 of SEQID NO: 196) in 10 µg of total DNA by complementation with a pUC vector (pCLS0002, SEQID NO: 191) using Amaxa (LONZA) according to the manufacturer's instructions. Three days following transfection, cells were harvested for genomic DNA extraction.

### g) Deep-sequencing at CAPNS1 locus

PCR for deep-sequencing were performed using the following primers: 5'-CCATCTCATCCCTGCGTGTCTCCGAC-(forward adaptor sequence)-10N-(sequences needed for PCR product identification)-CGAGTCAGGGCGGGATTAAG-3'-(locus specific forward sequence) (SEQ ID NO: 199) and the reverse primer 5'-CCTATCCCCTGTGTGCCTTGGCAGTCTCAG-(reverse adaptor sequence)-CGAGACTTCACGGTTTCGCC-3' (endogenous locus specific reverse sequence) (SEQ ID NO: 200). PCR products were sequenced by a 454 sequencing system (454 Life Sciences). Approximately 10,000 sequences were obtained per PCR product and then analyzed for the presence of site-specific insertion or deletion events.

### Results

### 3- In immortalized 293H cell line

Wild-type 293H cells were transfected by SC_CAPNS1 or Trex-SC_CAPNS1 in order to determine if those constructs could stimulate engineered meganuclease-induced targeted mutagenesis at an endogenous locus. Transfection with SC_CAPNS1 led to 1.6% of targeted mutagenesis (TM) whereas transfection with the fusion Trex-SC_CAPNS1 stimulated TM up to 12.4% (Figure 10, Panel A). Moreover, the analysis of the mutagenic sequences showed that the proportion of small deletions events of 2, 3 and 4 base pairs was increased from 2% of the TM events with SC_CAPNS1 to 67% with the fusion Trex-SC_CAPNS1 (Figure 10, Panel B).

### 4- In primary Detroit cell line

Wild type Detroit551 cells were transfected by SC_CAPNS1 or Trex-SC_CAPNS1 in order to determine if those constructs could also stimulate engineered meganuclease-induced targeted mutagenesis at an endogenous locus in primary cells. Transfection with SC_CAPNS1 led to 1.1% of TM whereas transfection with the fusion Trex-CAPNS1 stimulated TM up to 12.5% (Figure 11, Panel A). Moreover, the analysis of the mutagenic sequences showed that the proportion of small deletions events of 2, 3 and 4 base pairs was increased from 35% of the TM events with SC_CAPNS1 to 90% with the fusion Trex-SC_CAPNS1 (Figure 11, Panel B).

### Example 6: Effect of Terminal deoxynucleotidyl transferase (Tdt) expression on meganuclease-induced mutagenesis (Comparative)

Homing endonucleases from the LAGLIDADG family or meganucleases recognize long DNA sequences and cleave the two DNA strands, creating a four nucleotides 3' overhang. The cell can repair the double strand break (DSB) mainly through two mechanisms: by homologous recombination using an intact homologous template or by non homologous end joining (NHEJ). NHEJ is considered as an error prone mechanism that can induce mutations (insertion or deletion of DNA fragments) after DSB repair. Hence, after the transfection of a meganuclease into the cell, the measurement of the mutagenesis frequency at the meganuclease locus is a way to assess the meganuclease activity. Meganucleases derived from the I-CreI protein have been shown to induce mutagenesis at the genomic site, for which they have been designed (Munoz *et al.,* 2011).

The human Tdt protein (SEQ ID NO: 201) is a 508 amino acids protein that catalyzes the addition of deoxynucleotides to the 3'-hydroxyl terminus of DNA ends. The encoded protein is expressed in a restricted population of normal and malignant pre-B and pre-T lymphocytes during early differentiation. It generates antigen receptor diversity by synthesizing non-germ line elements at DSB site after RAG1 and RAG2 endonucleases cleavage. After a meganuclease DSB induced event, such an activity could add DNA sequences at the targeted site and would thus stimulate targeted mutagenesis induced by meganuclease.

### Example 6A: Co-transfection of Tdt (SEQ ID NO: 201) with meganucleases

To test this hypothesis, vector encoding meganuclease SC_GS (pCLS2690, SEQ ID NO: 153) was co-transfected on a cell line monitoring mutagenic NHEJ events in presence or absence of a vector encoding Tdt (pCLS3841 of SEQID NO: 202 encoding the protein of SEQ ID NO: 201). The SC_GS meganuclease (SEQ ID NO: 193) is a single chain protein where two I-CreI variants have been fused. It recognizes a 22bp DNA sequence (5'-TGCCCCAGGGTGAGAAAGTCCA-3': GS_CHO.1 target, SEQ ID NO: 155) located in the first exon of *Cricetulus griseus* glutamine synthetase gene. Moreover, two different meganucleases SC_RAG1 (pCLS2222, SEQID NO: 156 encoding SC_RAG1 of SEQ ID NO: 58), and SC_CAPNS1 (pCLS6163, SEQID NO: 158 encoding SC_CAPNS1 of SEQ ID NO: 192) were co-transfected with or without Tdt expression plasmid (pCLS3841, SEQID NO: 202) and the effects on meganuclease-induced mutagenesis at the endogenous loci were analyzed by deep-sequencing.

### Material and Methods

### d) Cellular model to monitor meganuclease-induced mutagenesis

The plasmid pCLS6810 (SEQID NO: 159) was designed to quantify NHEJ repair frequency induced by the SC_GS meganuclease (SEQ ID NO: 193). The sequence used to measure SC_GS-induced mutagenesis is made of an ATG start codon followed by i) 2 codons for alanine, ii) the tag HA sequence, iii) the SC_GS recognition site, iv) a glycine serine stretch, v) the same 2 codons for alanine as in i) and finally vi) a GFP reporter gene lacking its ATG start codon. Since by itself GFP reporter gene is inactive due to a frame-shift introduced by GS recognition sites, creation of a DNA double strand break (DSB) by SC_GS meganuclease followed by a mutagenic DSB repair event by NHEJ can lead to restoration of GFP gene expression in frame with the ATG start codon. These sequences were placed in a plasmid used to target the final construct at the RAG1 locus in 293H cell line using the hsRAG1 Integration Matrix CMV Neo from cGPS® Custom Human Full Kit DD (Cellectis Bioresearch). Using this kit, a stable cell line containing a single copy of the transgene at the RAG1 locus was obtained. Thus, after transfection of this cell line by the SC_GS meganuclease and with or without a plasmid encoding Tdt (pCLS3841, SEQ ID NO: 202), the percentage of GFP positive cells is directly correlated to the mutagenesis frequency induced by the transfected specie.

### e) Transfection on cellular model monitoring meganuclease-induced mutagenesis

One million of cells were seeded one day prior to transfection. Cells were co-transfected either with 1µg of SC_GS encoding vector (pCLS2690 , SEQID NO: 153) and with 0, 4, 6 or 9 µg of plasmid encoding Tdt (pCLS3841 SEQ ID NO: 202) or with 3 µg of SC_GS encoding plasmid with 0 or 2 µg of Tdt encoding vector in 5 or 10 µg of total DNA, respectively, by complementation with a pUC vector (pCLS0002, SEQ ID NO: 191) using 25 µl of lipofectamine (Invitrogen) according to the manufacturer's instructions. Three days following transfection, cells were harvested for flow cytometry analysis using Guava instrumentation. Conditions corresponding to 3 µg of SC_GS encoding vector with 0 or 2 µg of Tdt encoding plasmid were harvested for genomic DNA extraction. PCR for deep-sequencing were performed using the following primers: 5'-CCATCTCATCCCTGCGTGTCTCCGACTCAG (forward adaptor sequence)-10N-(sequences needed for PCR product identification)-GCTCTCTGGCTAACTAGAGAACCC (transgenic locus specific forward sequence) -3' (SEQ ID NO: 160) and 5'-CCTATCCCCTGTGTGCCTTGGCAGTCTCAG-(reverse adaptor sequence)-TCGATCAGCACGGGCACGATGCC (transgenic locus specific reverse sequence)- 3' (SEQ ID NO: 161). PCR products were sequenced by a 454 sequencing system (454 Life Sciences). Approximately 10,000 sequences were obtained per PCR product and then analyzed for the presence of site-specific insertion or deletion events.

### f) Transfection on 293H cells to monitor meganuclease-induced mutagenesis at endogenous loci

One million of cells were seeded one day prior to transfection. Cells were co-transfected with 3µg of SC_RAG1 encoding vector (pCLS2222, SEQ ID NO: 156) with 0.5, 1 and 2 µg or with 1, 3 and 7 µg of plasmid encoding Tdt (pCLS3841, SEQ ID NO: 202) in, respectively, 5 or 10 µg of total DNA by complementation with a pUC vector (pCLS0002, SEQID NO: 191) using 25 µl of lipofectamine (Invitrogen) according to the manufacturer's instructions. Three µg of SC_CAPNS1 encoding vector (pCLS6163 SEQ ID NO: 158) were co-transfected with 2 µg of empty vector plasmid (pCLS0002, SEQ ID NO: 191) or Tdt encoding plasmid (pCSL3841, SEQ ID NO: 202) using 25 µl of lipofectamine (Invitrogen) according to the manufacturer's instructions. Seven days following transfection, cells were harvested for genomic DNA extraction. PCR for deep-sequencing were performed using the following primers: 5'-CCATCTCATCCCTGCGTGTCTCCGACTCAG-(forward adaptor sequence)-10N-(sequences needed for PCR product identification)(SEQ ID NO: 5) - locus specific forward sequence for RAG1: GGCAAAGATGAATCAAAGATTCTGTCC-3' (SEQ ID NO: 162) and for CAPNS1: CGAGTCAGGGCGGGATTAAG-3'(SEQ ID NO: 164) and the reverse primer 5'-CCTATCCCCTGTGTGCCTTGGCAGTCTCAG-(reverse adaptor sequence)(SEQ ID NO: 6)-(endogenous locus specific reverse sequence for RAG1:-GATCTCACCCGGAACAGCTTAAATTTC-3' (SEQ ID NO: 165) and for CAPNS 1: - CGAGACTTCACGGTTTCGCC-3' (SEQ ID NO: 167). PCR products were sequenced by a 454 sequencing system (454 Life Sciences). Approximately 10,000 sequences were obtained per PCR product and then analyzed for the presence of site-specific insertion or deletion events.

### Results

### 1- On cellular model measuring meganuclease-induced mutagenic NHEJ repair

A cell line measuring mutagenic NHEJ repair induced by SC_GS was created. The percentage of GFP+ cells, monitoring the mutagenic NHEJ repair, was analyzed 96h after a transfection with SC_GS (pCLS2690, SEQ ID NO: 153) alone or with an increasing dose of Tdt encoding vector (pCLS3841, SEQ ID NO: 202). Without the presence of Tdt, SC_GS transfection led to 0.2 +/-0.1 % of GFP+ cells whereas all doses of Tdt encoding plasmid led to 1.0 +/- 0.4 % of GFP+ cells (Figure 12, panel A). Transfection with 3µg of SC_GS encoding plasmid with pUC vector led to 0.6 +/- 0.1 % of GFP+ cells while in presence of 2 µg of Tdt encoding plasmid the percentage of GFP+ cells was stimulated to 1.9 +/-0.3 % of GFP+ cells. Conditions corresponding to 3 µg of SC_GS with 2 µg of empty or Tdt encoding vector were analyzed by deep-sequencing. Transfection with SC_GS and an empty vector led to 3.2% of Targeted Mutagenesis (TM) while in presence of Tdt expressing plasmid, TM was stimulated up to 26.0% (Figure 12, panel B). In absence of Tdt the insertion events represented 29% of total TM events while in presence of Tdt these insertion events were increased up to 95.3 % (Figure 12, panel C). Finally, the analysis of insertion sizes in presence of Tdt encoding plasmid led to a specific hallmark of insertion with small insertions ranging from 2 to 8 bp (Figure 12, panel D).

### 2- At endogenous RAG1 locus

Wild type 293H cells were transfected by SC_RAG1 encoding vector (pCLS2222, SEQ ID NO: 156) with different doses of Tdt encoding plasmid (pCLS3841, SEQ ID NO: 202) in order to determine if Tdt could stimulate engineered meganuclease-induced targeted mutagenesis at an endogenous locus. Two different transfections were performed with 3 µg of SC_RAG1 encoding vector (pCLS2222, SEQ ID NO: 156) with either 0.5, 1 and 2 µg or 1, 3 and 7 µg of plasmid expressing Tdt (pCLS3841, SEQID NO: 202) in 5 or 10 µg of total DNA by complementation with an empty vector (pCLS0002, SEQID NO: 191) respectively. In absence of Tdt expressing vector, the targeted mutagenesis (TM) varies between 0.5 and 0.8%. When Tdt was present TM was stimulated up to 1.6% (Figure 13, panel A). The nature of mutagenic DSB repair was analyzed and showed a modification of the pattern of the TM events induced by the meganuclease. As showed in Figure 13, panel B, the percentage of insertion was almost null in absence of Tdt whereas in presence of Tdt expressing vector this percentage represents 50 up to 70% of the TM events. The sizes of insertions were also analyzed and in presence of Tdt a specific pattern of insertions appeared corresponding to small insertions ranging from 2 to 8 bp (Figure 13, panel C). Finally the sequences of these insertions seem to show that they are apparently random (Table 8).

**Table 8: Example of sequences with insertion at RAG1 endogenous locus in presence of Tdt.**

| Sequences with insertion | Insertion size | Insertion position |
|---|---|---|
| **attgttctcag**gc**gtacctcagccagc** | 2 | 5' |
| **attgttctcaggtac**at**ctcagccagc** | 2 | 3' |
| **attgttctcaggtac**cc**ctcagccagc** | 2 | 3' |
| **attgttctcaggtac**ggg**ctcagccagc** | 3 | 3' |
| **attgttctcag**ggc**gtacctcagccagc** | 3 | 5' |
| **attgttctcaggtac**agt**ctcagccagc** | 3 | 3' |
| **attgttctcaggtac**gggg**ctcagccag** | 4 | 3' |
| **attgttctcag**accc**gtacctcagccagc** | 4 | 5' |
| **attgttctcag**cctc**gtacctcagccagc** | 4 | 5' |
| **attgttctcag**cttc**gtacctcagccagc** | 4 | 5' |
| **attgttctcaggtac**tgga**ctcagccagc** | 4 | 3' |
| **attgttctcaggtac**aggg**ctcagccagc** | 4 | 3' |
| **attgttctcaggtac**gggaa**ctcagccagc** | 5 | 3' |
| **attgttctcaggtac**gaagg**ctcagccagc** | 5 | 3' |
| **attgttctcag**ttcct**gtacctcagccagc** | 5 | 5' |
| **attgttctcaggtac**gggtgg**ctcagccagc** | 6 | 3' |
| **attgttctcaggtac**tggtta**ctcagccagc** | 6 | 3' |
| **attgttctcaggtac**ccatac**ctcagccagc** | 6 | 3' |
| **attgttctcag**gttacct**gtacctcagccagc** | 7 | 5' |
| **attgttctcaggtac**aaggggg**ctcagccagc** | 7 | 3' |
| **attgttctcag**ggccgccc**gtacctcagccagc** | 8 | 5' |

### 3- At endogenous CAPNS1 locus

Wild type 293H cells were transfected with 3µg of plasmid encoding SC_CAPNS1 meganuclease (pCLS6163, SEQ ID NO: 158) with 0 or 2 µg of Tdt encoding plasmid (pCLS3841, SEQ ID NO: 202) (in 5µg of total DNA) in order to determine Tdt expression effect at another endogenous locus. In absence of Tdt expressing vector, the targeted mutagenesis (TM) was 7.4%. When Tdt was present TM was stimulated up to 13.9% (Figure 14, panel A). The nature of mutagenic DSB repair was analyzed and showed a modification of the pattern of the TM events induced by the meganuclease. As showed in Figure 14, panel B, insertion events represented 10% of total TM events in absence of Tdt whereas in presence of Tdt expressing vector insertion events represented 65% of the TM events. The sizes of insertions were also analyzed and in presence of Tdt a specific pattern of insertions appeared corresponding to small insertions ranging from 2 to 6 bp. Finally, the sequence analysis of these insertions seems to show that they are apparently random (Table 9).

**Table 9: Example of sequences with insertion at CAPNS 1 endogenous locus in presence of Tdt.**

| Sequences with insertion | Insertion size | Insertion position |
|---|---|---|
| **cagggccgcggtgc**gc**agtgtccgac** | 2 | 3' |
| **cagggccgcg**cc**gtgcagtgtccgac** | 2 | 5' |
| **cagggccgcg**gc**gtgcagtgtccgac** | 2 | 5' |
| **cagggccgcggtgc**ac**agtgtccgac** | 2 | 3' |
| **cagggccgcg**gcc**gtgcagtgtccgac** | 3 | 5' |
| **cagggccgcggtgc**tgc**agtgtccgac** | 3 | 3' |
| **cagggccgcg**cct**gtgcagtgtccgac** | 3 | 5' |
| **cagggccgcg**ttct**gtgcagtgtccgac** | 4 | 5' |
| **cagggccgcggtgc**gggc**agtgtccgac** | 4 | 3' |
| **cagggccgcg**gtcc**gtgcagtgtccgac** | 4 | 5' |
| **cagggccgcggtgc**aggc**agtgtccgac** | 4 | 3' |
| **cagggccgcggtgc**aaagc**agtgtccgac** | 5 | 3' |
| **cagggccgcg**gtgca**gtgcagtgtccgac** | 5 | 5' |
| **cagggccgc**ggtgcg**gtgcagtgtccgac** | 6 | 5' |
| **cagggccgcg**tgtct**gtgcagtgtccgac** | 5 | 5' |
| **cagggccgcggtgc**aaggtc**agtgtccgac** | 6 | 3' |
| **cagggccgcg**gtgccc**gtgcagtgtccgac** | 6 | 5' |
| **cagggccgcg**gtgcaa**gtgcagtgtccgac** | 6 | 5' |
| **cagggccgcggtgc**aagcaggg**agtgtccgac** | 8 | 3' |

### Example 6B: Fusion of the human Tdt to meganucleases: effect on targeted mutagenesis

Co-transfection of Tdt (SEQ ID NO: 201) with meganuclease encoding plasmids was shown to increase the rate of mutagenesis induced by meganucleases. However, this strategy implies the presence of two plasmids within the cell at the same time. Moreover it would be of benefit to target the Tdt activity at the newly created DSB upon Meganuclease's cleavage. Thus, a chimeric protein comprising TdT and Meganuclease proteins is engineered. The human Tdt protein (SEQ ID NO: 201) is fused to the N- or C-terminus of different Single chain engineered meganucleases SC_MN such as SC_GS (SEQ ID NO: 193), SC_RAG (SEQ ID NO: 58) and SC_CAPNS1 (SEQ ID NO: 192). Two SC_MN fused to Tdt protein are made: either at the N terminal domain or C terminal domain of the considered meganuclease. Those constructed are tested for their ability to increase mutagenic activity at the locus of interest.

### Material and Methods

### h) Making of SC MN/Tdt fusion proteins

The Tdt protein is fused to the SC_MN meganuclease either to its C-terminus or to its N-terminus using a ten amino acids linker (GGGGS)₂ (SEQ ID NO: 170). This yields to two protein constructs named respectively SC_MN-Tdt or Tdt-SC_MN. All SC_MN were initially cloned into the AscI / XhoI restriction sites of the pCLS1853 (Figure 7, SEQ ID NO: 175), a derivative of the pcDNA3.1 (Invitrogen), which drives the expression of a gene of interest under the control of the CMV promoter. The two fusion proteins for each SC_MN/Tdt constructs are obtained by amplifying separately the two ORFs using specific primers. The following table 10 gives the oligonucleotidic sequences that are used to create the different SC_GS/Tdt constructs.

**Table 10: Oligonucleotides to create different SC_GS/Tdt constructs**

| **Construct** | **Amplified ORF** | **Forward primer** | **SEQ ID NO:** | **Reverse primer** | **SEQ ID NO:** |
|---|---|---|---|---|---|
| SC_MN-TDT | SC_MN | CMVfor | 176 | Link10GSRev | 181 |
| | TDT | LinkTDTFor | 203 | TDTRev | 204 |
| TDT-SC_MN | SC_MN | Link10GSFor | 184 | V5rev | 177 |
| | TDT | TDTFor | 205 | Link10TDTRev | 206 |

Then, after a gel purification of the two PCR fragments, a PCR assembly is realized using the CMVfor (SEQ ID NO: 176) and TDTRev (SEQ ID NO: 204) oligonucleotides for Cter fusion of Tdt to SC_MN or using TDTFor (SEQ ID NO: 205) and V5Rev (SEQ ID NO: 177) for Nter fusion of Tdt to SC_MN. The final PCR product is cloned in a pTOPO vector then digested by AscI and XhoI and ligated into the pCLS1853 vector (SEQ ID NO: 175) predigested with these same enzymes.

### Example 7: Impact of co-transfection with two nucleases targeting two sequences separated by 173 base pairs (bp) on mutagenesis frequency. (Comparative)

To investigate the impact on mutagenesis frequency induced by two nucleases targeting two nearby sites, co-transfection with two engineered nucleases targeting DNA sequences within the RAG1 gene was performed. Nucleases consist of an engineered meganuclease (Nl) (SC_RAG of SEQ ID NO: 216) encoded by pCLS2222 (SEQ ID NO: 156) cleaving the DNA sequence 5'-TTGTTCTCAGGTACCTCAGCCAGC-3' (T1) (SEQ ID NO: 207) and a TALEN (N2) [SEQ ID NO: 209-210 respectively encoded by pCLS8964 (SEQ ID NO: 211) and pCLS8965 (SEQ ID NO: 212)] targeting DNA sequence 5'-TATATTTAAGCACTTATATGTGTGTAACAGGTATAAGTAACCATAAACA-3' (T2) (SEQ ID NO: 208). These two recognition sites are separated by 173 bp.

### Material and methods

### Cells transfection

The human 293H cells (ATCC) were plated at a density of 1.2 x 10⁶ cells per 10 cm dish in complete medium [DMEM supplemented with 2 mM L-glutamine, penicillin (100 IU/ml), streptomycin (100 µg/ml), amphotericin B (Fongizone: 0.25 µg/ml, Invitrogen-Life Science) and 10% FBS]. The next day, cells were transfected with 10µg of total DNA containing both nucleases expressing plasmids (3µg of N1 and 0.25µg of each monomer of N2), with Lipofectamine 2000 transfection reagent (Invitrogen) according to the manufacturer's protocol. As control, each nuclease was expressed alone. For all conditions, samples were completed at 10µg of total DNA with an empty vector pCLS0003 (SEQ ID NO: 213).

Two days after, cells were collected and genomic extraction was performed. The mutagenesis frequency was determined by Deep sequencing. The T1 and T2 targets were amplified with specific primers flanked by specific adaptator needed for High Throughput Sequencing on the 454 sequencing system (454 Life Sciences)
At T1 and T2 loci, primers F_T2:
R_T1: were used. 5,000 to 10,000 sequences per sample were analyzed.

### Results

The rate of mutations induced by the nucleases N1 and N2 at the targets T1 and T2 was measured by deep sequencing. Results are presented in Table 11. 0.63% of PCR fragments carried a mutation in samples corresponding to cells transfected with the N1 nuclease. Similarly, 1.46% of PCR fragments carried a mutation in sample corresponding to cells transfected with the N2 nuclease. The rate of induced mutagenesis increased up to 1.33% on T1 target and up to 2.48% on T2 target when the cells were transfected with plasmids expressing both N1 and N2, showing that the presence of two nucleases targeting two nearby sequences stimulates up to about two folds the frequency of mutagenesis. Interestingly, within the samples transfected with only one nuclease plasmid, the majority of deletions observed are small deletions. In contrast, within the sample co-transfected with both nucleases expressing plasmids a large fraction of deletions are large deletions (>197 bp), corresponding to the intervening sequences between the two cleavage sites.

Thus, it was observed that co-transfection of two nucleases targeting two nearby sequences separated by 173 bp stimulates the mutagenesis frequency.

**Table 11: Mutagenesis rate induction by two nucleases targeting two nearby sequences**

| Nucleases | % of Mutagenesis at T1 target | % of Mutagenesis at T2 target |
|---|---|---|
| N1 | 0.63 | 0 |
| N2 | 0 | 1.46 |
| N1 + N2 | 1.33 | 2.48 |

### List of cited references

Arimondo, P. B., C. J. Thomas, et al. (2006). "Exploring the cellular activity of camptothecin-triple-helix-forming oligonucleotide conjugates." Mol Cell Biol 26(1): 324-33.
Arnould, S., P. Chames, et al. (2006). "Engineering of large numbers of highly specific homing endonucleases that induce recombination on novel DNA targets." J Mol Biol 355(3): 443-58.
Arnould, S., C. Perez, et al. (2007). "Engineered I-Crel derivatives cleaving sequences from the human XPC gene can induce highly efficient gene correction in mammalian cells." J Mol Biol 371(1): 49-65.
Ashworth, J., J. J. Havranek, et al. (2006). "Computational redesign of endonuclease DNA binding and cleavage specificity." Nature 441(7093): 656-9.
Beumer, K. J., J. K. Trautman, et al. (2008). "Efficient gene targeting in Drosophila by direct embryo injection with zinc-finger nucleases." Proc Natl Acad Sci U S A 105(50): 19821-6.
Boch, J., H. Scholze, et al. (2009). "Breaking the code of DNA binding specificity of TAL-type III effectors." Science 326(5959): 1509-12.
Bolduc, J. M., P. C. Spiegel, et al. (2003). "Structural and biochemical analyses of DNA and RNA binding by a bifunctional homing endonuclease and group I intron splicing factor." Genes Dev 17(23): 2875-88.
Britt, A. B. (1999). "Molecular genetics of DNA repair in higher plants." Trends Plant Sci 4(1): 20-25.
Burden and O. N. (1998). "Mechanism of action of eukaryotic topoisomerase II and drugs targeted to the enzyme." Biochim Biophys Acta. 1400(1-3): 139-154.
Capecchi, M. R. (1989). "The new mouse genetics: altering the genome by gene targeting." Trends Genet 5(3): 70-6.
Cathomen, T. and J. K. Joung (2008). "Zinc-finger nucleases: the next generation emerges." Mol Ther 16(7): 1200-7.
Chames, P., J. C. Epinat, et al. (2005). "In vivo selection of engineered homing endonucleases using double-strand break induced homologous recombination." Nucleic Acids Res 33(20): e178.
Chevalier, B., M. Turmel, et al. (2003). "Flexible DNA target site recognition by divergent homing endonuclease isoschizomers I-Crel and I-MsoI." J Mol Biol 329(2): 253-69.
Chevalier, B. S., T. Kortemme, et al. (2002). "Design, activity, and structure of a highly specific artificial endonuclease." Mol Cell 10(4): 895-905.
Chevalier, B. S., R. J. Monnat, Jr., et al. (2001). "The homing endonuclease I-Crel uses three metals, one of which is shared between the two active sites." Nat Struct Biol 8(4): 312-6.
Chevalier, B. S. and B. L. Stoddard (2001). "Homing endonucleases: structural and functional insight into the catalysts of intron/intein mobility." Nucleic Acids Res 29(18): 3757-74.
Choulika, A., A. Perrin, et al. (1995). "Induction of homologous recombination in mammalian chromosomes by using the I-Scel system of Saccharomyces cerevisiae." Mol Cell Biol 15(4): 1968-73.
Christian, M., T. Cermak, et al. (2010). "Targeting DNA double-strand breaks with TAL effector nucleases." Genetics 186(2): 757-61.
Cohen-Tannoudji, M., S. Robine, et al. (1998). "I-SceI-induced gene replacement at a natural locus in embryonic stem cells." Mol Cell Biol 18(3): 1444-8.
Critchlow, S. E. and S. P. Jackson (1998). "DNA end-joining: from yeast to man." Trends Biochem Sci 23(10): 394-8.
Donoho, G., M. Jasin, et al. (1998). "Analysis of gene targeting and intrachromosomal homologous recombination stimulated by genomic double-strand breaks in mouse embryonic stem cells." Mol Cell Biol 18(7): 4070-8.
Doyon, J. B., V. Pattanayak, et al. (2006). "Directed evolution and substrate specificity profile of homing endonuclease I-SceI." J Am Chem Soc 128(7): 2477-84.
Doyon, Y., J. M. McCammon, et al. (2008). "Heritable targeted gene disruption in zebrafish using designed zinc-finger nucleases." Nat Biotechnol 26(6): 702-8.
Dujon, B., L. Colleaux, et al. (1986). "Mitochondrial introns as mobile genetic elements: the role of intron-encoded proteins." Basic Life Sci 40: 5-27.
Eisenschmidt, K., T. Lanio, et al. (2005). "Developing a programmed restriction endonuclease for highly specific DNA cleavage." Nucleic Acids Res 33(22): 7039-47.
Endo, M., K. Osakabe, et al. (2006). "Molecular characterization of true and ectopic gene targeting events at the acetolactate synthase gene in Arabidopsis." Plant Cell Physiol 47(3): 372-9.
Endo, M., K. Osakabe, et al. (2007). "Molecular breeding of a novel herbicide-tolerant rice by gene targeting." Plant J 52(1): 157-66.
Epinat, J. C., S. Arnould, et al. (2003). "A novel engineered meganuclease induces homologous recombination in yeast and mammalian cells." Nucleic Acids Res 31(11): 2952-62.
Feldmann, E., V. Schmiemann, et al. (2000). "DNA double-strand break repair in cell-free extracts from Ku80-deficient cells: implications for Ku serving as an alignment factor in non-homologous DNA end joining." Nucleic Acids Res 28(13): 2585-96.
Gimble, F. S., C. M. Moure, et al. (2003). "Assessing the plasticity of DNA target site recognition of the PI-SceI homing endonuclease using a bacterial two-hybrid selection system." J Mol Biol 334(5): 993-1008.
Gouble, A., J. Smith, et al. (2006). "Efficient in toto targeted recombination in mouse liver by meganuclease-induced double-strand break." J Gene Med 8(5): 616-22.
Grizot, S., J. Smith, et al. (2009). "Efficient targeting of a SCID gene by an engineered single-chain homing endonuclease." Nucleic Acids Res 37(16): 5405-19.
Haber, J. (2000). "Partners and pathways repairing a double-strand break." Trends Genet. 16(6): 259-264.
Haber, J. E. (1995). "In vivo biochemistry: physical monitoring of recombination induced by site-specific endonucleases." Bioessays 17(7): 609-20.
Hanin, M., S. Volrath, et al. (2001). "Gene targeting in Arabidopsis." Plant J 28(6): 671-7.
Ichiyanagi, K., Y. Ishino, et al. (2000). "Crystal structure of an archaeal intein-encoded homing endonuclease PI-PfuI." J Mol Biol 300(4): 889-901.
Kalish, J. M. and P. M. Glazer (2005). "Targeted genome modification via triple helix formation." Ann N Y Acad Sci 1058: 151-61.
Kim, Y. G., J. Cha, et al. (1996). "Hybrid restriction enzymes: zinc finger fusions to Fok I cleavage domain." Proc Natl Acad Sci U S A 93(3): 1156-60.
Kirik, A., S. Salomon, et al. (2000). "Species-specific double-strand break repair and genome evolution in plants." Embo J 19(20): 5562-6.
Li, H., H. Vogel, et al. (2007). "Deletion of Ku70, Ku80, or both causes early aging without substantially increased cancer." Mol Cell Biol 27(23): 8205-14.
Li, T., S. Huang, et al. (2010). "TAL nucleases (TALNs): hybrid proteins composed of TAL effectors and Fokl DNA-cleavage domain." Nucleic Acids Res 39(1): 359-72.
Lieber, M. R. and Z. E. Karanjawala (2004). "Ageing, repetitive genomes and DNA damage." Nat Rev Mol Cell Biol. 5(1): 69-75.
Lloyd, A., C. L. Plaisier, et al. (2005). "Targeted mutagenesis using zinc-finger nucleases in Arabidopsis." Proc Natl Acad Sci U S A 102(6): 2232-7.
Ma, J., E. Kim, et al. (2003). "Yeast Mre11 and Rad1 proteins define a Ku-independent mechanism to repair double-strand breaks lacking overlapping end sequences." Mol Cell Biol. 23(23): 8820-8828.
Meng, X., M. B. Noyes, et al. (2008). "Targeted gene inactivation in zebrafish using engineered zinc-finger nucleases." Nat Biotechnol 26(6): 695-701.
Moore, I., M. Samalova, et al. (2006). "Transactivated and chemically inducible gene expression in plants." Plant J 45(4): 651-83.
Moore, J. K. and J. E. Haber (1996). "Cell cycle and genetic requirements of two pathways of nonhomologous end-joining repair of double-strand breaks in Saccharomyces cerevisiae." Mol Cell Biol 16(5): 2164-73.
Moscou, M. J. and A. J. Bogdanove (2009). "A simple cipher governs DNA recognition by TAL effectors." Science 326(5959): 1501.
Moure, C. M., F. S. Gimble, et al. (2002). "Crystal structure of the intein homing endonuclease PI-Scel bound to its recognition sequence." Nat Struct Biol 9(10): 764-70.
Moure, C. M., F. S. Gimble, et al. (2003). "The crystal structure of the gene targeting homing endonuclease I-SceI reveals the origins of its target site specificity." J Mol Biol 334(4): 685-95.
Nagy, Z. and E. Soutoglou (2009). "DNA repair: easy to visualize, difficult to elucidate." Trends Cell Biol 19(11): 617-29.
Nouspikel, T. (2009). "DNA repair in mammalian cells : Nucleotide excision repair: variations on versatility." Cell Mol Life Sci 66(6): 994-1009.
Padidam, M. (2003). "Chemically regulated gene expression in plants." Curr Opin Plant Biol 6(2): 169-77.
Paques, F. and P. Duchateau (2007). "Meganucleases and DNA double-strand break-induced recombination: perspectives for gene therapy." Curr Gene Ther 7(1): 49-66.
Paques, F. and J. E. Haber (1999). "Multiple pathways of recombination induced by double-strand breaks in Saccharomyces cerevisiae." Microbiol Mol Biol Rev 63(2): 349-404.
Pingoud, A. and G. H. Silva (2007). "Precision genome surgery." Nat Biotechnol 25(7): 743-4.
Porteus, M. H. and D. Carroll (2005). "Gene targeting using zinc finger nucleases." Nat Biotechnol 23(8): 967-73.
Posfai, G., V. Kolisnychenko, et al. (1999). "Markerless gene replacement in Escherichia coli stimulated by a double-strand break in the chromosome." Nucleic Acids Res 27(22): 4409-15.
Povirk, L. F. (1996). "DNA damage and mutagenesis by radiomimetic DNA-cleaving agents: bleomycin, neocarzinostatin and other enediynes." Mutat Res 355(1-2): 71-89.
Puchta, H., B. Dujon, et al. (1996). "Two different but related mechanisms are used in plants for the repair of genomic double-strand breaks by homologous recombination." Proc Natl Acad Sci U S A 93(10): 5055-60.
Rosen, L. E., H. A. Morrison, et al. (2006). "Homing endonuclease I-Crel derivatives with novel DNA target specificities." Nucleic Acids Res.
Rothstein, R. (1991). "Targeting, disruption, replacement, and allele rescue: integrative DNA transformation in yeast." Methods Enzymol 194: 281-301.
Rouet, P., F. Smih, et al. (1994). "Expression of a site-specific endonuclease stimulates homologous recombination in mammalian cells." Proc Natl Acad Sci U S A 91(13): 6064-8.
Rouet, P., F. Smih, et al. (1994). "Introduction of double-strand breaks into the genome of mouse cells by expression of a rare-cutting endonuclease." Mol Cell Biol 14(12): 8096-106.
Sargent, R. G., M. A. Brenneman, et al. (1997). "Repair of site-specific double-strand breaks in a mammalian chromosome by homologous and illegitimate recombination." Mol Cell Biol 17(1): 267-77.
Seligman, L. M., K. M. Stephens, et al. (1997). "Genetic analysis of the Chlamydomonas reinhardtii I-Crel mobile intron homing system in Escherichia coli." Genetics 147(4): 1653-64.
Siebert, R. and H. Puchta (2002). "Efficient Repair of Genomic Double-Strand Breaks by Homologous Recombination between Directly Repeated Sequences in the Plant Genome." Plant Cell 14(5): 1121-31.
Silva, G. H., J. Z. Dalgaard, et al. (1999). "Crystal structure of the thermostable archaeal intron-encoded endonuclease I-Dmol." J Mol Biol 286(4): 1123-36.
Simon, P., F. Cannata, et al. (2008). "Sequence-specific DNA cleavage mediated by bipyridine polyamide conjugates." Nucleic Acids Res 36(11): 3531-8.
Smith, J., S. Grizot, et al. (2006). "A combinatorial approach to create artificial homing endonucleases cleaving chosen sequences." Nucleic Acids Res 34(22): e149.
Sonoda, E., H. Hochegger, et al. (2006). "Differential usage of non-homologous end-joining and homologous recombination in double strand break repair." DNA Repair (Amst) 5(9-10): 1021-9.
Spiegel, P. C., B. Chevalier, et al. (2006). "The structure of I-Ceul homing endonuclease: Evolving asymmetric DNA recognition from a symmetric protein scaffold." Structure 14(5): 869-80.
Stoddard, B. L. (2005). "Homing endonuclease structure and function." Q Rev Biophys 38(1): 49-95.
Sussman, D., M. Chadsey, et al. (2004). "Isolation and characterization of new homing endonuclease specificities at individual target site positions." J Mol Biol 342(1): 31-41.
Teicher, B. A. (2008). "Next generation topoisomerase I inhibitors: Rationale and biomarker strategies." Biochem Pharmacol 75(6): 1262-71.
Terada, R., Y. Johzuka-Hisatomi, et al. (2007). "Gene targeting by homologous recombination as a biotechnological tool for rice functional genomics." Plant Physiol 144(2): 846-56.
Terada, R., H. Urawa, et al. (2002). "Efficient gene targeting by homologous recombination in rice." Nat Biotechnol 20(10): 1030-4.
Wang, R., X. Zhou, et al. (2003). "Chemically regulated expression systems and their applications in transgenic plants." Transgenic Res 12(5): 529-40.
Zuo, J. and N. H. Chua (2000). "Chemical-inducible systems for regulated expression of plant genes." Curr Opin Biotechnol 11(2): 146-51.
Grizot, S., J. C. Epinat, et al. (2009). "Generation of redesigned homing endonucleases comprising DNA-binding domains derived from two different scaffolds." Nucleic Acids Res 38(6): 2006-18.
Mazur, D. J. and F. W. Perrino (2001). "Structure and expression of the TREX1 and TREX2 3' --> 5' exonuclease genes." J Biol Chem 276(18): 14718-27.
Perrino, F.W., de Silva U, Harvey S, Pryor E.E. Jr., Cole D.W. and Hollis T (2008). "Cooperative DNA binding and communication across the dimer interface in the TREX2 3' --> 5'-exonuclease." J Biol Chem 283 (31): 21441-52.

## Claims

1. A method for increasing double-strand break induced mutagenesis at a genomic locus of interest in a cell comprising the steps of:
(i) identifying at said genomic locus of interest at least one DNA target sequence cleavable by one rare-cutting endonuclease;
(ii) engineering said at least one rare-cutting endonuclease to provide a chimeric rare-cutting endonuclease able to generate one double-strand break in the genomic locus of interest and a loss of genetic information within the genomic locus of interest by another enzymatic activity, said another enzymatic activity being an exonuclease activity; and
(iii) contacting said DNA target sequence with said at least one chimeric rare-cutting endonuclease to generate one DNA double-strand break in the genomic locus of interest, and a loss of genetic information around said DNA target sequence within the genomic locus of interest,
wherein said chimeric rare-cutting endonuclease is a rare-cutting endonuclease fused to an exonuclease domain,
thereby obtaining a cell in which double-strand break induced mutagenesis at said genomic locus of interest is increased.

2. The method according to claim 1, wherein said rare-cutting endonuclease is a meganuclease.

3. The method according to claim 2, wherein said rare-cutting endonuclease is derived from I-*Cre*I meganuclease.

4. The method according to claim 2, wherein said rare-cutting endonuclease is a single chain meganuclease derived from I-*Cre*I meganuclease.

5. The method according to claim 1, wherein said rare-cutting endonuclease is a ZFN.

6. A chimeric rare-cutting endonuclease for increasing double-strand break induced mutagenesis into a genomic locus of interest comprising:
i) a rare-cutting endonuclease;
ii) an exonuclease domain.

7. The chimeric rare-cutting endonuclease according to claim 6, wherein said rare-cutting endonuclease is a meganuclease.

8. The chimeric rare-cutting endonuclease according to claim 6, wherein said rare-cutting endonuclease is derived from I-*Cre*I meganuclease.

9. The chimeric rare-cutting endonuclease according to claim 6, wherein said rare-cutting endonuclease is a single-chain meganuclease derived from I-*Cre*I meganuclease.

10. The chimeric rare-cutting endonuclease according to claim 6, wherein said rare-cutting endonuclease is a ZFN.

11. A recombinant polynucleotide encoding a chimeric rare-cutting endonuclease according to claims 6 to 10.

12. A vector comprising a recombinant polynucleotide according to claims 6 to 10.

13. A composition comprising a chimeric rare-cutting endonuclease according to claims 6 to 10 and a carrier.

14. A kit comprising a chimeric rare-cutting endonuclease according to claims 6 to 10 and instructions for use in increasing double-strand break-induced mutagenesis in a eukaryotic cell and optionally packaging materials, containers for the ingredients, and other components used for increasing double-strand break-induced mutagenesis.

15. Use of a chimeric rare-cutting endonuclease according to any one of claims 6 to 10 for increasing double-strand break induced mutagenesis at a genomic locus of interest in a cell.

## Patentansprüche

1. Verfahren zur Erhöhung doppelstrangbruchinduzierter Mutagenese an einem interessierenden genetischen Lokus in einer Zelle, umfassend die Schritte:
(i) Identifizieren zumindest einer Ziel-DNA-Sequenz am interessierenden genetischen Lokus, die durch eine selten schneidende Endonuklease spaltbar ist;
(ii) gentechnisches Verändern der mindestens einen selten schneidenden Endonuklease, um eine chimäre selten schneidende Endonuklease bereitzustellen, die in der Lage ist, einen Doppelstrangbruch in dem interessierenden genetischen Lokus und durch eine weitere enzymatische Aktivität einen Verlust genetischer Information innerhalb des interessierenden genetischen Lokus zu bewirken, wobei die weitere enzymatische Aktivität eine Exonuklease-Aktivität ist; und
(iii) in Kontakt Bringen der Ziel-DNA-Sequenz mit der mindestens einen chimären selten schneidenden Endonuklease, um einen DNA-Doppelstrangbruch und einen Verlust genetischer Information um die Ziel-DNA-Sequenz innerhalb des interessierenden genetischen Lokus zu bewirken,
wobei die chimäre selten schneidende Endonuklease eine selten schneidende Endonuklease ist, die an eine Exonuklease-Domäne fusioniert ist,
wodurch eine Zelle erhalten wird, in welcher doppelstrangbruchinduzierte Mutagenese am interessierenden genetischen Lokus erhöht ist.

2. Verfahren nach Anspruch 1, wobei die selten schneidende Endonuklease eine Meganuklease ist.

3. Verfahren nach Anspruch 2, wobei die selten schneidende Endonuklease von der I-*Cre*I-Meganuklease abgeleitet ist.

4. Verfahren nach Anspruch 2, wobei die selten schneidende Endonuklease eine von der I-*Cre*I-Meganuklease abgeleitete Einzelketten-Meganuklease ist.

5. Verfahren nach Anspruch 1, wobei die selten schneidende Endonuklease eine ZFN ist.

6. Chimäre selten schneidende Endonuklease zur Erhöhung doppelstrangbruchinduzierter Mutagenese in einem interessierenden genetischen Lokus, umfassend:
i) eine selten schneidende Endonuklease;
ii) eine Exonuklease-Domäne.

7. Chimäre selten schneidende Endonuklease nach Anspruch 6, wobei die selten schneidende Endonuklease eine Meganuklease ist.

8. Chimäre selten schneidende Endonuklease nach Anspruch 6, wobei die selten schneidende Endonuklease von der I-*Cre*I-Meganuklease abgeleitet ist.

9. Chimäre selten schneidende Endonuklease nach Anspruch 6, wobei die selten schneidende Endonuklease eine von der I-*Cre*I-Meganuklease abgeleitete Einzelketten-Meganuklease ist.

10. Chimäre selten schneidende Endonuklease nach Anspruch 6, wobei die selten schneidende Endonuklease eine ZFN ist.

11. Rekombinantes Polynukleotid, das für eine chimäre selten schneidende Endonuklease nach den Ansprüchen 6 bis 10 codiert.

12. Vektor, umfassend ein rekombinantes Polynukleotid nach den Ansprüchen 6 bis 10.

13. Zusammensetzung, umfassend eine chimäre selten schneidende Endonuklease nach den Ansprüchen 6 bis 10 und einen Träger.

14. Kit, umfassend eine chimäre selten schneidende Endonuklease nach den Ansprüchen 6 bis 10 und eine Anleitung zur Verwendung bei der Erhöhung doppelstrangbruchinduzierter Mutagenese in einer eukaryotischen Zelle und optional Verpackungsmaterial, Behälter für die Bestandteile, und weitere zur Erhöhung doppelstrangbruchinduzierter Mutagenese verwendete Komponenten.

15. Verwendung einer chimären selten schneidenden Endonuklease nach einem der Ansprüche 6 bis 10 zur Erhöhung doppelstrangbruchinduzierter Mutagenese an einem interessierenden genetischen Lokus in einer Zelle.

## Revendications

1. Procédé pour augmenter la mutagenèse induite par une cassure double brin au niveau d'un locus génomique d'intérêt dans une cellule, comprenant les étapes consistant à :
(i) identifier, au niveau dudit locus génomique d'intérêt, au moins une séquence cible d'ADN pouvant être clivée par une endonucléase à coupure rare ;
(ii) manipuler ladite au moins une endonucléase à coupure rare afin d'obtenir une endonucléase à coupure rare chimérique capable de générer une cassure double brin dans le locus génomique d'intérêt et une perte d'information génétique au sein du locus génomique d'intérêt par une autre activité enzymatique, ladite autre activité enzymatique étant une activité exonucléase ; et
(iii) mettre en contact ladite séquence cible d'ADN avec ladite au moins une endonucléase à coupure rare chimérique afin de générer une cassure d'ADN double brin dans le locus génomique d'intérêt, et une perte d'information génétique autour de ladite séquence cible d'ADN au sein du locus génomique d'intérêt,
dans lequel ladite endonucléase à coupure rare chimérique est une endonucléase à coupure rare fusionnée à un domaine exonucléase,
en obtenant ainsi une cellule dans laquelle la mutagenèse induite par une cassure double brin au niveau dudit locus génomique d'intérêt est augmentée.

2. Procédé selon la revendication 1, dans lequel ladite endonucléase à coupure rare est une méganucléase.

3. Procédé selon la revendication 2, dans lequel ladite endonucléase à coupure rare est dérivée d'une méganucléase I-*Cre*I.

4. Procédé selon la revendication 2, dans lequel ladite endonucléase à coupure rare est une méganucléase à chaîne unique dérivée d'une méganucléase I-*Cre*I.

5. Procédé selon la revendication 1, dans lequel ladite endonucléase à coupure rare est une ZFN.

6. Endonucléase à coupure rare chimérique pour augmenter la mutagenèse induite par une cassure double brin dans un locus génomique d'intérêt comprenant :
i) une endonucléase à coupure rare ;
ii) un domaine exonucléase.

7. Endonucléase à coupure rare chimérique selon la revendication 6, où ladite endonucléase à coupure rare est une méganucléase.

8. Endonucléase à coupure rare chimérique selon la revendication 6, où ladite endonucléase à coupure rare est dérivée d'une méganucléase I-*Cre*I.

9. Endonucléase à coupure rare chimérique selon la revendication 6, où ladite endonucléase à coupure rare est une méganucléase à chaîne unique dérivée d'une méganucléase I-*Cre*I.

10. Endonucléase à coupure rare chimérique selon la revendication 6, où ladite endonucléase à coupure rare est une ZFN.

11. Polynucléotide recombinant codant pour une endonucléase à coupure rare chimérique selon les revendications 6 à 10.

12. Vecteur comprenant un polynucléotide recombinant selon les revendications 6 à 10.

13. Composition comprenant une endonucléase à coupure rare chimérique selon les revendications 6 à 10 et un véhicule.

14. Kit comprenant une endonucléase à coupure rare chimérique selon les revendications 6 à 10 et un mode d'emploi pour augmenter la mutagenèse induite par une cassure double brin dans une cellule eucaryote, et facultativement des matériaux de conditionnement, des récipients pour les ingrédients, et d'autres composants utilisés pour augmenter la mutagenèse induite par une cassure double brin.

15. Utilisation d'une endonucléase à coupure rare chimérique selon l'une quelconque des revendications 6 à 10 pour augmenter la mutagenèse induite par une cassure double brin au niveau d'un locus génomique d'intérêt dans une cellule.


### SEQUENCE LISTING

<110> DUCHATEAU Philippe
   JUILLERAT ALEXANDRE
   SILVA GEORGE H.
   EPINAT JEAN-CHARLES
<120> METHOD FOR INCREASING THE EFFICIENCY OF DOUBLE-STRAND BREAK-INDUCED MUTAGENESIS
<130>
<160> 216
<170> PatentIn version 3.5
<210> 1
   <211> 163
   <212> PRT
   <213> Chlamydomonas reinhardtii
<400> 1
   Met Asn Thr Lys Tyr Asn Lys Glu Phe Leu Leu Tyr Leu Ala Gly Phe
   1               5                   10                  15
   Val Asp Gly Asp Gly Ser Ile Ile Ala Gln Ile Lys Pro Asn Gln Ser
               20                  25                  30
   Tyr Lys Phe Lys His Gln Leu Ser Leu Thr Phe Gln Val Thr Gln Lys
           35                  40                  45
   Thr Gln Arg Arg Trp Phe Leu Asp Lys Leu Val Asp Glu Ile Gly Val
       50                  55                  60
   Gly Tyr Val Arg Asp Arg Gly Ser Val Ser Asp Tyr Ile Leu Ser Glu
   65                  70                  75                  80
   Ile Lys Pro Leu His Asn Phe Leu Thr Gln Leu Gln Pro Phe Leu Lys
                   85                  90                  95
   Leu Lys Gln Lys Gln Ala Asn Leu Val Leu Lys Ile Ile Glu Gln Leu
               100                 105                 110
   Pro Ser Ala Lys Glu Ser Pro Asp Lys Phe Leu Glu Val Cys Thr Trp
           115                 120                 125
   Val Asp Gln Ile Ala Ala Leu Asn Asp Ser Lys Thr Arg Lys Thr Thr
       130                 135                 140
   Ser Glu Thr Val Arg Ala Val Leu Asp Ser Leu Ser Glu Lys Lys Lys
   145                 150                 155                 160
   Ser Ser Pro
<210> 2
   <211> 22
   <212> DNA
   <213> Artificial
<220>
   <223> C1221 target
<400> 2
   caaaacgtcg tacgacgttt tg 22
<210> 3
   <211> 22
   <212> DNA
   <213> Artificial
<220>
   <223> R1 target
<400> 3
   Tgttctcagg tacctcagcc ag 22
<210> 4
   <211> 22
   <212> DNA
   <213> Artificial
<220>
   <223> D21 target
<400> 4
   aaacctcaag taccaaatgt aa 22
<210> 5
   <211> 30
   <212> DNA
   <213> Artificial
<220>
   <223> Adaptor A Deep sequencing primer
<400> 5
   ccatctcatccctgcgtgtctccgacnnnn 30
<210> 6
   <211> 30
   <212> DNA
   <213> Artificial
<220>
   <223> Adaptor B Deep sequencing primer
<400> 6
   cctatcccctgtgtgccttggcagtctcag 30
<210> 7
   <211> 3
   <212> **PRT**
   <213> Artificial
<220>
   <223> 1a8h_1 peptidic linker
<400> 7
   Asn Val Gly
   1
<210> 8
   <211> 4
   <212> PRT
   <213> Artificial
<220>
   <223> 1dnpA_1 peptidic linker
<400> 8
   Asp Ser Val Ile
   1
<210> 9
   <211> 4
   <212> PRT
   <213> Artificial
<220>
   <223> ld8cA_2 peptidic linker
<400> 9
   Ile Val Glu Ala
   1
<210> 10
   <211> 4
   <212> PRT
   <213> Artificial
<220>
   <223> 1ckqA_3 peptidic linker
<400> 10
   Leu Glu Gly Ser
   1
<210> 11
   <211> 4
   <212> PRT
   <213> Artificial
<220>
   <223> 1sbp_1 peptidic linker
<400> 11
   Tyr Thr Ser Thr
   1
<210> 12
   <211> 5
   <212> PRT
   <213> Artificial
<220>
   <223> 1ev7A_1 peptidic linker
<400> 12
   Leu Gln Glu Asn Leu
   1 5
<210> 13
   <211> 5
   <212> PRT
   <213> Artificial
<220>
   <223> 1alo_3 peptidic linker
<400> 13
   Val Gly Arg Gln Pro
   1 5
<210> 14
   <211> 5
   <212> PRT
   <213> Artificial
<220>
   <223> 1amf_1 peptidic linker
<400> 14
   Leu Gly Asn Ser Leu
   1 5
<210> 15
   <211> 6
   <212> PRT
   <213> Artificial
<220>
   <223> ladjA_3 peptidic linker
<400> 15
   Leu Pro Glu Glu Lys Gly
   1 5
<210> 16
   <211> 6
   <212> PRT
   <213> Artificial
<220>
   <223> 1fcdC_1 peptidic linker
<400> 16
   Gln Thr Tyr Gln Pro Ala
   1 5
<210> 17
   <211> 6
   <212> PRT
   <213> Artificial
<220>
   <223> 1a13_2 peptidic linker
<400> 17
   Phe Ser His Ser Thr Thr
   1 5
<210> 18
   <211> 7
   <212> PRT
   <213> Artificial
<220>
   <223> 1g3p_1 peptidic linker
<400> 18
   Gly Tyr Thr Tyr Ile Asn Pro
   1 5
<210> 19
   <211> 7
   <212> PRT
   <213> Artificial
<220>
   <223> 1acc_3 peptidic linker
<400> 19
   Leu Thr Lys Tyr Lys Ser Ser
   1 5
<210> 20
   <211> 8
   <212> PRT
   <213> Artificial
<220>
   <223> 1ahjB_1 peptidic linker
<400> 20
   Ser Arg Pro Ser Glu Ser Glu Gly
   1 5
<210> 21
   <211> 8
   <212> PRT
   <213> Artificial
<220>
   <223> 1acc_1 peptidic linker
<400> 21
   Pro Glu Leu Lys Gln Lys Ser Ser
   1 5
<210> 22
   <211> 8
   <212> PRT
   <213> Artificial
<220>
   <223> 1af7_1 peptidic linker
<400> 22
   Leu Thr Thr Asn Leu Thr Ala Phe
   1 5
<210> 23
   <211> 9
   <212> PRT
   <213> Artificial
<220>
   <223> 1heiA_1 peptidic linker
<400> 23
   Thr Ala Thr Pro Pro Gly Ser Val Thr
   1 5
<210> 24
   <211> 9
   <212> PRT
   <213> Artificial
<220>
   <223> 1bia_2 peptidic linker
<400> 24
   Leu Asp Asn Phe Ile Asn Arg Pro Val
   1 5
<210> 25
   <211> 9
   <212> PRT
   <213> Artificial
<220>
   <223> 1igtB_1 peptidic linker
<400> 25
   Val Ser Ser Ala Lys Thr Thr Ala Pro
   1 5
<210> 26
   <211> 10
   <212> PRT
   <213> Artificial
<220>
   <223> 1nfkA_1 peptidic linker
<400> 26
   Asp Ser Lys Ala Pro Asn Ala Ser Asn Leu
   1 5 10
<210> 27
   <211> 10
   <212> PRT
   <213> Artificial
<220>
   <223> 1au7A_1 peptidic linker
<400> 27
   Lys Arg Arg Thr Thr Ile Ser Ile Ala Ala
   1 5
<210> 28
   <211> 11
   <212> PRT
   <213> Artificial
<220>
   <223> 1bpoB_1 peptidic linker
<400> 28
<210> 29
   <211> 11
   <212> PRT
   <213> Artificial
<220>
   <223> 1b0pA_2 peptidic linker
<400> 29
   Ala Pro Ala Glu Thr Lys Ala Glu Pro Met Thr
   1 5 10
<210> 30
   <211> 14
   <212> PRT
   <213> Artificial
<220>
   <223> 1c05A_2 peptidic linker
<400> 30
   Tyr Thr Arg Leu Pro Glu Arg Ser Glu Leu Pro Ala Glu Ile
   1 5 10
<210> 31
   <211> 14
   <212> PRT
   <213> Artificial
<220>
   <223> 1gcb_1 peptidic linker
<400> 31
   Val Ser Thr Asp Ser Thr Pro Val Thr Asn Gln Lys Ser Ser
   1               5                   10
<210> 32
   <211> 14
   <212> PRT
   <213> Artificial
<220>
   <223> 1bt3A_1 peptidic linker
<400> 32
   Tyr Lys Leu Pro Ala Val Thr Thr Met Lys Val Arg Pro Ala
   1               5                   10
<210> 33
   <211> 15
   <212> PRT
   <213> Artificial
<220>
   <223> 1b3oB_2 peptidic linker
<400> 33
   Ile Ala Arg Thr Asp Leu Lys Lys Asn Arg Asp Tyr Pro Leu Ala
   1               5                   10                  15
<210> 34
   <211> 21
   <212> PRT
   <213> Artificial
<220>
   <223> 16vpA_6 peptidic linker
<400> 34
   Thr Glu Glu Pro Gly Ala Pro Leu Thr Thr Pro Pro Thr Leu His Gly
   1               5                   10                  15
   Asn Gln Ala Arg Ala
               20
<210> 35
   <211> 21
   <212> PRT
   <213> Artificial
<220>
   <223> 1dhx_1 peptidic linker
<400> 35
   Ala Arg Phe Thr Leu Ala Val Gly Asp Asn Arg Val Leu Asp Met Ala
   1               5                   10                  15
   Ser Thr Tyr Phe Asp
               20
<210> 36
   <211> 26
   <212> PRT
   <213> Artificial
<220>
   <223> 1b8aA_1 peptidic linker
<400> 36
<210> 37
   <211> 28
   <212> PRT
   <213> Artificial
<220>
   <223> 1qu6A_1 peptidic linker
<400> 37
<210> 38
   <211> 158
   <212> PRT
   <213> Methylophilus methylotrophus
<220>
   <223> GenBank ACC85607.1 residues 2 to 159
<400> 38
   Ala Leu Ser Trp Asn Glu Ile Arg Arg Lys Ala Ile Glu Phe Ser Lys
   1               5                   10                  15
   Arg Trp Glu Asp Ala Ser Asp Glu Asn Ser Gln Ala Lys Pro Phe Leu
               20                  25                  30
   Ile Asp Phe Phe Glu Val Phe Gly Ile Thr Asn Lys Arg Val Ala Thr
           35                  40                  45
   Phe Glu His Ala Val Lys Lys Phe Ala Lys Ala His Lys Glu Gln Ser
       50                  55                  60
   Arg Gly Phe Val Asp Leu Phe Trp Pro Gly Ile Leu Leu Ile Glu Met
   65                  70                  75                  80
   Lys Ser Arg Gly Lys Asp Leu Asp Lys Ala Tyr Asp Gln Ala Leu Asp
                   85                  90                  95
   Tyr Phe Ser Gly Ile Ala Glu Arg Asp Leu Pro Arg Tyr Val Leu Val
               100                 105                 110
   Cys Asp Phe Gln Arg Phe Arg Leu Thr Asp Leu Ile Thr Lys Glu Ser
           115                 120                 125
   Val Glu Phe Leu Leu Lys Asp Leu Tyr Gln Asn Val Arg Ser Phe Gly
       130                 135                 140
   Phe Ile Ala Gly Tyr Gln Thr Gln Val Ile Lys Pro Gln Asp
   145                 150                 155
<210> 39
   <211> 155
   <212> PRT
   <213> EsaSSI
<220>
   <223> GenBank EAJ03172.1 residues 2 to 156
<400> 39
   Ala Ala Leu Ser Phe Pro Glu Ile Arg Thr Arg Leu Gln Ala Phe Ala
   1               5                   10                  15
   Lys Gln Trp Lys Gln Ala Glu Arg Glu Asn Ala Asp Ala Lys Leu Phe
               20                  25                  30
   Trp Ala Arg Phe Tyr Glu Cys Phe Gly Ile Arg Pro Glu Ser Ala Thr
           35                  40                  45
   Ile Tyr Glu Lys Ala Val Asp Lys Leu Asp Gly Ser Arg Gly Phe Ile
       50                  55                  60
   Asp Ser Phe Ile Pro Gly Leu Leu Ile Val Glu His Lys Ser Lys Gly
   65                  70                  75                  80
   Lys Asp Leu Asn Ser Ala Phe Thr Gln Ala Ser Asp Tyr Phe Thr Ala
                   85                  90                  95
   Leu Ala Glu Gly Glu Arg Pro Arg Tyr Ile Ile Val Ser Asp Phe Ala
               100                 105                 110
   Arg Phe Arg Leu Tyr Asp Leu Lys Thr Asp Thr Gln Val Glu Cys Lys
           115                 120                 125
   Leu Ala Asp Ile Ser Lys His Ala Gly Trp Phe Arg Phe Leu Val Glu
       130                 135                 140
   Gly Glu Ala Thr Pro Glu Ile Val Glu Glu Ser
   145                 150                 155
<210> 40
   <211> 179
   <212> PRT
   <213> Corynebacterium striatum
<220>
   <223> NCBI Reference Sequence NP_862240 residues 2 to 180
<400> 40
   Val Met Ala Pro Thr Thr Val Phe Asp Arg Ala Thr Ile Arg His Asn
   1               5                   10                  15
   Leu Thr Glu Phe Lys Leu Arg Trp Leu Asp Arg Ile Lys Gln Trp Glu
   20 25 30
   Ala Glu Asn Arg Pro Ala Thr Glu Ser Ser His Asp Gln Gln Phe Trp
   35 40 45
   Gly Asp Leu Leu Asp Cys Phe Gly Val Asn Ala Arg Asp Leu Tyr Leu
   50 55 60
   Tyr Gln Arg Ser Ala Lys Arg Ala Ser Thr Gly Arg Thr Gly Lys Ile
   65 70 75 80
   Asp Met Phe Met Pro Gly Lys Val Ile Gly Glu Ala Lys Ser Leu Gly
   85 90 95
   Val Pro Leu Asp Asp Ala Tyr Ala Gln Ala Leu Asp Tyr Leu Leu Gly
   100 105 110
   Gly Thr Ile Ala Asn Ser His Met Pro Ala Tyr Val Val Cys Ser Asn
   115 120 125
   Phe Glu Thr Leu Arg Val Thr Arg Leu Asn Arg Thr Tyr Val Gly Asp
   130 135 140
   Ser Ala Asp Trp Asp Ile Thr Phe Pro Leu Ala Glu Ile Asp Glu His
   145 150 155 160
   Ile Glu Gln Leu Ala Phe Leu Ala Asp Tyr Glu Thr Ser Ala Tyr Arg
   165 170 175
   Glu Glu Glu
<210> 41
   <211> 250
   <212> PRT
   <213> Nostoc sp. PCC 7120 (Anabaena sp. PCC 7120)
<220>
   <223> GenBank CAA45962.1 residues 25 to 274
<400> 41
   Gln Val Pro Pro Leu Thr Glu Leu Ser Pro Ser Ile Ser Val His Leu
   1 5 10 15
   Leu Leu Gly Asn Pro Ser Gly Ala Thr Pro Thr Lys Leu Thr Pro Asp
   20 25 30
   Asn Tyr Leu Met Val Lys Asn Gln Tyr Ala Leu Ser Tyr Asn Asn Ser
           35                  40                  45
   Lys Gly Thr Ala Asn Trp Val Ala Trp Gln Leu Asn Ser Ser Trp Leu
       50                  55                  60
   Gly Asn Ala Glu Arg Gln Asp Asn Phe Arg Pro Asp Lys Thr Leu Pro
   65                  70                  75                  80
   Ala Gly Trp Val Arg Val Thr Pro Ser Met Tyr Ser Gly Ser Gly Tyr
                   85                  90                  95
   Asp Arg Gly His Ile Ala Pro Ser Ala Asp Arg Thr Lys Thr Thr Glu
               100                 105                 110
   Asp Asn Ala Ala Thr Phe Leu Met Thr Asn Met Met Pro Gln Thr Pro
           115                 120                 125
   Asp Asn Asn Arg Asn Thr Trp Gly Asn Leu Glu Asp Tyr Cys Arg Glu
       130                 135                 140
   Leu Val Ser Gln Gly Lys Glu Leu Tyr Ile Val Ala Gly Pro Asn Gly
   145                 150                 155                 160
   Ser Leu Gly Lys Pro Leu Lys Gly Lys Val Thr Val Pro Lys Ser Thr
                   165                 170                 175
   Trp Lys Ile Val Val Val Leu Asp Ser Pro Gly Ser Gly Leu Glu Gly
               180                 185                 190
   Ile Thr Ala Asn Thr Arg Val Ile Ala Val Asn Ile Pro Asn Asp Pro
           195                 200                 205
   Glu Leu Asn Asn Asp Trp Arg Ala Tyr Lys Val Ser Val Asp Glu Leu
       210                 215                 220
   Glu Ser Leu Thr Gly Tyr Asp Phe Leu Ser Asn Val Ser Pro Asn Ile
   225                 230                 235                 240
   Gln Thr Ser Ile Glu Ser Lys Val Asp Asn
                   245                 250
<210> 42
   <211> 250
   <212> PRT
   <213> Escherichia coli (strain K12)
<220>
   <223> UniProtKB/Swiss Prot P25736, Endonucleasel, residues 23 to 235
<400> 42
<210> 43
   <211> 247
   <212> PRT
   <213> Dickeya dadantii (strain 3937) (Erwinia chrysanthemi (strain 3937))
   <220>
   <223> UniProtKB/Swiss Prot P37994, nuclease NucM, residues 20 to 266
<400> 43
   Ala Ala Gly Gln Asp Ile Asn Asn Phe Thr Gln Ala Lys Ala Ala Ala
   1               5                   10                  15
   Ala Lys Ile His Gln Asp Ala Pro Gly Thr Phe Tyr Cys Gly Cys Lys
               20                  25                  30
   Ile Asn Trp Gln Gly Lys Lys Gly Thr Pro Asp Leu Ala Ser Cys Gly
           35                  40                  45
   Tyr Gln Val Arg Lys Asp Ala Asn Arg Ala Ser Arg Ile Glu Trp Glu
       50                  55                  60
   His Val Val Pro Ala Trp Gln Phe Gly His Gln Arg Gln Cys Trp Gln
   65                  70                  75                  80
   Asp Gly Gly Arg Lys Asn Cys Thr Lys Asp Asp Val Tyr Arg Gln Ile
                   85                  90                  95
   Glu Thr Asp Leu His Asn Leu Gln Pro Ala Ile Gly Glu Val Asn Gly
               100                 105                 110
   Asp Arg Gly Asn Phe Met Tyr Ser Gln Trp Asn Gly Gly Glu Arg Gln
           115                 120                 125
   Tyr Gly Gln Cys Glu Met Lys Ile Asp Phe Lys Ser Gln Leu Ala Glu
       130                 135                 140
   Pro Pro Glu Arg Ala Arg Gly Ala Ile Ala Arg Thr Tyr Phe Tyr Met
   145                 150                 155                 160
   Arg Asp Arg Tyr Asn Leu Asn Leu Ser Arg Gln Gln Thr Gln Leu Phe
                   165                 170                 175
   Asp Ala Trp Asn Lys Gln Tyr Pro Ala Thr Thr Trp Glu Cys Thr Arg
               180                 185                 190
   Glu Lys Arg Ile Ala Ala Val Gln Gly Asn His Asn Pro Tyr Val Gln
           195                 200                 205
   Gln Ala Cys Ser Pro Asp Ala Ala Pro Tyr Tyr Asn Gly Leu Ser Leu
       210                 215                 220
   Ile Met Ile Ala Ala Val Ala Thr Val Ala Ala Arg Trp Leu Thr Pro
   225 230 235 240
   Ala Gly His Leu Pro Ser Asp
   245
<210> 44
   <211> 250
   <212> PRT
   <213> Streptococcus pneumoniae
<220>
   <223> UniProtKB/Swiss Prot P0A3S3, DNA-entry nuclease, residues 25 to 274
<400> 44
   Ile Lys Gln Met Pro Ser Ala Pro Asn Ser Pro Lys Thr Asn Leu Ser
   1 5 10 15
   Gln Lys Lys Gln Ala Ser Glu Ala Pro Ser Gln Ala Leu Ala Glu Ser
   20 25 30
   Val Leu Thr Asp Ala Val Lys Ser Gln Ile Lys Gly Ser Leu Glu Trp
   35 40 45
   Asn Gly Ser Gly Ala Phe Ile Val Asn Gly Asn Lys Thr Asn Leu Asp
   50 55 60
   Ala Lys Val Ser Ser Lys Pro Tyr Ala Asp Asn Lys Thr Lys Thr Val
   65 70 75 80
   Gly Lys Glu Thr Val Pro Thr Val Ala Asn Ala Leu Leu Ser Lys Ala
   85 90 95
   Thr Arg Gln Tyr Lys Asn Arg Lys Glu Thr Gly Asn Gly Ser Thr Ser
   100 105 110
   Trp Thr Pro Pro Gly Trp His Gln Val Lys Asn Leu Lys Gly Ser Tyr
   115 120 125
   Thr His Ala Val Asp Arg Gly His Leu Leu Gly Tyr Ala Leu Ile Gly
   130 135 140
   Gly Leu Asp Gly Phe Asp Ala Ser Thr Ser Asn Pro Lys Asn Ile Ala
   145 150 155 160
   Val Gln Thr Ala Trp Ala Asn Gln Ala Gln Ala Glu Tyr Ser Thr Gly
   165 170 175
   Gln Asn Tyr Tyr Glu Ser Lys Val Arg Lys Ala Leu Asp Gln Asn Lys
   180 185 190
   Arg Val Arg Tyr Arg Val Thr Leu Tyr Tyr Ala Ser Asn Glu Asp Leu
   195 200 205
   Val Pro Ser Ala Ser Gln Ile Glu Ala Lys Ser Ser Asp Gly Glu Leu
   210 215 220
   Glu Phe Asn Val Leu Val Pro Asn Val Gln Lys Gly Leu Gln Leu Asp
   225 230 235 240
   Tyr Arg Thr Gly Glu Val Thr Val Thr Gln
   245 250
<210> 45
   <211> 149
   <212> PRT
   <213> Staphylococcus aureus
<220>
   <223> UniProtKB/Swiss Prot P00644, thermonuclease, residues 83 to 231
<400> 45
   Ala Thr Ser Thr Lys Lys Leu His Lys Glu Pro Ala Thr Leu Ile Lys
   1 5 10 15
   Ala Ile Asp Gly Asp Thr Val Lys Leu Met Tyr Lys Gly Gln Pro Met
   20 25 30
   Thr Phe Arg Leu Leu Leu Val Asp Thr Pro Glu Thr Lys His Pro Lys
   35 40 45
   Lys Gly Val Glu Lys Tyr Gly Pro Glu Ala Ser Ala Phe Thr Lys Lys
   50 55 60
   Met Val Glu Asn Ala Lys Lys Ile Glu Val Glu Phe Asp Lys Gly Gln
   65 70 75 80
   Arg Thr Asp Lys Tyr Gly Arg Gly Leu Ala Tyr Ile Tyr Ala Asp Gly
   85 90 95
   Lys Met Val Asn Glu Ala Leu Val Arg Gln Gly Leu Ala Lys Val Ala
   100 105 110
   Tyr Val Tyr Lys Pro Asn Asn Thr His Glu Gln His Leu Arg Lys Ser
   115 120 125
   Glu Ala Gln Ala Lys Lys Glu Lys Leu Asn Ile Trp Ser Glu Asp Asn
   130 135 140
   Ala Asp Ser Gly Gln
   145
<210> 46
   <211> 143
   <212> PRT
   <213> Staphylococcus hyicus
<220>
   <223> UniProtKB/Swiss Prot P43270, thermonuclease, residues 27 to 169
<400> 46
   Gly Pro Phe Lys Ser Ala Gly Leu Ser Asn Ala Asn Glu Gln Thr Tyr
   1 5 10 15
   Lys Val Ile Arg Val Ile Asp Gly Asp Thr Ile Ile Val Asp Lys Asp
   20 25 30
   Gly Lys Gln Gln Asn Leu Arg Met Ile Gly Val Asp Thr Pro Glu Thr
   35 40 45
   Val Lys Pro Asn Thr Pro Val Gln Pro Tyr Gly Lys Glu Ala Ser Asp
   50 55 60
   Phe Thr Lys Arg His Leu Thr Asn Gln Lys Val Arg Leu Glu Tyr Asp
   65 70 75 80
   Lys Gln Glu Lys Asp Arg Tyr Gly Arg Thr Leu Ala Tyr Val Trp Leu
   85 90 95
   Gly Lys Glu Met Phe Asn Glu Lys Leu Ala Lys Glu Gly Leu Ala Arg
   100 105 110
   Ala Lys Phe Tyr Arg Pro Asn Tyr Lys Tyr Gln Glu Arg Ile Glu Gln
   115 120 125
   Ala Gln Lys Gln Ala Gln Lys Leu Lys Lys Asn Ile Trp Ser Asn
   130 135 140
<210> 47
   <211> 151
   <212> PRT
   <213> Shigella flexneri
<220>
   <223> UniProtKB/Swiss Prot P29769, micrococcal nuclease, residues 24 to 174
<400> 47
   Trp Ala Asp Phe Arg Gly Glu Val Val Arg Ile Leu Asp Gly Asp Thr
   1               5                   10                  15
   Ile Asp Val Leu Val Asn Arg Gln Thr Ile Arg Val Arg Leu Ala Asp
               20                  25                  30
   Ile Asp Ala Pro Glu Ser Gly Gln Ala Phe Gly Ser Arg Ala Arg Gln
           35                  40                  45
   Arg Leu Ala Asp Leu Thr Phe Arg Gln Glu Val Gln Val Thr Glu Lys
       50                  55                  60
   Glu Val Asp Arg Tyr Gly Arg Thr Leu Gly Val Val Tyr Ala Pro Leu
   65                  70                  75                  80
   Gln Tyr Pro Gly Gly Gln Thr Gln Leu Thr Asn Ile Asn Ala Ile Met
                   85                  90                  95
   Val Gln Glu Gly Met Ala Trp Ala Tyr Arg Tyr Tyr Gly Lys Pro Thr
               100                 105                 110
   Asp Ala Gln Met Tyr Glu Tyr Glu Lys Glu Ala Arg Arg Gln Arg Leu
           115                 120                 125
   Gly Leu Trp Ser Asp Pro Asn Ala Gln Glu Pro Trp Lys Trp Arg Arg
       130                 135                 140
   Ala Ser Lys Asn Ala Thr Asn
   145                 150
<210> 48
   <211> 192
   <212> PRT
   <213> Bacillus subtilis
<220>
   <223> UniProtKB/Swiss Prot P94492, endonuclease yncB, residues 20 to 211
<400> 48
   Cys Gly Ser Asn His Ala Ala Lys Asn His Ser Asp Ser Asn Gly Thr
   1               5                   10                  15
   Glu Gln Val Ser Gln Asp Thr His Ser Asn Glu Tyr Asn Gln Thr Glu
               20                  25                  30
   Gln Lys Ala Gly Thr Pro His Ser Lys Asn Gln Lys Lys Leu Val Asn
           35                  40                  45
   Val Thr Leu Asp Arg Ala Ile Asp Gly Asp Thr Ile Lys Val Ile Tyr
       50                  55                  60
   Asn Gly Lys Lys Asp Thr Val Arg Tyr Leu Leu Val Asp Thr Pro Glu
   65                  70                  75                  80
   Thr Lys Lys Pro Asn Ser Cys Val Gln Pro Tyr Gly Glu Asp Ala Ser
                   85                  90                  95
   Lys Arg Asn Lys Glu Leu Val Asn Ser Gly Lys Leu Gln Leu Glu Phe
               100                 105                 110
   Asp Lys Gly Asp Arg Arg Asp Lys Tyr Gly Arg Leu Leu Ala Tyr Val
           115                 120                 125
   Tyr Val Asp Gly Lys Ser Val Gln Glu Thr Leu Leu Lys Glu Gly Leu
       130                 135                 140
   Ala Arg Val Ala Tyr Val Tyr Glu Pro Asn Thr Lys Tyr Ile Asp Gln
   145                 150                 155                 160
   Phe Arg Leu Asp Glu Gln Glu Ala Lys Ser Asp Lys Leu Ser Ile Trp
                   165                 170                 175
   Ser Lys Ser Gly Tyr Val Thr Asn Arg Gly Phe Asn Gly Cys Val Lys
               180                 185                 190
<210> 49
   <211> 148
   <212> PRT
   <213> Enterobacteria phage T7 (Bacteriophage T7)
<220>
   <223> UniProtKB/Swiss Prot P00641, endodeoxyribonuclease 1, residues 2 to
   149
<400> 49
   Ala Gly Tyr Gly Ala Lys Gly Ile Arg Lys Val Gly Ala Phe Arg Ser
   1               5                   10                  15
   Gly Leu Glu Asp Lys Val Ser Lys Gln Leu Glu Ser Lys Gly Ile Lys
               20                  25                  30
   Phe Glu Tyr Glu Glu Trp Lys Val Pro Tyr Val Ile Pro Ala Ser Asn
   35 40 45
   His Thr Tyr Thr Pro Asp Phe Leu Leu Pro Asn Gly Ile Phe Val Glu
   50 55 60
   Thr Lys Gly Leu Trp Glu Ser Asp Asp Arg Lys Lys His Leu Leu Ile
   65 70 75 80
   Arg Glu Gln His Pro Glu Leu Asp Ile Arg Ile Val Phe Ser Ser Ser
   85 90 95
   Arg Thr Lys Leu Tyr Lys Gly Ser Pro Thr Ser Tyr Gly Glu Phe Cys
   100 105 110
   Glu Lys His Gly Ile Lys Phe Ala Asp Lys Leu Ile Pro Ala Glu Trp
   115 120 125
   Ile Lys Glu Pro Lys Lys Glu Val Pro Phe Asp Arg Leu Lys Arg Lys
   130 135 140
   Gly Gly Lys Lys
   145
<210> 50
   <211> 251
   <212> PRT
   <213> Bos taurus
<220>
   <223> UniProtKB/Swiss Prot, P38447, endonuclease G mitochondrial, residues 49 to 299
<400> 50
   Ala Gly Leu Pro Ala Val Pro Gly Ala Pro Ala Gly Gly Gly Pro Gly
   1 5 10 15
   Glu Leu Ala Lys Tyr Gly Leu Pro Gly Val Ala Gln Leu Lys Ser Arg
   20 25 30
   Ala Ser Tyr Val Leu Cys Tyr Asp Pro Arg Thr Arg Gly Ala Leu Trp
   35 40 45
   Val Val Glu Gln Leu Arg Pro Glu Gly Leu Arg Gly Asp Gly Asn Arg
   50 55 60
   Ser Ser Cys Asp Phe His Glu Asp Asp Ser Val His Ala Tyr His Arg
   65 70 75 80
   Ala Thr Asn Ala Asp Tyr Arg Gly Ser Gly Phe Asp Arg Gly His Leu
   85 90 95
   Ala Ala Ala Ala Asn His Arg Trp Ser Gln Lys Ala Met Asp Asp Thr
   100 105 110
   Phe Tyr Leu Ser Asn Val Ala Pro Gln Val Pro His Leu Asn Gln Asn
   115 120 125
   Ala Trp Asn Asn Leu Glu Lys Tyr Ser Arg Ser Leu Thr Arg Thr Tyr
   130 135 140
   Gln Asn Val Tyr Val Cys Thr Gly Pro Leu Phe Leu Pro Arg Thr Glu
   145 150 155 160
   Ala Asp Gly Lys Ser Tyr Val Lys Tyr Gln Val Ile Gly Lys Asn His
   165 170 175
   Val Ala Val Pro Thr His Phe Phe Lys Val Leu Ile Leu Glu Ala Ala
   180 185 190
   Gly Gly Gln Ile Glu Leu Arg Ser Tyr Val Met Pro Asn Ala Pro Val
   195 200 205
   Asp Glu Ala Ile Pro Leu Glu His Phe Leu Val Pro Ile Glu Ser Ile
   210 215 220
   Glu Arg Ala Ser Gly Leu Leu Phe Val Pro Asn Ile Leu Ala Arg Ala
   225 230 235 240
   Gly Ser Leu Lys Ala Ile Thr Ala Gly Ser Lys
   245 250
<210> 51
   <211> 129
   <212> PRT
   <213> Thermus thermophilus (strain HB8 / ATCC 27634 / DSM 579)
<220>
   <223> UniProtKB/Swiss Prot, Q56239, residues 2 to 130 DNA mismatch repair protein mutS
<400> 51
   Gly Gly Tyr Gly Gly Val Lys Met Glu Gly Met Leu Lys Gly Glu Gly
   1 5 10 15
   Pro Gly Pro Leu Pro Pro Leu Leu Gln Gln Tyr Val Glu Leu Arg Asp
   20 25 30
   Arg Tyr Pro Asp Tyr Leu Leu Leu Phe Gln Val Gly Asp Phe Tyr Glu
           35                  40                  45
   Cys Phe Gly Glu Asp Ala Glu Arg Leu Ala Arg Ala Leu Gly Leu Val
       50                  55                  60
   Leu Thr His Lys Thr Ser Lys Asp Phe Thr Thr Pro Met Ala Gly Ile
   65                  70                  75                  80
   Pro Ile Arg Ala Phe Asp Ala Tyr Ala Glu Arg Leu Leu Lys Met Gly
                   85                  90                  95
   Phe Arg Leu Ala Val Ala Asp Gln Val Glu Pro Ala Glu Glu Ala Glu
               100                 105                 110
   Gly Leu Val Arg Arg Glu Val Thr Gln Leu Leu Thr Pro Gly Thr Leu
           115                 120                 125
   Thr
<210> 52
   <211> 239
   <212> PRT
   <213> Homo sapiens
<220>
   <223> UniProtKB/Swiss Prot, Q53H47, residues 433 to 671 Histone-lysine N-methyltransferase SETMAR
<400> 52
   His Leu Lys Gln Ile Gly Lys Val Lys Lys Leu Asp Lys Trp Val Pro
   1               5                   10                  15
   His Glu Leu Thr Glu Asn Gln Lys Asn Arg Arg Phe Glu Val Ser Ser
               20                  25                  30
   Ser Leu Ile Leu Arg Asn His Asn Glu Pro Phe Leu Asp Arg Ile Val
           35                  40                  45
   Thr Cys Asp Glu Lys Trp Ile Leu Tyr Asp Asn Arg Arg Arg Ser Ala
       50                  55                  60
   Gln Trp Leu Asp Gln Glu Glu Ala Pro Lys His Phe Pro Lys Pro Ile
   65                  70                  75                  80
   Leu His Pro Lys Lys Val Met Val Thr Ile Trp Trp Ser Ala Ala Gly
                   85                  90                  95
   Leu Ile His Tyr Ser Phe Leu Asn Pro Gly Glu Thr Ile Thr Ser Glu
               100                 105                 110
   Lys Tyr Ala Gln Glu Ile Asp Glu Met Asn Gln Lys Leu Gln Arg Leu
           115                 120                 125
   Gln Leu Ala Leu Val Asn Arg Lys Gly Pro Ile Leu Leu His Asp Asn
       130                 135                 140
   Ala Arg Pro His Val Ala Gln Pro Thr Leu Gln Lys Leu Asn Glu Leu
   145                 150                 155                 160
   Gly Tyr Glu Val Leu Pro His Pro Pro Tyr Ser Pro Asp Leu Leu Pro
                   165                 170                 175
   Thr Asn Tyr His Val Phe Lys His Leu Asn Asn Phe Leu Gln Gly Lys
               180                 185                 190
   Arg Phe His Asn Gln Gln Asp Ala Glu Asn Ala Phe Gln Glu Phe Val
           195                 200                 205
   Glu Ser Gln Ser Thr Asp Phe Tyr Ala Thr Gly Ile Asn Gln Leu Ile
       210                 215                 220
   Ser Arg Trp Gln Lys Cys Val Asp Cys Asn Gly Ser Tyr Phe Asp
   225                 230                 235
<210> 53
   <211> 213
   <212> PRT
   <213> Vibrio vulnificus
<220>
   <223> GenBank AAF19759.1 residues 18 to 231
<400> 53
   Ala Pro Pro Ser Ser Phe Ser Ala Ala Lys Gln Gln Ala Val Lys Ile
   1               5                   10                  15
   Tyr Gln Asp His Pro Ile Ser Phe Tyr Cys Gly Cys Asp Ile Glu Trp
               20                  25                  30
   Gln Gly Lys Lys Gly Ile Pro Asn Leu Glu Thr Cys Gly Tyr Gln Val
           35                  40                  45
   Arg Lys Gln Gln Thr Arg Ala Ser Arg Ile Glu Trp Glu His Val Val
       50                  55                  60
   Pro Ala Trp Gln Phe Gly His His Arg Gln Cys Trp Gln Lys Gly Gly
   65 70 75 80
   Arg Lys Asn Cys Ser Lys Asn Asp Gln Gln Phe Arg Leu Met Glu Ala
   85 90 95
   Asp Leu His Asn Leu Thr Pro Ala Ile Gly Glu Val Asn Gly Asp Arg
   100 105 110
   Ser Asn Phe Asn Phe Ser Gln Trp Asn Gly Val Asp Gly Val Ser Tyr
   115 120 125
   Gly Arg Cys Glu Met Gln Val Asn Phe Lys Gln Arg Lys Val Met Pro
   130 135 140
   Pro Asp Arg Ala Arg Gly Ser Ile Ala Arg Thr Tyr Leu Tyr Met Ser
   145 150 155 160
   Gln Glu Tyr Gly Phe Gln Leu Ser Lys Gln Gln Gln Gln Leu Met Gln
   165 170 175
   Ala Trp Asn Lys Ser Tyr Pro Val Asp Glu Trp Glu Cys Thr Arg Asp
   180 185 190
   Asp Arg Ile Ala Lys Ile Gln Gly Asn His Asn Pro Phe Val Gln Gln
   195 200 205
   Ser Cys Gln Thr Gln
   210
<210> 54
   <211> 131
   <212> PRT
   <213> Escherichia coli
<220>
   <223> UniProtKB/Swiss Prot, Q47112, Colicin-E7, residues 446 to 576
<400> 54
   Lys Arg Asn Lys Pro Gly Lys Ala Thr Gly Lys Gly Lys Pro Val Asn
   1 5 10 15
   Asn Lys Trp Leu Asn Asn Ala Gly Lys Asp Leu Gly Ser Pro Val Pro
   20 25 30
   Asp Arg Ile Ala Asn Lys Leu Arg Asp Lys Glu Phe Lys Ser Phe Asp
   35 40 45
   Asp Phe Arg Lys Lys Phe Trp Glu Glu Val Ser Lys Asp Pro Glu Leu
       50                  55                  60
   Ser Lys Gln Phe Ser Arg Asn Asn Asn Asp Arg Met Lys Val Gly Lys
   65                  70                  75                  80
   Ala Pro Lys Thr Arg Thr Gln Asp Val Ser Gly Lys Arg Thr Ser Phe
                   85                  90                  95
   Glu Leu His His Glu Lys Pro Ile Ser Gln Asn Gly Gly Val Tyr Asp
               100                 105                 110
   Met Asp Asn Ile Ser Val Val Thr Pro Lys Arg His Ile Asp Ile His
           115                 120                 125
   Arg Gly Lys
       130
<210> 55
   <211> 174
   <212> PRT
   <213> Bacillus phage SP01 (Bacteriophage SP01)
<220>
   <223> UniProtKB/Swiss Prot, P34081, DNA endonuclease I-HmuI residues 1 to 174
<400> 55
   Met Glu Trp Lys Asp Ile Lys Gly Tyr Glu Gly His Tyr Gln Val Ser
   1               5                   10                  15
   Asn Thr Gly Glu Val Tyr Ser Ile Lys Ser Gly Lys Thr Leu Lys His
               20                  25                  30
   Gln Ile Pro Lys Asp Gly Tyr His Arg Ile Gly Leu Phe Lys Gly Gly
           35                  40                  45
   Lys Gly Lys Thr Phe Gln Val His Arg Leu Val Ala Ile His Phe Cys
       50                  55                  60
   Glu Gly Tyr Glu Glu Gly Leu Val Val Asp His Lys Asp Gly Asn Lys
   65                  70                  75                  80
   Asp Asn Asn Leu Ser Thr Asn Leu Arg Trp Val Thr Gln Lys Ile Asn
                   85                  90                  95
   Val Glu Asn Gln Met Ser Arg Gly Thr Leu Asn Val Ser Lys Ala Gln
               100                 105                 110
   Gln Ile Ala Lys Ile Lys Asn Gln Lys Pro Ile Ile Val Ile Ser Pro
           115                 120                 125
   Asp Gly Ile Glu Lys Glu Tyr Pro Ser Thr Lys Cys Ala Cys Glu Glu
       130                 135                 140
   Leu Gly Leu Thr Arg Gly Lys Val Thr Asp Val Leu Lys Gly His Arg
   145                 150                 155                 160
   Ile His His Lys Gly Tyr Thr Phe Arg Tyr Lys Leu Asn Gly
                   165                 170
<210> 56
   <211> 169
   <212> PRT
   <213> Enterobacteria phage T4 (Bacteriophage T4)
<220>
   <223> UniProtKB/Swiss Prot, P13299, Intron-associated endonuclease 1
   (I-TevI) residues 2 to 170
<400> 56
   Lys Ser Gly Ile Tyr Gln Ile Lys Asn Thr Leu Asn Asn Lys Val Tyr
   1               5                   10                  15
   Val Gly Ser Ala Lys Asp Phe Glu Lys Arg Trp Lys Arg His Phe Lys
               20                  25                  30
   Asp Leu Glu Lys Gly Cys His Ser Ser Ile Lys Leu Gln Arg Ser Phe
           35                  40                  45
   Asn Lys His Gly Asn Val Phe Glu Cys Ser Ile Leu Glu Glu Ile Pro
       50                  55                  60
   Tyr Glu Lys Asp Leu Ile Ile Glu Arg Glu Asn Phe Trp Ile Lys Glu
   65                  70                  75                  80
   Leu Asn Ser Lys Ile Asn Gly Tyr Asn Ile Ala Asp Ala Thr Phe Gly
                   85                  90                  95
   Asp Thr Cys Ser Thr His Pro Leu Lys Glu Glu Ile Ile Lys Lys Arg
               100                 105                 110
   Ser Glu Thr Val Lys Ala Lys Met Leu Lys Leu Gly Pro Asp Gly Arg
           115                 120                 125
   Lys Ala Leu Tyr Ser Lys Pro Gly Ser Lys Asn Gly Arg Trp Asn Pro
       130                 135                 140
   Glu Thr His Lys Phe Cys Lys Cys Gly Val Arg Ile Gln Thr Ser Ala
   145 150 155 160
   Tyr Thr Cys Ser Lys Cys Arg Asn Arg
   165
<210> 57
   <211> 145
   <212> PRT
   <213> Enterobacteria phage RB3 (Bacteriophage RB3)
<220>
   <223> UniProtKB/Swiss Prot, Q38419, Intron-associated endonuclease 3
   (I-TevIII) residues 2 to 146
<400> 57
   Asn Tyr Arg Lys Ile Trp Ile Asp Ala Asn Gly Pro Ile Pro Lys Asp
   1 5 10 15
   Ser Asp Gly Arg Thr Asp Glu Ile His His Lys Asp Gly Asn Arg Glu
   20 25 30
   Asn Asn Asp Leu Asp Asn Leu Met Cys Leu Ser Ile Gln Glu His Tyr
   35 40 45
   Asp Ile His Leu Ala Gln Lys Asp Tyr Gln Ala Cys His Ala Ile Lys
   50 55 60
   Leu Arg Met Lys Tyr Ser Pro Glu Glu Ile Ser Glu Leu Ala Ser Lys
   65 70 75 80
   Ala Ala Lys Ser Arg Glu Ile Gln Ile Phe Asn Ile Pro Glu Val Arg
   85 90 95
   Ala Lys Asn Ile Ala Ser Ile Lys Ser Lys Ile Glu Asn Gly Thr Phe
   100 105 110
   His Leu Leu Asp Gly Glu Ile Gln Arg Lys Ser Asn Leu Asn Arg Val
   115 120 125
   Ala Leu Gly Ile His Asn Phe Gln Gln Ala Glu His Ile Ala Lys Val
   130 135 140
   Lys
   145
<210> 58
   <211> 354
   <212> PRT
   <213> artificial sequence
<220>
   <223> R1 single chain meganuclease
<400> 58
   Met Ala Asn Thr Lys Tyr Asn Glu Glu Phe Leu Leu Tyr Leu Ala Gly
   1               5                   10                  15
   Phe Val Asp Gly Asp Gly Ser Ile Ile Ala Gln Ile Asn Pro Asn Gln
               20                  25                  30
   Ser Ser Lys Phe Lys His Arg Leu Arg Leu Thr Phe Tyr Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Ser Gly Ser Val Ser Gln Tyr Val Leu Ser
   65                  70                  75                  80
   Glu Ile Lys Pro Leu His Asn Phe Leu Thr Gln Leu Gln Pro Phe Leu
                   85                  90                  95
   Glu Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Gly Lys Lys
   145                 150                 155                 160
   Lys Ser Ser Pro Ala Ala Gly Gly Ser Asp Lys Tyr Asn Gln Ala Leu
                   165                 170                 175
   Ser Lys Tyr Asn Gln Ala Leu Ser Lys Tyr Asn Gln Ala Leu Ser Gly
               180                 185                 190
   Gly Gly Gly Ser Asn Lys Lys Phe Leu Leu Tyr Leu Ala Gly Phe Val
           195                 200                 205
   Asp Ser Asp Gly Ser Ile Ile Ala Gln Ile Lys Pro Arg Gln Ser Asn
   210 215 220
   Lys Phe Lys His Gln Leu Ser Leu Thr Phe Ala Val Thr Gln Lys Thr
   225 230 235 240
   Gln Arg Arg Trp Phe Leu Asp Lys Leu Val Asp Arg Ile Gly Val Gly
   245 250 255
   Tyr Val Tyr Asp Ser Gly Ser Val Ser Asp Tyr Arg Leu Ser Glu Ile
   260 265 270
   Lys Pro Leu His Asn Phe Leu Thr Gln Leu Gln Pro Phe Leu Lys Leu
   275 280 285
   Lys Gln Lys Gln Ala Asn Leu Val Leu Lys Ile Ile Glu Gln Leu Pro
   290 295 300
   Ser Ala Lys Glu Ser Pro Asp Lys Phe Leu Glu Val Cys Thr Trp Val
   305 310 315 320
   Asp Gln Ile Ala Ala Leu Asn Asp Ser Lys Thr Arg Lys Thr Thr Ser
   325 330 335
   Glu Thr Val Arg Ala Val Leu Asp Ser Leu Ser Glu Lys Lys Lys Ser
   340 345 350
   Ser Pro
<210> 59
   <211> 354
   <212> PRT
   <213> artificial sequence
<220>
   <223> D21 single chain meganuclease
<400> 59
   Met Ala Asn Thr Lys Tyr Asn Glu Glu Phe Leu Leu Tyr Leu Ala Gly
   1 5 10 15
   Phe Val Asp Gly Asp Gly Ser Ile Ile Ala Gln Ile Lys Pro Arg Gln
   20 25 30
   Ser Tyr Lys Phe Lys His Gln Leu Glu Leu Thr Phe Thr Val Gly Gln
   35 40 45
   Lys Thr Gln Arg Arg Trp Phe Leu Asp Lys Leu Val Asp Glu Ile Gly
       50                  55                  60
   Val Gly Tyr Val Thr Asp Ser Gly Ser Met Ser Ala Tyr Arg Leu Ser
   65                  70                  75                  80
   Lys Ile Lys Pro Leu His Asn Phe Leu Thr Gln Leu Gln Pro Phe Leu
                   85                  90                  95
   Glu Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Val Ala Ala Leu Asn Asp Ser Lys Thr Arg Lys Thr
       130                 135                 140
   Thr Ser Glu Thr Val Arg Ala Val Leu Asp Asn Leu Ser Glu Lys Lys
   145                 150                 155                 160
   Lys Ser Ser Pro Ala Ala Gly Gly Ser Asp Lys Tyr Asn Gln Ala Leu
                   165                 170                 175
   Ser Lys Tyr Asn Gln Ala Leu Ser Lys Tyr Asn Gln Ala Leu Ser Gly
               180                 185                 190
   Gly Gly Gly Ser Asn Lys Lys Phe Leu Leu Tyr Leu Ala Gly Phe Val
           195                 200                 205
   Asp Ser Asp Gly Ser Ile Ile Ala Gln Ile Arg Pro Asn Gln Ser Ala
       210                 215                 220
   Lys Phe Lys His Tyr Leu Gln Leu Thr Phe Gln Val Thr Gln Lys Thr
   225                 230                 235                 240
   Gln Arg Arg Trp Phe Leu Asp Lys Leu Val Asp Arg Ile Gly Val Gly
                   245                 250                 255
   Tyr Val Arg Asp Ser Gly Ser Val Ser Asp Tyr Lys Leu Ser Glu Ile
               260                 265                 270
   Lys Pro Leu His Asn Phe Leu Thr Gln Leu Gln Pro Phe Leu Lys Leu
           275                 280                 285
   Lys Gln Lys Gln Ala Asn Leu Val Leu Lys Ile Ile Glu Gln Leu Pro
       290                 295                 300
   Ser Ala Lys Glu Ser Pro Asp Lys Phe Leu Glu Val Cys Thr Trp Val
   305 310 315 320
   Asp Gln Val Ala Ala Leu Asn Asp Ser Lys Thr Arg Lys Thr Thr Ser
   325 330 335
   Glu Thr Val Arg Ala Val Leu Asp Ser Leu Ser Glu Lys Lys Lys Ser
   340 345 350
   Ser Pro
<210> 60
   <211> 245
   <212> PRT
   <213> artificial sequence
<220>
   <223> I-TevI
<400> 60
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Lys Phe Cys Lys Cys Gly Val Arg Ile Gln Thr Ser
   145                 150                 155                 160
   Ala Tyr Thr Cys Ser Lys Cys Arg Asn Arg Ser Gly Glu Asn Asn Ser
                   165                 170                 175
   Phe Phe Asn His Lys His Ser Asp Ile Thr Lys Ser Lys Ile Ser Glu
               180                 185                 190
   Lys Met Lys Gly Lys Lys Pro Ser Asn Ile Lys Lys Ile Ser Cys Asp
           195                 200                 205
   Gly Val Ile Phe Asp Cys Ala Ala Asp Ala Ala Arg His Phe Lys Ile
       210                 215                 220
   Ser Ser Gly Leu Val Thr Tyr Arg Val Lys Ser Asp Lys Trp Asn Trp
   225                 230                 235                 240
   Phe Tyr Ile Asn Ala
                   245
<210> 61
   <211> 366
   <212> PRT
   <213> artificial sequence
<220>
   <223> hTevCre_D01
<400> 61
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Lys Phe Cys Lys Cys Gly Val Arg Ile Gln Thr Ser
   145                 150                 155                 160
   Ala Tyr Thr Cys Ser Lys Cys Arg Asn Arg Ser Gly Glu Asn Asn Ser
                   165                 170                 175
   Phe Phe Asn His Lys His Ser Asp Ile Thr Lys Ser Lys Ile Ser Gly
               180                 185                 190
   Lys Met Lys Gly Gln Gly Pro Ser Gly Asn Thr Lys Tyr Asn Lys Glu
           195                 200                 205
   Phe Leu Leu Tyr Leu Ala Gly Phe Val Asp Gly Asp Gly Ser Ile Ile
       210                 215                 220
   Ala Gln Ile Lys Pro Asn Gln Ser Tyr Lys Phe Lys His Gln Leu Ser
   225                 230                 235                 240
   Leu Thr Phe Gln Val Thr Gln Lys Thr Gln Arg Arg Trp Phe Leu Asp
                   245                 250                 255
   Lys Leu Val Asp Glu Ile Gly Val Gly Tyr Val Arg Asp Arg Gly Ser
               260                 265                 270
   Val Ser Asp Tyr Ile Leu Ser Glu Ile Lys Pro Leu His Asn Phe Leu
           275                 280                 285
   Thr Gln Leu Gln Pro Phe Leu Lys Leu Lys Gln Lys Gln Ala Asn Leu
       290                 295                 300
   Val Leu Lys Ile Ile Glu Gln Leu Pro Ser Ala Lys Glu Ser Pro Asp
   305                 310                 315                 320
   Lys Phe Leu Glu Val Cys Thr Trp Val Asp Gln Ile Ala Ala Leu Asn
                   325                 330                 335
   Asp Ser Lys Thr Arg Lys Thr Thr Ser Glu Thr Val Arg Ala Val Leu
               340                 345                 350
   Asp Ser Leu Ser Glu Lys Lys Lys Ser Ser Pro Ala Ala Asp
   355 360 365
<210> 62
   <211> 353
   <212> PRT
   <213> artificial sequence
<220>
   <223> hTevCre_D02
<400> 62
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Lys Phe Cys Lys Cys Gly Val Arg Ile Gln Thr Ser
   145                 150                 155                 160
   Ala Tyr Thr Cys Ser Lys Cys Arg Asn Arg Ser Gly Glu Asn Asn Ser
                   165                 170                 175
   Phe Phe Asn His Lys His Ser Gln Gly Pro Ser Gly Asn Thr Lys Tyr
               180                 185                 190
   Asn Lys Glu Phe Leu Leu Tyr Leu Ala Gly Phe Val Asp Gly Asp Gly
           195                 200                 205
   Ser Ile Ile Ala Gln Ile Lys Pro Asn Gln Ser Tyr Lys Phe Lys His
       210                 215                 220
   Gln Leu Ser Leu Thr Phe Gln Val Thr Gln Lys Thr Gln Arg Arg Trp
   225                 230                 235                 240
   Phe Leu Asp Lys Leu Val Asp Glu Ile Gly Val Gly Tyr Val Arg Asp
                   245                 250                 255
   Arg Gly Ser Val Ser Asp Tyr Ile Leu Ser Glu Ile Lys Pro Leu His
               260                 265                 270
   Asn Phe Leu Thr Gln Leu Gln Pro Phe Leu Lys Leu Lys Gln Lys Gln
           275                 280                 285
   Ala Asn Leu Val Leu Lys Ile Ile Glu Gln Leu Pro Ser Ala Lys Glu
       290                 295                 300
   Ser Pro Asp Lys Phe Leu Glu Val Cys Thr Trp Val Asp Gln Ile Ala
   305                 310                 315                 320
   Ala Leu Asn Asp Ser Lys Thr Arg Lys Thr Thr Ser Glu Thr Val Arg
                   325                 330                 335
   Ala Val Leu Asp Ser Leu Ser Glu Lys Lys Lys Ser Ser Pro Ala Ala
               340                 345                 350
   Asp
<210> 63
   <211> 339
   <212> PRT
   <213> artificial sequence
<220>
   <223> hTevCre_D03
<400> 63
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Lys Phe Cys Lys Cys Gly Val Arg Ile Gln Thr Ser
   145                 150                 155                 160
   Ala Tyr Thr Cys Ser Lys Cys Arg Asn Gln Gly Pro Ser Gly Asn Thr
                   165                 170                 175
   Lys Tyr Asn Lys Glu Phe Leu Leu Tyr Leu Ala Gly Phe Val Asp Gly
               180                 185                 190
   Asp Gly Ser Ile Ile Ala Gln Ile Lys Pro Asn Gln Ser Tyr Lys Phe
           195                 200                 205
   Lys His Gln Leu Ser Leu Thr Phe Gln Val Thr Gln Lys Thr Gln Arg
       210                 215                 220
   Arg Trp Phe Leu Asp Lys Leu Val Asp Glu Ile Gly Val Gly Tyr Val
   225                 230                 235                 240
   Arg Asp Arg Gly Ser Val Ser Asp Tyr Ile Leu Ser Glu Ile Lys Pro
                   245                 250                 255
   Leu His Asn Phe Leu Thr Gln Leu Gln Pro Phe Leu Lys Leu Lys Gln
               260                 265                 270
   Lys Gln Ala Asn Leu Val Leu Lys Ile Ile Glu Gln Leu Pro Ser Ala
           275                 280                 285
   Lys Glu Ser Pro Asp Lys Phe Leu Glu Val Cys Thr Trp Val Asp Gln
       290                 295                 300
   Ile Ala Ala Leu Asn Asp Ser Lys Thr Arg Lys Thr Thr Ser Glu Thr
   305 310 315 320
   Val Arg Ala Val Leu Asp Ser Leu Ser Glu Lys Lys Lys Ser Ser Pro
   325 330 335
   Ala Ala Asp
<210> 64
   <211> 318
   <212> PRT
   <213> artificial sequence
<220>
   <223> hTevCre_D04
<400> 64
   Met Lys Ser Gly Ile Tyr Gln Ile Lys Asn Thr Leu Asn Asn Lys Val
   1 5 10 15
   Tyr Val Gly Ser Ala Lys Asp Phe Glu Lys Arg Trp Lys Arg His Phe
   20 25 30
   Lys Asp Leu Glu Lys Gly Cys His Ser Ser Ile Lys Leu Gln Arg Ser
   35 40 45
   Phe Asn Lys His Gly Asn Val Phe Glu Cys Ser Ile Leu Glu Glu Ile
   50 55 60
   Pro Tyr Glu Lys Asp Leu Ile Ile Glu Arg Glu Asn Phe Trp Ile Lys
   65 70 75 80
   Glu Leu Asn Ser Lys Ile Asn Gly Tyr Asn Ile Ala Asp Ala Thr Phe
   85 90 95
   Gly Asp Thr Cys Ser Thr His Pro Leu Lys Glu Glu Ile Ile Lys Lys
   100 105 110
   Arg Ser Glu Thr Val Lys Ala Lys Met Leu Lys Leu Gly Pro Asp Gly
   115 120 125
   Arg Lys Ala Leu Tyr Ser Lys Pro Gly Ser Lys Asn Gly Arg Trp Asn
   130 135 140
   Pro Glu Thr His Gln Gly Pro Ser Gly Asn Thr Lys Tyr Asn Lys Glu
   145 150 155 160
   Phe Leu Leu Tyr Leu Ala Gly Phe Val Asp Gly Asp Gly Ser Ile Ile
   165 170 175
   Ala Gln Ile Lys Pro Asn Gln Ser Tyr Lys Phe Lys His Gln Leu Ser
   180 185 190
   Leu Thr Phe Gln Val Thr Gln Lys Thr Gln Arg Arg Trp Phe Leu Asp
   195 200 205
   Lys Leu Val Asp Glu Ile Gly Val Gly Tyr Val Arg Asp Arg Gly Ser
   210 215 220
   Val Ser Asp Tyr Ile Leu Ser Glu Ile Lys Pro Leu His Asn Phe Leu
   225 230 235 240
   Thr Gln Leu Gln Pro Phe Leu Lys Leu Lys Gln Lys Gln Ala Asn Leu
   245 250 255
   Val Leu Lys Ile Ile Glu Gln Leu Pro Ser Ala Lys Glu Ser Pro Asp
   260 265 270
   Lys Phe Leu Glu Val Cys Thr Trp Val Asp Gln Ile Ala Ala Leu Asn
   275 280 285
   Asp Ser Lys Thr Arg Lys Thr Thr Ser Glu Thr Val Arg Ala Val Leu
   290 295 300
   Asp Ser Leu Ser Glu Lys Lys Lys Ser Ser Pro Ala Ala Asp
   305 310 315
<210> 65
   <211> 293
   <212> PRT
   <213> artificial sequence
<220>
   <223> hTevCre_D05
<400> 65
   Met Lys Ser Gly Ile Tyr Gln Ile Lys Asn Thr Leu Asn Asn Lys Val
   1 5 10 15
   Tyr Val Gly Ser Ala Lys Asp Phe Glu Lys Arg Trp Lys Arg His Phe
   20 25 30
   Lys Asp Leu Glu Lys Gly Cys His Ser Ser Ile Lys Leu Gln Arg Ser
   35 40 45
   Phe Asn Lys His Gly Asn Val Phe Glu Cys Ser Ile Leu Glu Glu Ile
   50 55 60
   Pro Tyr Glu Lys Asp Leu Ile Ile Glu Arg Glu Asn Phe Trp Ile Lys
   65 70 75 80
   Glu Leu Asn Ser Lys Ile Asn Gly Tyr Asn Ile Ala Asp Ala Thr Phe
   85 90 95
   Gly Asp Thr Cys Ser Thr His Pro Leu Lys Glu Glu Ile Ile Lys Lys
   100 105 110
   Arg Ser Glu Thr Val Lys Ala Lys Met Leu Lys Gln Gly Pro Ser Gly
   115 120 125
   Asn Thr Lys Tyr Asn Lys Glu Phe Leu Leu Tyr Leu Ala Gly Phe Val
   130 135 140
   Asp Gly Asp Gly Ser Ile Ile Ala Gln Ile Lys Pro Asn Gln Ser Tyr
   145 150 155 160
   Lys Phe Lys His Gln Leu Ser Leu Thr Phe Gln Val Thr Gln Lys Thr
   165 170 175
   Gln Arg Arg Trp Phe Leu Asp Lys Leu Val Asp Glu Ile Gly Val Gly
   180 185 190
   Tyr Val Arg Asp Arg Gly Ser Val Ser Asp Tyr Ile Leu Ser Glu Ile
   195 200 205
   Lys Pro Leu His Asn Phe Leu Thr Gln Leu Gln Pro Phe Leu Lys Leu
   210 215 220
   Lys Gln Lys Gln Ala Asn Leu Val Leu Lys Ile Ile Glu Gln Leu Pro
   225 230 235 240
   Ser Ala Lys Glu Ser Pro Asp Lys Phe Leu Glu Val Cys Thr Trp Val
   245 250 255
   Asp Gln Ile Ala Ala Leu Asn Asp Ser Lys Thr Arg Lys Thr Thr Ser
   260 265 270
   Glu Thr Val Arg Ala Val Leu Asp Ser Leu Ser Glu Lys Lys Lys Ser
   275 280 285
   Ser Pro Ala Ala Asp
   290
<210> 66
   <211> 296
   <212> PRT
   <213> artificial sequence
<220>
   <223> hTevCre_D06
<400> 66
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Asn Thr Lys Tyr Asn Lys Glu Phe Leu Leu Tyr Leu Ala
       130                 135                 140
   Gly Phe Val Asp Gly Asp Gly Ser Ile Ile Ala Gln Ile Lys Pro Asn
   145                 150                 155                 160
   Gln Ser Tyr Lys Phe Lys His Gln Leu Ser Leu Thr Phe Gln Val Thr
                   165                 170                 175
   Gln Lys Thr Gln Arg Arg Trp Phe Leu Asp Lys Leu Val Asp Glu Ile
               180                 185                 190
   Gly Val Gly Tyr Val Arg Asp Arg Gly Ser Val Ser Asp Tyr Ile Leu
           195                 200                 205
   Ser Glu Ile Lys Pro Leu His Asn Phe Leu Thr Gln Leu Gln Pro Phe
   210 215 220
   Leu Lys Leu Lys Gln Lys Gln Ala Asn Leu Val Leu Lys Ile Ile Glu
   225 230 235 240
   Gln Leu Pro Ser Ala Lys Glu Ser Pro Asp Lys Phe Leu Glu Val Cys
   245 250 255
   Thr Trp Val Asp Gln Ile Ala Ala Leu Asn Asp Ser Lys Thr Arg Lys
   260 265 270
   Thr Thr Ser Glu Thr Val Arg Ala Val Leu Asp Ser Leu Ser Glu Lys
   275 280 285
   Lys Lys Ser Ser Pro Ala Ala Asp
   290 295
<210> 67
   <211> 5
   <212> PRT
   <213> artificial sequence
<220>
   <223> QGPSG peptidic linker
<400> 67
   Gln Gly Pro Ser Gly
   1 5
<210> 68
   <211> 8
   <212> PRT
   <213> artificial sequence
<220>
   <223> LGPDGRKA peptidic linker
<400> 68
   Leu Gly Pro Asp Gly Arg Lys Ala
   1 5
<210> 69
   <211> 167
   <212> PRT
   <213> artificial sequence
<220>
   <223> I-CreI_N20
<400> 69
   Met Ala Asn Thr Lys Tyr Asn Lys Glu Phe Leu Leu Tyr Leu Ala Gly
   1 5 10 15
   Phe Val Asp Gly Asn Gly Ser Ile Ile Ala Gln Ile Lys Pro Asn Gln
   20 25 30
   Ser Tyr Lys Phe Lys His Gln Leu Ser Leu Thr Phe Gln Val Thr Gln
   35 40 45
   Lys Thr Gln Arg Arg Trp Phe Leu Asp Lys Leu Val Asp Glu Ile Gly
   50 55 60
   Val Gly Tyr Val Arg Asp Arg Gly Ser Val Ser Asp Tyr Ile Leu Ser
   65 70 75 80
   Glu Ile Lys Pro Leu His Asn Phe Leu Thr Gln Leu Gln Pro Phe Leu
   85 90 95
   Lys Leu Lys Gln Lys Gln Ala Asn Leu Val Leu Lys Ile Ile Glu Gln
   100 105 110
   Leu Pro Ser Ala Lys Glu Ser Pro Asp Lys Phe Leu Glu Val Cys Thr
   115 120 125
   Trp Val Asp Gln Ile Ala Ala Leu Asn Asp Ser Lys Thr Arg Lys Thr
   130 135 140
   Thr Ser Glu Thr Val Arg Ala Val Leu Asp Ser Leu Ser Glu Lys Lys
   145 150 155 160
   Lys Ser Ser Pro Ala Ala Asp
   165
<210> 70
   <211> 366
   <212> PRT
   <213> artificial sequence
<220>
   <223> hTevCre_D01_N20
<400> 70
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Lys Phe Cys Lys Cys Gly Val Arg Ile Gln Thr Ser
   145                 150                 155                 160
   Ala Tyr Thr Cys Ser Lys Cys Arg Asn Arg Ser Gly Glu Asn Asn Ser
                   165                 170                 175
   Phe Phe Asn His Lys His Ser Asp Ile Thr Lys Ser Lys Ile Ser Gly
               180                 185                 190
   Lys Met Lys Gly Gln Gly Pro Ser Gly Asn Thr Lys Tyr Asn Lys Glu
           195                 200                 205
   Phe Leu Leu Tyr Leu Ala Gly Phe Val Asp Gly Asn Gly Ser Ile Ile
       210                 215                 220
   Ala Gln Ile Lys Pro Asn Gln Ser Tyr Lys Phe Lys His Gln Leu Ser
   225                 230                 235                 240
   Leu Thr Phe Gln Val Thr Gln Lys Thr Gln Arg Arg Trp Phe Leu Asp
                   245                 250                 255
   Lys Leu Val Asp Glu Ile Gly Val Gly Tyr Val Arg Asp Arg Gly Ser
               260                 265                 270
   Val Ser Asp Tyr Ile Leu Ser Glu Ile Lys Pro Leu His Asn Phe Leu
           275                 280                 285
   Thr Gln Leu Gln Pro Phe Leu Lys Leu Lys Gln Lys Gln Ala Asn Leu
   290 295 300
   Val Leu Lys Ile Ile Glu Gln Leu Pro Ser Ala Lys Glu Ser Pro Asp
   305 310 315 320
   Lys Phe Leu Glu Val Cys Thr Trp Val Asp Gln Ile Ala Ala Leu Asn
   325 330 335
   Asp Ser Lys Thr Arg Lys Thr Thr Ser Glu Thr Val Arg Ala Val Leu
   340 345 350
   Asp Ser Leu Ser Glu Lys Lys Lys Ser Ser Pro Ala Ala Asp
   355 360 365
<210> 71
   <211> 353
   <212> PRT
   <213> artificial sequence
<220>
   <223> hTevCre_D02_N20
<400> 71
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Lys Phe Cys Lys Cys Gly Val Arg Ile Gln Thr Ser
   145 150 155 160
   Ala Tyr Thr Cys Ser Lys Cys Arg Asn Arg Ser Gly Glu Asn Asn Ser
   165 170 175
   Phe Phe Asn His Lys His Ser Gln Gly Pro Ser Gly Asn Thr Lys Tyr
   180 185 190
   Asn Lys Glu Phe Leu Leu Tyr Leu Ala Gly Phe Val Asp Gly Asn Gly
   195 200 205
   Ser Ile Ile Ala Gln Ile Lys Pro Asn Gln Ser Tyr Lys Phe Lys His
   210 215 220
   Gln Leu Ser Leu Thr Phe Gln Val Thr Gln Lys Thr Gln Arg Arg Trp
   225 230 235 240
   Phe Leu Asp Lys Leu Val Asp Glu Ile Gly Val Gly Tyr Val Arg Asp
   245 250 255
   Arg Gly Ser Val Ser Asp Tyr Ile Leu Ser Glu Ile Lys Pro Leu His
   260 265 270
   Asn Phe Leu Thr Gln Leu Gln Pro Phe Leu Lys Leu Lys Gln Lys Gln
   275 280 285
   Ala Asn Leu Val Leu Lys Ile Ile Glu Gln Leu Pro Ser Ala Lys Glu
   290 295 300
   Ser Pro Asp Lys Phe Leu Glu Val Cys Thr Trp Val Asp Gln Ile Ala
   305 310 315 320
   Ala Leu Asn Asp Ser Lys Thr Arg Lys Thr Thr Ser Glu Thr Val Arg
   325 330 335
   Ala Val Leu Asp Ser Leu Ser Glu Lys Lys Lys Ser Ser Pro Ala Ala
   340 345 350
   Asp
<210> 72
   <211> 339
   <212> PRT
   <213> artificial sequence
<220>
   <223> hTevCre_D03_N20
<400> 72
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Lys Phe Cys Lys Cys Gly Val Arg Ile Gln Thr Ser
   145                 150                 155                 160
   Ala Tyr Thr Cys Ser Lys Cys Arg Asn Gln Gly Pro Ser Gly Asn Thr
                   165                 170                 175
   Lys Tyr Asn Lys Glu Phe Leu Leu Tyr Leu Ala Gly Phe Val Asp Gly
               180                 185                 190
   Asn Gly Ser Ile Ile Ala Gln Ile Lys Pro Asn Gln Ser Tyr Lys Phe
           195                 200                 205
   Lys His Gln Leu Ser Leu Thr Phe Gln Val Thr Gln Lys Thr Gln Arg
       210                 215                 220
   Arg Trp Phe Leu Asp Lys Leu Val Asp Glu Ile Gly Val Gly Tyr Val
   225                 230                 235                 240
   Arg Asp Arg Gly Ser Val Ser Asp Tyr Ile Leu Ser Glu Ile Lys Pro
   245 250 255
   Leu His Asn Phe Leu Thr Gln Leu Gln Pro Phe Leu Lys Leu Lys Gln
   260 265 270
   Lys Gln Ala Asn Leu Val Leu Lys Ile Ile Glu Gln Leu Pro Ser Ala
   275 280 285
   Lys Glu Ser Pro Asp Lys Phe Leu Glu Val Cys Thr Trp Val Asp Gln
   290 295 300
   Ile Ala Ala Leu Asn Asp Ser Lys Thr Arg Lys Thr Thr Ser Glu Thr
   305 310 315 320
   Val Arg Ala Val Leu Asp Ser Leu Ser Glu Lys Lys Lys Ser Ser Pro
   325 330 335
   Ala Ala Asp
<210> 73
   <211> 318
   <212> PRT
   <213> artificial sequence
<220>
   <223> hTevCre_D04_N20
<400> 73
   Met Lys Ser Gly Ile Tyr Gln Ile Lys Asn Thr Leu Asn Asn Lys Val
   1 5 10 15
   Tyr Val Gly Ser Ala Lys Asp Phe Glu Lys Arg Trp Lys Arg His Phe
   20 25 30
   Lys Asp Leu Glu Lys Gly Cys His Ser Ser Ile Lys Leu Gln Arg Ser
   35 40 45
   Phe Asn Lys His Gly Asn Val Phe Glu Cys Ser Ile Leu Glu Glu Ile
   50 55 60
   Pro Tyr Glu Lys Asp Leu Ile Ile Glu Arg Glu Asn Phe Trp Ile Lys
   65 70 75 80
   Glu Leu Asn Ser Lys Ile Asn Gly Tyr Asn Ile Ala Asp Ala Thr Phe
   85 90 95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Gln Gly Pro Ser Gly Asn Thr Lys Tyr Asn Lys Glu
   145                 150                 155                 160
   Phe Leu Leu Tyr Leu Ala Gly Phe Val Asp Gly Asn Gly Ser Ile Ile
                   165                 170                 175
   Ala Gln Ile Lys Pro Asn Gln Ser Tyr Lys Phe Lys His Gln Leu Ser
               180                 185                 190
   Leu Thr Phe Gln Val Thr Gln Lys Thr Gln Arg Arg Trp Phe Leu Asp
           195                 200                 205
   Lys Leu Val Asp Glu Ile Gly Val Gly Tyr Val Arg Asp Arg Gly Ser
       210                 215                 220
   Val Ser Asp Tyr Ile Leu Ser Glu Ile Lys Pro Leu His Asn Phe Leu
   225                 230                 235                 240
   Thr Gln Leu Gln Pro Phe Leu Lys Leu Lys Gln Lys Gln Ala Asn Leu
                   245                 250                 255
   Val Leu Lys Ile Ile Glu Gln Leu Pro Ser Ala Lys Glu Ser Pro Asp
               260                 265                 270
   Lys Phe Leu Glu Val Cys Thr Trp Val Asp Gln Ile Ala Ala Leu Asn
           275                 280                 285
   Asp Ser Lys Thr Arg Lys Thr Thr Ser Glu Thr Val Arg Ala Val Leu
       290                 295                 300
   Asp Ser Leu Ser Glu Lys Lys Lys Ser Ser Pro Ala Ala Asp
   305                 310                 315
<210> 74
   <211> 293
   <212> PRT
   <213> artificial sequence
<220>
   <223> hTevCre_D05_N20
<400> 74
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Gln Gly Pro Ser Gly
           115                 120                 125
   Asn Thr Lys Tyr Asn Lys Glu Phe Leu Leu Tyr Leu Ala Gly Phe Val
       130                 135                 140
   Asp Gly Asn Gly Ser Ile Ile Ala Gln Ile Lys Pro Asn Gln Ser Tyr
   145                 150                 155                 160
   Lys Phe Lys His Gln Leu Ser Leu Thr Phe Gln Val Thr Gln Lys Thr
                   165                 170                 175
   Gln Arg Arg Trp Phe Leu Asp Lys Leu Val Asp Glu Ile Gly Val Gly
               180                 185                 190
   Tyr Val Arg Asp Arg Gly Ser Val Ser Asp Tyr Ile Leu Ser Glu Ile
           195                 200                 205
   Lys Pro Leu His Asn Phe Leu Thr Gln Leu Gln Pro Phe Leu Lys Leu
       210                 215                 220
   Lys Gln Lys Gln Ala Asn Leu Val Leu Lys Ile Ile Glu Gln Leu Pro
   225                 230                 235                 240
   Ser Ala Lys Glu Ser Pro Asp Lys Phe Leu Glu Val Cys Thr Trp Val
   245 250 255
   Asp Gln Ile Ala Ala Leu Asn Asp Ser Lys Thr Arg Lys Thr Thr Ser
   260 265 270
   Glu Thr Val Arg Ala Val Leu Asp Ser Leu Ser Glu Lys Lys Lys Ser
   275 280 285
   Ser Pro Ala Ala Asp
   290
<210> 75
   <211> 296
   <212> PRT
   <213> artificial sequence
<220>
   <223> hTevCre_D06_N20
<400> 75
   Met Lys Ser Gly Ile Tyr Gln Ile Lys Asn Thr Leu Asn Asn Lys Val
   1 5 10 15
   Tyr Val Gly Ser Ala Lys Asp Phe Glu Lys Arg Trp Lys Arg His Phe
   20 25 30
   Lys Asp Leu Glu Lys Gly Cys His Ser Ser Ile Lys Leu Gln Arg Ser
   35 40 45
   Phe Asn Lys His Gly Asn Val Phe Glu Cys Ser Ile Leu Glu Glu Ile
   50 55 60
   Pro Tyr Glu Lys Asp Leu Ile Ile Glu Arg Glu Asn Phe Trp Ile Lys
   65 70 75 80
   Glu Leu Asn Ser Lys Ile Asn Gly Tyr Asn Ile Ala Asp Ala Thr Phe
   85 90 95
   Gly Asp Thr Cys Ser Thr His Pro Leu Lys Glu Glu Ile Ile Lys Lys
   100 105 110
   Arg Ser Glu Thr Val Lys Ala Lys Met Leu Lys Leu Gly Pro Asp Gly
   115 120 125
   Arg Lys Ala Asn Thr Lys Tyr Asn Lys Glu Phe Leu Leu Tyr Leu Ala
   130 135 140
   Gly Phe Val Asp Gly Asn Gly Ser Ile Ile Ala Gln Ile Lys Pro Asn
   145 150 155 160
   Gln Ser Tyr Lys Phe Lys His Gln Leu Ser Leu Thr Phe Gln Val Thr
   165 170 175
   Gln Lys Thr Gln Arg Arg Trp Phe Leu Asp Lys Leu Val Asp Glu Ile
   180 185 190
   Gly Val Gly Tyr Val Arg Asp Arg Gly Ser Val Ser Asp Tyr Ile Leu
   195 200 205
   Ser Glu Ile Lys Pro Leu His Asn Phe Leu Thr Gln Leu Gln Pro Phe
   210 215 220
   Leu Lys Leu Lys Gln Lys Gln Ala Asn Leu Val Leu Lys Ile Ile Glu
   225 230 235 240
   Gln Leu Pro Ser Ala Lys Glu Ser Pro Asp Lys Phe Leu Glu Val Cys
   245 250 255
   Thr Trp Val Asp Gln Ile Ala Ala Leu Asn Asp Ser Lys Thr Arg Lys
   260 265 270
   Thr Thr Ser Glu Thr Val Arg Ala Val Leu Asp Ser Leu Ser Glu Lys
   275 280 285
   Lys Lys Ser Ser Pro Ala Ala Asp
   290 295
<210> 76
   <211> 167
   <212> PRT
   <213> artificial sequence
<220>
   <223> I-CreI
<400> 76
   Met Ala Asn Thr Lys Tyr Asn Lys Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Arg Gly Ser Val Ser Asp Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Asp
                   165
<210> 77
   <211> 157
   <212> PRT
   <213> artificial sequence
<220>
   <223> I-CreI_X
<400> 77
   Met Ala Asn Thr Lys Tyr Asn Lys Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Arg Gly Ser Val Ser Asp Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Ala Asp
   145                 150                 155
<210> 78
   <211> 20
   <212> PRT
   <213> artificial sequence
<220>
   <223> NFS1 peptidic linker
<400> 78
   Gly Ser Asp Ile Thr Lys Ser Lys Ile Ser Glu Lys Met Lys Gly Gln
   1               5                   10                  15
   Gly Pro Ser Gly
               20
<210> 79
   <211> 23
   <212> PRT
   <213> artificial sequence
<220>
   <223> NFS2 peptidic linker
<400> 79
   Gly Ser Asp Ile Thr Lys Ser Lys Ile Ser Glu Lys Met Lys Gly Leu
   1               5                   10                  15
   Gly Pro Asp Gly Arg Lys Ala
               20
<210> 80
   <211> 10
   <212> PRT
   <213> artificial sequence
<220>
   <223> CFS1 peptidic linker
<400> 80
   Ser Leu Thr Lys Ser Lys Ile Ser Gly Ser
   1               5                   10
<210> 81
   <211> 177
   <212> PRT
   <213> artificial sequence
<220>
   <223> I-CreI_NFS1
<400> 81
   Met Ala Gly Ser Asp Ile Thr Lys Ser Lys Ile Ser Glu Lys Met Lys
   1               5                   10                  15
   Gly Gln Gly Pro Ser Gly Asn Thr Lys Tyr Asn Lys Glu Phe Leu Leu
               20                  25                  30
   Tyr Leu Ala Gly Phe Val Asp Gly Asp Gly Ser Ile Ile Ala Gln Ile
           35                  40                  45
   Lys Pro Asn Gln Ser Tyr Lys Phe Lys His Gln Leu Ser Leu Thr Phe
       50                  55                  60
   Gln Val Thr Gln Lys Thr Gln Arg Arg Trp Phe Leu Asp Lys Leu Val
   65                  70                  75                  80
   Asp Glu Ile Gly Val Gly Tyr Val Arg Asp Arg Gly Ser Val Ser Asp
                   85                  90                  95
   Tyr Ile Leu Ser Glu Ile Ala Pro Leu His Asn Phe Leu Thr Gln Leu
               100                 105                 110
   Gln Pro Phe Leu Lys Leu Lys Gln Lys Gln Ala Asn Leu Val Leu Lys
           115                 120                 125
   Ile Ile Glu Gln Leu Pro Ser Ala Lys Glu Ser Pro Asp Lys Phe Leu
       130                 135                 140
   Glu Val Cys Thr Trp Val Asp Gln Ile Ala Ala Leu Asn Asp Ser Lys
   145                 150                 155                 160
   Thr Arg Lys Thr Thr Ser Glu Thr Val Arg Ala Val Leu Asp Ser Ala
                   165                 170                 175
   Asp
<210> 82
   <211> 180
   <212> PRT
   <213> artificial sequence
<220>
   <223> I-CreI_NFS2
<400> 82
   Met Ala Gly Ser Asp Ile Thr Lys Ser Lys Ile Ser Glu Lys Met Lys
   1               5                   10                  15
   Gly Leu Gly Pro Asp Gly Arg Lys Ala Asn Thr Lys Tyr Asn Lys Glu
               20                  25                  30
   Phe Leu Leu Tyr Leu Ala Gly Phe Val Asp Gly Asp Gly Ser Ile Ile
           35                  40                  45
   Ala Gln Ile Lys Pro Asn Gln Ser Tyr Lys Phe Lys His Gln Leu Ser
       50                  55                  60
   Leu Thr Phe Gln Val Thr Gln Lys Thr Gln Arg Arg Trp Phe Leu Asp
   65                  70                  75                  80
   Lys Leu Val Asp Glu Ile Gly Val Gly Tyr Val Arg Asp Arg Gly Ser
                   85                  90                  95
   Val Ser Asp Tyr Ile Leu Ser Glu Ile Ala Pro Leu His Asn Phe Leu
               100                 105                 110
   Thr Gln Leu Gln Pro Phe Leu Lys Leu Lys Gln Lys Gln Ala Asn Leu
           115                 120                 125
   Val Leu Lys Ile Ile Glu Gln Leu Pro Ser Ala Lys Glu Ser Pro Asp
       130                 135                 140
   Lys Phe Leu Glu Val Cys Thr Trp Val Asp Gln Ile Ala Ala Leu Asn
   145                 150                 155                 160
   Asp Ser Lys Thr Arg Lys Thr Thr Ser Glu Thr Val Arg Ala Val Leu
                   165                 170                 175
   Asp Ser Ala Asp
               180
<210> 83
   <211> 166
   <212> PRT
   <213> artificial sequence
<220>
   <223> I-CreI_CFS1
<400> 83
   Met Ala Asn Thr Lys Tyr Asn Lys Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Arg Gly Ser Val Ser Asp Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Thr Lys Ser Lys
   145                 150                 155                 160
   Ile Ser Gly Ser Ala Asp
                   165
<210> 84
   <211> 141
   <212> PRT
   <213> artificial sequence
<220>
   <223> ColE7
<400> 84
   Met Ala Ser Gly Ser Lys Arg Asn Lys Pro Gly Lys Ala Thr Gly Lys
   1               5                   10                  15
   Gly Lys Pro Val Asn Asn Lys Trp Leu Asn Asn Ala Gly Lys Asp Leu
               20                  25                  30
   Gly Ser Pro Val Pro Asp Arg Ile Ala Asn Lys Leu Arg Asp Lys Glu
           35                  40                  45
   Phe Lys Ser Phe Asp Asp Phe Arg Lys Lys Phe Trp Glu Glu Val Ser
       50                  55                  60
   Lys Asp Pro Glu Leu Ser Lys Gln Phe Ser Arg Asn Asn Asn Asp Arg
   65                  70                  75                  80
   Met Lys Val Gly Lys Ala Pro Lys Thr Arg Thr Gln Asp Val Ser Gly
                   85                  90                  95
   Lys Arg Thr Ser Phe Glu Leu His His Glu Lys Pro Ile Ser Gln Asn
               100                 105                 110
   Gly Gly Val Tyr Asp Met Asp Asn Ile Ser Val Val Thr Pro Lys Arg
           115                 120                 125
   His Ile Asp Ile His Arg Gly Lys Gly Ser Ser Ala Asp
       130                 135                 140
<210> 85
   <211> 311
   <212> PRT
   <213> artificial sequence
<220>
   <223> hColE7Cre_D0101
<400> 85
   Met Ala Ser Gly Ser Lys Arg Asn Lys Pro Gly Lys Ala Thr Gly Lys
   1               5                   10                  15
   Gly Lys Pro Val Asn Asn Lys Trp Leu Asn Asn Ala Gly Lys Asp Leu
               20                  25                  30
   Gly Ser Pro Val Pro Asp Arg Ile Ala Asn Lys Leu Arg Asp Lys Glu
           35                  40                  45
   Phe Lys Ser Phe Asp Asp Phe Arg Lys Lys Phe Trp Glu Glu Val Ser
       50                  55                  60
   Lys Asp Pro Glu Leu Ser Lys Gln Phe Ser Arg Asn Asn Asn Asp Arg
   65                  70                  75                  80
   Met Lys Val Gly Lys Ala Pro Lys Thr Arg Thr Gln Asp Val Ser Gly
   85 90 95
   Lys Arg Thr Ser Phe Glu Leu His His Glu Lys Pro Ile Ser Gln Asn
   100 105 110
   Gly Gly Val Tyr Asp Met Asp Asn Ile Ser Val Val Thr Pro Lys Arg
   115 120 125
   His Ile Asp Ile His Arg Gly Lys Gly Ser Asp Ile Thr Lys Ser Lys
   130 135 140
   Ile Ser Glu Lys Met Lys Gly Gln Gly Pro Ser Gly Asn Thr Lys Tyr
   145 150 155 160
   Asn Lys Glu Phe Leu Leu Tyr Leu Ala Gly Phe Val Asp Gly Asp Gly
   165 170 175
   Ser Ile Ile Ala Gln Ile Lys Pro Asn Gln Ser Tyr Lys Phe Lys His
   180 185 190
   Gln Leu Ser Leu Thr Phe Gln Val Thr Gln Lys Thr Gln Arg Arg Trp
   195 200 205
   Phe Leu Asp Lys Leu Val Asp Glu Ile Gly Val Gly Tyr Val Arg Asp
   210 215 220
   Arg Gly Ser Val Ser Asp Tyr Ile Leu Ser Glu Ile Ala Pro Leu His
   225 230 235 240
   Asn Phe Leu Thr Gln Leu Gln Pro Phe Leu Lys Leu Lys Gln Lys Gln
   245 250 255
   Ala Asn Leu Val Leu Lys Ile Ile Glu Gln Leu Pro Ser Ala Lys Glu
   260 265 270
   Ser Pro Asp Lys Phe Leu Glu Val Cys Thr Trp Val Asp Gln Ile Ala
   275 280 285
   Ala Leu Asn Asp Ser Lys Thr Arg Lys Thr Thr Ser Glu Thr Val Arg
   290 295 300
   Ala Val Leu Asp Ser Ala Asp
   305 310
<210> 86
   <211> 314
   <212> PRT
   <213> artificial sequence
<220>
   <223> hColE7Cre_D0102
<400> 86
   Met Ala Ser Gly Ser Lys Arg Asn Lys Pro Gly Lys Ala Thr Gly Lys
   1               5                   10                  15
   Gly Lys Pro Val Asn Asn Lys Trp Leu Asn Asn Ala Gly Lys Asp Leu
               20                  25                  30
   Gly Ser Pro Val Pro Asp Arg Ile Ala Asn Lys Leu Arg Asp Lys Glu
           35                  40                  45
   Phe Lys Ser Phe Asp Asp Phe Arg Lys Lys Phe Trp Glu Glu Val Ser
       50                  55                  60
   Lys Asp Pro Glu Leu Ser Lys Gln Phe Ser Arg Asn Asn Asn Asp Arg
   65                  70                  75                  80
   Met Lys Val Gly Lys Ala Pro Lys Thr Arg Thr Gln Asp Val Ser Gly
                   85                  90                  95
   Lys Arg Thr Ser Phe Glu Leu His His Glu Lys Pro Ile Ser Gln Asn
               100                 105                 110
   Gly Gly Val Tyr Asp Met Asp Asn Ile Ser Val Val Thr Pro Lys Arg
           115                 120                 125
   His Ile Asp Ile His Arg Gly Lys Gly Ser Asp Ile Thr Lys Ser Lys
       130                 135                 140
   Ile Ser Glu Lys Met Lys Gly Leu Gly Pro Asp Gly Arg Lys Ala Asn
   145                 150                 155                 160
   Thr Lys Tyr Asn Lys Glu Phe Leu Leu Tyr Leu Ala Gly Phe Val Asp
                   165                 170                 175
   Gly Asp Gly Ser Ile Ile Ala Gln Ile Lys Pro Asn Gln Ser Tyr Lys
               180                 185                 190
   Phe Lys His Gln Leu Ser Leu Thr Phe Gln Val Thr Gln Lys Thr Gln
           195                 200                 205
   Arg Arg Trp Phe Leu Asp Lys Leu Val Asp Glu Ile Gly Val Gly Tyr
       210                 215                 220
   Val Arg Asp Arg Gly Ser Val Ser Asp Tyr Ile Leu Ser Glu Ile Ala
   225                 230                 235                 240
   Pro Leu His Asn Phe Leu Thr Gln Leu Gln Pro Phe Leu Lys Leu Lys
                   245                 250                 255
   Gln Lys Gln Ala Asn Leu Val Leu Lys Ile Ile Glu Gln Leu Pro Ser
               260                 265                 270
   Ala Lys Glu Ser Pro Asp Lys Phe Leu Glu Val Cys Thr Trp Val Asp
           275                 280                 285
   Gln Ile Ala Ala Leu Asn Asp Ser Lys Thr Arg Lys Thr Thr Ser Glu
       290                 295                 300
   Thr Val Arg Ala Val Leu Asp Ser Ala Asp
   305                 310
<210> 87
   <211> 300
   <212> PRT
   <213> artificial sequence
<220>
   <223> hCreColE7_D0101
<400> 87
   Met Ala Asn Thr Lys Tyr Asn Lys Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Arg Gly Ser Val Ser Asp Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Thr Lys Ser Lys
   145                 150                 155                 160
   Ile Ser Gly Ser Lys Arg Asn Lys Pro Gly Lys Ala Thr Gly Lys Gly
                   165                 170                 175
   Lys Pro Val Asn Asn Lys Trp Leu Asn Asn Ala Gly Lys Asp Leu Gly
               180                 185                 190
   Ser Pro Val Pro Asp Arg Ile Ala Asn Lys Leu Arg Asp Lys Glu Phe
           195                 200                 205
   Lys Ser Phe Asp Asp Phe Arg Lys Lys Phe Trp Glu Glu Val Ser Lys
       210                 215                 220
   Asp Pro Glu Leu Ser Lys Gln Phe Ser Arg Asn Asn Asn Asp Arg Met
   225                 230                 235                 240
   Lys Val Gly Lys Ala Pro Lys Thr Arg Thr Gln Asp Val Ser Gly Lys
                   245                 250                 255
   Arg Thr Ser Phe Glu Leu His His Glu Lys Pro Ile Ser Gln Asn Gly
               260                 265                 270
   Gly Val Tyr Asp Met Asp Asn Ile Ser Val Val Thr Pro Lys Arg His
           275                 280                 285
   Ile Asp Ile His Arg Gly Lys Gly Ser Ser Ala Asp
       290                 295                 300
<210> 88
   <211> 177
   <212> PRT
   <213> artificial sequence
<220>
   <223> I-CreI_NFS1_N20
<400> 88
   Met Ala Gly Ser Asp Ile Thr Lys Ser Lys Ile Ser Glu Lys Met Lys
   1               5                   10                  15
   Gly Gln Gly Pro Ser Gly Asn Thr Lys Tyr Asn Lys Glu Phe Leu Leu
               20                  25                  30
   Tyr Leu Ala Gly Phe Val Asp Gly Asn Gly Ser Ile Ile Ala Gln Ile
   35 40 45
   Lys Pro Asn Gln Ser Tyr Lys Phe Lys His Gln Leu Ser Leu Thr Phe
   50 55 60
   Gln Val Thr Gln Lys Thr Gln Arg Arg Trp Phe Leu Asp Lys Leu Val
   65 70 75 80
   Asp Glu Ile Gly Val Gly Tyr Val Arg Asp Arg Gly Ser Val Ser Asp
   85 90 95
   Tyr Ile Leu Ser Glu Ile Ala Pro Leu His Asn Phe Leu Thr Gln Leu
   100 105 110
   Gln Pro Phe Leu Lys Leu Lys Gln Lys Gln Ala Asn Leu Val Leu Lys
   115 120 125
   Ile Ile Glu Gln Leu Pro Ser Ala Lys Glu Ser Pro Asp Lys Phe Leu
   130 135 140
   Glu Val Cys Thr Trp Val Asp Gln Ile Ala Ala Leu Asn Asp Ser Lys
   145 150 155 160
   Thr Arg Lys Thr Thr Ser Glu Thr Val Arg Ala Val Leu Asp Ser Ala
   165 170 175
   Asp
<210> 89
   <211> 180
   <212> PRT
   <213> artificial sequence
<220>
   <223> I-CreI_NFS2_N20
<400> 89
   Met Ala Gly Ser Asp Ile Thr Lys Ser Lys Ile Ser Glu Lys Met Lys
   1 5 10 15
   Gly Leu Gly Pro Asp Gly Arg Lys Ala Asn Thr Lys Tyr Asn Lys Glu
   20 25 30
   Phe Leu Leu Tyr Leu Ala Gly Phe Val Asp Gly Asn Gly Ser Ile Ile
   35 40 45
   Ala Gln Ile Lys Pro Asn Gln Ser Tyr Lys Phe Lys His Gln Leu Ser
       50                  55                  60
   Leu Thr Phe Gln Val Thr Gln Lys Thr Gln Arg Arg Trp Phe Leu Asp
   65                  70                  75                  80
   Lys Leu Val Asp Glu Ile Gly Val Gly Tyr Val Arg Asp Arg Gly Ser
                   85                  90                  95
   Val Ser Asp Tyr Ile Leu Ser Glu Ile Ala Pro Leu His Asn Phe Leu
               100                 105                 110
   Thr Gln Leu Gln Pro Phe Leu Lys Leu Lys Gln Lys Gln Ala Asn Leu
           115                 120                 125
   Val Leu Lys Ile Ile Glu Gln Leu Pro Ser Ala Lys Glu Ser Pro Asp
       130                 135                 140
   Lys Phe Leu Glu Val Cys Thr Trp Val Asp Gln Ile Ala Ala Leu Asn
   145                 150                 155                 160
   Asp Ser Lys Thr Arg Lys Thr Thr Ser Glu Thr Val Arg Ala Val Leu
                   165                 170                 175
   Asp Ser Ala Asp
               180
<210> 90
   <211> 166
   <212> PRT
   <213> artificial sequence
<220>
   <223> I-CreI_CFS1_N20
<400> 90
   Met Ala Asn Thr Lys Tyr Asn Lys Glu Phe Leu Leu Tyr Leu Ala Gly
   1               5                   10                  15
   Phe Val Asp Gly Asn Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Arg Gly Ser Val Ser Asp Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Thr Lys Ser Lys
   145                 150                 155                 160
   Ile Ser Gly Ser Ala Asp
                   165
<210> 91
   <211> 311
   <212> PRT
   <213> artificial sequence
<220>
   <223> hColE7Cre_D0101_N20
<400> 91
   Met Ala Ser Gly Ser Lys Arg Asn Lys Pro Gly Lys Ala Thr Gly Lys
   1               5                   10                  15
   Gly Lys Pro Val Asn Asn Lys Trp Leu Asn Asn Ala Gly Lys Asp Leu
               20                  25                  30
   Gly Ser Pro Val Pro Asp Arg Ile Ala Asn Lys Leu Arg Asp Lys Glu
           35                  40                  45
   Phe Lys Ser Phe Asp Asp Phe Arg Lys Lys Phe Trp Glu Glu Val Ser
       50                  55                  60
   Lys Asp Pro Glu Leu Ser Lys Gln Phe Ser Arg Asn Asn Asn Asp Arg
   65                  70                  75                  80
   Met Lys Val Gly Lys Ala Pro Lys Thr Arg Thr Gln Asp Val Ser Gly
                   85                  90                  95
   Lys Arg Thr Ser Phe Glu Leu His His Glu Lys Pro Ile Ser Gln Asn
               100                 105                 110
   Gly Gly Val Tyr Asp Met Asp Asn Ile Ser Val Val Thr Pro Lys Arg
   115 120 125
   His Ile Asp Ile His Arg Gly Lys Gly Ser Asp Ile Thr Lys Ser Lys
   130 135 140
   Ile Ser Glu Lys Met Lys Gly Gln Gly Pro Ser Gly Asn Thr Lys Tyr
   145 150 155 160
   Asn Lys Glu Phe Leu Leu Tyr Leu Ala Gly Phe Val Asp Gly Asn Gly
   165 170 175
   Ser Ile Ile Ala Gln Ile Lys Pro Asn Gln Ser Tyr Lys Phe Lys His
   180 185 190
   Gln Leu Ser Leu Thr Phe Gln Val Thr Gln Lys Thr Gln Arg Arg Trp
   195 200 205
   Phe Leu Asp Lys Leu Val Asp Glu Ile Gly Val Gly Tyr Val Arg Asp
   210 215 220
   Arg Gly Ser Val Ser Asp Tyr Ile Leu Ser Glu Ile Ala Pro Leu His
   225 230 235 240
   Asn Phe Leu Thr Gln Leu Gln Pro Phe Leu Lys Leu Lys Gln Lys Gln
   245 250 255
   Ala Asn Leu Val Leu Lys Ile Ile Glu Gln Leu Pro Ser Ala Lys Glu
   260 265 270
   Ser Pro Asp Lys Phe Leu Glu Val Cys Thr Trp Val Asp Gln Ile Ala
   275 280 285
   Ala Leu Asn Asp Ser Lys Thr Arg Lys Thr Thr Ser Glu Thr Val Arg
   290 295 300
   Ala Val Leu Asp Ser Ala Asp
   305 310
<210> 92
   <211> 314
   <212> PRT
   <213> artificial sequence
<220>
   <223> hColE7Cre_D0102_N20
<400> 92
   Met Ala Ser Gly Ser Lys Arg Asn Lys Pro Gly Lys Ala Thr Gly Lys
   1               5                   10                  15
   Gly Lys Pro Val Asn Asn Lys Trp Leu Asn Asn Ala Gly Lys Asp Leu
               20                  25                  30
   Gly Ser Pro Val Pro Asp Arg Ile Ala Asn Lys Leu Arg Asp Lys Glu
           35                  40                  45
   Phe Lys Ser Phe Asp Asp Phe Arg Lys Lys Phe Trp Glu Glu Val Ser
       50                  55                  60
   Lys Asp Pro Glu Leu Ser Lys Gln Phe Ser Arg Asn Asn Asn Asp Arg
   65                  70                  75                  80
   Met Lys Val Gly Lys Ala Pro Lys Thr Arg Thr Gln Asp Val Ser Gly
                   85                  90                  95
   Lys Arg Thr Ser Phe Glu Leu His His Glu Lys Pro Ile Ser Gln Asn
               100                 105                 110
   Gly Gly Val Tyr Asp Met Asp Asn Ile Ser Val Val Thr Pro Lys Arg
           115                 120                 125
   His Ile Asp Ile His Arg Gly Lys Gly Ser Asp Ile Thr Lys Ser Lys
       130                 135                 140
   Ile Ser Glu Lys Met Lys Gly Leu Gly Pro Asp Gly Arg Lys Ala Asn
   145                 150                 155                 160
   Thr Lys Tyr Asn Lys Glu Phe Leu Leu Tyr Leu Ala Gly Phe Val Asp
                   165                 170                 175
   Gly Asn Gly Ser Ile Ile Ala Gln Ile Lys Pro Asn Gln Ser Tyr Lys
               180                 185                 190
   Phe Lys His Gln Leu Ser Leu Thr Phe Gln Val Thr Gln Lys Thr Gln
           195                 200                 205
   Arg Arg Trp Phe Leu Asp Lys Leu Val Asp Glu Ile Gly Val Gly Tyr
       210                 215                 220
   Val Arg Asp Arg Gly Ser Val Ser Asp Tyr Ile Leu Ser Glu Ile Ala
   225                 230                 235                 240
   Pro Leu His Asn Phe Leu Thr Gln Leu Gln Pro Phe Leu Lys Leu Lys
                   245                 250                 255
   Gln Lys Gln Ala Asn Leu Val Leu Lys Ile Ile Glu Gln Leu Pro Ser
   260 265 270
   Ala Lys Glu Ser Pro Asp Lys Phe Leu Glu Val Cys Thr Trp Val Asp
   275 280 285
   Gln Ile Ala Ala Leu Asn Asp Ser Lys Thr Arg Lys Thr Thr Ser Glu
   290 295 300
   Thr Val Arg Ala Val Leu Asp Ser Ala Asp
   305 310
<210> 93
   <211> 300
   <212> PRT
   <213> artificial sequence
<220>
   <223> hCreColE7_D0101_N20
<400> 93
   Met Ala Asn Thr Lys Tyr Asn Lys Glu Phe Leu Leu Tyr Leu Ala Gly
   1 5 10 15
   Phe Val Asp Gly Asn Gly Ser Ile Ile Ala Gln Ile Lys Pro Asn Gln
   20 25 30
   Ser Tyr Lys Phe Lys His Gln Leu Ser Leu Thr Phe Gln Val Thr Gln
   35 40 45
   Lys Thr Gln Arg Arg Trp Phe Leu Asp Lys Leu Val Asp Glu Ile Gly
   50 55 60
   Val Gly Tyr Val Arg Asp Arg Gly Ser Val Ser Asp Tyr Ile Leu Ser
   65 70 75 80
   Glu Ile Lys Pro Leu His Asn Phe Leu Thr Gln Leu Gln Pro Phe Leu
   85 90 95
   Lys Leu Lys Gln Lys Gln Ala Asn Leu Val Leu Lys Ile Ile Glu Gln
   100 105 110
   Leu Pro Ser Ala Lys Glu Ser Pro Asp Lys Phe Leu Glu Val Cys Thr
   115 120 125
   Trp Val Asp Gln Ile Ala Ala Leu Asn Asp Ser Lys Thr Arg Lys Thr
   130 135 140
   Thr Ser Glu Thr Val Arg Ala Val Leu Asp Ser Leu Thr Lys Ser Lys
   145                 150                 155                 160
   Ile Ser Gly Ser Lys Arg Asn Lys Pro Gly Lys Ala Thr Gly Lys Gly
                   165                 170                 175
   Lys Pro Val Asn Asn Lys Trp Leu Asn Asn Ala Gly Lys Asp Leu Gly
               180                 185                 190
   Ser Pro Val Pro Asp Arg Ile Ala Asn Lys Leu Arg Asp Lys Glu Phe
           195                 200                 205
   Lys Ser Phe Asp Asp Phe Arg Lys Lys Phe Trp Glu Glu Val Ser Lys
       210                 215                 220
   Asp Pro Glu Leu Ser Lys Gln Phe Ser Arg Asn Asn Asn Asp Arg Met
   225                 230                 235                 240
   Lys Val Gly Lys Ala Pro Lys Thr Arg Thr Gln Asp Val Ser Gly Lys
                   245                 250                 255
   Arg Thr Ser Phe Glu Leu His His Glu Lys Pro Ile Ser Gln Asn Gly
               260                 265                 270
   Gly Val Tyr Asp Met Asp Asn Ile Ser Val Val Thr Pro Lys Arg His
           275                 280                 285
   Ile Asp Ile His Arg Gly Lys Gly Ser Ser Ala Asp
       290                 295                 300
<210> 94
   <211> 32
   <212> PRT
   <213> artificial sequence
<220>
   <223> RM2 peptidic linker
<400> 94
   Ala Ala Gly Gly Ser Ala Leu Thr Ala Gly Ala Leu Ser Leu Thr Ala
   1               5                   10                  15
   Gly Ala Leu Ser Leu Thr Ala Gly Ala Leu Ser Gly Gly Gly Gly Ser
               20                  25                  30
<210> 95
   <211> 27
   <212> PRT
   <213> artificial sequence
<220>
   <223> BQY peptidic linker
<400> 95
   Ala Ala Gly Ala Ser Ser Val Ser Ala Ser Gly His Ile Ala Pro Leu
   1               5                   10                  15
   Ser Leu Pro Ser Ser Pro Pro Ser Val Gly Ser
               20                  25
<210> 96
   <211> 919
   <212> PRT
   <213> Methylophilus methylotrophus
<220>
   <223> MmeI ACC85607.1
<400> 96
   Met Ala Leu Ser Trp Asn Glu Ile Arg Arg Lys Ala Ile Glu Phe Ser
   1               5                   10                  15
   Lys Arg Trp Glu Asp Ala Ser Asp Glu Asn Ser Gln Ala Lys Pro Phe
               20                  25                  30
   Leu Ile Asp Phe Phe Glu Val Phe Gly Ile Thr Asn Lys Arg Val Ala
           35                  40                  45
   Thr Phe Glu His Ala Val Lys Lys Phe Ala Lys Ala His Lys Glu Gln
       50                  55                  60
   Ser Arg Gly Phe Val Asp Leu Phe Trp Pro Gly Ile Leu Leu Ile Glu
   65                  70                  75                  80
   Met Lys Ser Arg Gly Lys Asp Leu Asp Lys Ala Tyr Asp Gln Ala Leu
                   85                  90                  95
   Asp Tyr Phe Ser Gly Ile Ala Glu Arg Asp Leu Pro Arg Tyr Val Leu
               100                 105                 110
   Val Cys Asp Phe Gln Arg Phe Arg Leu Thr Asp Leu Ile Thr Lys Glu
           115                 120                 125
   Ser Val Glu Phe Leu Leu Lys Asp Leu Tyr Gln Asn Val Arg Ser Phe
       130                 135                 140
   Gly Phe Ile Ala Gly Tyr Gln Thr Gln Val Ile Lys Pro Gln Asp Pro
   145                 150                 155                 160
   Ile Asn Ile Lys Ala Ala Glu Arg Met Gly Lys Leu His Asp Thr Leu
                   165                 170                 175
   Lys Leu Val Gly Tyr Glu Gly His Ala Leu Glu Leu Tyr Leu Val Arg
               180                 185                 190
   Leu Leu Phe Cys Leu Phe Ala Glu Asp Thr Thr Ile Phe Glu Lys Ser
           195                 200                 205
   Leu Phe Gln Glu Tyr Ile Glu Thr Lys Thr Leu Glu Asp Gly Ser Asp
       210                 215                 220
   Leu Ala His His Ile Asn Thr Leu Phe Tyr Val Leu Asn Thr Pro Glu
   225                 230                 235                 240
   Gln Lys Arg Leu Lys Asn Leu Asp Glu His Leu Ala Ala Phe Pro Tyr
                   245                 250                 255
   Ile Asn Gly Lys Leu Phe Glu Glu Pro Leu Pro Pro Ala Gln Phe Asp
               260                 265                 270
   Lys Ala Met Arg Glu Ala Leu Leu Asp Leu Cys Ser Leu Asp Trp Ser
           275                 280                 285
   Arg Ile Ser Pro Ala Ile Phe Gly Ser Leu Phe Gln Ser Ile Met Asp
       290                 295                 300
   Ala Lys Lys Arg Arg Asn Leu Gly Ala His Tyr Thr Ser Glu Ala Asn
   305                 310                 315                 320
   Ile Leu Lys Leu Ile Lys Pro Leu Phe Leu Asp Glu Leu Trp Val Glu
                   325                 330                 335
   Phe Glu Lys Val Lys Asn Asn Lys Asn Lys Leu Leu Ala Phe His Lys
               340                 345                 350
   Lys Leu Arg Gly Leu Thr Phe Phe Asp Pro Ala Cys Gly Cys Gly Asn
           355                 360                 365
   Phe Leu Val Ile Thr Tyr Arg Glu Leu Arg Leu Leu Glu Ile Glu Val
       370                 375                 380
   Leu Arg Gly Leu His Arg Gly Gly Gln Gln Val Leu Asp Ile Glu His
   385                 390                 395                 400
   Leu Ile Gln Ile Asn Val Asp Gln Phe Phe Gly Ile Glu Ile Glu Glu
                   405                 410                 415
   Phe Pro Ala Gln Ile Ala Gln Val Ala Leu Trp Leu Thr Asp His Gln
               420                 425                 430
   Met Asn Met Lys Ile Ser Asp Glu Phe Gly Asn Tyr Phe Ala Arg Ile
           435                 440                 445
   Pro Leu Lys Ser Thr Pro His Ile Leu Asn Ala Asn Ala Leu Gln Ile
       450                 455                 460
   Asp Trp Asn Asp Val Leu Glu Ala Lys Lys Cys Cys Phe Ile Leu Gly
   465                 470                 475                 480
   Asn Pro Pro Phe Val Gly Lys Ser Lys Gln Thr Pro Gly Gln Lys Ala
                   485                 490                 495
   Asp Leu Leu Ser Val Phe Gly Asn Leu Lys Ser Ala Ser Asp Leu Asp
               500                 505                 510
   Leu Val Ala Ala Trp Tyr Pro Lys Ala Ala His Tyr Ile Gln Thr Asn
           515                 520                 525
   Ala Asn Ile Arg Cys Ala Phe Val Ser Thr Asn Ser Ile Thr Gln Gly
       530                 535                 540
   Glu Gln Val Ser Leu Leu Trp Pro Leu Leu Leu Ser Leu Gly Ile Lys
   545                 550                 555                 560
   Ile Asn Phe Ala His Arg Thr Phe Ser Trp Thr Asn Glu Ala Ser Gly
                   565                 570                 575
   Val Ala Ala Val His Cys Val Ile Ile Gly Phe Gly Leu Lys Asp Ser
               580                 585                 590
   Asp Glu Lys Ile Ile Tyr Glu Tyr Glu Ser Ile Asn Gly Glu Pro Leu
           595                 600                 605
   Ala Ile Lys Ala Lys Asn Ile Asn Pro Tyr Leu Arg Asp Gly Val Asp
       610                 615                 620
   Val Ile Ala Cys Lys Arg Gln Gln Pro Ile Ser Lys Leu Pro Ser Met
   625                 630                 635                 640
   Arg Tyr Gly Asn Lys Pro Thr Asp Asp Gly Asn Phe Leu Phe Thr Asp
                   645                 650                 655
   Glu Glu Lys Asn Gln Phe Ile Thr Asn Glu Pro Ser Ser Glu Lys Tyr
   660 665 670
   Phe Arg Arg Phe Val Gly Gly Asp Glu Phe Ile Asn Asn Thr Ser Arg
   675 680 685
   Trp Cys Leu Trp Leu Asp Gly Ala Asp Ile Ser Glu Ile Arg Ala Met
   690 695 700
   Pro Leu Val Leu Ala Arg Ile Lys Lys Val Gln Glu Phe Arg Leu Lys
   705 710 715 720
   Ser Ser Ala Lys Pro Thr Arg Gln Ser Ala Ser Thr Pro Met Lys Phe
   725 730 735
   Phe Tyr Ile Ser Gln Pro Asp Thr Asp Tyr Leu Leu Ile Pro Glu Thr
   740 745 750
   Ser Ser Glu Asn Arg Gln Phe Ile Pro Ile Gly Phe Val Asp Arg Asn
   755 760 765
   Val Ile Ser Ser Asn Ala Thr Tyr His Ile Pro Ser Ala Glu Pro Leu
   770 775 780
   Ile Phe Gly Leu Leu Ser Ser Thr Met His Asn Cys Trp Met Arg Asn
   785 790 795 800
   Val Gly Gly Arg Leu Glu Ser Arg Tyr Arg Tyr Ser Ala Ser Leu Val
   805 810 815
   Tyr Asn Thr Phe Pro Trp Ile Gln Pro Asn Glu Lys Gln Ser Lys Ala
   820 825 830
   Ile Glu Glu Ala Ala Phe Ala Ile Leu Lys Ala Arg Ser Asn Tyr Pro
   835 840 845
   Asn Glu Ser Leu Ala Gly Leu Tyr Asp Pro Lys Thr Met Pro Ser Glu
   850 855 860
   Leu Leu Lys Ala His Gln Lys Leu Asp Lys Ala Val Asp Ser Val Tyr
   865 870 875 880
   Gly Phe Lys Gly Pro Asn Thr Glu Ile Ala Arg Ile Ala Phe Leu Phe
   885 890 895
   Glu Thr Tyr Gln Lys Met Thr Ser Leu Leu Pro Pro Glu Lys Glu Ile
   900 905 910
   Lys Lys Ser Lys Gly Lys Asn
   915
<210> 97
   <211> 576
   <212> PRT
   <213> CEA7_ECOLX
<220>
   <223> Colicin-E7 (CEA7_ECOLX) Q47112.2
<400> 97
   Met Ser Gly Gly Asp Gly Arg Gly His Asn Ser Gly Ala His Asn Thr
   1               5                   10                  15
   Gly Gly Asn Ile Asn Gly Gly Pro Thr Gly Leu Gly Gly Asn Gly Gly
               20                  25                  30
   Ala Ser Asp Gly Ser Gly Trp Ser Ser Glu Asn Asn Pro Trp Gly Gly
           35                  40                  45
   Gly Ser Gly Ser Gly Val His Trp Gly Gly Gly Ser Gly His Gly Asn
       50                  55                  60
   Gly Gly Gly Asn Ser Asn Ser Gly Gly Gly Ser Asn Ser Ser Val Ala
   65                  70                  75                  80
   Ala Pro Met Ala Phe Gly Phe Pro Ala Leu Ala Ala Pro Gly Ala Gly
                   85                  90                  95
   Thr Leu Gly Ile Ser Val Ser Gly Glu Ala Leu Ser Ala Ala Ile Ala
               100                 105                 110
   Asp Ile Phe Ala Ala Leu Lys Gly Pro Phe Lys Phe Ser Ala Trp Gly
           115                 120                 125
   Ile Ala Leu Tyr Gly Ile Leu Pro Ser Glu Ile Ala Lys Asp Asp Pro
       130                 135                 140
   Asn Met Met Ser Lys Ile Val Thr Ser Leu Pro Ala Glu Thr Val Thr
   145                 150                 155                 160
   Asn Val Gln Val Ser Thr Leu Pro Leu Asp Gln Ala Thr Val Ser Val
                   165                 170                 175
   Thr Lys Arg Val Thr Asp Val Val Lys Asp Thr Arg Gln His Ile Ala
               180                 185                 190
   Val Val Ala Gly Val Pro Met Ser Val Pro Val Val Asn Ala Lys Pro
           195                 200                 205
   Thr Arg Thr Pro Gly Val Phe His Ala Ser Phe Pro Gly Val Pro Ser
       210                 215                 220
   Leu Thr Val Ser Thr Val Lys Gly Leu Pro Val Ser Thr Thr Leu Pro
   225                 230                 235                 240
   Arg Gly Ile Thr Glu Asp Lys Gly Arg Thr Ala Val Pro Ala Gly Phe
                   245                 250                 255
   Thr Phe Gly Gly Gly Ser His Glu Ala Val Ile Arg Phe Pro Lys Glu
               260                 265                 270
   Ser Gly Gln Lys Pro Val Tyr Val Ser Val Thr Asp Val Leu Thr Pro
           275                 280                 285
   Ala Gln Val Lys Gln Arg Gln Asp Glu Glu Lys Arg Leu Gln Gln Glu
       290                 295                 300
   Trp Asn Asp Ala His Pro Val Glu Val Ala Glu Arg Asn Tyr Glu Gln
   305                 310                 315                 320
   Ala Arg Ala Glu Leu Asn Gln Ala Asn Lys Asp Val Ala Arg Asn Gln
                   325                 330                 335
   Glu Arg Gln Ala Lys Ala Val Gln Val Tyr Asn Ser Arg Lys Ser Glu
               340                 345                 350
   Leu Asp Ala Ala Asn Lys Thr Leu Ala Asp Ala Lys Ala Glu Ile Lys
           355                 360                 365
   Gln Phe Glu Arg Phe Ala Arg Glu Pro Met Ala Ala Gly His Arg Met
       370                 375                 380
   Trp Gln Met Ala Gly Leu Lys Ala Gln Arg Ala Gln Thr Asp Val Asn
   385                 390                 395                 400
   Asn Lys Lys Ala Ala Phe Asp Ala Ala Ala Lys Glu Lys Ser Asp Ala
                   405                 410                 415
   Asp Val Ala Leu Ser Ser Ala Leu Glu Arg Arg Lys Gln Lys Glu Asn
               420                 425                 430
   Lys Glu Lys Asp Ala Lys Ala Lys Leu Asp Lys Glu Ser Lys Arg Asn
           435                 440                 445
   Lys Pro Gly Lys Ala Thr Gly Lys Gly Lys Pro Val Asn Asn Lys Trp
   450 455 460
   Leu Asn Asn Ala Gly Lys Asp Leu Gly Ser Pro Val Pro Asp Arg Ile
   465 470 475 480
   Ala Asn Lys Leu Arg Asp Lys Glu Phe Lys Ser Phe Asp Asp Phe Arg
   485 490 495
   Lys Lys Phe Trp Glu Glu Val Ser Lys Asp Pro Glu Leu Ser Lys Gln
   500 505 510
   Phe Ser Arg Asn Asn Asn Asp Arg Met Lys Val Gly Lys Ala Pro Lys
   515 520 525
   Thr Arg Thr Gln Asp Val Ser Gly Lys Arg Thr Ser Phe Glu Leu His
   530 535 540
   His Glu Lys Pro Ile Ser Gln Asn Gly Gly Val Tyr Asp Met Asp Asn
   545 550 555 560
   Ile Ser Val Val Thr Pro Lys Arg His Ile Asp Ile His Arg Gly Lys
   565 570 575
<210> 98
   <211> 274
   <212> PRT
   <213> Streptococcus pneumoniae
<220>
   <223> End A CAA38134.1
<400> 98
<210> 99
   <211> 235
   <212> PRT
   <213> Escherichia coli
<220>
   <223> Endo I (END1_ECOLI) P25736.1
<400> 99
   Met Tyr Arg Tyr Leu Ser Ile Ala Ala Val Val Leu Ser Ala Ala Phe
   1 5 10 15
   Ser Gly Pro Ala Leu Ala Glu Gly Ile Asn Ser Phe Ser Gln Ala Lys
               20                  25                  30
   Ala Ala Ala Val Lys Val His Ala Asp Ala Pro Gly Thr Phe Tyr Cys
           35                  40                  45
   Gly Cys Lys Ile Asn Trp Gln Gly Lys Lys Gly Val Val Asp Leu Gln
       50                  55                  60
   Ser Cys Gly Tyr Gln Val Arg Lys Asn Glu Asn Arg Ala Ser Arg Val
   65                  70                  75                  80
   Glu Trp Glu His Val Val Pro Ala Trp Gln Phe Gly His Gln Arg Gln
                   85                  90                  95
   Cys Trp Gln Asp Gly Gly Arg Lys Asn Cys Ala Lys Asp Pro Val Tyr
               100                 105                 110
   Arg Lys Met Glu Ser Asp Met His Asn Leu Gln Pro Ser Val Gly Glu
           115                 120                 125
   Val Asn Gly Asp Arg Gly Asn Phe Met Tyr Ser Gln Trp Asn Gly Gly
       130                 135                 140
   Glu Gly Gln Tyr Gly Gln Cys Ala Met Lys Val Asp Phe Lys Glu Lys
   145                 150                 155                 160
   Ala Ala Glu Pro Pro Ala Arg Ala Arg Gly Ala Ile Ala Arg Thr Tyr
                   165                 170                 175
   Phe Tyr Met Arg Asp Gln Tyr Asn Leu Thr Leu Ser Arg Gln Gln Thr
               180                 185                 190
   Gln Leu Phe Asn Ala Trp Asn Lys Met Tyr Pro Val Thr Asp Trp Glu
           195                 200                 205
   Cys Glu Arg Asp Glu Arg Ile Ala Lys Val Gln Gly Asn His Asn Pro
       210                 215                 220
   Tyr Val Gln Arg Ala Cys Gln Ala Arg Lys Ser
   225                 230                 235
<210> 100
   <211> 297
   <212> PRT
   <213> Homo sapiens
<220>
   <223> Human Endo G (NUCG_HUMAN) Q14249.4
<400> 100
   Met Arg Ala Leu Arg Ala Gly Leu Thr Leu Ala Ser Gly Ala Gly Leu
   1               5                   10                  15
   Gly Ala Val Val Glu Gly Trp Arg Arg Arg Arg Glu Asp Ala Arg Ala
               20                  25                  30
   Ala Pro Gly Leu Leu Gly Arg Leu Pro Val Leu Pro Val Ala Ala Ala
           35                  40                  45
   Ala Glu Leu Pro Pro Val Pro Gly Gly Pro Arg Gly Pro Gly Glu Leu
       50                  55                  60
   Ala Lys Tyr Gly Leu Pro Gly Leu Ala Gln Leu Lys Ser Arg Glu Ser
   65                  70                  75                  80
   Tyr Val Leu Cys Tyr Asp Pro Arg Thr Arg Gly Ala Leu Trp Val Val
                   85                  90                  95
   Glu Gln Leu Arg Pro Glu Arg Leu Arg Gly Asp Gly Asp Arg Arg Glu
               100                 105                 110
   Cys Asp Phe Arg Glu Asp Asp Ser Val His Ala Tyr His Arg Ala Thr
           115                 120                 125
   Asn Ala Asp Tyr Arg Gly Ser Gly Phe Asp Arg Gly His Leu Ala Ala
       130                 135                 140
   Ala Ala Asn His Arg Trp Ser Gln Lys Ala Met Asp Asp Thr Phe Tyr
   145                 150                 155                 160
   Leu Ser Asn Val Ala Pro Gln Val Pro His Leu Asn Gln Asn Ala Trp
                   165                 170                 175
   Asn Asn Leu Glu Lys Tyr Ser Arg Ser Leu Thr Arg Ser Tyr Gln Asn
               180                 185                 190
   Val Tyr Val Cys Thr Gly Pro Leu Phe Leu Pro Arg Thr Glu Ala Asp
           195                 200                 205
   Gly Lys Ser Tyr Val Lys Tyr Gln Val Ile Gly Lys Asn His Val Ala
       210                 215                 220
   Val Pro Thr His Phe Phe Lys Val Leu Ile Leu Glu Ala Ala Gly Gly
   225                 230                 235                 240
   Gln Ile Glu Leu Arg Thr Tyr Val Met Pro Asn Ala Pro Val Asp Glu
                   245                 250                 255
   Ala Ile Pro Leu Glu Arg Phe Leu Val Pro Ile Glu Ser Ile Glu Arg
               260                 265                 270
   Ala Ser Gly Leu Leu Phe Val Pro Asn Ile Leu Ala Arg Ala Gly Ser
           275                 280                 285
   Leu Lys Ala Ile Thr Ala Gly Ser Lys
       290                 295
<210> 101
   <211> 299
   <212> PRT
   <213> Bos taurus
<220>
   <223> Bovine Endo G (NUCG_BOVIN) P38447.1
<400> 101
   Met Gln Leu Leu Arg Ala Gly Leu Thr Leu Ala Leu Gly Ala Gly Leu
   1               5                   10                  15
   Gly Ala Ala Ala Glu Ser Trp Trp Arg Gln Arg Ala Asp Ala Arg Ala
               20                  25                  30
   Thr Pro Gly Leu Leu Ser Arg Leu Pro Val Leu Pro Val Ala Ala Ala
           35                  40                  45
   Ala Gly Leu Pro Ala Val Pro Gly Ala Pro Ala Gly Gly Gly Pro Gly
       50                  55                  60
   Glu Leu Ala Lys Tyr Gly Leu Pro Gly Val Ala Gln Leu Lys Ser Arg
   65                  70                  75                  80
   Ala Ser Tyr Val Leu Cys Tyr Asp Pro Arg Thr Arg Gly Ala Leu Trp
                   85                  90                  95
   Val Val Glu Gln Leu Arg Pro Glu Gly Leu Arg Gly Asp Gly Asn Arg
               100                 105                 110
   Ser Ser Cys Asp Phe His Glu Asp Asp Ser Val His Ala Tyr His Arg
           115                 120                 125
   Ala Thr Asn Ala Asp Tyr Arg Gly Ser Gly Phe Asp Arg Gly His Leu
       130                 135                 140
   Ala Ala Ala Ala Asn His Arg Trp Ser Gln Lys Ala Met Asp Asp Thr
   145                 150                 155                 160
   Phe Tyr Leu Ser Asn Val Ala Pro Gln Val Pro His Leu Asn Gln Asn
                   165                 170                 175
   Ala Trp Asn Asn Leu Glu Lys Tyr Ser Arg Ser Leu Thr Arg Thr Tyr
               180                 185                 190
   Gln Asn Val Tyr Val Cys Thr Gly Pro Leu Phe Leu Pro Arg Thr Glu
           195                 200                 205
   Ala Asp Gly Lys Ser Tyr Val Lys Tyr Gln Val Ile Gly Lys Asn His
       210                 215                 220
   Val Ala Val Pro Thr His Phe Phe Lys Val Leu Ile Leu Glu Ala Ala
   225                 230                 235                 240
   Gly Gly Gln Ile Glu Leu Arg Ser Tyr Val Met Pro Asn Ala Pro Val
                   245                 250                 255
   Asp Glu Ala Ile Pro Leu Glu His Phe Leu Val Pro Ile Glu Ser Ile
               260                 265                 270
   Glu Arg Ala Ser Gly Leu Leu Phe Val Pro Asn Ile Leu Ala Arg Ala
           275                 280                 285
   Gly Ser Leu Lys Ala Ile Thr Ala Gly Ser Lys
       290                 295
<210> 102
   <211> 247
   <212> PRT
   <213> Haemophilus influenzae
<220>
   <223> R.HinP1I AAW33811.1
<400> 102
   Met Asn Leu Val Glu Leu Gly Ser Lys Thr Ala Lys Asp Gly Phe Lys
   1               5                   10                  15
   Asn Glu Lys Asp Ile Ala Asp Arg Phe Glu Asn Trp Lys Glu Asn Ser
               20                  25                  30
   Glu Ala Gln Asp Trp Leu Val Thr Met Gly His Asn Leu Asp Glu Ile
           35                  40                  45
   Lys Ser Val Lys Ala Val Val Leu Ser Gly Tyr Lys Ser Asp Ile Asn
       50                  55                  60
   Val Gln Val Leu Val Phe Tyr Lys Asp Ala Leu Asp Ile His Asn Ile
   65                  70                  75                  80
   Gln Val Lys Leu Val Ser Asn Lys Arg Gly Phe Asn Gln Ile Asp Lys
                   85                  90                  95
   His Trp Leu Ala His Tyr Gln Glu Met Trp Lys Phe Asp Asp Asn Leu
               100                 105                 110
   Leu Arg Ile Leu Arg His Phe Thr Gly Glu Leu Pro Pro Tyr His Ser
           115                 120                 125
   Asn Thr Lys Asp Lys Arg Arg Met Phe Met Thr Glu Phe Ser Gln Glu
       130                 135                 140
   Glu Gln Asn Ile Val Leu Asn Trp Leu Glu Lys Asn Arg Val Leu Val
   145                 150                 155                 160
   Leu Thr Asp Ile Leu Arg Gly Arg Gly Asp Phe Ala Ala Glu Trp Val
                   165                 170                 175
   Leu Val Ala Gln Lys Val Ser Asn Asn Ala Arg Trp Ile Leu Arg Asn
               180                 185                 190
   Ile Asn Glu Val Leu Gln His Tyr Gly Ser Gly Asp Ile Ser Leu Ser
           195                 200                 205
   Pro Arg Gly Ser Ile Asn Phe Gly Arg Val Thr Ile Gln Arg Lys Gly
       210                 215                 220
   Gly Asp Asn Gly Arg Glu Thr Ala Asn Met Leu Gln Phe Lys Ile Asp
   225                 230                 235                 240
   Pro Thr Glu Leu Phe Asp Ile
                   245
<210> 103
   <211> 188
   <212> PRT
   <213> Bacillus phage Bastille
<220>
   <223> I-BasI AAO93095.1
<400> 103
   Met Phe Gln Glu Glu Trp Lys Asp Val Thr Gly Phe Glu Asp Tyr Tyr
   1 5 10 15
   Glu Val Ser Asn Lys Gly Arg Val Ala Ser Lys Arg Thr Gly Val Ile
   20 25 30
   Met Ala Gln Tyr Lys Ile Asn Ser Gly Tyr Leu Cys Ile Lys Phe Thr
   35 40 45
   Val Asn Lys Lys Arg Thr Ser His Leu Val His Arg Leu Val Ala Arg
   50 55 60
   Glu Phe Cys Glu Gly Tyr Ser Pro Glu Leu Asp Val Asn His Lys Asp
   65 70 75 80
   Thr Asp Arg Met Asn Asn Asn Tyr Asp Asn Leu Glu Trp Leu Thr Arg
   85 90 95
   Ala Asp Asn Leu Lys Asp Val Arg Glu Arg Gly Lys Leu Asn Thr His
   100 105 110
   Thr Ala Arg Glu Ala Leu Ala Lys Val Ser Lys Lys Ala Val Asp Val
   115 120 125
   Tyr Thr Lys Asp Gly Ser Glu Tyr Ile Ala Thr Tyr Pro Ser Ala Thr
   130 135 140
   Glu Ala Ala Glu Ala Leu Gly Val Gln Gly Ala Lys Ile Ser Thr Val
   145 150 155 160
   Cys His Gly Lys Arg Gln His Thr Gly Gly Tyr His Phe Lys Phe Asn
   165 170 175
   Ser Ser Val Asp Pro Asn Arg Ser Val Ser Lys Lys
   180 185
<210> 104
   <211> 266
   <212> PRT
   <213> Bacillus mojavensis
<220>
   <223> I-BmoI AAK09365.1
<400> 104
   Met Lys Ser Gly Val Tyr Lys Ile Thr Asn Lys Asn Thr Gly Lys Phe
   1               5                   10                  15
   Tyr Ile Gly Ser Ser Glu Asp Cys Glu Ser Arg Leu Lys Val His Phe
               20                  25                  30
   Arg Asn Leu Lys Asn Asn Arg His Ile Asn Arg Tyr Leu Asn Asn Ser
   35 40 45
   Phe Asn Lys His Gly Glu Gln Val Phe Ile Gly Glu Val Ile His Ile
   50 55 60
   Leu Pro Ile Glu Glu Ala Ile Ala Lys Glu Gln Trp Tyr Ile Asp Asn
   65 70 75 80
   Phe Tyr Glu Glu Met Tyr Asn Ile Ser Lys Ser Ala Tyr His Gly Gly
   85 90 95
   Asp Leu Thr Ser Tyr His Pro Asp Lys Arg Asn Ile Ile Leu Lys Arg
   100 105 110
   Ala Asp Ser Leu Lys Lys Val Tyr Leu Lys Met Thr Ser Glu Glu Lys
   115 120 125
   Ala Lys Arg Trp Gln Cys Val Gln Gly Glu Asn Asn Pro Met Phe Gly
   130 135 140
   Arg Lys His Thr Glu Thr Thr Lys Leu Lys Ile Ser Asn His Asn Lys
   145 150 155 160
   Leu Tyr Tyr Ser Thr His Lys Asn Pro Phe Lys Gly Lys Lys His Ser
   165 170 175
   Glu Glu Ser Lys Thr Lys Leu Ser Glu Tyr Ala Ser Gln Arg Val Gly
   180 185 190
   Glu Lys Asn Pro Phe Tyr Gly Lys Thr His Ser Asp Glu Phe Lys Thr
   195 200 205
   Tyr Met Ser Lys Lys Phe Lys Gly Arg Lys Pro Lys Asn Ser Arg Pro
   210 215 220
   Val Ile Ile Asp Gly Thr Glu Tyr Glu Ser Ala Thr Glu Ala Ser Arg
   225 230 235 240
   Gln Leu Asn Val Val Pro Ala Thr Ile Leu His Arg Ile Lys Ser Lys
   245 250 255
   Asn Glu Lys Tyr Ser Gly Tyr Phe Tyr Lys
   260 265
<210> 105
   <211> 174
   <212> PRT
   <213> HMUI_BPSP1
<220>
   <223> I-HmuI P34081.1
<400> 105
   Met Glu Trp Lys Asp Ile Lys Gly Tyr Glu Gly His Tyr Gln Val Ser
   1               5                   10                  15
   Asn Thr Gly Glu Val Tyr Ser Ile Lys Ser Gly Lys Thr Leu Lys His
               20                  25                  30
   Gln Ile Pro Lys Asp Gly Tyr His Arg Ile Gly Leu Phe Lys Gly Gly
           35                  40                  45
   Lys Gly Lys Thr Phe Gln Val His Arg Leu Val Ala Ile His Phe Cys
       50                  55                  60
   Glu Gly Tyr Glu Glu Gly Leu Val Val Asp His Lys Asp Gly Asn Lys
   65                  70                  75                  80
   Asp Asn Asn Leu Ser Thr Asn Leu Arg Trp Val Thr Gln Lys Ile Asn
                   85                  90                  95
   Val Glu Asn Gln Met Ser Arg Gly Thr Leu Asn Val Ser Lys Ala Gln
               100                 105                 110
   Gln Ile Ala Lys Ile Lys Asn Gln Lys Pro Ile Ile Val Ile Ser Pro
           115                 120                 125
   Asp Gly Ile Glu Lys Glu Tyr Pro Ser Thr Lys Cys Ala Cys Glu Glu
       130                 135                 140
   Leu Gly Leu Thr Arg Gly Lys Val Thr Asp Val Leu Lys Gly His Arg
   145                 150                 155                 160
   Ile His His Lys Gly Tyr Thr Phe Arg Tyr Lys Leu Asn Gly
                   165                 170
<210> 106
   <211> 245
   <212> PRT
   <213> TEV1_BPT4
<220>
   <223> I-TevI P13299.2
<400> 106
   Met Lys Ser Gly Ile Tyr Gln Ile Lys Asn Thr Leu Asn Asn Lys Val
   1               5                   10                  15
   Tyr Val Gly Ser Ala Lys Asp Phe Glu Lys Arg Trp Lys Arg His Phe
               20                  25                  30
   Lys Asp Leu Glu Lys Gly Cys His Ser Ser Ile Lys Leu Gln Arg Ser
           35                  40                  45
   Phe Asn Lys His Gly Asn Val Phe Glu Cys Ser Ile Leu Glu Glu Ile
       50                  55                  60
   Pro Tyr Glu Lys Asp Leu Ile Ile Glu Arg Glu Asn Phe Trp Ile Lys
   65                  70                  75                  80
   Glu Leu Asn Ser Lys Ile Asn Gly Tyr Asn Ile Ala Asp Ala Thr Phe
                   85                  90                  95
   Gly Asp Thr Cys Ser Thr His Pro Leu Lys Glu Glu Ile Ile Lys Lys
               100                 105                 110
   Arg Ser Glu Thr Val Lys Ala Lys Met Leu Lys Leu Gly Pro Asp Gly
           115                 120                 125
   Arg Lys Ala Leu Tyr Ser Lys Pro Gly Ser Lys Asn Gly Arg Trp Asn
       130                 135                 140
   Pro Glu Thr His Lys Phe Cys Lys Cys Gly Val Arg Ile Gln Thr Ser
   145                 150                 155                 160
   Ala Tyr Thr Cys Ser Lys Cys Arg Asn Arg Ser Gly Glu Asn Asn Ser
                   165                 170                 175
   Phe Phe Asn His Lys His Ser Asp Ile Thr Lys Ser Lys Ile Ser Glu
               180                 185                 190
   Lys Met Lys Gly Lys Lys Pro Ser Asn Ile Lys Lys Ile Ser Cys Asp
           195                 200                 205
   Gly Val Ile Phe Asp Cys Ala Ala Asp Ala Ala Arg His Phe Lys Ile
       210                 215                 220
   Ser Ser Gly Leu Val Thr Tyr Arg Val Lys Ser Asp Lys Trp Asn Trp
   225                 230                 235                 240
   Phe Tyr Ile Asn Ala
                   245
<210> 107
   <211> 258
   <212> PRT
   <213> TEV2_BPT4
<220>
   <223> I-TevII P07072.2
<400> 107
   Met Lys Trp Lys Leu Arg Lys Ser Leu Lys Ile Ala Asn Ser Val Ala
   1               5                   10                  15
   Phe Thr Tyr Met Val Arg Phe Pro Asp Lys Ser Phe Tyr Ile Gly Phe
               20                  25                  30
   Lys Lys Phe Lys Thr Ile Tyr Gly Lys Asp Thr Asn Trp Lys Glu Tyr
           35                  40                  45
   Asn Ser Ser Ser Lys Leu Val Lys Glu Lys Leu Lys Asp Tyr Lys Ala
       50                  55                  60
   Lys Trp Ile Ile Leu Gln Val Phe Asp Ser Tyr Glu Ser Ala Leu Lys
   65                  70                  75                  80
   His Glu Glu Met Leu Ile Arg Lys Tyr Phe Asn Asn Glu Phe Ile Leu
                   85                  90                  95
   Asn Lys Ser Ile Gly Gly Tyr Lys Phe Asn Lys Tyr Pro Asp Ser Glu
               100                 105                 110
   Glu His Lys Gln Lys Leu Ser Asn Ala His Lys Gly Lys Ile Leu Ser
           115                 120                 125
   Leu Lys His Lys Asp Lys Ile Arg Glu Lys Leu Ile Glu His Tyr Lys
       130                 135                 140
   Asn Asn Ser Arg Ser Glu Ala His Val Lys Asn Asn Ile Gly Ser Arg
   145                 150                 155                 160
   Thr Ala Lys Lys Thr Val Ser Ile Ala Leu Lys Ser Gly Asn Lys Phe
                   165                 170                 175
   Arg Ser Phe Lys Ser Ala Ala Lys Phe Leu Lys Cys Ser Glu Glu Gln
               180                 185                 190
   Val Ser Asn His Pro Asn Val Ile Asp Ile Lys Ile Thr Ile His Pro
           195                 200                 205
   Val Pro Glu Tyr Val Lys Ile Asn Asp Asn Ile Tyr Lys Ser Phe Val
       210                 215                 220
   Asp Ala Ala Lys Asp Leu Lys Leu His Pro Ser Arg Ile Lys Asp Leu
   225                 230                 235                 240
   Cys Leu Asp Asp Asn Tyr Pro Asn Tyr Ile Val Ser Tyr Lys Arg Val
                   245                 250                 255
   Glu Lys
<210> 108
   <211> 269
   <212> PRT
   <213> TEV3_BPR03
<220>
   <223> I-TevIII Q38419.1
<400> 108
   Met Asn Tyr Arg Lys Ile Trp Ile Asp Ala Asn Gly Pro Ile Pro Lys
   1               5                   10                  15
   Asp Ser Asp Gly Arg Thr Asp Glu Ile His His Lys Asp Gly Asn Arg
               20                  25                  30
   Glu Asn Asn Asp Leu Asp Asn Leu Met Cys Leu Ser Ile Gln Glu His
           35                  40                  45
   Tyr Asp Ile His Leu Ala Gln Lys Asp Tyr Gln Ala Cys His Ala Ile
       50                  55                  60
   Lys Leu Arg Met Lys Tyr Ser Pro Glu Glu Ile Ser Glu Leu Ala Ser
   65                  70                  75                  80
   Lys Ala Ala Lys Ser Arg Glu Ile Gln Ile Phe Asn Ile Pro Glu Val
                   85                  90                  95
   Arg Ala Lys Asn Ile Ala Ser Ile Lys Ser Lys Ile Glu Asn Gly Thr
               100                 105                 110
   Phe His Leu Leu Asp Gly Glu Ile Gln Arg Lys Ser Asn Leu Asn Arg
           115                 120                 125
   Val Ala Leu Gly Ile His Asn Phe Gln Gln Ala Glu His Ile Ala Lys
       130                 135                 140
   Val Lys Glu Arg Asn Ile Ala Ala Ile Lys Glu Gly Thr His Val Phe
   145 150 155 160
   Cys Gly Gly Lys Met Gln Ser Glu Thr Gln Ser Lys Arg Val Asn Asp
   165 170 175
   Gly Ser His His Phe Leu Ser Glu Asp His Lys Lys Arg Thr Ser Ala
   180 185 190
   Lys Thr Leu Glu Met Val Lys Asn Gly Thr His Pro Ala Gln Lys Glu
   195 200 205
   Ile Thr Cys Asp Phe Cys Gly His Ile Gly Lys Gly Pro Gly Phe Tyr
   210 215 220
   Leu Lys His Asn Asp Arg Cys Lys Leu Asn Pro Asn Arg Ile Gln Leu
   225 230 235 240
   Asn Cys Pro Tyr Cys Asp Lys Lys Asp Leu Ser Pro Ser Thr Tyr Lys
   245 250 255
   Arg Trp His Gly Asp Asn Cys Lys Ala Arg Phe Asn Asp
   260 265
<210> 109
   <211> 243
   <212> PRT
   <213> Staphylococcus phage Twort
<220>
   <223> I-TwoI AAM00817.1
<400> 109
   Met Glu Glu Leu Trp Lys Glu Ile Pro Gly Phe Asn Ser Tyr Met Ile
   1               5                   10                  15
   Ser Asn Lys Gly Gln Val Tyr Ser Arg Lys Arg Asn Lys Ile Leu Ala
               20                  25                  30
   Leu Arg Thr Asp Lys Asn Gly Tyr Lys Arg Ile Ser Ile Phe Asn Asn
           35                  40                  45
   Glu Gly Lys Arg Ile Leu Leu Gly Val His Lys Leu Val Leu Leu Gly
       50                  55                  60
   Phe Lys Gly Ile Asn Thr Glu Lys Pro Ile Pro His His Lys Asn Asn
   65                  70                  75                  80
   Ile Lys Asp Asp Asn Arg Leu Glu Asn Leu Glu Trp Val Thr Val Ser
                   85                  90                  95
   Glu Asn Thr Lys His Ala Tyr Asp Ile Gly Ala Leu Lys Ser Pro Arg
               100                 105                 110
   Arg Val Thr Cys Thr Leu Tyr Tyr Lys Gly Glu Pro Leu Ser Cys Tyr
           115                 120                 125
   Asp Ser Leu Phe Asp Leu Ala Lys Ala Leu Lys Val Ser Arg Ser Val
       130                 135                 140
   Ile Glu Ser Pro Arg Asn Gly Leu Val Leu Ser Thr Phe Glu Val Lys
   145                 150                 155                 160
   Arg Glu Pro Thr Ile Gln Gly Leu Pro Leu Asn Lys Glu Ile Phe Glu
                   165                 170                 175
   His Ser Leu Ile Lys Gly Leu Gly Asn Pro Pro Leu Lys Val Tyr Asn
               180                 185                 190
   Glu Asp Glu Thr Tyr Tyr Phe Leu Thr Leu Met Asp Ile Ser Lys Tyr
           195                 200                 205
   Phe Asn Glu Ser Tyr Ser Lys Val Gln Arg Gly Tyr Tyr Lys Gly Lys
       210                 215                 220
   Trp Lys Ser Tyr Ile Ile Glu His Ile Asp Phe Tyr Glu Tyr Tyr Lys
   225                 230                 235                 240
   Gln Thr His
<210> 110
   <211> 262
   <212> PRT
   <213> T2M1_MORSP
<220>
   <223> R.MspI P11405.1
<400> 110
   Met Arg Thr Glu Leu Leu Ser Lys Leu Tyr Asp Asp Phe Gly Ile Asp
   1               5                   10                  15
   Gln Leu Pro His Thr Gln His Gly Val Thr Ser Asp Arg Leu Gly Lys
               20                  25                  30
   Leu Tyr Glu Lys Tyr Ile Leu Asp Ile Phe Lys Asp Ile Glu Ser Leu
           35                  40                  45
   Lys Lys Tyr Asn Thr Asn Ala Phe Pro Gln Glu Lys Asp Ile Ser Ser
       50                  55                  60
   Lys Leu Leu Lys Ala Leu Asn Leu Asp Leu Asp Asn Ile Ile Asp Val
   65                  70                  75                  80
   Ser Ser Ser Asp Thr Asp Leu Gly Arg Thr Ile Ala Gly Gly Ser Pro
                   85                  90                  95
   Lys Thr Asp Ala Thr Ile Arg Phe Thr Phe His Asn Gln Ser Ser Arg
               100                 105                 110
   Leu Val Pro Leu Asn Ile Lys His Ser Ser Lys Lys Lys Val Ser Ile
           115                 120                 125
   Ala Glu Tyr Asp Val Glu Thr Ile Cys Thr Gly Val Gly Ile Ser Asp
       130                 135                 140
   Gly Glu Leu Lys Glu Leu Ile Arg Lys His Gln Asn Asp Gln Ser Ala
   145                 150                 155                 160
   Lys Leu Phe Thr Pro Val Gln Lys Gln Arg Leu Thr Glu Leu Leu Glu
                   165                 170                 175
   Pro Tyr Arg Glu Arg Phe Ile Arg Trp Cys Val Thr Leu Arg Ala Glu
               180                 185                 190
   Lys Ser Glu Gly Asn Ile Leu His Pro Asp Leu Leu Ile Arg Phe Gln
           195                 200                 205
   Val Ile Asp Arg Glu Tyr Val Asp Val Thr Ile Lys Asn Ile Asp Asp
       210                 215                 220
   Tyr Val Ser Asp Arg Ile Ala Glu Gly Ser Lys Ala Arg Lys Pro Gly
   225                 230                 235                 240
   Phe Gly Thr Gly Leu Asn Trp Thr Tyr Ala Ser Gly Ser Lys Ala Lys
                   245                 250                 255
   Lys Met Gln Phe Lys Gly
               260
<210> 111
   <211> 246
   <212> PRT
   <213> Kocuria varians
<220>
   <223> R.MvaI
<400> 111
   Met Ser Glu Tyr Leu Asn Leu Leu Lys Glu Ala Ile Gln Asn Val Val
   1               5                   10                  15
   Asp Gly Gly Trp His Glu Thr Lys Arg Lys Gly Asn Thr Gly Ile Gly
               20                  25                  30
   Lys Thr Phe Glu Asp Leu Leu Glu Lys Glu Glu Asp Asn Leu Asp Ala
           35                  40                  45
   Pro Asp Phe His Asp Ile Glu Ile Lys Thr His Glu Thr Ala Ala Lys
       50                  55                  60
   Ser Leu Leu Thr Leu Phe Thr Lys Ser Pro Thr Asn Pro Arg Gly Ala
   65                  70                  75                  80
   Asn Thr Met Leu Arg Asn Arg Tyr Gly Lys Lys Asp Glu Tyr Gly Asn
                   85                  90                  95
   Asn Ile Leu His Gln Thr Val Ser Gly Asn Arg Lys Thr Asn Ser Asn
               100                 105                 110
   Ser Tyr Asn Tyr Asp Phe Lys Ile Asp Ile Asp Trp Glu Ser Gln Val
           115                 120                 125
   Val Arg Leu Glu Val Phe Asp Lys Gln Asp Ile Met Ile Asp Asn Ser
       130                 135                 140
   Val Tyr Trp Ser Phe Asp Ser Leu Gln Asn Gln Leu Asp Lys Lys Leu
   145                 150                 155                 160
   Lys Tyr Ile Ala Val Ile Ser Ala Glu Ser Lys Ile Glu Asn Glu Lys
                   165                 170                 175
   Lys Tyr Tyr Lys Tyr Asn Ser Ala Asn Leu Phe Thr Asp Leu Thr Val
               180                 185                 190
   Gln Ser Leu Cys Arg Gly Ile Glu Asn Gly Asp Ile Lys Val Asp Ile
           195                 200                 205
   Arg Ile Gly Ala Tyr His Ser Gly Lys Lys Lys Gly Lys Thr His Asp
       210                 215                 220
   His Gly Thr Ala Phe Arg Ile Asn Met Glu Lys Leu Leu Glu Tyr Gly
   225                 230                 235                 240
   Glu Val Lys Val Ile Val
               245
<210> 112
   <211> 274
   <212> PRT
   <213> Nostoc sp. PCC 7120
<220>
   <223> NucA CAA45962.1
<400> 112
   Met Gly Ile Cys Gly Lys Leu Gly Val Ala Ala Leu Val Ala Leu Ile
   1               5                   10                  15
   Val Gly Cys Ser Pro Val Gln Ser Gln Val Pro Pro Leu Thr Glu Leu
               20                  25                  30
   Ser Pro Ser Ile Ser Val His Leu Leu Leu Gly Asn Pro Ser Gly Ala
           35                  40                  45
   Thr Pro Thr Lys Leu Thr Pro Asp Asn Tyr Leu Met Val Lys Asn Gln
       50                  55                  60
   Tyr Ala Leu Ser Tyr Asn Asn Ser Lys Gly Thr Ala Asn Trp Val Ala
   65                  70                  75                  80
   Trp Gln Leu Asn Ser Ser Trp Leu Gly Asn Ala Glu Arg Gln Asp Asn
                   85                  90                  95
   Phe Arg Pro Asp Lys Thr Leu Pro Ala Gly Trp Val Arg Val Thr Pro
               100                 105                 110
   Ser Met Tyr Ser Gly Ser Gly Tyr Asp Arg Gly His Ile Ala Pro Ser
           115                 120                 125
   Ala Asp Arg Thr Lys Thr Thr Glu Asp Asn Ala Ala Thr Phe Leu Met
       130                 135                 140
   Thr Asn Met Met Pro Gln Thr Pro Asp Asn Asn Arg Asn Thr Trp Gly
   145                 150                 155                 160
   Asn Leu Glu Asp Tyr Cys Arg Glu Leu Val Ser Gln Gly Lys Glu Leu
                   165                 170                 175
   Tyr Ile Val Ala Gly Pro Asn Gly Ser Leu Gly Lys Pro Leu Lys Gly
               180                 185                 190
   Lys Val Thr Val Pro Lys Ser Thr Trp Lys Ile Val Val Val Leu Asp
           195                 200                 205
   Ser Pro Gly Ser Gly Leu Glu Gly Ile Thr Ala Asn Thr Arg Val Ile
       210                 215                 220
   Ala Val Asn Ile Pro Asn Asp Pro Glu Leu Asn Asn Asp Trp Arg Ala
   225                 230                 235                 240
   Tyr Lys Val Ser Val Asp Glu Leu Glu Ser Leu Thr Gly Tyr Asp Phe
                   245                 250                 255
   Leu Ser Asn Val Ser Pro Asn Ile Gln Thr Ser Ile Glu Ser Lys Val
               260                 265                 270
   Asp Asn
<210> 113
   <211> 232
   <212> PRT
   <213> NUCM_DICD3
<220>
   <223> NucM P37994.2
<400> 113
   Met Leu Arg Asn Leu Val Ile Phe Ala Val Leu Gly Ala Gly Leu Thr
   1               5                   10                  15
   Thr Leu Ala Ala Ala Gly Gln Asp Ile Asn Asn Phe Thr Gln Ala Lys
               20                  25                  30
   Ala Ala Ala Ala Lys Ile His Gln Asp Ala Pro Gly Thr Phe Tyr Cys
           35                  40                  45
   Gly Cys Lys Ile Asn Trp Gln Gly Lys Lys Gly Thr Pro Asp Leu Ala
       50                  55                  60
   Ser Cys Gly Tyr Gln Val Arg Lys Asp Ala Asn Arg Ala Ser Arg Ile
   65                  70                  75                  80
   Glu Trp Glu His Val Val Pro Ala Trp Gln Phe Gly His Gln Arg Gln
                   85                  90                  95
   Cys Trp Gln Asp Gly Gly Arg Lys Asn Cys Thr Lys Asp Asp Val Tyr
               100                 105                 110
   Arg Gln Ile Glu Thr Asp Leu His Asn Leu Gln Pro Ala Ile Gly Glu
           115                 120                 125
   Val Asn Gly Asp Arg Gly Asn Phe Met Tyr Ser Gln Trp Asn Gly Gly
       130                 135                 140
   Glu Arg Gln Tyr Gly Gln Cys Glu Met Lys Ile Asp Phe Lys Ser Gln
   145                 150                 155                 160
   Leu Ala Glu Pro Pro Glu Arg Ala Arg Gly Ala Ile Ala Arg Thr Tyr
                   165                 170                 175
   Phe Tyr Met Arg Asp Arg Tyr Asn Leu Asn Leu Ser Arg Gln Gln Thr
               180                 185                 190
   Gln Leu Phe Asp Ala Trp Asn Lys Gln Tyr Pro Ala Thr Thr Trp Glu
           195                 200                 205
   Cys Thr Arg Glu Lys Arg Ile Ala Ala Val Gln Gly Asn His Asn Pro
       210                 215                 220
   Tyr Val Gln Gln Ala Cys Gln Pro
   225                 230
<210> 114
   <211> 231
   <212> PRT
   <213> Vibrio vulnificus
<220>
   <223> Vvn AAF19759.1
<400> 114
   Met Lys Arg Leu Phe Ile Phe Ile Ala Ser Phe Thr Ala Phe Ala Ile
   1               5                   10                  15
   Gln Ala Ala Pro Pro Ser Ser Phe Ser Ala Ala Lys Gln Gln Ala Val
               20                  25                  30
   Lys Ile Tyr Gln Asp His Pro Ile Ser Phe Tyr Cys Gly Cys Asp Ile
           35                  40                  45
   Glu Trp Gln Gly Lys Lys Gly Ile Pro Asn Leu Glu Thr Cys Gly Tyr
       50                  55                  60
   Gln Val Arg Lys Gln Gln Thr Arg Ala Ser Arg Ile Glu Trp Glu His
   65                  70                  75                  80
   Val Val Pro Ala Trp Gln Phe Gly His His Arg Gln Cys Trp Gln Lys
                   85                  90                  95
   Gly Gly Arg Lys Asn Cys Ser Lys Asn Asp Gln Gln Phe Arg Leu Met
   100 105 110
   Glu Ala Asp Leu His Asn Leu Thr Pro Ala Ile Gly Glu Val Asn Gly
   115 120 125
   Asp Arg Ser Asn Phe Asn Phe Ser Gln Trp Asn Gly Val Asp Gly Val
   130 135 140
   Ser Tyr Gly Arg Cys Glu Met Gln Val Asn Phe Lys Gln Arg Lys Val
   145 150 155 160
   Met Pro Gln Thr Glu Leu Arg Gly Ser Ile Ala Arg Thr Tyr Leu Tyr
   165 170 175
   Met Ser Gln Glu Tyr Gly Phe Gln Leu Ser Lys Gln Gln Gln Gln Leu
   180 185 190
   Met Gln Ala Trp Asn Lys Ser Tyr Pro Val Asp Glu Trp Glu Cys Thr
   195 200 205
   Arg Asp Asp Arg Ile Ala Lys Ile Gln Gly Asn His Asn Pro Phe Val
   210 215 220
   Gln Gln Ser Cys Gln Thr Gln
   225 230
<210> 115
   <211> 222
   <212> PRT
   <213> artificial sequence
<220>
   <223> Vvn_CLS
<400> 115
   Met Ala Ser Gly Ala Pro Pro Ser Ser Phe Ser Ala Ala Lys Gln Gln
   1 5 10 15
   Ala Val Lys Ile Tyr Gln Asp His Pro Ile Ser Phe Tyr Cys Gly Cys
   20 25 30
   Asp Ile Glu Trp Gln Gly Lys Lys Gly Ile Pro Asn Leu Glu Thr Cys
   35 40 45
   Gly Tyr Gln Val Arg Lys Gln Gln Thr Arg Ala Ser Arg Ile Glu Trp
   50 55 60
   Glu His Val Val Pro Ala Trp Gln Phe Gly His His Arg Gln Cys Trp
   65 70 75 80
   Gln Lys Gly Gly Arg Lys Asn Cys Ser Lys Asn Asp Gln Gln Phe Arg
   85 90 95
   Leu Met Glu Ala Asp Leu His Asn Leu Thr Pro Ala Ile Gly Glu Val
   100 105 110
   Asn Gly Asp Arg Ser Asn Phe Asn Phe Ser Gln Trp Asn Gly Val Asp
   115 120 125
   Gly Val Ser Tyr Gly Arg Cys Glu Met Gln Val Asn Phe Lys Gln Arg
   130 135 140
   Lys Val Met Pro Pro Asp Arg Ala Arg Gly Ser Ile Ala Arg Thr Tyr
   145 150 155 160
   Leu Tyr Met Ser Gln Glu Tyr Gly Phe Gln Leu Ser Lys Gln Gln Gln
   165 170 175
   Gln Leu Met Gln Ala Trp Asn Lys Ser Tyr Pro Val Asp Glu Trp Glu
   180 185 190
   Cys Thr Arg Asp Asp Arg Ile Ala Lys Ile Gln Gly Asn His Asn Pro
   195 200 205
   Phe Val Gln Gln Ser Cys Gln Thr Gln Gly Ser Ser Ala Asp
   210 215 220
<210> 116
   <211> 231
   <212> PRT
   <213> NUC_STAAU
<220>
   <223> Staphylococcal nuclease (NUC_STAAU) P00644.1
<400> 116
   Met Leu Val Met Thr Glu Tyr Leu Leu Ser Ala Gly Ile Cys Met Ala
   1               5                   10                  15
   Ile Val Ser Ile Leu Leu Ile Gly Met Ala Ile Ser Asn Val Ser Lys
               20                  25                  30
   Gly Gln Tyr Ala Lys Arg Phe Phe Phe Phe Ala Thr Ser Cys Leu Val
           35                  40                  45
   Leu Thr Leu Val Val Val Ser Ser Leu Ser Ser Ser Ala Asn Ala Ser
       50                  55                  60
   Gln Thr Asp Asn Gly Val Asn Arg Ser Gly Ser Glu Asp Pro Thr Val
   65                  70                  75                  80
   Tyr Ser Ala Thr Ser Thr Lys Lys Leu His Lys Glu Pro Ala Thr Leu
                   85                  90                  95
   Ile Lys Ala Ile Asp Gly Asp Thr Val Lys Leu Met Tyr Lys Gly Gln
               100                 105                 110
   Pro Met Thr Phe Arg Leu Leu Leu Val Asp Thr Pro Glu Thr Lys His
           115                 120                 125
   Pro Lys Lys Gly Val Glu Lys Tyr Gly Pro Glu Ala Ser Ala Phe Thr
       130                 135                 140
   Lys Lys Met Val Glu Asn Ala Lys Lys Ile Glu Val Glu Phe Asp Lys
   145                 150                 155                 160
   Gly Gln Arg Thr Asp Lys Tyr Gly Arg Gly Leu Ala Tyr Ile Tyr Ala
                   165                 170                 175
   Asp Gly Lys Met Val Asn Glu Ala Leu Val Arg Gln Gly Leu Ala Lys
               180                 185                 190
   Val Ala Tyr Val Tyr Lys Pro Asn Asn Thr His Glu Gln His Leu Arg
           195                 200                 205
   Lys Ser Glu Ala Gln Ala Lys Lys Glu Lys Leu Asn Ile Trp Ser Glu
       210                 215                 220
   Asp Asn Ala Asp Ser Gly Gln
   225                 230
<210> 117
   <211> 169
   <212> PRT
   <213> NUC_STAHY
<220>
   <223> Staphylococcal nuclease (NUC_STAHY) P43270.1
<400> 117
   Met Lys Lys Ile Thr Thr Gly Leu Ile Ile Val Val Ala Ala Ile Ile
   1               5                   10                  15
   Val Leu Ser Ile Gln Phe Met Thr Glu Ser Gly Pro Phe Lys Ser Ala
               20                  25                  30
   Gly Leu Ser Asn Ala Asn Glu Gln Thr Tyr Lys Val Ile Arg Val Ile
           35                  40                  45
   Asp Gly Asp Thr Ile Ile Val Asp Lys Asp Gly Lys Gln Gln Asn Leu
       50                  55                  60
   Arg Met Ile Gly Val Asp Thr Pro Glu Thr Val Lys Pro Asn Thr Pro
   65                  70                  75                  80
   Val Gln Pro Tyr Gly Lys Glu Ala Ser Asp Phe Thr Lys Arg His Leu
                   85                  90                  95
   Thr Asn Gln Lys Val Arg Leu Glu Tyr Asp Lys Gln Glu Lys Asp Arg
               100                 105                 110
   Tyr Gly Arg Thr Leu Ala Tyr Val Trp Leu Gly Lys Glu Met Phe Asn
           115                 120                 125
   Glu Lys Leu Ala Lys Glu Gly Leu Ala Arg Ala Lys Phe Tyr Arg Pro
       130                 135                 140
   Asn Tyr Lys Tyr Gln Glu Arg Ile Glu Gln Ala Gln Lys Gln Ala Gln
   145                 150                 155                 160
   Lys Leu Lys Lys Asn Ile Trp Ser Asn
                   165
<210> 118
   <211> 174
   <212> PRT
   <213> NUC_SHIFL
<220>
   <223> Micrococcal nuclease (NUC_SHIFL)P29769.1
<400> 118
   Met Lys Ser Ala Leu Ala Ala Leu Arg Ala Val Ala Ala Ala Val Val
   1               5                   10                  15
   Leu Ile Val Ser Val Pro Ala Trp Ala Asp Phe Arg Gly Glu Val Val
               20                  25                  30
   Arg Ile Leu Asp Gly Asp Thr Ile Asp Val Leu Val Asn Arg Gln Thr
           35                  40                  45
   Ile Arg Val Arg Leu Ala Asp Ile Asp Ala Pro Glu Ser Gly Gln Ala
       50                  55                  60
   Phe Gly Ser Arg Ala Arg Gln Arg Leu Ala Asp Leu Thr Phe Arg Gln
   65                  70                  75                  80
   Glu Val Gln Val Thr Glu Lys Glu Val Asp Arg Tyr Gly Arg Thr Leu
                   85                  90                  95
   Gly Val Val Tyr Ala Pro Leu Gln Tyr Pro Gly Gly Gln Thr Gln Leu
               100                 105                 110
   Thr Asn Ile Asn Ala Ile Met Val Gln Glu Gly Met Ala Trp Ala Tyr
           115                 120                 125
   Arg Tyr Tyr Gly Lys Pro Thr Asp Ala Gln Met Tyr Glu Tyr Glu Lys
       130                 135                 140
   Glu Ala Arg Arg Gln Arg Leu Gly Leu Trp Ser Asp Pro Asn Ala Gln
   145                 150                 155                 160
   Glu Pro Trp Lys Trp Arg Arg Ala Ser Lys Asn Ala Thr Asn
                   165                 170
<210> 119
   <211> 211
   <212> PRT
   <213> YNCB_BACSU
<220>
   <223> Endonuclease yncB P94492.1
<400> 119
   Met Lys Lys Ile Leu Ile Ser Met Ile Ala Ile Val Leu Ser Ile Thr
   1               5                   10                  15
   Leu Ala Ala Cys Gly Ser Asn His Ala Ala Lys Asn His Ser Asp Ser
               20                  25                  30
   Asn Gly Thr Glu Gln Val Ser Gln Asp Thr His Ser Asn Glu Tyr Asn
           35                  40                  45
   Gln Thr Glu Gln Lys Ala Gly Thr Pro His Ser Lys Asn Gln Lys Lys
       50                  55                  60
   Leu Val Asn Val Thr Leu Asp Arg Ala Ile Asp Gly Asp Thr Ile Lys
   65                  70                  75                  80
   Val Ile Tyr Asn Gly Lys Lys Asp Thr Val Arg Tyr Leu Leu Val Asp
                   85                  90                  95
   Thr Pro Glu Thr Lys Lys Pro Asn Ser Cys Val Gln Pro Tyr Gly Glu
               100                 105                 110
   Asp Ala Ser Lys Arg Asn Lys Glu Leu Val Asn Ser Gly Lys Leu Gln
           115                 120                 125
   Leu Glu Phe Asp Lys Gly Asp Arg Arg Asp Lys Tyr Gly Arg Leu Leu
       130                 135                 140
   Ala Tyr Val Tyr Val Asp Gly Lys Ser Val Gln Glu Thr Leu Leu Lys
   145                 150                 155                 160
   Glu Gly Leu Ala Arg Val Ala Tyr Val Tyr Glu Pro Asn Thr Lys Tyr
                   165                 170                 175
   Ile Asp Gln Phe Arg Leu Asp Glu Gln Glu Ala Lys Ser Asp Lys Leu
               180                 185                 190
   Ser Ile Trp Ser Lys Ser Gly Tyr Val Thr Asn Arg Gly Phe Asn Gly
           195                 200                 205
   Cys Val Lys
       210
<210> 120
   <211> 149
   <212> PRT
   <213> ENRN_BPT7
<220>
   <223> Endodeoxyribonuclease I (ENRN_BPT7)P00641.1
<400> 120
   Met Ala Gly Tyr Gly Ala Lys Gly Ile Arg Lys Val Gly Ala Phe Arg
   1               5                   10                  15
   Ser Gly Leu Glu Asp Lys Val Ser Lys Gln Leu Glu Ser Lys Gly Ile
               20                  25                  30
   Lys Phe Glu Tyr Glu Glu Trp Lys Val Pro Tyr Val Ile Pro Ala Ser
           35                  40                  45
   Asn His Thr Tyr Thr Pro Asp Phe Leu Leu Pro Asn Gly Ile Phe Val
       50                  55                  60
   Glu Thr Lys Gly Leu Trp Glu Ser Asp Asp Arg Lys Lys His Leu Leu
   65                  70                  75                  80
   Ile Arg Glu Gln His Pro Glu Leu Asp Ile Arg Ile Val Phe Ser Ser
                   85                  90                  95
   Ser Arg Thr Lys Leu Tyr Lys Gly Ser Pro Thr Ser Tyr Gly Glu Phe
               100                 105                 110
   Cys Glu Lys His Gly Ile Lys Phe Ala Asp Lys Leu Ile Pro Ala Glu
           115                 120                 125
   Trp Ile Lys Glu Pro Lys Lys Glu Val Pro Phe Asp Arg Leu Lys Arg
       130                 135                 140
   Lys Gly Gly Lys Lys
   145
<210> 121
   <211> 671
   <212> PRT
   <213> Homo sapiens
<220>
   <223> Metnase Q53H47.1
<400> 121
   Met Ala Glu Phe Lys Glu Lys Pro Glu Ala Pro Thr Glu Gln Leu Asp
   1               5                   10                  15
   Val Ala Cys Gly Gln Glu Asn Leu Pro Val Gly Ala Trp Pro Pro Gly
               20                  25                  30
   Ala Ala Pro Ala Pro Phe Gln Tyr Thr Pro Asp His Val Val Gly Pro
           35                  40                  45
   Gly Ala Asp Ile Asp Pro Thr Gln Ile Thr Phe Pro Gly Cys Ile Cys
       50                  55                  60
   Val Lys Thr Pro Cys Leu Pro Gly Thr Cys Ser Cys Leu Arg His Gly
   65                  70                  75                  80
   Glu Asn Tyr Asp Asp Asn Ser Cys Leu Arg Asp Ile Gly Ser Gly Gly
                   85                  90                  95
   Lys Tyr Ala Glu Pro Val Phe Glu Cys Asn Val Leu Cys Arg Cys Ser
               100                 105                 110
   Asp His Cys Arg Asn Arg Val Val Gln Lys Gly Leu Gln Phe His Phe
           115                 120                 125
   Gln Val Phe Lys Thr His Lys Lys Gly Trp Gly Leu Arg Thr Leu Glu
       130                 135                 140
   Phe Ile Pro Lys Gly Arg Phe Val Cys Glu Tyr Ala Gly Glu Val Leu
   145 150 155 160
   Gly Phe Ser Glu Val Gln Arg Arg Ile His Leu Gln Thr Lys Ser Asp
   165 170 175
   Ser Asn Tyr Ile Ile Ala Ile Arg Glu His Val Tyr Asn Gly Gln Val
   180 185 190
   Met Glu Thr Phe Val Asp Pro Thr Tyr Ile Gly Asn Ile Gly Arg Phe
   195 200 205
   Leu Asn His Ser Cys Glu Pro Asn Leu Leu Met Ile Pro Val Arg Ile
   210 215 220
   Asp Ser Met Val Pro Lys Leu Ala Leu Phe Ala Ala Lys Asp Ile Val
   225 230 235 240
   Pro Glu Glu Glu Leu Ser Tyr Asp Tyr Ser Gly Arg Tyr Leu Asn Leu
   245 250 255
   Thr Val Ser Glu Asp Lys Glu Arg Leu Asp His Gly Lys Leu Arg Lys
   260 265 270
   Pro Cys Tyr Cys Gly Ala Lys Ser Cys Thr Ala Phe Leu Pro Phe Asp
   275 280 285
   Ser Ser Leu Tyr Cys Pro Val Glu Lys Ser Asn Ile Ser Cys Gly Asn
   290 295 300
   Glu Lys Glu Pro Ser Met Cys Gly Ser Ala Pro Ser Val Phe Pro Ser
   305 310 315 320
   Cys Lys Arg Leu Thr Leu Glu Thr Met Lys Met Met Leu Asp Lys Lys
   325 330 335
   Gln Ile Arg Ala Ile Phe Leu Phe Glu Phe Lys Met Gly Arg Lys Ala
   340 345 350
   Ala Glu Thr Thr Arg Asn Ile Asn Asn Ala Phe Gly Pro Gly Thr Ala
   355 360 365
   Asn Glu Arg Thr Val Gln Trp Trp Phe Lys Lys Phe Cys Lys Gly Asp
   370 375 380
   Glu Ser Leu Glu Asp Glu Glu Arg Ser Gly Arg Pro Ser Glu Val Asp
   385 390 395 400
   Asn Asp Gln Leu Arg Ala Ile Ile Glu Ala Asp Pro Leu Thr Thr Thr
   405 410 415
   Arg Glu Val Ala Glu Glu Leu Asn Val Asn His Ser Thr Val Val Arg
   420 425 430
   His Leu Lys Gln Ile Gly Lys Val Lys Lys Leu Asp Lys Trp Val Pro
   435 440 445
   His Glu Leu Thr Glu Asn Gln Lys Asn Arg Arg Phe Glu Val Ser Ser
   450 455 460
   Ser Leu Ile Leu Arg Asn His Asn Glu Pro Phe Leu Asp Arg Ile Val
   465 470 475 480
   Thr Cys Asp Glu Lys Trp Ile Leu Tyr Asp Asn Arg Arg Arg Ser Ala
   485 490 495
   Gln Trp Leu Asp Gln Glu Glu Ala Pro Lys His Phe Pro Lys Pro Ile
   500 505 510
   Leu His Pro Lys Lys Val Met Val Thr Ile Trp Trp Ser Ala Ala Gly
   515 520 525
   Leu Ile His Tyr Ser Phe Leu Asn Pro Gly Glu Thr Ile Thr Ser Glu
   530 535 540
   Lys Tyr Ala Gln Glu Ile Asp Glu Met Asn Gln Lys Leu Gln Arg Leu
   545 550 555 560
   Gln Leu Ala Leu Val Asn Arg Lys Gly Pro Ile Leu Leu His Asp Asn
   565 570 575
   Ala Arg Pro His Val Ala Gln Pro Thr Leu Gln Lys Leu Asn Glu Leu
   580 585 590
   Gly Tyr Glu Val Leu Pro His Pro Pro Tyr Ser Pro Asp Leu Leu Pro
   595 600 605
   Thr Asn Tyr His Val Phe Lys His Leu Asn Asn Phe Leu Gln Gly Lys
   610 615 620
   Arg Phe His Asn Gln Gln Asp Ala Glu Asn Ala Phe Gln Glu Phe Val
   625 630 635 640
   Glu Ser Gln Ser Thr Asp Phe Tyr Ala Thr Gly Ile Asn Gln Leu Ile
   645 650 655
   Ser Arg Trp Gln Lys Cys Val Asp Cys Asn Gly Ser Tyr Phe Asp
   660 665 670
<210> 122
   <211> 488
   <212> PRT
   <213> Geobacillus stearothermophilus
<220>
   <223> Nb.BsrDI ABD15132.1
<400> 122
   Met Thr Glu Tyr Asp Leu His Leu Tyr Ala Asp Ser Phe His Glu Gly
   1               5                   10                  15
   His Trp Cys Cys Glu Asn Leu Ala Lys Ile Ala Gln Ser Asp Gly Gly
               20                  25                  30
   Lys His Gln Ile Asp Tyr Leu Gln Gly Phe Ile Pro Arg His Ser Leu
           35                  40                  45
   Ile Phe Ser Asp Leu Ile Ile Asn Ile Thr Val Phe Gly Ser Tyr Lys
       50                  55                  60
   Ser Trp Lys His Leu Pro Lys Gln Ile Lys Asp Leu Leu Phe Trp Gly
   65                  70                  75                  80
   Lys Pro Asp Phe Ile Ala Tyr Asp Pro Lys Asn Asp Lys Ile Leu Phe
                   85                  90                  95
   Ala Val Glu Glu Thr Gly Ala Val Pro Thr Gly Asn Gln Ala Leu Gln
               100                 105                 110
   Arg Cys Glu Arg Ile Tyr Gly Ser Ala Arg Lys Gln Ile Pro Phe Trp
           115                 120                 125
   Tyr Leu Leu Ser Glu Phe Gly Gln His Lys Asp Gly Gly Thr Arg Arg
       130                 135                 140
   Asp Ser Ile Trp Pro Thr Ile Met Gly Leu Lys Leu Thr Gln Leu Val
   145                 150                 155                 160
   Lys Thr Pro Ser Ile Ile Leu His Tyr Ser Asp Ile Asn Asn Pro Glu
                   165                 170                 175
   Asp Tyr Asn Ser Gly Asn Gly Leu Lys Phe Leu Phe Lys Ser Leu Leu
               180                 185                 190
   Gln Ile Ile Ile Asn Tyr Cys Thr Leu Lys Asn Pro Leu Lys Gly Met
           195                 200                 205
   Leu Glu Leu Leu Ser Ile Gln Tyr Glu Asn Met Leu Glu Phe Ile Lys
       210                 215                 220
   Ser Gln Trp Lys Glu Gln Ile Asp Phe Leu Pro Gly Glu Glu Ile Leu
   225                 230                 235                 240
   Asn Thr Lys Thr Lys Glu Leu Ala Arg Met Tyr Ala Ser Leu Ala Ile
                   245                 250                 255
   Gly Gln Thr Val Lys Ile Pro Glu Glu Leu Phe Asn Trp Pro Arg Thr
               260                 265                 270
   Asp Lys Val Asn Phe Lys Ser Pro Gln Gly Leu Ile Lys Tyr Asp Glu
           275                 280                 285
   Leu Cys Tyr Gln Leu Glu Lys Ala Val Gly Ser Lys Lys Ala Tyr Cys
       290                 295                 300
   Leu Ser Asn Asn Ala Gly Ala Lys Pro Gln Lys Leu Glu Ser Leu Lys
   305                 310                 315                 320
   Glu Trp Ile Asn Ser Gln Lys Lys Leu Phe Asp Lys Ala Pro Lys Leu
                   325                 330                 335
   Thr Pro Pro Ala Glu Phe Asn Met Lys Leu Asp Ala Phe Pro Val Thr
               340                 345                 350
   Ser Asn Asn Asn Tyr Tyr Val Thr Thr Ser Lys Asn Ile Leu Tyr Leu
           355                 360                 365
   Phe Asp Tyr Trp Lys Asp Leu Arg Ile Ala Ile Glu Thr Ala Phe Pro
       370                 375                 380
   Arg Leu Lys Gly Lys Leu Pro Thr Asp Ile Asp Glu Lys Pro Ala Leu
   385                 390                 395                 400
   Ile Tyr Ile Cys Asn Ser Val Lys Pro Gly Arg Leu Phe Gly Asp Pro
                   405                 410                 415
   Phe Thr Gly Gln Leu Ser Ala Phe Ser Thr Ile Phe Gly Lys Lys Asn
               420                 425                 430
   Ile Asp Met Pro Arg Ile Val Val Ala Tyr Tyr Pro His Gln Ile Tyr
           435                 440                 445
   Ser Gln Ala Leu Pro Lys Asn Asn Lys Ser Asn Lys Gly Ile Thr Leu
       450                 455                 460
   Lys Lys Glu Leu Thr Asp Phe Leu Ile Phe His Gly Gly Val Val Val
   465                 470                 475                 480
   Lys Leu Asn Glu Gly Lys Ala Tyr
                   485
<210> 123
   <211> 217
   <212> PRT
   <213> Geobacillus stearothermophilus
<220>
   <223> BsrDI A ABD15133.1
<400> 123
   Met Thr Asp Tyr Arg Tyr Ser Phe Glu Leu Ser Glu Glu Ile Ala Arg
   1               5                   10                  15
   Trp Ala Phe Glu Ile Lys Thr Lys Asn Thr Asp Trp Phe Val Ala Phe
               20                  25                  30
   Ser Asn Pro Thr Ala Gly Pro Trp Lys Arg Val Met Ala Ile Asp Lys
           35                  40                  45
   Ala Ser Asn Arg Glu Gly Glu Val His Arg Phe Gly Arg Glu Asp Glu
       50                  55                  60
   Arg Pro Asp Ile Ile Leu Val Asn Asp Asn Ile Ser Leu Ile Leu Ile
   65                  70                  75                  80
   Leu Glu Ala Lys Glu Lys Leu Asn Gln Leu Ile Ser Lys Ser Gln Val
                   85                  90                  95
   Asp Lys Ser Val Asp Val Phe Leu Thr Leu Ser Ser Ile Leu Lys Glu
               100                 105                 110
   Lys Ser Asp Asn Asn Tyr Trp Gly Asp Arg Thr Lys Tyr Ile Asn Val
           115                 120                 125
   Leu Gly Ile Leu Trp Gly Ser Glu Gln Glu Thr Ser Gln Lys Asp Ile
       130                 135                 140
   Asp Asn Ala Phe Arg Val Tyr Arg Asp Ser Leu Val Lys Asn Leu Lys
   145                 150                 155                 160
   Glu Ile Asn Pro Thr Pro Thr Asn Ile Cys Thr Asp Ile Leu Val Gly
                   165                 170                 175
   Val Glu Ser Ile Lys Asn Lys Lys Glu Glu Ile Ser Ile Lys Ile His
               180                 185                 190
   Val Ser Asn Ile Tyr Ala Glu Ile Tyr Pro Lys Phe Thr Gly Lys His
           195                 200                 205
   Leu Leu Glu Lys Leu Ala Val Leu Asn
       210                 215
<210> 124
   <211> 604
   <212> PRT
   <213> Bacillus sp. D6
<220>
   <223> Nt.BspD6I ABN42182.1 (R.BspD6I large subunit)
<400> 124
   Met Ala Lys Lys Val Asn Trp Tyr Val Ser Cys Ser Pro Arg Ser Pro
   1               5                   10                  15
   Glu Lys Ile Gln Pro Glu Leu Lys Val Leu Ala Asn Phe Glu Gly Ser
               20                  25                  30
   Tyr Trp Lys Gly Val Lys Gly Tyr Lys Ala Gln Glu Ala Phe Ala Lys
           35                  40                  45
   Glu Leu Ala Ala Leu Pro Gln Phe Leu Gly Thr Thr Tyr Lys Lys Glu
       50                  55                  60
   Ala Ala Phe Ser Thr Arg Asp Arg Val Ala Pro Met Lys Thr Tyr Gly
   65                  70                  75                  80
   Phe Val Phe Val Asp Glu Glu Gly Tyr Leu Arg Ile Thr Glu Ala Gly
                   85                  90                  95
   Lys Met Leu Ala Asn Asn Arg Arg Pro Lys Asp Val Phe Leu Lys Gln
               100                 105                 110
   Leu Val Lys Trp Gln Tyr Pro Ser Phe Gln His Lys Gly Lys Glu Tyr
           115                 120                 125
   Pro Glu Glu Glu Trp Ser Ile Asn Pro Leu Val Phe Val Leu Ser Leu
       130                 135                 140
   Leu Lys Lys Val Gly Gly Leu Ser Lys Leu Asp Ile Ala Met Phe Cys
   145 150 155 160
   Leu Thr Ala Thr Asn Asn Asn Gln Val Asp Glu Ile Ala Glu Glu Ile
   165 170 175
   Met Gln Phe Arg Asn Glu Arg Glu Lys Ile Lys Gly Gln Asn Lys Lys
   180 185 190
   Leu Glu Phe Thr Glu Asn Tyr Phe Phe Lys Arg Phe Glu Lys Ile Tyr
   195 200 205
   Gly Asn Val Gly Lys Ile Arg Glu Gly Lys Ser Asp Ser Ser His Lys
   210 215 220
   Ser Lys Ile Glu Thr Lys Met Arg Asn Ala Arg Asp Val Ala Asp Ala
   225 230 235 240
   Thr Thr Arg Tyr Phe Arg Tyr Thr Gly Leu Phe Val Ala Arg Gly Asn
   245 250 255
   Gln Leu Val Leu Asn Pro Glu Lys Ser Asp Leu Ile Asp Glu Ile Ile
   260 265 270
   Ser Ser Ser Lys Val Val Lys Asn Tyr Thr Arg Val Glu Glu Phe His
   275 280 285
   Glu Tyr Tyr Gly Asn Pro Ser Leu Pro Gln Phe Ser Phe Glu Thr Lys
   290 295 300
   Glu Gln Leu Leu Asp Leu Ala His Arg Ile Arg Asp Glu Asn Thr Arg
   305 310 315 320
   Leu Ala Glu Gln Leu Val Glu His Phe Pro Asn Val Lys Val Glu Ile
   325 330 335
   Gln Val Leu Glu Asp Ile Tyr Asn Ser Leu Asn Lys Lys Val Asp Val
   340 345 350
   Glu Thr Leu Lys Asp Val Ile Tyr His Ala Lys Glu Leu Gln Leu Glu
   355 360 365
   Leu Lys Lys Lys Lys Leu Gln Ala Asp Phe Asn Asp Pro Arg Gln Leu
   370 375 380
   Glu Glu Val Ile Asp Leu Leu Glu Val Tyr His Glu Lys Lys Asn Val
   385                 390                 395                 400
   Ile Glu Glu Lys Ile Lys Ala Arg Phe Ile Ala Asn Lys Asn Thr Val
                   405                 410                 415
   Phe Glu Trp Leu Thr Trp Asn Gly Phe Ile Ile Leu Gly Asn Ala Leu
               420                 425                 430
   Glu Tyr Lys Asn Asn Phe Val Ile Asp Glu Glu Leu Gln Pro Val Thr
           435                 440                 445
   His Ala Ala Gly Asn Gln Pro Asp Met Glu Ile Ile Tyr Glu Asp Phe
       450                 455                 460
   Ile Val Leu Gly Glu Val Thr Thr Ser Lys Gly Ala Thr Gln Phe Lys
   465                 470                 475                 480
   Met Glu Ser Glu Pro Val Thr Arg His Tyr Leu Asn Lys Lys Lys Glu
                   485                 490                 495
   Leu Glu Lys Gln Gly Val Glu Lys Glu Leu Tyr Cys Leu Phe Ile Ala
               500                 505                 510
   Pro Glu Ile Asn Lys Asn Thr Phe Glu Glu Phe Met Lys Tyr Asn Ile
           515                 520                 525
   Val Gln Asn Thr Arg Ile Ile Pro Leu Ser Leu Lys Gln Phe Asn Met
       530                 535                 540
   Leu Leu Met Val Gln Lys Lys Leu Ile Glu Lys Gly Arg Arg Leu Ser
   545                 550                 555                 560
   Ser Tyr Asp Ile Lys Asn Leu Met Val Ser Leu Tyr Arg Thr Thr Ile
                   565                 570                 575
   Glu Cys Glu Arg Lys Tyr Thr Gln Ile Lys Ala Gly Leu Glu Glu Thr
               580                 585                 590
   Leu Asn Asn Trp Val Val Asp Lys Glu Val Arg Phe
           595                 600
<210> 125
   <211> 186
   <212> PRT
   <213> Bacillus sp. D6
<220>
   <223> ss.BspD6I (R.BspD6I small subunit)
<400> 125
   Met Gln Asp Ile Leu Asp Phe Tyr Glu Glu Val Glu Lys Thr Ile Asn
   1               5                   10                  15
   Pro Pro Asn Tyr Phe Glu Trp Asn Thr Tyr Arg Val Phe Lys Lys Leu
               20                  25                  30
   Gly Ser Tyr Lys Asn Leu Val Pro Asn Phe Lys Leu Asp Asp Ser Gly
           35                  40                  45
   His Pro Ile Gly Asn Ala Ile Pro Gly Val Glu Asp Ile Leu Val Glu
       50                  55                  60
   Tyr Glu His Phe Ser Ile Leu Ile Glu Cys Ser Leu Thr Ile Gly Glu
   65                  70                  75                  80
   Lys Gln Leu Asp Tyr Glu Gly Asp Ser Val Val Arg His Leu Gln Glu
                   85                  90                  95
   Tyr Lys Lys Lys Gly Ile Glu Ala Tyr Thr Leu Phe Leu Gly Lys Ser
               100                 105                 110
   Ile Asp Leu Ser Phe Ala Arg His Ile Gly Phe Asn Lys Glu Ser Glu
           115                 120                 125
   Pro Val Ile Pro Leu Thr Val Asp Gln Phe Lys Lys Leu Val Thr Gln
       130                 135                 140
   Leu Lys Gly Asp Gly Glu His Phe Asn Pro Asn Lys Leu Lys Glu Ile
   145                 150                 155                 160
   Leu Ile Lys Leu Leu Arg Ser Asp Leu Gly Tyr Asp Gln Ala Glu Glu
                   165                 170                 175
   Trp Leu Thr Phe Ile Glu Tyr Asn Leu Lys
               180                 185
<210> 126
   <211> 555
   <212> PRT
   <213> Paucimonas lemoignei
<220>
   <223> R.PleI AAK27215.1
<400> 126
   Met Ala Lys Pro Ile Asp Ser Lys Val Leu Phe Ile Thr Thr Ser Pro
   1               5                   10                  15
   Arg Thr Pro Glu Lys Met Val Pro Glu Ile Glu Leu Leu Asp Lys Asn
               20                  25                  30
   Phe Asn Gly Asp Val Trp Asn Lys Asp Thr Gln Thr Ala Phe Met Lys
           35                  40                  45
   Ile Leu Lys Glu Glu Ser Phe Phe Asp Gly Glu Gly Lys Asn Asp Pro
       50                  55                  60
   Ala Phe Ser Ala Arg Asp Arg Ile Asn Arg Ala Pro Lys Ser Leu Gly
   65                  70                  75                  80
   Phe Val Ile Leu Thr Pro Lys Leu Ser Leu Thr Asp Ala Gly Val Glu
                   85                  90                  95
   Leu Ile Lys Ala Lys Arg Lys Asp Asp Ile Phe Leu Arg Gln Met Leu
               100                 105                 110
   Lys Phe Gln Leu Pro Ser Pro Tyr His Lys Leu Ser Asp Lys Ala Ala
           115                 120                 125
   Leu Phe Tyr Val Lys Pro Tyr Leu Glu Ile Phe Arg Leu Val Arg His
       130                 135                 140
   Phe Gly Ser Leu Thr Phe Asp Glu Leu Met Ile Phe Gly Leu Gln Ile
   145                 150                 155                 160
   Ile Asp Phe Arg Ile Phe Asn Gln Ile Val Asp Lys Ile Glu Asp Phe
                   165                 170                 175
   Arg Val Gly Lys Ile Glu Asn Lys Gly Arg Tyr Lys Thr Tyr Lys Lys
               180                 185                 190
   Glu Arg Phe Glu Glu Glu Leu Gly Lys Ile Tyr Lys Asp Glu Leu Phe
           195                 200                 205
   Gly Leu Thr Glu Ala Ser Ala Lys Thr Leu Ile Thr Lys Lys Gly Asn
       210                 215                 220
   Asn Met Arg Asp Tyr Ala Asp Ala Cys Val Arg Tyr Leu Arg Ala Thr
   225                 230                 235                 240
   Gly Met Val Asn Val Ser Tyr Gln Gly Lys Ser Leu Ser Ile Val Gln
                   245                 250                 255
   Glu Lys Lys Glu Glu Val Asp Phe Phe Leu Lys Asn Thr Glu Arg Glu
               260                 265                 270
   Pro Cys Phe Ile Asn Asp Glu Ala Ser Tyr Val Ser Tyr Leu Gly Asn
   275 280 285
   Pro Asn Tyr Pro Lys Leu Phe Val Asp Asp Val Asp Arg Ile Lys Lys
   290 295 300
   Lys Leu Arg Phe Asp Phe Lys Lys Thr Asn Lys Val Asn Ala Leu Thr
   305 310 315 320
   Leu Pro Glu Leu Lys Glu Glu Leu Glu Asn Glu Ile Leu Ser Arg Lys
   325 330 335
   Glu Asn Ile Leu Lys Ser Gln Ile Ser Asp Ile Lys Asn Phe Lys Leu
   340 345 350
   Tyr Glu Asp Ile Gln Glu Val Phe Glu Lys Ile Glu Asn Asp Arg Thr
   355 360 365
   Leu Ser Asp Ala Pro Leu Met Leu Glu Trp Asn Thr Trp Arg Ala Met
   370 375 380
   Thr Met Leu Asp Gly Gly Glu Ile Lys Ala Asn Leu Lys Phe Asp Asp
   385 390 395 400
   Phe Gly Ser Pro Met Ser Thr Ala Ile Gly Asn Met Pro Asp Ile Val
   405 410 415
   Cys Glu Tyr Asp Asp Phe Gln Leu Ser Val Glu Val Thr Met Ala Ser
   420 425 430
   Gly Gln Lys Gln Tyr Glu Met Glu Gly Glu Pro Val Ser Arg His Leu
   435 440 445
   Gly Lys Leu Lys Lys Ser Ser Glu Lys Pro Val Tyr Cys Leu Phe Ile
   450 455 460
   Ala Pro Lys Ile Asn Pro Ser Ser Val Ala His Phe Phe Met Ser His
   465 470 475 480
   Lys Val Asp Ile Glu Tyr Tyr Gly Gly Lys Ser Leu Ile Ile Pro Leu
   485 490 495
   Glu Leu Ser Val Phe Arg Lys Met Ile Glu Asp Thr Phe Lys Ala Ser
   500 505 510
   Tyr Ile Pro Lys Ser Asp Asn Val His Lys Leu Phe Lys Asn Phe Ala
   515 520 525
   Ser Ile Ala Asp Glu Ala Gly Asn Glu Lys Val Trp Tyr Glu Gly Val
   530 535 540
   Lys Arg Thr Ala Met Asn Trp Leu Ser Leu Ser
   545 550 555
<210> 127
   <211> 556
   <212> PRT
   <213> Micrococcus lylae
<220>
   <223> MlyI AAK39546.1
<400> 127
   Met Ala Ser Leu Ser Lys Thr Lys His Leu Phe Gly Phe Thr Ser Pro
   1               5                   10                  15
   Arg Thr Ile Glu Lys Ile Ile Pro Glu Leu Asp Ile Leu Ser Gln Gln
               20                  25                  30
   Phe Ser Gly Lys Val Trp Gly Glu Asn Gln Ile Asn Phe Phe Asp Ala
           35                  40                  45
   Ile Phe Asn Ser Asp Phe Tyr Glu Gly Thr Thr Tyr Pro Gln Asp Pro
       50                  55                  60
   Ala Leu Ala Ala Arg Asp Arg Ile Thr Arg Ala Pro Lys Ala Leu Gly
   65                  70                  75                  80
   Phe Ile Gln Leu Lys Pro Val Ile Gln Leu Thr Lys Ala Gly Asn Gln
                   85                  90                  95
   Leu Val Asn Gln Lys Arg Leu Pro Glu Leu Phe Thr Lys Gln Leu Leu
               100                 105                 110
   Lys Phe Gln Leu Pro Ser Pro Tyr His Thr Gln Ser Pro Thr Val Asn
           115                 120                 125
   Phe Asn Val Arg Pro Tyr Leu Glu Leu Leu Arg Leu Ile Asn Glu Leu
       130                 135                 140
   Gly Ser Ile Ser Lys Thr Glu Ile Ala Leu Phe Phe Leu Gln Leu Val
   145                 150                 155                 160
   Asn Tyr Asn Lys Phe Asp Glu Ile Lys Asn Lys Ile Leu Lys Phe Arg
                   165                 170                 175
   Glu Thr Arg Lys Asn Asn Arg Ser Val Ser Trp Lys Thr Tyr Val Ser
               180                 185                 190
   Gln Glu Phe Glu Lys Gln Ile Ser Ile Ile Phe Ala Asp Glu Val Thr
           195                 200                 205
   Ala Lys Asn Phe Arg Thr Arg Glu Ser Ser Asp Glu Ser Phe Lys Lys
       210                 215                 220
   Phe Val Lys Thr Lys Glu Gly Asn Met Lys Asp Tyr Ala Asp Ala Phe
   225                 230                 235                 240
   Phe Arg Tyr Ile Arg Gly Thr Gln Leu Val Thr Ile Asp Lys Asn Leu
                   245                 250                 255
   His Leu Lys Ile Ser Ser Leu Lys Gln Asp Ser Val Asp Phe Leu Leu
               260                 265                 270
   Lys Asn Thr Asp Arg Asn Ala Leu Asn Leu Ser Leu Met Glu Tyr Glu
           275                 280                 285
   Asn Tyr Leu Phe Asp Pro Asp Gln Leu Ile Val Leu Glu Asp Asn Ser
       290                 295                 300
   Gly Leu Ile Asn Ser Lys Ile Lys Gln Leu Asp Asp Ser Ile Asn Val
   305                 310                 315                 320
   Glu Ser Leu Lys Ile Asp Asp Ala Lys Asp Leu Leu Asn Asp Leu Glu
                   325                 330                 335
   Ile Gln Arg Lys Ala Lys Thr Ile Glu Asp Thr Val Asn His Leu Lys
               340                 345                 350
   Leu Arg Ser Asp Ile Glu Asp Ile Leu Asp Val Phe Ala Lys Ile Lys
           355                 360                 365
   Lys Arg Asp Val Pro Asp Val Pro Leu Phe Leu Glu Trp Asn Ile Trp
       370                 375                 380
   Arg Ala Phe Ala Ala Leu Asn His Thr Gln Ala Ile Glu Gly Asn Phe
   385                 390                 395                 400
   Ile Val Asp Leu Asp Gly Met Pro Leu Asn Thr Ala Pro Gly Lys Lys
                   405                 410                 415
   Pro Asp Ile Glu Ile Asn Tyr Gly Ser Phe Ser Cys Ile Val Glu Val
                   420                 425                 430
   Thr Met Ser Ser Gly Glu Thr Gln Phe Asn Met Glu Gly Ser Ser Val
           435                 440                 445
   Pro Arg His Tyr Gly Asp Leu Val Arg Lys Val Asp His Asp Ala Tyr
       450                 455                 460
   Cys Ile Phe Ile Ala Pro Lys Val Ala Pro Gly Thr Lys Ala His Phe
   465                 470                 475                 480
   Phe Asn Leu Asn Arg Leu Ser Thr Lys His Tyr Gly Gly Lys Thr Lys
                   485                 490                 495
   Ile Ile Pro Met Ser Leu Asp Asp Phe Ile Cys Phe Leu Gln Val Gly
               500                 505                 510
   Ile Thr His Asn Phe Gln Asp Ile Asn Lys Leu Lys Asn Trp Leu Asp
           515                 520                 525
   Asn Leu Ile Asn Phe Asn Leu Glu Ser Glu Asp Glu Glu Ile Trp Phe
       530                 535                 540
   Glu Glu Ile Ile Ser Lys Ile Ser Thr Trp Ala Ile
   545                 550                 555
<210> 128
   <211> 543
   <212> PRT
   <213> Geobacillus sp. Y412MC52
<220>
   <223> AlwI YP_004134094.1
<400> 128
   Met Asn Lys Lys Asn Thr Arg Lys Val Trp Phe Ile Thr Arg Pro Glu
   1               5                   10                  15
   Arg Asp Pro Arg Phe His Gln Glu Ala Leu Leu Ala Leu Gln Lys Ala
               20                  25                  30
   Thr Asp Asp Phe Arg Leu Lys Trp Ala Gly Asn Arg Glu Val His Lys
           35                  40                  45
   Arg Tyr Glu Glu Glu Leu Ala Asn Met Gly Ile Lys Arg Asn Asn Val
       50                  55                  60
   Ser His Asp Gly Ser Gly Gly Arg Thr Trp Met Ala Met Leu Lys Thr
   65                  70                  75                  80
   Phe Ser Tyr Cys Tyr Val Asp Asp Asp Gly Tyr Ile Arg Leu Thr Lys
                   85                  90                  95
   Val Gly Glu Lys Leu Ile Gln Gly Glu Lys Val Tyr Glu Asn Thr Arg
               100                 105                 110
   Lys Gln Val Leu Thr Leu Gln Tyr Pro Asn Ala Tyr Phe Leu Glu Pro
           115                 120                 125
   Gly Phe Arg Pro Lys Phe Asp Glu Gly Phe Arg Ile Arg Pro Val Leu
       130                 135                 140
   Phe Leu Ile Lys Leu Ala Asn Asp Glu Arg Leu Asp Phe Tyr Val Thr
   145                 150                 155                 160
   Lys Glu Glu Ile Thr Tyr Phe Ala Met Thr Ala Gln Lys Asp Ser Gln
                   165                 170                 175
   Leu Asp Glu Ile Val His Lys Ile Leu Ala Phe Arg Lys Ala Gly Pro
               180                 185                 190
   Arg Glu Arg Glu Glu Met Lys Gln Asp Ile Ala Ala Lys Phe Asp His
           195                 200                 205
   Arg Glu Arg Ser Asp Lys Gly Ala Arg Asp Phe Tyr Glu Ala His Ser
       210                 215                 220
   Asp Val Ala His Thr Phe Met Leu Ile Ser Asp Tyr Thr Gly Leu Val
   225                 230                 235                 240
   Glu Tyr Ile Arg Gly Lys Ala Leu Lys Gly Asp Ser Ser Lys Ile Asn
                   245                 250                 255
   Glu Ile Lys Gln Glu Ile Ala Glu Ile Glu Lys Arg Tyr Pro Phe Asn
               260                 265                 270
   Thr Arg Tyr Met Ile Ser Leu Glu Arg Met Ala Glu Asn Ser Gly Leu
           275                 280                 285
   Asp Val Asp Ser Tyr Lys Ala Ser Arg Tyr Gly Asn Ile Lys Pro Ala
       290                 295                 300
   Ala Asn Ser Ser Lys Leu Arg Ala Lys Ala Glu Arg Ile Leu Ala Gln
   305                 310                 315                 320
   Phe Pro Ser Ile Glu Ser Met Ser Lys Glu Glu Ile Ala Gly Ala Leu
                   325                 330                 335
   Gln Lys Tyr Leu Ser Pro Arg Asp Ile Glu Lys Val Ile His Glu Ile
               340                 345                 350
   Val Glu Asn Lys Asp Asp Phe Glu Gly Ile Asn Ser Asp Phe Val Glu
           355                 360                 365
   Thr Tyr Leu Asn Glu Lys Asp Asn Leu Ala Phe Glu Asp Lys Thr Gly
       370                 375                 380
   Gln Ile Phe Ser Ala Leu Gly Phe Asp Val Ala Met Arg Pro Lys Ala
   385                 390                 395                 400
   Lys Asn Gly Glu Arg Thr Glu Ile Glu Ile Ile Ala Arg Tyr Gly Gly
                   405                 410                 415
   Ser Lys Phe Gly Ile Ile Asp Ala Lys Asn Tyr Ala Gly Lys Phe Pro
               420                 425                 430
   Leu Ser Ser Ser Leu Val Ser His Met Ala Ser Glu Tyr Ile Pro Asn
           435                 440                 445
   Tyr Thr Gly Tyr Glu Gly Lys Glu Leu Thr Phe Phe Gly Tyr Val Thr
       450                 455                 460
   Ala Asn Asp Phe Ser Gly Glu Arg Asn Leu Glu Lys Ile Ser Asp Lys
   465                 470                 475                 480
   Ala Lys Arg Ile Thr Gly Asn Pro Ile Ser Gly Phe Leu Val Thr Ala
                   485                 490                 495
   Arg Thr Leu Leu Gly Phe Leu Asp Tyr Cys Ile Glu Asn Asp Val Pro
               500                 505                 510
   Leu Glu Asp Arg Ala Glu Leu Phe Val Lys Ala Val Lys Asn Lys Gly
           515                 520                 525
   Tyr Lys Ser Leu Glu Ala Leu Leu Arg Glu Leu Lys Glu Thr Ile
       530                 535                 540
<210> 129
   <211> 685
   <212> PRT
   <213> Kocuria varians
<220>
   <223> Mva1269I AAY97906.1
<400> 129
   Met Tyr Leu Asn Thr Ala Val Phe Asn Ile Tyr Gly Asp Asn Ile Val
   1 5 10 15
   Glu Cys Ser Arg Ala Phe His Tyr Ile Leu Glu Gly Phe Lys Leu Ala
   20 25 30
   Asn Ile Ser Ile Thr Gln Glu Tyr Asp Leu Gln Asn Ile Thr Thr Pro
   35 40 45
   Lys Phe Cys Ile Tyr Thr Asp Lys Phe Arg Tyr Ile Phe Ile Phe Ile
   50 55 60
   Pro Gly Thr Ser Ala Ser Arg Trp Asn Lys Asp Ile Tyr Lys Glu Leu
   65 70 75 80
   Val Leu Asn Asn Gly Gly Pro Leu Lys Glu Gly Ala Asp Ala Ile Ile
   85 90 95
   Thr Arg Ile Phe Ser Glu Asp Ser Glu Leu Val Leu Ala Ser Met Glu
   100 105 110
   Phe Ser Ala Ala Leu Pro Ala Gly Asn Asn Thr Trp Gln Arg Ser Gly
   115 120 125
   Arg Ala Tyr Ser Leu Thr Ala Ala Asn Ile Pro Tyr Phe Tyr Ile Val
   130 135 140
   Gln Leu Gly Gly Lys Glu Ile Lys Lys Gly Lys Asp Gly Lys Ser Asp
   145 150 155 160
   Lys Phe Ala Thr Arg Leu Pro Asn Pro Ala Leu Ser Leu Ser Phe Thr
   165 170 175
   Leu Asn Thr Ile Lys Lys Pro Ala Pro Ser Leu Ile Val Tyr Asp Gln
   180 185 190
   Ala Pro Glu Ala Asp Ser Ala Ile Ser Asp Leu Tyr Ser Asn Cys Tyr
   195 200 205
   Gly Ile Asp Asp Phe Ser Leu Tyr Leu Phe Lys Leu Ile Thr Glu Glu
   210 215 220
   Asn Asn Leu His Glu Leu Lys Asn Ile Tyr Asn Lys Asn Val Glu Phe
   225 230 235 240
   Leu Gln Leu Arg Ser Val Asp Glu Lys Gly Lys Asn Phe Ser Gly Lys
   245 250 255
   Asp Tyr Lys Tyr Ile Phe Glu His Lys Asp Pro Tyr Lys Gly Leu Thr
   260 265 270
   Glu Val Val Lys Glu Arg Lys Ile Pro Trp Lys Lys Lys Thr Ala Thr
   275 280 285
   Lys Thr Phe Glu Asn Phe Pro Leu Arg Asn Gln Ala Pro Ile Phe Arg
   290 295 300
   Leu Ile Asp Phe Leu Ser Thr Lys Ser Tyr Gly Ile Val Ser Lys Asp
   305 310 315 320
   Ser Leu Pro Leu Thr Phe Ile Pro Ser Glu His Arg Val Glu Val Ala
   325 330 335
   Asn Tyr Ile Cys Asn Gln Leu Tyr Ile Asp Lys Val Ser Asp Glu Phe
   340 345 350
   Val Lys Trp Ile Tyr Lys Lys Glu Asp Leu Ala Ile Cys Ile Ile Asn
   355 360 365
   Gly Phe Lys Pro Gly Gly Asp Asp Ser Arg Pro Asp Arg Gly Leu Pro
   370 375 380
   Pro Phe Thr Lys Met Leu Thr Asn Leu Asp Ile Leu Thr Leu Met Phe
   385 390 395 400
   Gly Pro Ala Pro Pro Thr Gln Trp Asp Tyr Leu Asp Ser Asp Pro Glu
   405 410 415
   Lys Leu Asn Lys Thr Asn Gly Leu Trp Gln Ser Ile Phe Ala Phe Ser
   420 425 430
   Asp Ala Ile Leu Val Asp Ser Ser Thr Arg Asp Asn Asn Lys Phe Val
   435 440 445
   Tyr Asn Ala Tyr Leu Lys Glu His Trp Val Val Gln Arg Glu Lys Lys
   450 455 460
   Glu Ser Asn Thr Pro Ile Ser Tyr Phe Pro Lys Ser Val Gly Glu His
   465 470 475 480
   Asp Val Asp Thr Ser Leu His Ile Leu Phe Thr Tyr Ile Gly Lys His
   485 490 495
   Phe Glu Ser Ala Cys Asn Pro Pro Gly Gly Asp Trp Ser Gly Val Ser
   500 505 510
   Leu Leu Lys Asn Asn Ile Glu Tyr Arg Trp Thr Ser Met Tyr Arg Val
   515 520 525
   Ser Gln Asp Gly Thr Lys Arg Pro Asp His Ile Tyr Gln Leu Val Tyr
   530 535 540
   Asn Ser Thr Asp Thr Leu Leu Leu Ile Glu Ser Lys Gly Ile Lys Asn
   545 550 555 560
   Asp Leu Leu Lys Ser Lys Glu Ala Asn Val Gly Ile Gly Met Ile Asn
   565 570 575
   Tyr Leu Lys Asn Leu Met Ala Arg Asp Tyr Thr Ala Val Lys Lys Asp
   580 585 590
   Gly Glu Trp Lys Asn Ile His Gly Gln Met Thr Leu Asp Lys Phe Leu
   595 600 605
   Thr Phe Ser Ala Val Ala Tyr Leu Phe Thr Thr Asp Phe Asp Asn Glu
   610 615 620
   Tyr Thr Ser Ala Ala Glu Leu Leu Val His Ser Asn Thr Gln Leu Ala
   625 630 635 640
   Phe Ala Leu Glu Ile Lys Glu Lys Asn Ser Val Met His Ile Phe Thr
   645 650 655
   Ala Asn Thr Val Ala Tyr Asn Phe Ala Glu Tyr Leu Leu Glu Thr Met
   660 665 670
   Arg Asn Ser His Leu Pro Leu Lys Ile Tyr Lys Pro Ile
   675 680 685
<210> 130
   <211> 599
   <212> PRT
   <213> Geobacillus stearothermophilus
<220>
   <223> BsrI ADR72996.1
<400> 130
   Met Arg Asn Ile Arg Ile Tyr Ser Glu Val Lys Glu Gln Gly Ile Phe
   1               5                   10                  15
   Phe Lys Glu Val Ile Gln Ser Val Leu Glu Lys Ala Asn Val Glu Val
   20 25 30
   Val Leu Val Asn Ser Ala Met Leu Asp Tyr Ser Asp Val Ser Val Ile
   35 40 45
   Ser Leu Ile Arg Asn Gln Lys Lys Phe Asp Leu Leu Val Ser Glu Val
   50 55 60
   Arg Asp Lys Arg Glu Ile Pro Ile Val Met Val Glu Phe Ser Thr Ala
   65 70 75 80
   Val Thr Thr Asp Asp His Glu Leu Gln Arg Ala Asp Ala Met Phe Trp
   85 90 95
   Ala Tyr Lys Tyr Lys Ile Pro Tyr Leu Lys Ile Ser Pro Met Glu Lys
   100 105 110
   Lys Ser Gln Thr Ala Asp Asp Lys Phe Gly Gly Gly Arg Leu Leu Ser
   115 120 125
   Val Asn Asp Gln Ile Ile His Met Tyr Arg Thr Asp Gly Val Met Tyr
   130 135 140
   His Ile Glu Trp Glu Ser Met Asp Asn Ser Ala Tyr Val Lys Asn Ala
   145 150 155 160
   Glu Leu Tyr Pro Ser Cys Pro Asp Cys Ala Pro Glu Leu Ala Ser Leu
   165 170 175
   Phe Arg Cys Leu Leu Glu Thr Ile Glu Lys Cys Glu Asn Ile Glu Asp
   180 185 190
   Tyr Tyr Arg Ile Leu Leu Asp Lys Leu Gly Lys Gln Lys Val Ala Val
   195 200 205
   Lys Trp Gly Asn Phe Arg Glu Glu Lys Thr Leu Glu Gln Trp Lys His
   210 215 220
   Glu Lys Phe Asp Leu Leu Glu Arg Phe Ser Lys Ser Ser Ser Arg Met
   225 230 235 240
   Glu Tyr Asp Lys Asp Lys Lys Glu Leu Lys Ile Lys Val Asn Arg Tyr
   245 250 255
   Gly His Ala Met Asp Pro Glu Arg Gly Ile Leu Ala Phe Trp Lys Leu
   260 265 270
   Val Leu Gly Asp Glu Trp Lys Ile Val Ala Glu Phe Gln Leu Gln Arg
   275 280 285
   Lys Thr Leu Lys Gly Arg Gln Ser Tyr Gln Ser Leu Phe Asp Glu Val
   290 295 300
   Ser Gln Glu Glu Lys Leu Met Asn Ile Ala Ser Glu Ile Ile Lys Asn
   305 310 315 320
   Gly Asn Val Ile Ser Pro Asp Lys Ala Ile Glu Ile His Lys Leu Ala
   325 330 335
   Thr Ser Ser Thr Met Ile Ser Thr Ile Asp Leu Gly Thr Pro Glu Arg
   340 345 350
   Lys Tyr Ile Thr Asp Asp Ser Leu Lys Gly Tyr Leu Gln His Gly Leu
   355 360 365
   Ile Thr Asn Ile Tyr Lys Asn Leu Leu Tyr Tyr Val Asp Glu Ile Arg
   370 375 380
   Phe Thr Asp Leu Gln Arg Lys Thr Ile Ala Ser Leu Thr Trp Asn Lys
   385 390 395 400
   Glu Ile Val Asn Asp Tyr Tyr Lys Ser Leu Met Asp Gln Leu Leu Asp
   405 410 415
   Lys Asn Leu Arg Val Leu Pro Leu Thr Ser Ile Lys Asn Ile Ser Glu
   420 425 430
   Asp Leu Ile Thr Trp Ser Ser Lys Glu Ile Leu Ile Asn Leu Gly Tyr
   435 440 445
   Lys Ile Leu Ala Ala Ser Tyr Pro Glu Ala Gln Gly Asp Arg Cys Ile
   450 455 460
   Leu Val Gly Pro Thr Gly Lys Lys Thr Glu Arg Lys Phe Ile Asp Leu
   465 470 475 480
   Ile Ala Ile Ser Pro Lys Ser Lys Gly Val Ile Leu Leu Glu Cys Lys
   485 490 495
   Asp Lys Leu Ser Lys Ser Lys Asp Asp Cys Glu Lys Met Asn Asp Leu
   500 505 510
   Leu Asn His Asn Tyr Asp Lys Val Thr Lys Leu Ile Asn Val Leu Asn
   515 520 525
   Ile Asn Asn Tyr Asn Tyr Asn Asn Ile Ile Tyr Thr Gly Val Ala Gly
   530 535 540
   Leu Ile Gly Arg Lys Asn Val Asp Asn Leu Pro Val Asp Phe Val Ile
   545 550 555 560
   Lys Phe Lys Tyr Asp Ala Lys Asn Leu Lys Leu Asn Trp Glu Ile Asn
   565 570 575
   Ser Asp Ile Leu Gly Lys His Ser Gly Ser Phe Ser Met Glu Asp Val
   580 585 590
   Ala Val Val Arg Lys Arg Ser
   595
<210> 131
   <211> 676
   <212> PRT
   <213> Geobacillus stearothermophilus
<220>
   <223> BsmI AAL86024.1
<400> 131
   Met Asn Val Phe Arg Ile His Gly Asp Asn Ile Ile Glu Cys Glu Arg
   1 5 10 15
   Val Ile Asp Leu Ile Leu Ser Lys Ile Asn Pro Gln Lys Val Lys Arg
   20 25 30
   Gly Phe Ile Ser Leu Ser Cys Pro Phe Ile Glu Ile Ile Phe Lys Glu
   35 40 45
   Gly His Asp Tyr Phe His Trp Arg Phe Asp Met Phe Pro Gly Phe Asn
   50 55 60
   Lys Asn Thr Asn Asp Arg Trp Asn Ser Asn Ile Leu Asp Leu Leu Ser
   65 70 75 80
   Gln Lys Gly Ser Phe Leu Tyr Glu Thr Pro Asp Val Ile Ile Thr Ser
   85 90 95
   Leu Asn Asn Gly Lys Glu Glu Ile Leu Met Ala Ile Glu Phe Cys Ser
   100 105 110
   Ala Leu Gln Ala Gly Asn Gln Ala Trp Gln Arg Ser Gly Arg Ala Tyr
   115 120 125
   Ser Val Gly Arg Thr Gly Tyr Pro Tyr Ile Tyr Ile Val Asp Phe Val
       130                 135                 140
   Lys Tyr Glu Leu Asn Asn Ser Asp Arg Ser Arg Lys Asn Leu Arg Phe
   145                 150                 155                 160
   Pro Asn Pro Ala Ile Pro Tyr Ser Tyr Ile Ser His Ser Lys Asn Thr
                   165                 170                 175
   Gly Asn Phe Ile Val Gln Ala Tyr Phe Arg Gly Glu Glu Tyr Gln Pro
               180                 185                 190
   Lys Tyr Asp Lys Lys Leu Lys Phe Phe Asp Glu Thr Ile Phe Ala Glu
           195                 200                 205
   Asp Asp Ile Ala Asp Tyr Ile Ile Ala Lys Leu Gln His Arg Asp Thr
       210                 215                 220
   Ser Asn Ile Glu Gln Leu Leu Ile Asn Lys Asn Leu Lys Met Val Glu
   225                 230                 235                 240
   Phe Leu Ser Lys Asn Thr Lys Asn Asp Asn Asn Phe Thr Tyr Ser Glu
                   245                 250                 255
   Trp Glu Ser Ile Tyr Asn Gly Thr Tyr Arg Ile Thr Asn Leu Pro Ser
               260                 265                 270
   Leu Gly Arg Phe Lys Phe Arg Lys Lys Ile Ala Glu Lys Ser Leu Ser
           275                 280                 285
   Gly Lys Val Lys Glu Phe Asn Asn Ile Val Gln Arg Tyr Ser Val Gly
       290                 295                 300
   Leu Ala Ser Ser Asp Leu Pro Phe Gly Val Ile Arg Lys Glu Ser Arg
   305                 310                 315                 320
   Asn Asp Phe Ile Asn Asp Val Cys Lys Leu Tyr Asn Ile Asn Asp Met
                   325                 330                 335
   Lys Ile Ile Lys Glu Leu Lys Glu Asp Ala Asp Leu Ile Val Cys Met
               340                 345                 350
   Leu Lys Gly Phe Lys Pro Arg Gly Asp Asp Asn Arg Pro Asp Arg Gly
           355                 360                 365
   Ala Leu Pro Leu Val Ala Met Leu Ala Gly Glu Asn Ala Gln Ile Phe
       370                 375                 380
   Thr Phe Ile Tyr Gly Pro Leu Ile Lys Gly Ala Ile Asn Leu Ile Asp
   385                 390                 395                 400
   Gln Asp Ile Asn Lys Leu Ala Lys Arg Asn Gly Leu Trp Lys Ser Phe
                   405                 410                 415
   Val Ser Leu Ser Asp Phe Ile Val Leu Asp Cys Pro Ile Ile Gly Glu
               420                 425                 430
   Ser Tyr Asn Glu Phe Arg Leu Ile Ile Asn Lys Asn Asn Lys Glu Ser
           435                 440                 445
   Ile Leu Arg Lys Thr Ser Lys Gln Gln Asn Ile Leu Val Asp Pro Thr
       450                 455                 460
   Pro Asn His Tyr Gln Glu Asn Asp Val Asp Thr Val Ile Tyr Ser Ile
   465                 470                 475                 480
   Phe Lys Tyr Ile Val Pro Asn Cys Phe Ser Gly Met Cys Asn Pro Pro
                   485                 490                 495
   Gly Gly Asp Trp Ser Gly Leu Ser Ile Ile Arg Asn Gly His Glu Phe
               500                 505                 510
   Arg Trp Leu Ser Leu Pro Arg Val Ser Glu Asn Gly Lys Arg Pro Asp
           515                 520                 525
   His Val Ile Gln Ile Leu Asp Leu Phe Glu Lys Pro Leu Leu Leu Ser
       530                 535                 540
   Ile Glu Ser Lys Glu Lys Pro Asn Asp Leu Glu Pro Lys Ile Gly Val
   545                 550                 555                 560
   Gln Leu Ile Lys Tyr Ile Glu Tyr Leu Phe Asp Phe Thr Pro Ser Val
                   565                 570                 575
   Gln Arg Lys Ile Ala Gly Gly Asn Trp Glu Phe Gly Asn Lys Ser Leu
               580                 585                 590
   Val Pro Asn Asp Phe Ile Leu Leu Ser Ala Gly Ala Phe Ile Asp Tyr
           595                 600                 605
   Asp Asn Leu Thr Glu Asn Asp Tyr Glu Lys Ile Phe Glu Val Thr Gly
       610                 615                 620
   Cys Asp Leu Leu Ile Ala Ile Lys Asn Gln Asn Asn Pro Gln Lys Trp
   625                 630                 635                 640
   Val Ile Lys Phe Lys Pro Lys Asn Thr Ile Ala Glu Lys Leu Val Asn
   645 650 655
   Tyr Ile Lys Leu Asn Phe Lys Ser Asn Ile Phe Asp Thr Gly Phe Phe
   660 665 670
   His Ile Glu Gly
   675
<210> 132
   <211> 465
   <212> PRT
   <213> artificial sequence
<220>
   <223> Nb.BtsCI ADI24225.1
<400> 132
   Met Lys Arg Ile Leu Tyr Leu Leu Thr Glu Glu Arg Pro Lys Ile Asn
   1 5 10 15
   Ile Ile His Gln Ile Ile Asn Leu Glu Tyr Lys Ala Thr Leu His Phe
   20 25 30
   Gly Ala Lys Ile Val Pro Val Met Asn Glu Glu Asn Lys Phe Thr Phe
   35 40 45
   Ile Tyr His Val Lys Gly Ile Glu Val Glu Gly Phe Asp Ala Val Leu
   50 55 60
   Ile Lys Ile Val Ser Gly His Ser Ser Phe Val Asp Tyr Leu Val Phe
   65 70 75 80
   Asp Ser Asn Asp Leu Lys Pro Glu Lys Asn Thr Ile Thr Leu Phe Asp
   85 90 95
   Leu Asp Gln Tyr Glu Leu Asp Leu Ser Tyr Tyr Phe Gly Lys Gly Trp
   100 105 110
   Ile Val Arg Ile Pro Ser Pro Ser Asp Leu Pro Lys Tyr Val Val Glu
   115 120 125
   Glu Thr Lys Thr Asp Asp His Glu Ser Arg Asn Thr Asn Ala Tyr Gln
   130 135 140
   Arg Ser Ser Lys Phe Val Phe Cys Glu Leu Tyr Tyr Gly Lys Glu Val
   145 150 155 160
   Lys Lys Tyr Met Leu Tyr Asp Ile Ser Asp Gly Arg Thr Leu Ser Gly
                   165                 170                 175
   Thr Asp Thr His Asn Phe Gly Met Arg Met Leu Val Thr Asn Asn Val
               180                 185                 190
   Asn Leu Val Gly Val Pro Asn Met Tyr Leu Pro Phe Thr Asp Ile Lys
           195                 200                 205
   Glu Phe Ile Asn Glu Lys Asn Arg Ile Ala Asp Asn Gly Pro Ser His
       210                 215                 220
   Asn Val Pro Ile Arg Leu Lys Leu Asp Lys Glu Lys Asn Val Ile Tyr
   225                 230                 235                 240
   Ile Ser Ala Lys Leu Asp Lys Gly Asn Gly Lys Asn Lys Asn Lys Ile
                   245                 250                 255
   Ser Asn Asp Pro Asn Ile Gly Ala Val Ala Ile Ile Ser Ala Thr Leu
               260                 265                 270
   Arg Asn Leu Asn Trp Lys Gly Asp Ile Glu Ile Ile Asn His Asn Leu
           275                 280                 285
   Leu Pro Ser Ser Ile Ser Ser Arg Ser Asn Gly Asn Lys Leu Leu Tyr
       290                 295                 300
   Ile Met Lys Lys Leu Gly Val Arg Phe Asn Asn Ile Asn Val Asn Trp
   305                 310                 315                 320
   Asn Asn Ile Lys Asn Asn Ile Asn Tyr Phe Phe Tyr Asn Ile Thr Ser
                   325                 330                 335
   Glu Lys Ile Val Ser Ile Tyr Tyr His Leu Tyr Val Glu Asp Lys Leu
               340                 345                 350
   Ser Asn Ala Arg Val Ile Phe Asp Asn His Ala Gly Cys Gly Lys Ser
           355                 360                 365
   Tyr Phe Arg Thr Leu Asn Asn Lys Ile Ile Pro Val Gly Lys Glu Ile
       370                 375                 380
   Pro Leu Pro Ala Leu Val Ile Phe Asp Ser Asp Gln Asn Ile Val Lys
   385                 390                 395                 400
   Val Ile Ala Ala Ala Lys Ala Glu Asn Val Tyr Asn Gly Val Glu Gln
                   405                 410                 415
   Leu Ser Thr Phe Asp Lys Phe Ile Glu Ser Tyr Ile Asn Lys Tyr Tyr
   420 425 430
   Pro Gly Ala Ala Val Glu Cys Ser Val Ile Thr Trp Gly Lys Ser Ser
   435 440 445
   Asn Pro Tyr Val Ser Phe Tyr Leu Asp Lys Asp Gly Ser Ala Val Phe
   450 455 460
   Leu
   465
<210> 133
   <211> 465
   <212> PRT
   <213> artificial sequence
<220>
   <223> Nt.BtsCI ADI24224.1
<400> 133
   Met Lys Arg Ile Leu Tyr Leu Leu Thr Glu Glu Arg Pro Lys Ile Asn
   1 5 10 15
   Ile Ile His Gln Ile Ile Asn Leu Glu Tyr Lys Ala Thr Leu His Phe
   20 25 30
   Gly Ala Lys Ile Val Pro Val Met Asn Glu Glu Asn Lys Phe Thr Phe
   35 40 45
   Ile Tyr His Val Lys Gly Ile Glu Val Glu Gly Phe Asp Ala Val Leu
   50 55 60
   Ile Lys Ile Val Ser Gly His Ser Ser Phe Val Asp Tyr Leu Val Phe
   65 70 75 80
   Asp Ser Asn Asp Leu Lys Pro Glu Lys Asn Thr Ile Thr Leu Phe Asp
   85 90 95
   Leu Asp Gln Tyr Glu Leu Asp Leu Ser Tyr Tyr Phe Gly Lys Gly Trp
   100 105 110
   Ile Val Arg Ile Pro Ser Pro Ser Asp Leu Pro Lys Tyr Val Val Phe
   115 120 125
   Glu Thr Lys Thr Asp Asp His Glu Ser Arg Asn Thr Asn Ala Tyr Gln
   130 135 140
   Arg Ser Ser Lys Phe Val Phe Cys Glu Leu Tyr Tyr Gly Lys Glu Val
   145                 150                 155                 160
   Lys Lys Tyr Met Leu Tyr Asp Ile Ser Asp Gly Arg Thr Leu Ser Gly
                   165                 170                 175
   Thr Asp Thr His Asn Phe Gly Met Arg Met Leu Val Thr Asn Asn Val
               180                 185                 190
   Asn Leu Val Gly Val Pro Asn Met Tyr Leu Pro Phe Thr Asp Ile Lys
           195                 200                 205
   Glu Phe Ile Asn Glu Lys Asn Arg Ile Ala Asp Asn Gly Pro Ser His
       210                 215                 220
   Asn Val Pro Ile Arg Leu Lys Leu Asp Lys Glu Lys Asn Val Ile Tyr
   225                 230                 235                 240
   Ile Ser Ala Lys Leu Asp Lys Gly Asn Gly Lys Asn Lys Asn Lys Ile
                   245                 250                 255
   Ser Asn Asp Pro Asn Ile Gly Ala Val Ala Ile Ile Ser Ala Thr Leu
               260                 265                 270
   Arg Asn Leu Asn Trp Lys Gly Asp Ile Glu Ile Ile Asn His Asn Leu
           275                 280                 285
   Leu Pro Ser Ser Ile Ser Ser Arg Ser Asn Gly Asn Lys Leu Leu Tyr
       290                 295                 300
   Ile Met Lys Lys Leu Gly Val Arg Phe Asn Asn Ile Asn Val Asn Trp
   305                 310                 315                 320
   Asn Asn Ile Lys Asn Asn Ile Asn Tyr Phe Phe Tyr Asn Ile Thr Ser
                   325                 330                 335
   Glu Lys Ile Val Ser Ile Tyr Tyr His Leu Tyr Val Glu Asp Lys Leu
               340                 345                 350
   Ser Asn Ala Arg Val Ile Phe Asp Asn His Ala Gly Cys Gly Lys Ser
           355                 360                 365
   Tyr Phe Arg Thr Leu Asn Asn Lys Ile Ile Pro Val Gly Lys Glu Ile
       370                 375                 380
   Pro Leu Pro Asp Leu Val Ile Phe Asp Ser Asp Gln Asn Ile Val Lys
   385                 390                 395                 400
   Val Ile Glu Ala Glu Lys Ala Glu Asn Val Tyr Asn Gly Val Glu Gln
   405 410 415
   Leu Ser Thr Phe Asp Lys Phe Ile Glu Ser Tyr Ile Asn Lys Tyr Tyr
   420 425 430
   Pro Gly Ala Ala Val Glu Cys Ser Val Ile Thr Trp Gly Lys Ser Ser
   435 440 445
   Asn Pro Tyr Val Ser Phe Tyr Leu Asp Lys Asp Gly Ser Ala Val Phe
   450 455 460
   Leu
   465
<210> 134
   <211> 164
   <212> PRT
   <213> Geobacillus thermoglucosidasius
<220>
   <223> R1.BtsI ABC75874.1
<400> 134
   Met Lys Ile Thr Glu Gly Ile Val His Val Ala Met Arg His Phe Leu
   1 5 10 15
   Lys Ser Asn Gly Trp Lys Leu Ile Ala Gly Gln Tyr Pro Gly Gly Ser
   20 25 30
   Asp Asp Glu Leu Thr Ala Leu Asn Ile Val Asp Pro Val Val Ala Arg
   35 40 45
   Asp Asn Ser Pro Asp Pro Arg Arg His Ser Leu Gly Lys Ile Val Pro
   50 55 60
   Asp Leu Ile Ala Tyr Lys Asn Asp Asp Leu Leu Val Ile Glu Ala Lys
   65 70 75 80
   Pro Lys Tyr Ser Gln Asp Asp Arg Asp Lys Leu Leu Tyr Leu Leu Ser
   85 90 95
   Glu Arg Lys His Asp Phe Tyr Ala Ala Leu Glu Lys Phe Ala Thr Glu
   100 105 110
   Arg Asn His Pro Glu Leu Leu Pro Val Ser Lys Leu Asn Ile Ile Pro
   115 120 125
   Gly Leu Ala Phe Ser Ala Ser Glu Asn Lys Phe Lys Lys Asp Pro Gly
   130 135 140
   Phe Val Tyr Ile Arg Val Ser Gly Ile Phe Glu Ala Phe Met Glu Gly
   145 150 155 160
   Tyr Asp Trp Gly
<210> 135
   <211> 328
   <212> PRT
   <213> Geobacillus thermoglucosidasius
<220>
   <223> R2.BtsI ABC75876.1
<400> 135
   Met Gln Ile Glu Gln Leu Met Lys Ser Leu Thr Ile Tyr Phe Asp Asp
   1               5                   10                  15
   Ile Gln Glu Gly Leu Trp Phe Lys Asn Leu His Pro Leu Leu Glu Ser
               20                  25                  30
   Ala Ser Leu Glu Ala Ile Thr Gly Ser Leu Lys Arg Asn Pro Asn Leu
           35                  40                  45
   Ala Asp Val Leu Lys Tyr Asp Arg Pro Asp Ile Ile Leu Thr Leu Asn
       50                  55                  60
   Gln Thr Pro Ile Leu Val Ile Glu Arg Thr Ile Glu Val Pro Ser Gly
   65                  70                  75                  80
   His Asn Val Gly Gln Arg Tyr Gly Arg Leu Ala Ala Ala Ser Glu Ala
                   85                  90                  95
   Gly Val Pro Leu Val Tyr Phe Gly Pro Tyr Ala Ala Arg Lys His Gly
               100                 105                 110
   Gly Ala Thr Glu Gly Pro Arg Tyr Met Asn Leu Arg Leu Phe Tyr Ala
           115                 120                 125
   Leu Asp Val Met Gln Lys Val Asn Gly Ser Ala Ile Thr Thr Ile Asn
       130                 135                 140
   Trp Pro Val Asp Gln Asn Phe Glu Ile Leu Gln Asp Pro Ser Lys Asp
   145                 150                 155                 160
   Lys Arg Met Lys Glu Tyr Leu Glu Met Phe Phe Asp Asn Leu Leu Lys
                   165                 170                 175
   Tyr Gly Ile Ala Gly Ile Asn Leu Ala Ile Arg Asn Ser Ser Phe Gln
               180                 185                 190
   Ala Glu Gln Leu Ala Glu Arg Glu Lys Phe Val Glu Thr Met Ile Thr
           195                 200                 205
   Asn Pro Glu Gln Tyr Asp Val Pro Pro Asp Ser Val Gln Ile Leu Asn
       210                 215                 220
   Ala Glu Arg Phe Phe Asn Glu Leu Gly Ile Ser Glu Asn Lys Arg Ile
   225                 230                 235                 240
   Ile Cys Asp Glu Val Val Leu Tyr Gln Val Gly Met Thr Tyr Val Arg
                   245                 250                 255
   Ser Asp Pro Tyr Thr Gly Met Ala Leu Leu Tyr Lys Tyr Leu Tyr Ile
               260                 265                 270
   Leu Gly Ser Glu Arg Asn Arg Cys Leu Ile Leu Lys Phe Pro Asn Ile
           275                 280                 285
   Thr Thr Asp Met Trp Lys Lys Val Ala Phe Gly Ser Arg Glu Arg Lys
       290                 295                 300
   Asp Val Arg Ile Tyr Arg Ser Val Ser Asp Gly Ile Leu Phe Ala Asp
   305                 310                 315                 320
   Gly Tyr Leu Ser Lys Glu Glu Leu
                   325
<210> 136
   <211> 275
   <212> PRT
   <213> Brevibacillus brevis
<220>
   <223> BbvCI subunit 1 AAX14652.1
<400> 136
   Met Ile Asn Glu Asp Phe Phe Ile Tyr Glu Gln Leu Ser His Lys Lys
   1               5                   10                  15
   Asn Leu Glu Gln Lys Gly Lys Asn Ala Phe Asp Glu Glu Thr Glu Glu
               20                  25                  30
   Leu Val Arg Gln Ala Lys Ser Gly Tyr His Ala Phe Ile Glu Gly Ile
           35                  40                  45
   Asn Tyr Asp Glu Val Thr Lys Leu Asp Leu Asn Ser Ser Val Ala Ala
       50                  55                  60
   Leu Glu Asp Tyr Ile Ser Ile Ala Lys Glu Ile Glu Lys Lys His Lys
   65                  70                  75                  80
   Met Phe Asn Trp Arg Ser Asp Tyr Ala Gly Ser Ile Ile Pro Glu Phe
                   85                  90                  95
   Leu Tyr Arg Ile Val His Val Ala Thr Val Lys Ala Gly Leu Lys Pro
               100                 105                 110
   Ile Phe Ser Thr Arg Asn Thr Ile Ile Glu Ile Ser Gly Ala Ala His
           115                 120                 125
   Arg Glu Gly Leu Gln Ile Arg Arg Lys Asn Glu Asp Phe Ala Leu Gly
       130                 135                 140
   Phe His Glu Val Asp Val Lys Ile Ala Ser Glu Ser His Arg Val Ile
   145                 150                 155                 160
   Ser Leu Ala Val Ala Cys Glu Val Lys Thr Asn Ile Asp Lys Asn Lys
                   165                 170                 175
   Leu Asn Gly Leu Asp Phe Ser Ala Glu Arg Met Lys Arg Thr Tyr Pro
               180                 185                 190
   Gly Ser Ala Tyr Phe Leu Ile Thr Glu Thr Leu Asp Phe Ser Pro Asp
           195                 200                 205
   Glu Asn His Ser Ser Gly Leu Ile Asp Glu Ile Tyr Val Leu Arg Lys
       210                 215                 220
   Gln Val Arg Thr Lys Asn Arg Val Gln Lys Ala Pro Leu Cys Pro Ser
   225                 230                 235                 240
   Val Phe Ala Glu Leu Leu Glu Asp Ile Leu Glu Ile Ser Tyr Arg Ala
                   245                 250                 255
   Ser Asn Val Lys Gly His Val Tyr Asp Arg Leu Glu Gly Gly Lys Leu
               260                 265                 270
   Ile Arg Val
           275
<210> 137
   <211> 285
   <212> PRT
   <213> Brevibacillus brevis
<220>
   <223> BbvCI subunit 2 AAX14653.1
<400> 137
   Met Phe Asn Gln Phe Asn Pro Leu Val Tyr Thr His Gly Gly Lys Leu
   1               5                   10                  15
   Glu Arg Lys Ser Lys Lys Asp Lys Thr Ala Ser Lys Val Phe Glu Glu
               20                  25                  30
   Phe Gly Val Met Glu Ala Tyr Asn Cys Trp Lys Glu Ala Ser Leu Cys
           35                  40                  45
   Ile Gln Gln Arg Asp Lys Asp Ser Val Leu Lys Leu Val Ala Ala Leu
       50                  55                  60
   Asn Thr Tyr Lys Asp Ala Val Glu Pro Ile Phe Asp Ser Arg Leu Asn
   65                  70                  75                  80
   Ser Ala Gln Glu Val Leu Gln Pro Ser Ile Leu Glu Glu Phe Phe Glu
                   85                  90                  95
   Tyr Leu Phe Ser Arg Ile Asp Ser Ile Val Gly Val Asn Ile Pro Ile
               100                 105                 110
   Arg His Pro Ala Lys Gly Tyr Leu Ser Leu Ser Phe Asn Pro His Asn
           115                 120                 125
   Ile Glu Thr Leu Ile Gln Ser Pro Glu Tyr Thr Val Arg Ala Lys Asp
       130                 135                 140
   His Asp Phe Ile Ile Gly Gly Ser Ala Lys Leu Thr Ile Gln Gly His
   145                 150                 155                 160
   Gly Gly Glu Gly Glu Thr Thr Asn Ile Val Val Pro Ala Val Ala Ile
                   165                 170                 175
   Glu Cys Lys Arg Tyr Leu Glu Arg Asn Met Leu Asp Glu Cys Ala Gly
               180                 185                 190
   Thr Ala Glu Arg Leu Lys Arg Ala Thr Pro Tyr Cys Leu Tyr Phe Val
           195                 200                 205
   Val Ala Glu Tyr Leu Lys Leu Asp Asp Gly Ala Pro Glu Leu Thr Glu
       210                 215                 220
   Ile Asp Glu Ile Tyr Ile Leu Arg His Gln Arg Asn Ser Glu Arg Asn
   225 230 235 240
   Lys Pro Gly Phe Lys Pro Asn Pro Ile Asp Gly Glu Leu Ile Trp Asp
   245 250 255
   Leu Tyr Gln Glu Val Met Asn His Leu Gly Lys Ile Trp Trp Asp Pro
   260 265 270
   Asn Ser Ala Leu Gln Arg Gly Lys Val Phe Asn Arg Pro
   275 280 285
<210> 138
   <211> 294
   <212> PRT
   <213> Bacillus pumilus
<220>
   <223> Bpu10I alpha subunit CAA74998.1
<400> 138
   Met Gly Val Glu Gln Glu Trp Ile Lys Asn Ile Thr Asp Met Tyr Gln
   1 5 10 15
   Ser Pro Glu Leu Ile Pro Ser His Ala Ser Asn Leu Leu His Gln Leu
   20 25 30
   Lys Arg Glu Lys Arg Asn Glu Lys Leu Lys Lys Ala Leu Glu Ile Ile
   35 40 45
   Thr Pro Asn Tyr Ile Ser Tyr Ile Ser Ile Leu Leu Asn Asn His Asn
   50 55 60
   Met Thr Arg Lys Glu Ile Val Ile Leu Val Asp Ala Leu Asn Glu Tyr
   65 70 75 80
   Met Asn Thr Leu Arg His Pro Ser Val Lys Ser Val Phe Ser His Gln
   85 90 95
   Ala Asp Phe Tyr Ser Ser Val Leu Pro Glu Phe Phe Asn Leu Leu Phe
   100 105 110
   Arg Asn Leu Ile Lys Gly Leu Asn Glu Lys Ile Lys Val Asn Ser Gln
   115 120 125
   Lys Asp Ile Ile Ile Asp Cys Ile Phe Asp Pro Tyr Asn Glu Gly Arg
   130 135 140
   Val Val Phe Lys Lys Lys Arg Val Asp Val Ala Ile Ile Leu Lys Asn
   145                 150                 155                 160
   Lys Phe Val Phe Asn Asn Val Glu Ile Ser Asp Phe Ala Ile Pro Leu
                   165                 170                 175
   Val Ala Ile Glu Ile Lys Thr Asn Leu Asp Lys Asn Met Leu Ser Gly
               180                 185                 190
   Ile Glu Gln Ser Val Asp Ser Leu Lys Glu Thr Phe Pro Leu Cys Leu
           195                 200                 205
   Tyr Tyr Cys Ile Thr Glu Leu Ala Asp Phe Ala Ile Glu Lys Gln Asn
       210                 215                 220
   Tyr Ala Ser Thr His Ile Asp Glu Val Phe Ile Leu Arg Lys Gln Lys
   225                 230                 235                 240
   Arg Gly Pro Val Arg Arg Gly Thr Pro Leu Glu Val Val His Ala Asp
                   245                 250                 255
   Leu Ile Leu Glu Val Val Glu Gln Val Gly Glu His Leu Ser Lys Phe
               260                 265                 270
   Lys Asp Pro Ile Lys Thr Leu Lys Ala Arg Met Thr Glu Gly Tyr Leu
           275                 280                 285
   Ile Lys Gly Lys Gly Lys
       290
<210> 139
   <211> 288
   <212> PRT
   <213> Bacillus pumilus
<220>
   <223> Bpu10I beta subunit CAA74999.1
<400> 139
   Met Thr Gln Ile Asp Leu Ser Asn Thr Lys His Gly Ser Ile Leu Phe
   1               5                   10                  15
   Glu Lys Gln Lys Asn Val Lys Glu Lys Tyr Leu Gln Gln Ala Tyr Lys
               20                  25                  30
   His Tyr Leu Tyr Phe Arg Arg Ser Ile Asp Gly Leu Glu Ile Thr Asn
           35                  40                  45
   Asp Glu Ala Ile Phe Lys Leu Thr Gln Ala Ala Asn Asn Tyr Arg Asp
       50                  55                  60
   Asn Val Leu Tyr Leu Phe Glu Ser Arg Pro Asn Ser Gly Gln Glu Ala
   65                  70                  75                  80
   Phe Arg Tyr Thr Ile Leu Glu Glu Phe Phe Tyr His Leu Phe Lys Asp
                   85                  90                  95
   Leu Val Lys Lys Lys Phe Asn Gln Glu Pro Ser Ser Ile Val Met Gly
               100                 105                 110
   Lys Ala Asn Ser Tyr Val Ser Leu Ser Phe Ser Pro Glu Ser Phe Leu
           115                 120                 125
   Gly Leu Tyr Glu Asn Pro Ile Pro Tyr Ile His Thr Lys Asp Gln Asp
       130                 135                 140
   Phe Val Leu Gly Cys Ala Val Asp Leu Lys Ile Ser Pro Lys Asn Glu
   145                 150                 155                 160
   Leu Asn Lys Glu Asn Glu Thr Glu Ile Val Val Pro Val Ile Ala Ile
                   165                 170                 175
   Glu Cys Lys Thr Tyr Ile Glu Arg Asn Met Leu Asp Ser Cys Ala Ala
               180                 185                 190
   Thr Ala Ser Arg Leu Lys Ala Ala Met Pro Tyr Cys Leu Tyr Ile Val
           195                 200                 205
   Ala Ser Glu Tyr Met Lys Met Asp Gln Ala Tyr Pro Glu Leu Thr Asp
       210                 215                 220
   Ile Asp Glu Val Phe Ile Leu Cys Lys Ala Ser Val Gly Glu Arg Thr
   225                 230                 235                 240
   Ala Leu Lys Lys Lys Gly Leu Pro Pro His Lys Leu Asp Glu Asn Leu
                   245                 250                 255
   Met Val Glu Leu Phe His Met Val Glu Arg His Leu Asn Arg Val Trp
               260                 265                 270
   Trp Ser Pro Asn Glu Ala Leu Ser Arg Gly Arg Val Ile Gly Arg Pro
           275                 280                 285
<210> 140
   <211> 358
   <212> PRT
   <213> Bacillus megaterium
<220>
   <223> BmrI ABM69266.1
<400> 140
   Met Asn Tyr Phe Ser Leu His Pro Asn Val Tyr Ala Thr Gly Arg Pro
   1               5                   10                  15
   Lys Gly Leu Ile Asn Met Leu Glu Ser Val Trp Ile Ser Asn Gln Lys
               20                  25                  30
   Pro Gly Asp Gly Thr Met Tyr Leu Ile Ser Gly Phe Ala Asn Tyr Asn
           35                  40                  45
   Gly Gly Ile Arg Phe Tyr Glu Thr Phe Thr Glu His Ile Asn His Gly
       50                  55                  60
   Gly Lys Val Ile Ala Ile Leu Gly Gly Ser Thr Ser Gln Arg Leu Ser
   65                  70                  75                  80
   Ser Lys Gln Val Val Ala Glu Leu Val Ser Arg Gly Val Asp Val Tyr
                   85                  90                  95
   Ile Ile Asn Arg Lys Arg Leu Leu His Ala Lys Leu Tyr Gly Ser Ser
               100                 105                 110
   Ser Asn Ser Gly Glu Ser Leu Val Val Ser Ser Gly Asn Phe Thr Gly
           115                 120                 125
   Pro Gly Met Ser Gln Asn Val Glu Ala Ser Leu Leu Leu Asp Asn Asn
       130                 135                 140
   Thr Thr Ser Ser Met Gly Phe Ser Trp Asn Gly Met Val Asn Ser Met
   145                 150                 155                 160
   Leu Asp Gln Lys Trp Gln Ile His Asn Leu Ser Asn Ser Asn Pro Thr
                   165                 170                 175
   Ser Pro Ser Trp Asn Leu Leu Tyr Asp Glu Arg Thr Thr Asn Leu Thr
               180                 185                 190
   Leu Asp Asp Thr Gln Lys Val Thr Leu Ile Leu Thr Leu Gly His Ala
           195                 200                 205
   Asp Thr Ala Arg Ile Gln Ala Ala Pro Lys Ser Lys Ala Gly Glu Gly
       210                 215                 220
   Ser Gln Tyr Phe Trp Leu Ser Lys Asp Ser Tyr Asp Phe Phe Pro Pro
   225 230 235 240
   Leu Thr Ile Arg Asn Lys Arg Gly Thr Lys Ala Thr Tyr Ser Cys Leu
   245 250 255
   Ile Asn Met Asn Tyr Leu Asp Ile Lys Tyr Ile Asp Ser Glu Cys Arg
   260 265 270
   Val Thr Phe Glu Ala Glu Asn Asn Phe Asp Phe Arg Leu Gly Thr Gly
   275 280 285
   Lys Leu Arg Tyr Thr Asn Val Ala Ala Ser Asp Asp Ile Ala Ala Ile
   290 295 300
   Thr Arg Val Gly Asp Ser Asp Tyr Glu Leu Arg Ile Ile Lys Lys Gly
   305 310 315 320
   Ser Ser Asn Tyr Asp Ala Leu Asp Ser Ala Ala Val Asn Phe Ile Gly
   325 330 335
   Asn Arg Gly Lys Arg Tyr Gly Tyr Ile Pro Asn Asp Glu Phe Gly Arg
   340 345 350
   Ile Ile Gly Ala Lys Phe
   355
<210> 141
   <211> 358
   <212> PRT
   <213> Bacillus firmus
<220>
   <223> BfiI CAC12783.1
<400> 141
   Met Asn Phe Phe Ser Leu His Pro Asn Val Tyr Ala Thr Gly Arg Pro
   1               5                   10                  15
   Lys Gly Leu Ile Gly Met Leu Glu Asn Val Trp Val Ser Asn His Thr
               20                  25                  30
   Pro Gly Glu Gly Thr Leu Tyr Leu Ile Ser Gly Phe Ser Asn Tyr Asn
           35                  40                  45
   Gly Gly Val Arg Phe Tyr Glu Thr Phe Thr Glu His Ile Asn Gln Gly
       50                  55                  60
   Gly Arg Val Ile Ala Ile Leu Gly Gly Ser Thr Ser Gln Arg Leu Ser
   65                  70                  75                  80
   Ser Arg Gln Val Val Glu Glu Leu Leu Asn Arg Gly Val Glu Val His
                   85                  90                  95
   Ile Ile Asn Arg Lys Arg Ile Leu His Ala Lys Leu Tyr Gly Thr Ser
               100                 105                 110
   Asn Asn Leu Gly Glu Ser Leu Val Val Ser Ser Gly Asn Phe Thr Gly
           115                 120                 125
   Pro Gly Met Ser Gln Asn Ile Glu Ala Ser Leu Leu Leu Asp Asn Asn
       130                 135                 140
   Thr Thr Gln Ser Met Gly Phe Ser Trp Asn Asp Met Ile Ser Glu Met
   145                 150                 155                 160
   Leu Asn Gln Asn Trp His Ile His Asn Met Thr Asn Ala Thr Asp Ala
                   165                 170                 175
   Ser Pro Gly Trp Asn Leu Leu Tyr Asp Glu Arg Thr Thr Asn Leu Thr
               180                 185                 190
   Leu Asp Glu Thr Glu Arg Val Thr Leu Ile Val Thr Leu Gly His Ala
           195                 200                 205
   Asp Thr Ala Arg Ile Gln Ala Ala Pro Gly Thr Thr Ala Gly Gln Gly
       210                 215                 220
   Thr Gln Tyr Phe Trp Leu Ser Lys Asp Ser Tyr Asp Phe Phe Pro Pro
   225                 230                 235                 240
   Leu Thr Ile Arg Asn Arg Arg Gly Thr Lys Ala Thr Tyr Ser Ser Leu
                   245                 250                 255
   Ile Asn Met Asn Tyr Ile Asp Ile Asn Tyr Thr Asp Thr Gln Cys Arg
               260                 265                 270
   Val Thr Phe Glu Ala Glu Asn Asn Phe Asp Phe Arg Leu Gly Thr Gly
           275                 280                 285
   Lys Leu Arg Tyr Thr Gly Val Ala Lys Ser Asn Asp Ile Ala Ala Ile
       290                 295                 300
   Thr Arg Val Gly Asp Ser Asp Tyr Glu Leu Arg Ile Ile Lys Gln Gly
   305                 310                 315                 320
   Thr Pro Glu His Ser Gln Leu Asp Pro Tyr Ala Val Ser Phe Ile Gly
   325 330 335
   Asn Arg Gly Lys Arg Phe Gly Tyr Ile Ser Asn Glu Glu Phe Gly Arg
   340 345 350
   Ile Ile Gly Val Thr Phe
   355
<210> 142
   <211> 846
   <212> PRT
   <213> Homo sapiens
<220>
   <223> hExoI (EXO1_HUMAN) Q9UQ84.2
<400> 142
   Met Gly Ile Gln Gly Leu Leu Gln Phe Ile Lys Glu Ala Ser Glu Pro
   1               5                   10                  15
   Ile His Val Arg Lys Tyr Lys Gly Gln Val Val Ala Val Asp Thr Tyr
               20                  25                  30
   Cys Trp Leu His Lys Gly Ala Ile Ala Cys Ala Glu Lys Leu Ala Lys
           35                  40                  45
   Gly Glu Pro Thr Asp Arg Tyr Val Gly Phe Cys Met Lys Phe Val Asn
       50                  55                  60
   Met Leu Leu Ser His Gly Ile Lys Pro Ile Leu Val Phe Asp Gly Cys
   65                  70                  75                  80
   Thr Leu Pro Ser Lys Lys Glu Val Glu Arg Ser Arg Arg Glu Arg Arg
                   85                  90                  95
   Gln Ala Asn Leu Leu Lys Gly Lys Gln Leu Leu Arg Glu Gly Lys Val
               100                 105                 110
   Ser Glu Ala Arg Glu Cys Phe Thr Arg Ser Ile Asn Ile Thr His Ala
           115                 120                 125
   Met Ala His Lys Val Ile Lys Ala Ala Arg Ser Gln Gly Val Asp Cys
       130                 135                 140
   Leu Val Ala Pro Tyr Glu Ala Asp Ala Gln Leu Ala Tyr Leu Asn Lys
   145                 150                 155                 160
   Ala Gly Ile Val Gln Ala Ile Ile Thr Glu Asp Ser Asp Leu Leu Ala
                   165                 170                 175
   Phe Gly Cys Lys Lys Val Ile Leu Lys Met Asp Gln Phe Gly Asn Gly
               180                 185                 190
   Leu Glu Ile Asp Gln Ala Arg Leu Gly Met Cys Arg Gln Leu Gly Asp
           195                 200                 205
   Val Phe Thr Glu Glu Lys Phe Arg Tyr Met Cys Ile Leu Ser Gly Cys
       210                 215                 220
   Asp Tyr Leu Ser Ser Leu Arg Gly Ile Gly Leu Ala Lys Ala Cys Lys
   225                 230                 235                 240
   Val Leu Arg Leu Ala Asn Asn Pro Asp Ile Val Lys Val Ile Lys Lys
                   245                 250                 255
   Ile Gly His Tyr Leu Lys Met Asn Ile Thr Val Pro Glu Asp Tyr Ile
               260                 265                 270
   Asn Gly Phe Ile Arg Ala Asn Asn Thr Phe Leu Tyr Gln Leu Val Phe
           275                 280                 285
   Asp Pro Ile Lys Arg Lys Leu Ile Pro Leu Asn Ala Tyr Glu Asp Asp
       290                 295                 300
   Val Asp Pro Glu Thr Leu Ser Tyr Ala Gly Gln Tyr Val Asp Asp Ser
   305                 310                 315                 320
   Ile Ala Leu Gln Ile Ala Leu Gly Asn Lys Asp Ile Asn Thr Phe Glu
                   325                 330                 335
   Gln Ile Asp Asp Tyr Asn Pro Asp Thr Ala Met Pro Ala His Ser Arg
               340                 345                 350
   Ser His Ser Trp Asp Asp Lys Thr Cys Gln Lys Ser Ala Asn Val Ser
           355                 360                 365
   Ser Ile Trp His Arg Asn Tyr Ser Pro Arg Pro Glu Ser Gly Thr Val
       370                 375                 380
   Ser Asp Ala Pro Gln Leu Lys Glu Asn Pro Ser Thr Val Gly Val Glu
   385                 390                 395                 400
   Arg Val Ile Ser Thr Lys Gly Leu Asn Leu Pro Arg Lys Ser Ser Ile
                   405                 410                 415
   Val Lys Arg Pro Arg Ser Ala Glu Leu Ser Glu Asp Asp Leu Leu Ser
   420 425 430
   Gln Tyr Ser Leu Ser Phe Thr Lys Lys Thr Lys Lys Asn Ser Ser Glu
   435 440 445
   Gly Asn Lys Ser Leu Ser Phe Ser Glu Val Phe Val Pro Asp Leu Val
   450 455 460
   Asn Gly Pro Thr Asn Lys Lys Ser Val Ser Thr Pro Pro Arg Thr Arg
   465 470 475 480
   Asn Lys Phe Ala Thr Phe Leu Gln Arg Lys Asn Glu Glu Ser Gly Ala
   485 490 495
   Val Val Val Pro Gly Thr Arg Ser Arg Phe Phe Cys Ser Ser Asp Ser
   500 505 510
   Thr Asp Cys Val Ser Asn Lys Val Ser Ile Gln Pro Leu Asp Glu Thr
   515 520 525
   Ala Val Thr Asp Lys Glu Asn Asn Leu His Glu Ser Glu Tyr Gly Asp
   530 535 540
   Gln Glu Gly Lys Arg Leu Val Asp Thr Asp Val Ala Arg Asn Ser Ser
   545 550 555 560
   Asp Asp Ile Pro Asn Asn His Ile Pro Gly Asp His Ile Pro Asp Lys
   565 570 575
   Ala Thr Val Phe Thr Asp Glu Glu Ser Tyr Ser Phe Glu Ser Ser Lys
   580 585 590
   Phe Thr Arg Thr Ile Ser Pro Pro Thr Leu Gly Thr Leu Arg Ser Cys
   595 600 605
   Phe Ser Trp Ser Gly Gly Leu Gly Asp Phe Ser Arg Thr Pro Ser Pro
   610 615 620
   Ser Pro Ser Thr Ala Leu Gln Gln Phe Arg Arg Lys Ser Asp Ser Pro
   625 630 635 640
   Thr Ser Leu Pro Glu Asn Asn Met Ser Asp Val Ser Gln Leu Lys Ser
   645 650 655
   Glu Glu Ser Ser Asp Asp Glu Ser His Pro Leu Arg Glu Glu Ala Cys
   660 665 670
   Ser Ser Gln Ser Gln Glu Ser Gly Glu Phe Ser Leu Gln Ser Ser Asn
           675                 680                 685
   Ala Ser Lys Leu Ser Gln Cys Ser Ser Lys Asp Ser Asp Ser Glu Glu
       690                 695                 700
   Ser Asp Cys Asn Ile Lys Leu Leu Asp Ser Gln Ser Asp Gln Thr Ser
   705                 710                 715                 720
   Lys Leu Arg Leu Ser His Phe Ser Lys Lys Asp Thr Pro Leu Arg Asn
                   725                 730                 735
   Lys Val Pro Gly Leu Tyr Lys Ser Ser Ser Ala Asp Ser Leu Ser Thr
               740                 745                 750
   Thr Lys Ile Lys Pro Leu Gly Pro Ala Arg Ala Ser Gly Leu Ser Lys
           755                 760                 765
   Lys Pro Ala Ser Ile Gln Lys Arg Lys His His Asn Ala Glu Asn Lys
       770                 775                 780
   Pro Gly Leu Gln Ile Lys Leu Asn Glu Leu Trp Lys Asn Phe Gly Phe
   785                 790                 795                 800
   Lys Lys Asp Ser Glu Lys Leu Pro Pro Cys Lys Lys Pro Leu Ser Pro
                   805                 810                 815
   Val Arg Asp Asn Ile Gln Leu Thr Pro Glu Ala Glu Glu Asp Ile Phe
               820                 825                 830
   Asn Lys Pro Glu Cys Gly Arg Val Gln Arg Ala Ile Phe Gln
           835                 840                 845
<210> 143
   <211> 702
   <212> PRT
   <213> Saccharomyces cerevisiae
<220>
   <223> Yeast ExoI (EXO1_YEAST) P39875.2
<400> 143
   Met Gly Ile Gln Gly Leu Leu Pro Gln Leu Lys Pro Ile Gln Asn Pro
   1               5                   10                  15
   Val Ser Leu Arg Arg Tyr Glu Gly Glu Val Leu Ala Ile Asp Gly Tyr
               20                  25                  30
   Ala Trp Leu His Arg Ala Ala Cys Ser Cys Ala Tyr Glu Leu Ala Met
           35                  40                  45
   Gly Lys Pro Thr Asp Lys Tyr Leu Gln Phe Phe Ile Lys Arg Phe Ser
       50                  55                  60
   Leu Leu Lys Thr Phe Lys Val Glu Pro Tyr Leu Val Phe Asp Gly Asp
   65                  70                  75                  80
   Ala Ile Pro Val Lys Lys Ser Thr Glu Ser Lys Arg Arg Asp Lys Arg
                   85                  90                  95
   Lys Glu Asn Lys Ala Ile Ala Glu Arg Leu Trp Ala Cys Gly Glu Lys
               100                 105                 110
   Lys Asn Ala Met Asp Tyr Phe Gln Lys Cys Val Asp Ile Thr Pro Glu
           115                 120                 125
   Met Ala Lys Cys Ile Ile Cys Tyr Cys Lys Leu Asn Gly Ile Arg Tyr
       130                 135                 140
   Ile Val Ala Pro Phe Glu Ala Asp Ser Gln Met Val Tyr Leu Glu Gln
   145                 150                 155                 160
   Lys Asn Ile Val Gln Gly Ile Ile Ser Glu Asp Ser Asp Leu Leu Val
                   165                 170                 175
   Phe Gly Cys Arg Arg Leu Ile Thr Lys Leu Asn Asp Tyr Gly Glu Cys
               180                 185                 190
   Leu Glu Ile Cys Arg Asp Asn Phe Ile Lys Leu Pro Lys Lys Phe Pro
           195                 200                 205
   Leu Gly Ser Leu Thr Asn Glu Glu Ile Ile Thr Met Val Cys Leu Ser
       210                 215                 220
   Gly Cys Asp Tyr Thr Asn Gly Ile Pro Lys Val Gly Leu Ile Thr Ala
   225                 230                 235                 240
   Met Lys Leu Val Arg Arg Phe Asn Thr Ile Glu Arg Ile Ile Leu Ser
                   245                 250                 255
   Ile Gln Arg Glu Gly Lys Leu Met Ile Pro Asp Thr Tyr Ile Asn Glu
               260                 265                 270
   Tyr Glu Ala Ala Val Leu Ala Phe Gln Phe Gln Arg Val Phe Cys Pro
           275                 280                 285
   Ile Arg Lys Lys Ile Val Ser Leu Asn Glu Ile Pro Leu Tyr Leu Lys
   290 295 300
   Asp Thr Glu Ser Lys Arg Lys Arg Leu Tyr Ala Cys Ile Gly Phe Val
   305 310 315 320
   Ile His Arg Glu Thr Gln Lys Lys Gln Ile Val His Phe Asp Asp Asp
   325 330 335
   Ile Asp His His Leu His Leu Lys Ile Ala Gln Gly Asp Leu Asn Pro
   340 345 350
   Tyr Asp Phe His Gln Pro Leu Ala Asn Arg Glu His Lys Leu Gln Leu
   355 360 365
   Ala Ser Lys Ser Asn Ile Glu Phe Gly Lys Thr Asn Thr Thr Asn Ser
   370 375 380
   Glu Ala Lys Val Lys Pro Ile Glu Ser Phe Phe Gln Lys Met Thr Lys
   385 390 395 400
   Leu Asp His Asn Pro Lys Val Ala Asn Asn Ile His Ser Leu Arg Gln
   405 410 415
   Ala Glu Asp Lys Leu Thr Met Ala Ile Lys Arg Arg Lys Leu Ser Asn
   420 425 430
   Ala Asn Val Val Gln Glu Thr Leu Lys Asp Thr Arg Ser Lys Phe Phe
   435 440 445
   Asn Lys Pro Ser Met Thr Val Val Glu Asn Phe Lys Glu Lys Gly Asp
   450 455 460
   Ser Ile Gln Asp Phe Lys Glu Asp Thr Asn Ser Gln Ser Leu Glu Glu
   465 470 475 480
   Pro Val Ser Glu Ser Gln Leu Ser Thr Gln Ile Pro Ser Ser Phe Ile
   485 490 495
   Thr Thr Asn Leu Glu Asp Asp Asp Asn Leu Ser Glu Glu Val Ser Glu
   500 505 510
   Val Val Ser Asp Ile Glu Glu Asp Arg Lys Asn Ser Glu Gly Lys Thr
   515 520 525
   Ile Gly Asn Glu Ile Tyr Asn Thr Asp Asp Asp Gly Asp Gly Asp Thr
   530 535 540
   Ser Glu Asp Tyr Ser Glu Thr Ala Glu Ser Arg Val Pro Thr Ser Ser
   545 550 555 560
   Thr Thr Ser Phe Pro Gly Ser Ser Gln Arg Ser Ile Ser Gly Cys Thr
   565 570 575
   Lys Val Leu Gln Lys Phe Arg Tyr Ser Ser Ser Phe Ser Gly Val Asn
   580 585 590
   Ala Asn Arg Gln Pro Leu Phe Pro Arg His Val Asn Gln Lys Ser Arg
   595 600 605
   Gly Met Val Tyr Val Asn Gln Asn Arg Asp Asp Asp Cys Asp Asp Asn
   610 615 620
   Asp Gly Lys Asn Gln Ile Thr Gln Arg Pro Ser Leu Arg Lys Ser Leu
   625 630 635 640
   Ile Gly Ala Arg Ser Gln Arg Ile Val Ile Asp Met Lys Ser Val Asp
   645 650 655
   Glu Arg Lys Ser Phe Asn Ser Ser Pro Ile Leu His Glu Glu Ser Lys
   660 665 670
   Lys Arg Asp Ile Glu Thr Thr Lys Ser Ser Gln Ala Arg Pro Ala Val
   675 680 685
   Arg Ser Ile Ser Leu Leu Ser Gln Phe Val Tyr Lys Gly Lys
   690 695 700
<210> 144
   <211> 475
   <212> PRT
   <213> Escherichia coli DH1
<220>
   <223> E.coli ExoI BAJ43803.1
<400> 144
   Met Met Asn Asp Gly Lys Gln Gln Ser Thr Phe Leu Phe His Asp Tyr
   1 5 10 15
   Glu Thr Phe Gly Thr His Pro Ala Leu Asp Arg Pro Ala Gln Phe Ala
   20 25 30
   Ala Ile Arg Thr Asp Ser Glu Phe Asn Val Ile Gly Glu Pro Glu Val
   35 40 45
   Phe Tyr Cys Lys Pro Ala Asp Asp Tyr Leu Pro Gln Pro Gly Ala Val
       50                  55                  60
   Leu Ile Thr Gly Ile Thr Pro Gln Glu Ala Arg Ala Lys Gly Glu Asn
   65                  70                  75                  80
   Glu Ala Ala Phe Ala Ala Arg Ile His Ser Leu Phe Thr Val Pro Lys
                   85                  90                  95
   Thr Cys Ile Leu Gly Tyr Asn Asn Val Arg Phe Asp Asp Glu Val Thr
               100                 105                 110
   Arg Asn Ile Phe Tyr Arg Asn Phe Tyr Asp Pro Tyr Ala Trp Ser Trp
           115                 120                 125
   Gln His Asp Asn Ser Arg Trp Asp Leu Leu Asp Val Met Arg Ala Cys
       130                 135                 140
   Tyr Ala Leu Arg Pro Glu Gly Ile Asn Trp Pro Glu Asn Asp Asp Gly
   145                 150                 155                 160
   Leu Pro Ser Phe Arg Leu Glu His Leu Thr Lys Ala Asn Gly Ile Glu
                   165                 170                 175
   His Ser Asn Ala His Asp Ala Met Ala Asp Val Tyr Ala Thr Ile Ala
               180                 185                 190
   Met Ala Lys Leu Val Lys Thr Arg Gln Pro Arg Leu Phe Asp Tyr Leu
           195                 200                 205
   Phe Thr His Arg Asn Lys His Lys Leu Met Ala Leu Ile Asp Val Pro
       210                 215                 220
   Gln Met Lys Pro Leu Val His Val Ser Gly Met Phe Gly Ala Trp Arg
   225                 230                 235                 240
   Gly Asn Thr Ser Trp Val Ala Pro Leu Ala Trp His Pro Glu Asn Arg
                   245                 250                 255
   Asn Ala Val Ile Met Val Asp Leu Ala Gly Asp Ile Ser Pro Leu Leu
               260                 265                 270
   Glu Leu Asp Ser Asp Thr Leu Arg Glu Arg Leu Tyr Thr Ala Lys Thr
           275                 280                 285
   Asp Leu Gly Asp Asn Ala Ala Val Pro Val Lys Leu Val His Ile Asn
       290                 295                 300
   Lys Cys Pro Val Leu Ala Gln Ala Asn Thr Leu Arg Pro Glu Asp Ala
   305 310 315 320
   Asp Arg Leu Gly Ile Asn Arg Gln His Cys Leu Asp Asn Leu Lys Ile
   325 330 335
   Leu Arg Glu Asn Pro Gln Val Arg Glu Lys Val Val Ala Ile Phe Ala
   340 345 350
   Glu Ala Glu Pro Phe Thr Pro Ser Asp Asn Val Asp Ala Gln Leu Tyr
   355 360 365
   Asn Gly Phe Phe Ser Asp Ala Asp Arg Ala Ala Met Lys Ile Val Leu
   370 375 380
   Glu Thr Glu Pro Arg Asn Leu Pro Ala Leu Asp Ile Thr Phe Val Asp
   385 390 395 400
   Lys Arg Ile Glu Lys Leu Leu Phe Asn Tyr Arg Ala Arg Asn Phe Pro
   405 410 415
   Gly Thr Leu Asp Tyr Ala Glu Gln Gln Arg Trp Leu Glu His Arg Arg
   420 425 430
   Gln Val Phe Thr Pro Glu Phe Leu Gln Gly Tyr Ala Asp Glu Leu Gln
   435 440 445
   Met Leu Val Gln Gln Tyr Ala Asp Asp Lys Glu Lys Val Ala Leu Leu
   450 455 460
   Lys Ala Leu Trp Gln Tyr Ala Glu Glu Ile Val
   465 470 475
<210> 145
   <211> 279
   <212> PRT
   <213> Homo sapiens
<220>
   <223> TREX2_HUMAN Q9BQ50.1
<400> 145
   Met Gly Arg Ala Gly Ser Pro Leu Pro Arg Ser Ser Trp Pro Arg Met
   1 5 10 15
   Asp Asp Cys Gly Ser Arg Ser Arg Cys Ser Pro Thr Leu Cys Ser Ser
   20 25 30
   Leu Arg Thr Cys Tyr Pro Arg Gly Asn Ile Thr Met Ser Glu Ala Pro
           35                  40                  45
   Arg Ala Glu Thr Phe Val Phe Leu Asp Leu Glu Ala Thr Gly Leu Pro
       50                  55                  60
   Ser Val Glu Pro Glu Ile Ala Glu Leu Ser Leu Phe Ala Val His Arg
   65                  70                  75                  80
   Ser Ser Leu Glu Asn Pro Glu His Asp Glu Ser Gly Ala Leu Val Leu
                   85                  90                  95
   Pro Arg Val Leu Asp Lys Leu Thr Leu Cys Met Cys Pro Glu Arg Pro
               100                 105                 110
   Phe Thr Ala Lys Ala Ser Glu Ile Thr Gly Leu Ser Ser Glu Gly Leu
           115                 120                 125
   Ala Arg Cys Arg Lys Ala Gly Phe Asp Gly Ala Val Val Arg Thr Leu
       130                 135                 140
   Gln Ala Phe Leu Ser Arg Gln Ala Gly Pro Ile Cys Leu Val Ala His
   145                 150                 155                 160
   Asn Gly Phe Asp Tyr Asp Phe Pro Leu Leu Cys Ala Glu Leu Arg Arg
                   165                 170                 175
   Leu Gly Ala Arg Leu Pro Arg Asp Thr Val Cys Leu Asp Thr Leu Pro
               180                 185                 190
   Ala Leu Arg Gly Leu Asp Arg Ala His Ser His Gly Thr Arg Ala Arg
           195                 200                 205
   Gly Arg Gln Gly Tyr Ser Leu Gly Ser Leu Phe His Arg Tyr Phe Arg
       210                 215                 220
   Ala Glu Pro Ser Ala Ala His Ser Ala Glu Gly Asp Val His Thr Leu
   225                 230                 235                 240
   Leu Leu Ile Phe Leu His Arg Ala Ala Glu Leu Leu Ala Trp Ala Asp
                   245                 250                 255
   Glu Gln Ala Arg Gly Trp Ala His Ile Glu Pro Met Tyr Leu Pro Pro
               260                 265                 270
   Asp Asp Pro Ser Leu Glu Ala
           275
<210> 146
   <211> 314
   <212> PRT
   <213> Mus musculus
<220>
   <223> TREX1_MOUSE Q91XB0.2
<400> 146
   Met Gly Ser Gln Thr Leu Pro His Gly His Met Gln Thr Leu Ile Phe
   1               5                   10                  15
   Leu Asp Leu Glu Ala Thr Gly Leu Pro Ser Ser Arg Pro Glu Val Thr
               20                  25                  30
   Glu Leu Cys Leu Leu Ala Val His Arg Arg Ala Leu Glu Asn Thr Ser
           35                  40                  45
   Ile Ser Gln Gly His Pro Pro Pro Val Pro Arg Pro Pro Arg Val Val
       50                  55                  60
   Asp Lys Leu Ser Leu Cys Ile Ala Pro Gly Lys Ala Cys Ser Pro Gly
   65                  70                  75                  80
   Ala Ser Glu Ile Thr Gly Leu Ser Lys Ala Glu Leu Glu Val Gln Gly
                   85                  90                  95
   Arg Gln Arg Phe Asp Asp Asn Leu Ala Ile Leu Leu Arg Ala Phe Leu
               100                 105                 110
   Gln Arg Gln Pro Gln Pro Cys Cys Leu Val Ala His Asn Gly Asp Arg
           115                 120                 125
   Tyr Asp Phe Pro Leu Leu Gln Thr Glu Leu Ala Arg Leu Ser Thr Pro
       130                 135                 140
   Ser Pro Leu Asp Gly Thr Phe Cys Val Asp Ser Ile Ala Ala Leu Lys
   145                 150                 155                 160
   Ala Leu Glu Gln Ala Ser Ser Pro Ser Gly Asn Gly Ser Arg Lys Ser
                   165                 170                 175
   Tyr Ser Leu Gly Ser Ile Tyr Thr Arg Leu Tyr Trp Gln Ala Pro Thr
               180                 185                 190
   Asp Ser His Thr Ala Glu Gly Asp Val Leu Thr Leu Leu Ser Ile Cys
           195                 200                 205
   Gln Trp Lys Pro Gln Ala Leu Leu Gln Trp Val Asp Glu His Ala Arg
   210 215 220
   Pro Phe Ser Thr Val Lys Pro Met Tyr Gly Thr Pro Ala Thr Thr Gly
   225 230 235 240
   Thr Thr Asn Leu Arg Pro His Ala Ala Thr Ala Thr Thr Pro Leu Ala
   245 250 255
   Thr Ala Asn Gly Ser Pro Ser Asn Gly Arg Ser Arg Arg Pro Lys Ser
   260 265 270
   Pro Pro Pro Glu Lys Val Pro Glu Ala Pro Ser Gln Glu Gly Leu Leu
   275 280 285
   Ala Pro Leu Ser Leu Leu Thr Leu Leu Thr Leu Ala Ile Ala Thr Leu
   290 295 300
   Tyr Gly Leu Phe Leu Ala Ser Pro Gly Gln
   305 310
<210> 147
   <211> 369
   <212> PRT
   <213> Homo sapiens
<220>
   <223> TREX1_HUMAN Q9NSU2.1
<400> 147
   Met Gly Pro Gly Ala Arg Arg Gln Gly Arg Ile Val Gln Gly Arg Pro
   1 5 10 15
   Glu Met Cys Phe Cys Pro Pro Pro Thr Pro Leu Pro Pro Leu Arg Ile
   20 25 30
   Leu Thr Leu Gly Thr His Thr Pro Thr Pro Cys Ser Ser Pro Gly Ser
   35 40 45
   Ala Ala Gly Thr Tyr Pro Thr Met Gly Ser Gln Ala Leu Pro Pro Gly
   50 55 60
   Pro Met Gln Thr Leu Ile Phe Phe Asp Met Glu Ala Thr Gly Leu Pro
   65 70 75 80
   Phe Ser Gln Pro Lys Val Thr Glu Leu Cys Leu Leu Ala Val His Arg
   85 90 95
   Cys Ala Leu Glu Ser Pro Pro Thr Ser Gln Gly Pro Pro Pro Thr Val
               100                 105                 110
   Pro Pro Pro Pro Arg Val Val Asp Lys Leu Ser Leu Cys Val Ala Pro
           115                 120                 125
   Gly Lys Ala Cys Ser Pro Ala Ala Ser Glu Ile Thr Gly Leu Ser Thr
       130                 135                 140
   Ala Val Leu Ala Ala His Gly Arg Gln Cys Phe Asp Asp Asn Leu Ala
   145                 150                 155                 160
   Asn Leu Leu Leu Ala Phe Leu Arg Arg Gln Pro Gln Pro Trp Cys Leu
                   165                 170                 175
   Val Ala His Asn Gly Asp Arg Tyr Asp Phe Pro Leu Leu Gln Ala Glu
               180                 185                 190
   Leu Ala Met Leu Gly Leu Thr Ser Ala Leu Asp Gly Ala Phe Cys Val
           195                 200                 205
   Asp Ser Ile Thr Ala Leu Lys Ala Leu Glu Arg Ala Ser Ser Pro Ser
       210                 215                 220
   Glu His Gly Pro Arg Lys Ser Tyr Ser Leu Gly Ser Ile Tyr Thr Arg
   225                 230                 235                 240
   Leu Tyr Gly Gln Ser Pro Pro Asp Ser His Thr Ala Glu Gly Asp Val
                   245                 250                 255
   Leu Ala Leu Leu Ser Ile Cys Gln Trp Arg Pro Gln Ala Leu Leu Arg
               260                 265                 270
   Trp Val Asp Ala His Ala Arg Pro Phe Gly Thr Ile Arg Pro Met Tyr
           275                 280                 285
   Gly Val Thr Ala Ser Ala Arg Thr Lys Pro Arg Pro Ser Ala Val Thr
       290                 295                 300
   Thr Thr Ala His Leu Ala Thr Thr Arg Asn Thr Ser Pro Ser Leu Gly
   305                 310                 315                 320
   Glu Ser Arg Gly Thr Lys Asp Leu Pro Pro Val Lys Asp Pro Gly Ala
                   325                 330                 335
   Leu Ser Arg Glu Gly Leu Leu Ala Pro Leu Gly Leu Leu Ala Ile Leu
               340                 345                 350
   Thr Leu Ala Val Ala Thr Leu Tyr Gly Leu Ser Leu Ala Thr Pro Gly
           355                 360                 365
   Glu
<210> 148
   <211> 315
   <212> PRT
   <213> Bos taurus
<220>
   <223> TREX1_BOVIN Q9BG99.1
<400> 148
   Met Gly Ser Arg Ala Leu Pro Pro Gly Pro Val Gln Thr Leu Ile Phe
   1               5                   10                  15
   Leu Asp Leu Glu Ala Thr Gly Leu Pro Phe Ser Gln Pro Lys Ile Thr
               20                  25                  30
   Glu Leu Cys Leu Leu Ala Val His Arg Tyr Ala Leu Glu Gly Leu Ser
           35                  40                  45
   Ala Pro Gln Gly Pro Ser Pro Thr Ala Pro Val Pro Pro Arg Val Leu
       50                  55                  60
   Asp Lys Leu Ser Leu Cys Val Ala Pro Gly Lys Val Cys Ser Pro Ala
   65                  70                  75                  80
   Ala Ser Glu Ile Thr Gly Leu Ser Thr Ala Val Leu Ala Ala His Gly
                   85                  90                  95
   Arg Arg Ala Phe Asp Ala Asp Leu Val Asn Leu Ile Arg Thr Phe Leu
               100                 105                 110
   Gln Arg Gln Pro Gln Pro Trp Cys Leu Val Ala His Asn Gly Asp Arg
           115                 120                 125
   Tyr Asp Phe Pro Leu Leu Arg Ala Glu Leu Ala Leu Leu Gly Leu Ala
       130                 135                 140
   Ser Ala Leu Asp Asp Ala Phe Cys Val Asp Ser Ile Ala Ala Leu Lys
   145                 150                 155                 160
   Ala Leu Glu Pro Thr Gly Ser Ser Ser Glu His Gly Pro Arg Lys Ser
                   165                 170                 175
   Tyr Ser Leu Gly Ser Val Tyr Thr Arg Leu Tyr Gly Gln Ala Pro Pro
   180 185 190
   Asp Ser His Thr Ala Glu Gly Asp Val Leu Ala Leu Leu Ser Val Cys
   195 200 205
   Gln Trp Arg Pro Arg Ala Leu Leu Arg Trp Val Asp Ala His Ala Lys
   210 215 220
   Pro Phe Ser Thr Val Lys Pro Met Tyr Val Ile Thr Thr Ser Thr Gly
   225 230 235 240
   Thr Asn Pro Arg Pro Ser Ala Val Thr Ala Thr Val Pro Leu Ala Arg
   245 250 255
   Ala Ser Asp Thr Gly Pro Asn Leu Arg Gly Asp Arg Ser Pro Lys Pro
   260 265 270
   Ala Pro Ser Pro Lys Met Cys Pro Gly Ala Pro Pro Gly Glu Gly Leu
   275 280 285
   Leu Ala Pro Leu Gly Leu Leu Ala Phe Leu Thr Leu Ala Val Ala Met
   290 295 300
   Leu Tyr Gly Leu Ser Leu Ala Met Pro Gly Gln
   305 310 315
<210> 149
   <211> 316
   <212> PRT
   <213> Rattus norvegicus
<220>
   <223> Rat TREX1 AAH91242.1
<400> 149
   Met Gly Ser Gln Ala Leu Pro His Gly His Met Gln Thr Leu Ile Phe
   1 5 10 15
   Leu Asp Leu Glu Ala Thr Gly Leu Pro Tyr Ser Gln Pro Lys Ile Thr
   20 25 30
   Glu Leu Cys Leu Leu Ala Val His Arg His Ala Leu Glu Asn Ser Ser
   35 40 45
   Met Ser Glu Gly Gln Pro Pro Pro Val Pro Lys Pro Pro Arg Val Val
   50 55 60
   Asp Lys Leu Ser Leu Cys Ile Ala Pro Gly Lys Pro Cys Ser Ser Gly
   65 70 75 80
   Ala Ser Glu Ile Thr Gly Leu Thr Thr Ala Gly Leu Glu Ala His Gly
                   85                  90                  95
   Arg Gln Arg Phe Asn Asp Asn Leu Ala Thr Leu Leu Gln Val Phe Leu
               100                 105                 110
   Gln Arg Gln Pro Gln Pro Cys Cys Leu Val Ala His Asn Gly Asp Arg
           115                 120                 125
   Tyr Asp Phe Pro Leu Leu Gln Ala Glu Leu Ala Ser Leu Ser Val Ile
       130                 135                 140
   Ser Pro Leu Asp Gly Thr Phe Cys Val Asp Ser Ile Ala Ala Leu Lys
   145                 150                 155                 160
   Thr Leu Glu Gln Ala Ser Ser Pro Ser Glu His Gly Pro Arg Lys Ser
                   165                 170                 175
   Tyr Ser Leu Gly Ser Ile Tyr Thr Arg Leu Tyr Gly Gln Ala Pro Thr
               180                 185                 190
   Asp Ser His Thr Ala Glu Gly Asp Val Leu Ala Leu Leu Ser Ile Cys
           195                 200                 205
   Gln Trp Lys Pro Gln Ala Leu Leu Gln Trp Val Asp Lys His Ala Arg
       210                 215                 220
   Pro Phe Ser Thr Ile Lys Pro Met Tyr Gly Met Ala Ala Thr Thr Gly
   225                 230                 235                 240
   Thr Ala Ser Pro Arg Leu Cys Ala Ala Thr Thr Ser Ser Pro Leu Ala
                   245                 250                 255
   Thr Ala Asn Leu Ser Pro Ser Asn Gly Arg Ser Arg Gly Lys Arg Pro
               260                 265                 270
   Thr Ser Pro Pro Pro Glu Asn Val Pro Glu Ala Pro Ser Arg Glu Gly
           275                 280                 285
   Leu Leu Ala Pro Leu Gly Leu Leu Thr Phe Leu Thr Leu Ala Ile Ala
       290                 295                 300
   Val Leu Tyr Gly Ile Phe Leu Ala Ser Pro Gly Gln
   305                 310                 315
<210> 150
   <211> 829
   <212> PRT
   <213> Homo sapiens
<220>
   <223> Human DNA2 AAH63664.1
<400> 150
   Phe Ala Ile Pro Ala Ser Arg Met Glu Gln Leu Asn Glu Leu Glu Leu
   1               5                   10                  15
   Leu Met Glu Lys Ser Phe Trp Glu Glu Ala Glu Leu Pro Ala Glu Leu
               20                  25                  30
   Phe Gln Lys Lys Val Val Ala Ser Phe Pro Arg Thr Val Leu Ser Thr
           35                  40                  45
   Gly Met Asp Asn Arg Tyr Leu Val Leu Ala Val Asn Thr Val Gln Asn
       50                  55                  60
   Lys Glu Gly Asn Cys Glu Lys Arg Leu Val Ile Thr Ala Ser Gln Ser
   65                  70                  75                  80
   Leu Glu Asn Lys Glu Leu Cys Ile Leu Arg Asn Asp Trp Cys Ser Val
                   85                  90                  95
   Pro Val Glu Pro Gly Asp Ile Ile His Leu Glu Gly Asp Cys Thr Ser
               100                 105                 110
   Asp Thr Trp Ile Ile Asp Lys Asp Phe Gly Tyr Leu Ile Leu Tyr Pro
           115                 120                 125
   Asp Met Leu Ile Ser Gly Thr Ser Ile Ala Ser Ser Ile Arg Cys Met
       130                 135                 140
   Arg Arg Ala Val Leu Ser Glu Thr Phe Arg Ser Ser Asp Pro Ala Thr
   145                 150                 155                 160
   Arg Gln Met Leu Ile Gly Thr Val Leu His Glu Val Phe Gln Lys Ala
                   165                 170                 175
   Ile Asn Asn Ser Phe Ala Pro Glu Lys Leu Gln Glu Leu Ala Phe Gln
               180                 185                 190
   Thr Ile Gln Glu Ile Arg His Leu Lys Glu Met Tyr Arg Leu Asn Leu
           195                 200                 205
   Ser Gln Asp Glu Ile Lys Gln Glu Val Glu Asp Tyr Leu Pro Ser Phe
       210                 215                 220
   Cys Lys Trp Ala Gly Asp Phe Met His Lys Asn Thr Ser Thr Asp Phe
   225                 230                 235                 240
   Pro Gln Met Gln Leu Ser Leu Pro Ser Asp Asn Ser Lys Asp Asn Ser
                   245                 250                 255
   Thr Cys Asn Ile Glu Val Val Lys Pro Met Asp Ile Glu Glu Ser Ile
               260                 265                 270
   Trp Ser Pro Arg Phe Gly Leu Lys Gly Lys Ile Asp Val Thr Val Gly
           275                 280                 285
   Val Lys Ile His Arg Gly Tyr Lys Thr Lys Tyr Lys Ile Met Pro Leu
       290                 295                 300
   Glu Leu Lys Thr Gly Lys Glu Ser Asn Ser Ile Glu His Arg Ser Gln
   305                 310                 315                 320
   Val Val Leu Tyr Thr Leu Leu Ser Gln Glu Arg Arg Ala Asp Pro Glu
                   325                 330                 335
   Ala Gly Leu Leu Leu Tyr Leu Lys Thr Gly Gln Met Tyr Pro Val Pro
               340                 345                 350
   Ala Asn His Leu Asp Lys Arg Glu Leu Leu Lys Leu Arg Asn Gln Met
           355                 360                 365
   Ala Phe Ser Leu Phe His Arg Ile Ser Lys Ser Ala Thr Arg Gln Lys
       370                 375                 380
   Thr Gln Leu Ala Ser Leu Pro Gln Ile Ile Glu Glu Glu Lys Thr Cys
   385                 390                 395                 400
   Lys Tyr Cys Ser Gln Ile Gly Asn Cys Ala Leu Tyr Ser Arg Ala Val
                   405                 410                 415
   Glu Gln Gln Met Asp Cys Ser Ser Val Pro Ile Val Met Leu Pro Lys
               420                 425                 430
   Ile Glu Glu Glu Thr Gln His Leu Lys Gln Thr His Leu Glu Tyr Phe
           435                 440                 445
   Ser Leu Trp Cys Leu Met Leu Thr Leu Glu Ser Gln Ser Lys Asp Asn
       450                 455                 460
   Lys Lys Asn His Gln Asn Ile Trp Leu Met Pro Ala Ser Glu Met Glu
   465 470 475 480
   Lys Ser Gly Ser Cys Ile Gly Asn Leu Ile Arg Met Glu His Val Lys
   485 490 495
   Ile Val Cys Asp Gly Gln Tyr Leu His Asn Phe Gln Cys Lys His Gly
   500 505 510
   Ala Ile Pro Val Thr Asn Leu Met Ala Gly Asp Arg Val Ile Val Ser
   515 520 525
   Gly Glu Glu Arg Ser Leu Phe Ala Leu Ser Arg Gly Tyr Val Lys Glu
   530 535 540
   Ile Asn Met Thr Thr Val Thr Cys Leu Leu Asp Arg Asn Leu Ser Val
   545 550 555 560
   Leu Pro Glu Ser Thr Leu Phe Arg Leu Asp Gln Glu Glu Lys Asn Cys
   565 570 575
   Asp Ile Asp Thr Pro Leu Gly Asn Leu Ser Lys Leu Met Glu Asn Thr
   580 585 590
   Phe Val Ser Lys Lys Leu Arg Asp Leu Ile Ile Asp Phe Arg Glu Pro
   595 600 605
   Gln Phe Ile Ser Tyr Leu Ser Ser Val Leu Pro His Asp Ala Lys Asp
   610 615 620
   Thr Val Ala Cys Ile Leu Lys Gly Leu Asn Lys Pro Gln Arg Gln Ala
   625 630 635 640
   Met Lys Lys Val Leu Leu Ser Lys Asp Tyr Thr Leu Ile Val Gly Met
   645 650 655
   Pro Gly Thr Gly Lys Thr Thr Thr Ile Cys Thr Leu Val Pro Ala Pro
   660 665 670
   Glu Gln Val Glu Lys Gly Gly Val Ser Asn Val Thr Glu Ala Lys Leu
   675 680 685
   Ile Val Phe Leu Thr Ser Ile Phe Val Lys Ala Gly Cys Ser Pro Ser
   690 695 700
   Asp Ile Gly Ile Ile Ala Pro Tyr Arg Gln Gln Leu Lys Ile Ile Asn
   705 710 715 720
   Asp Leu Leu Ala Arg Ser Ile Gly Met Val Glu Val Asn Thr Val Asp
                   725                 730                 735
   Lys Tyr Gln Gly Arg Asp Lys Ser Ile Val Leu Val Ser Phe Val Arg
               740                 745                 750
   Ser Asn Lys Asp Gly Thr Val Gly Glu Leu Leu Lys Asp Trp Arg Arg
           755                 760                 765
   Leu Asn Val Ala Ile Thr Arg Ala Lys His Lys Leu Ile Leu Leu Gly
       770                 775                 780
   Cys Val Pro Ser Leu Asn Cys Tyr Pro Pro Leu Glu Lys Leu Leu Asn
   785                 790                 795                 800
   His Leu Asn Ser Glu Lys Leu Ile Ile Asp Leu Pro Ser Arg Glu His
                   805                 810                 815
   Glu Ser Leu Cys His Ile Leu Gly Asp Phe Gln Arg Glu
               820                 825
<210> 151
   <211> 1522
   <212> PRT
   <213> Saccharomyces cerevisiae
<220>
   <223> DNA2YEAST P38859.1
<400> 151
   Met Pro Gly Thr Pro Gln Lys Asn Lys Arg Ser Ala Ser Ile Ser Val
   1               5                   10                  15
   Ser Pro Ala Lys Lys Thr Glu Glu Lys Glu Ile Ile Gln Asn Asp Ser
               20                  25                  30
   Lys Ala Ile Leu Ser Lys Gln Thr Lys Arg Lys Lys Lys Tyr Ala Phe
           35                  40                  45
   Ala Pro Ile Asn Asn Leu Asn Gly Lys Asn Thr Lys Val Ser Asn Ala
       50                  55                  60
   Ser Val Leu Lys Ser Ile Ala Val Ser Gln Val Arg Asn Thr Ser Arg
   65                  70                  75                  80
   Thr Lys Asp Ile Asn Lys Ala Val Ser Lys Ser Val Lys Gln Leu Pro
                   85                  90                  95
   Asn Ser Gln Val Lys Pro Lys Arg Glu Met Ser Asn Leu Ser Arg His
   100 105 110
   His Asp Phe Thr Gln Asp Glu Asp Gly Pro Met Glu Glu Val Ile Trp
   115 120 125
   Lys Tyr Ser Pro Leu Gln Arg Asp Met Ser Asp Lys Thr Thr Ser Ala
   130 135 140
   Ala Glu Tyr Ser Asp Asp Tyr Glu Asp Val Gln Asn Pro Ser Ser Thr
   145 150 155 160
   Pro Ile Val Pro Asn Arg Leu Lys Thr Val Leu Ser Phe Thr Asn Ile
   165 170 175
   Gln Val Pro Asn Ala Asp Val Asn Gln Leu Ile Gln Glu Asn Gly Asn
   180 185 190
   Glu Gln Val Arg Pro Lys Pro Ala Glu Ile Ser Thr Arg Glu Ser Leu
   195 200 205
   Arg Asn Ile Asp Asp Ile Leu Asp Asp Ile Glu Gly Asp Leu Thr Ile
   210 215 220
   Lys Pro Thr Ile Thr Lys Phe Ser Asp Leu Pro Ser Ser Pro Ile Lys
   225 230 235 240
   Ala Pro Asn Val Glu Lys Lys Ala Glu Val Asn Ala Glu Glu Val Asp
   245 250 255
   Lys Met Asp Ser Thr Gly Asp Ser Asn Asp Gly Asp Asp Ser Leu Ile
   260 265 270
   Asp Ile Leu Thr Gln Lys Tyr Val Glu Lys Arg Lys Ser Glu Ser Gln
   275 280 285
   Ile Thr Ile Gln Gly Asn Thr Asn Gln Lys Ser Gly Ala Gln Glu Ser
   290 295 300
   Cys Gly Lys Asn Asp Asn Thr Lys Ser Arg Gly Glu Ile Glu Asp His
   305 310 315 320
   Glu Asn Val Asp Asn Gln Ala Lys Thr Gly Asn Ala Phe Tyr Glu Asn
   325 330 335
   Glu Glu Asp Ser Asn Cys Gln Arg Ile Lys Lys Asn Glu Lys Ile Glu
   340 345 350
   Tyr Asn Ser Ser Asp Glu Phe Ser Asp Asp Ser Leu Ile Glu Leu Leu
           355                 360                 365
   Asn Glu Thr Gln Thr Gln Val Glu Pro Asn Thr Ile Glu Gln Asp Leu
       370                 375                 380
   Asp Lys Val Glu Lys Met Val Ser Asp Asp Leu Arg Ile Ala Thr Asp
   385                 390                 395                 400
   Ser Thr Leu Ser Ala Tyr Ala Leu Arg Ala Lys Ser Gly Ala Pro Arg
                   405                 410                 415
   Asp Gly Val Val Arg Leu Val Ile Val Ser Leu Arg Ser Val Glu Leu
               420                 425                 430
   Pro Lys Ile Gly Thr Gln Lys Ile Leu Glu Cys Ile Asp Gly Lys Gly
           435                 440                 445
   Glu Gln Ser Ser Val Val Val Arg His Pro Trp Val Tyr Leu Glu Phe
       450                 455                 460
   Glu Val Gly Asp Val Ile His Ile Ile Glu Gly Lys Asn Ile Glu Asn
   465                 470                 475                 480
   Lys Arg Leu Leu Ser Asp Asp Lys Asn Pro Lys Thr Gln Leu Ala Asn
                   485                 490                 495
   Asp Asn Leu Leu Val Leu Asn Pro Asp Val Leu Phe Ser Ala Thr Ser
               500                 505                 510
   Val Gly Ser Ser Val Gly Cys Leu Arg Arg Ser Ile Leu Gln Met Gln
           515                 520                 525
   Phe Gln Asp Pro Arg Gly Glu Pro Ser Leu Val Met Thr Leu Gly Asn
       530                 535                 540
   Ile Val His Glu Leu Leu Gln Asp Ser Ile Lys Tyr Lys Leu Ser His
   545                 550                 555                 560
   Asn Lys Ile Ser Met Glu Ile Ile Ile Gln Lys Leu Asp Ser Leu Leu
                   565                 570                 575
   Glu Thr Tyr Ser Phe Ser Ile Ile Ile Cys Asn Glu Glu Ile Gln Tyr
               580                 585                 590
   Val Lys Glu Leu Val Met Lys Glu His Ala Glu Asn Ile Leu Tyr Phe
           595                 600                 605
   Val Asn Lys Phe Val Ser Lys Ser Asn Tyr Gly Cys Tyr Thr Ser Ile
       610                 615                 620
   Ser Gly Thr Arg Arg Thr Gln Pro Ile Ser Ile Ser Asn Val Ile Asp
   625                 630                 635                 640
   Ile Glu Glu Asn Ile Trp Ser Pro Ile Tyr Gly Leu Lys Gly Phe Leu
                   645                 650                 655
   Asp Ala Thr Val Glu Ala Asn Val Glu Asn Asn Lys Lys His Ile Val
               660                 665                 670
   Pro Leu Glu Val Lys Thr Gly Lys Ser Arg Ser Val Ser Tyr Glu Val
           675                 680                 685
   Gln Gly Leu Ile Tyr Thr Leu Leu Leu Asn Asp Arg Tyr Glu Ile Pro
       690                 695                 700
   Ile Glu Phe Phe Leu Leu Tyr Phe Thr Arg Asp Lys Asn Met Thr Lys
   705                 710                 715                 720
   Phe Pro Ser Val Leu His Ser Ile Lys His Ile Leu Met Ser Arg Asn
                   725                 730                 735
   Arg Met Ser Met Asn Phe Lys His Gln Leu Gln Glu Val Phe Gly Gln
               740                 745                 750
   Ala Gln Ser Arg Phe Glu Leu Pro Pro Leu Leu Arg Asp Ser Ser Cys
           755                 760                 765
   Asp Ser Cys Phe Ile Lys Glu Ser Cys Met Val Leu Asn Lys Leu Leu
       770                 775                 780
   Glu Asp Gly Thr Pro Glu Glu Ser Gly Leu Val Glu Gly Glu Phe Glu
   785                 790                 795                 800
   Ile Leu Thr Asn His Leu Ser Gln Asn Leu Ala Asn Tyr Lys Glu Phe
                   805                 810                 815
   Phe Thr Lys Tyr Asn Asp Leu Ile Thr Lys Glu Glu Ser Ser Ile Thr
               820                 825                 830
   Cys Val Asn Lys Glu Leu Phe Leu Leu Asp Gly Ser Thr Arg Glu Ser
           835                 840                 845
   Arg Ser Gly Arg Cys Leu Ser Gly Leu Val Val Ser Glu Val Val Glu
       850                 855                 860
   His Glu Lys Thr Glu Gly Ala Tyr Ile Tyr Cys Phe Ser Arg Arg Arg
   865                 870                 875                 880
   Asn Asp Asn Asn Ser Gln Ser Met Leu Ser Ser Gln Ile Ala Ala Asn
                   885                 890                 895
   Asp Phe Val Ile Ile Ser Asp Glu Glu Gly His Phe Cys Leu Cys Gln
               900                 905                 910
   Gly Arg Val Gln Phe Ile Asn Pro Ala Lys Ile Gly Ile Ser Val Lys
           915                 920                 925
   Arg Lys Leu Leu Asn Asn Arg Leu Leu Asp Lys Glu Lys Gly Val Thr
       930                 935                 940
   Thr Ile Gln Ser Val Val Glu Ser Glu Leu Glu Gln Ser Ser Leu Ile
   945                 950                 955                 960
   Ala Thr Gln Asn Leu Val Thr Tyr Arg Ile Asp Lys Asn Asp Ile Gln
                   965                 970                 975
   Gln Ser Leu Ser Leu Ala Arg Phe Asn Leu Leu Ser Leu Phe Leu Pro
               980                 985                 990
   Ala Val Ser Pro Gly Val Asp Ile Val Asp Glu Arg Ser Lys Leu Cys
           995                 1000                1005
   Arg Lys Thr Lys Arg Ser Asp Gly Gly Asn Glu Ile Leu Arg Ser Leu
       1010                1015                1020
   Leu Val Asp Asn Arg Ala Pro Lys Phe Arg Asp Ala Asn Asp Asp Pro
   1025                1030                1035                1040
   Val Ile Pro Tyr Lys Leu Ser Lys Asp Thr Thr Leu Asn Leu Asn Gln
                   1045                1050                1055
   Lys Glu Ala Ile Asp Lys Val Met Arg Ala Glu Asp Tyr Ala Leu Ile
               1060                1065                1070
   Leu Gly Met Pro Gly Thr Gly Lys Thr Thr Val Ile Ala Glu Ile Ile
           1075                1080                1085
   Lys Ile Leu Val Ser Glu Gly Lys Arg Val Leu Leu Thr Ser Tyr Thr
       1090                1095                1100
   His Ser Ala Val Asp Asn Ile Leu Ile Lys Leu Arg Asn Thr Asn Ile
   1105 1110 1115 1120
   Ser Ile Met Arg Leu Gly Met Lys His Lys Val His Pro Asp Thr Gln
   1125 1130 1135
   Lys Tyr Val Pro Asn Tyr Ala Ser Val Lys Ser Tyr Asn Asp Tyr Leu
   1140 1145 1150
   Ser Lys Ile Asn Ser Thr Ser Val Val Ala Thr Thr Cys Leu Gly Ile
   1155 1160 1165
   Asn Asp Ile Leu Phe Thr Leu Asn Glu Lys Asp Phe Asp Tyr Val Ile
   1170 1175 1180
   Leu Asp Glu Ala Ser Gln Ile Ser Met Pro Val Ala Leu Gly Pro Leu
   1185 1190 1195 1200
   Arg Tyr Gly Asn Arg Phe Ile Met Val Gly Asp His Tyr Gln Leu Pro
   1205 1210 1215
   Pro Leu Val Lys Asn Asp Ala Ala Arg Leu Gly Gly Leu Glu Glu Ser
   1220 1225 1230
   Leu Phe Lys Thr Phe Cys Glu Lys His Pro Glu Ser Val Ala Glu Leu
   1235 1240 1245
   Thr Leu Gln Tyr Arg Met Cys Gly Asp Ile Val Thr Leu Ser Asn Phe
   1250 1255 1260
   Leu Ile Tyr Asp Asn Lys Leu Lys Cys Gly Asn Asn Glu Val Phe Ala
   1265 1270 1275 1280
   Gln Ser Leu Glu Leu Pro Met Pro Glu Ala Leu Ser Arg Tyr Arg Asn
   1285 1290 1295
   Glu Ser Ala Asn Ser Lys Gln Trp Leu Glu Asp Ile Leu Glu Pro Thr
   1300 1305 1310
   Arg Lys Val Val Phe Leu Asn Tyr Asp Asn Cys Pro Asp Ile Ile Glu
   1315 1320 1325
   Gln Ser Glu Lys Asp Asn Ile Thr Asn His Gly Glu Ala Glu Leu Thr
   1330 1335 1340
   Leu Gln Cys Val Glu Gly Met Leu Leu Ser Gly Val Pro Cys Glu Asp
   1345 1350 1355 1360
   Ile Gly Val Met Thr Leu Tyr Arg Ala Gln Leu Arg Leu Leu Lys Lys
                   1365                1370                1375
   Ile Phe Asn Lys Asn Val Tyr Asp Gly Leu Glu Ile Leu Thr Ala Asp
               1380                1385                1390
   Gln Phe Gln Gly Arg Asp Lys Lys Cys Ile Ile Ile Ser Met Val Arg
           1395                1400                1405
   Arg Asn Ser Gln Leu Asn Gly Gly Ala Leu Leu Lys Glu Leu Arg Arg
       1410                1415                1420
   Val Asn Val Ala Met Thr Arg Ala Lys Ser Lys Leu Ile Ile Ile Gly
   1425                1430                1435                1440
   Ser Lys Ser Thr Ile Gly Ser Val Pro Glu Ile Lys Ser Phe Val Asn
                   1445                1450                1455
   Leu Leu Glu Glu Arg Asn Trp Val Tyr Thr Met Cys Lys Asp Ala Leu
               1460                1465                1470
   Tyr Lys Tyr Lys Phe Pro Asp Arg Ser Asn Ala Ile Asp Glu Ala Arg
           1475                1480                1485
   Lys Gly Cys Gly Lys Arg Thr Gly Ala Lys Pro Ile Thr Ser Lys Ser
       1490                1495                1500
   Lys Phe Val Ser Asp Lys Pro Ile Ile Lys Glu Ile Leu Gln Glu Tyr
   1505                1510                1515                1520
   Glu Ser
<210> 152
   <211> 490
   <212> PRT
   <213> Human herpesvirus 2
<220>
   <223> VP16 AAA45863.1
<400> 152
   Met Asp Leu Leu Val Asp Asp Leu Phe Ala Asp Arg Asp Gly Val Ser
   1               5                   10                  15
   Pro Pro Pro Pro Arg Pro Ala Gly Gly Pro Lys Asn Thr Pro Ala Ala
               20                  25                  30
   Pro Pro Leu Tyr Ala Thr Gly Arg Leu Ser Gln Ala Gln Leu Met Pro
           35                  40                  45
   Ser Pro Pro Met Pro Val Pro Pro Ala Ala Leu Phe Asn Arg Leu Leu
   50 55 60
   Asp Asp Leu Gly Phe Ser Ala Gly Pro Ala Leu Cys Thr Met Leu Asp
   65 70 75 80
   Thr Trp Asn Glu Asp Leu Phe Ser Gly Phe Pro Thr Asn Ala Asp Met
   85 90 95
   Tyr Arg Glu Cys Lys Phe Leu Ser Thr Leu Pro Ser Asp Val Ile Asp
   100 105 110
   Trp Gly Asp Ala His Val Pro Glu Arg Ser Pro Ile Asp Ile Arg Ala
   115 120 125
   His Gly Asp Val Ala Phe Pro Thr Leu Pro Ala Thr Arg Asp Glu Leu
   130 135 140
   Pro Ser Tyr Tyr Glu Ala Met Ala Gln Phe Phe Arg Gly Glu Leu Arg
   145 150 155 160
   Ala Arg Glu Glu Ser Tyr Arg Thr Val Leu Ala Asn Phe Cys Ser Ala
   165 170 175
   Leu Tyr Arg Tyr Leu Arg Ala Ser Val Arg Gln Leu His Arg Gln Ala
   180 185 190
   His Met Arg Gly Arg Asn Arg Asp Leu Arg Glu Met Leu Arg Thr Thr
   195 200 205
   Ile Ala Asp Arg Tyr Tyr Arg Glu Thr Ala Arg Leu Ala Arg Val Leu
   210 215 220
   Phe Leu His Leu Tyr Leu Phe Leu Ser Arg Glu Ile Leu Trp Ala Ala
   225 230 235 240
   Tyr Ala Glu Gln Met Met Arg Pro Asp Leu Phe Asp Gly Leu Cys Cys
   245 250 255
   Asp Leu Glu Ser Trp Arg Gln Leu Ala Cys Leu Phe Gln Pro Leu Met
   260 265 270
   Phe Ile Asn Gly Ser Leu Thr Val Arg Gly Val Pro Val Glu Ala Arg
   275 280 285
   Arg Leu Arg Glu Leu Asn His Ile Arg Glu His Leu Asn Leu Pro Leu
   290 295 300
   Val Arg Ser Ala Ala Ala Glu Glu Pro Gly Ala Pro Leu Thr Thr Pro
   305 310 315 320
   Pro Val Leu Gln Gly Asn Gln Ala Arg Ser Ser Gly Tyr Phe Met Leu
   325 330 335
   Leu Ile Arg Ala Lys Leu Asp Ser Tyr Ser Ser Val Ala Thr Ser Glu
   340 345 350
   Gly Glu Ser Val Met Arg Glu His Ala Tyr Ser Arg Gly Arg Thr Arg
   355 360 365
   Asn Asn Tyr Gly Ser Thr Ile Glu Gly Leu Leu Asp Leu Pro Asp Asp
   370 375 380
   Asp Asp Ala Pro Ala Glu Ala Gly Leu Val Ala Pro Arg Met Ser Phe
   385 390 395 400
   Leu Ser Ala Gly Gln Arg Pro Arg Arg Leu Ser Thr Thr Ala Pro Ile
   405 410 415
   Thr Asp Val Ser Leu Gly Asp Glu Leu Arg Leu Asp Gly Glu Glu Val
   420 425 430
   Asp Met Thr Pro Ala Asp Ala Leu Asp Asp Phe Asp Leu Glu Met Leu
   435 440 445
   Gly Asp Val Glu Ser Pro Ser Pro Gly Met Thr His Asp Pro Val Ser
   450 455 460
   Tyr Gly Ala Leu Asp Val Asp Asp Phe Glu Phe Glu Gln Met Phe Thr
   465 470 475 480
   Asp Ala Met Gly Ile Asp Asp Phe Gly Gly
   485 490
<210> 153
   <211> 6101
   <212> NUCLEIC
   <213> Artificial sequence
<220>
   <223> pCLS2690
<400> 153
   gacggatcgg gagatctccc gatcccctat ggtgcactct cagtacaatc tgctctgatg 60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggag tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg    660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa atgaattccg tcgaccatgg ccaataccaa atataacgaa    900
   gagttcctgc tgtacctggc cggctttgtg gacgctgacg gtagcatcat cgctcagatt    960
   aaaccaagac agtctcggaa gtttaaacat gagctaagct tgacctttga tgtgactcaa   1020
   aagacccagc gccgttggtt tctggacaag ctagtggatg aaattggcgt tggttacgta   1080
   tatgattctg gatccgtttc ctattaccag ttaagcgaaa tcaagccgct gcacaacttc   1140
   ctgactcaac tgcagccgtt tctggaactg aaacagaaac aggcaaacct ggttctgaaa   1200
   attatcgaac agctgccgtc tgcaaaagaa tccccggcca aattcctgga agtttgtacc   1260
   tgggtggatc agattgcagc tctgaacgat tctaagacgc gtaaaaccac ttctgaaacc   1320
   gttcgtgctg tgctggacag cctgagcgag aagaagaaat cctccccggc ggccggtgga   1380
   tctgataagt ataatcaggc tctgtctaaa tacaaccaag cactgtccaa gtacaatcag   1440
   gccctgtctg gtggaggcgg ttccaacaaa aagttcctgc tgtatcttgc tggatttgtg   1500
   gatggtgatg gctccatcat tgctcagata aaaccacgtc aagggtataa gttcaaacac   1560
   cagctctcct tgacttttca ggtcactcag aagacacaaa gaaggtggtt cttggacaaa   1620
   ttggttgatc gtattggtgt gggctatgtc gctgaccgtg gctctgtgtc agactaccgc   1680
   ctgtctgaaa ttaagcctct tcataacttt ctcacccaac tgcaaccctt cttgaagctc   1740
   aaacagaagc aagcaaatct ggttttgaaa atcatcgagc aactgccatc tgccaaggag   1800
   tccctggaca agtttcttga agtgtgtact tgggtggatc agattgctgc cttgaatgac   1860
   tccaagacca gaaaaaccac ctctgagact gtgagggcag ttctggatag cctctctgag   1920
   aagaaaaagt cctctcctta gccatggccc gcggttcgaa ggtaagccta tccctaaccc   1980
   tctcctcggt ctcgattcta cgcgtaccgg ttagtaatga gtttaaacgg gggaggctaa   2040
   ctgaaacacg gaaggagaca ataccggaag gaacccgcgc tatgacggca ataaaaagac   2100
   agaataaaac gcacgggtgt tgggtcgttt gttcataaac gcggggttcg gtcccagggc   2160
   tggcactctg tcgatacccc accgagaccc cattggggcc aatacgcccg cgtttcttcc   2220
   ttttccccac cccacccccc aagttcgggt gaaggcccag ggctcgcagc caacgtcggg   2280
   gcggcaggcc ctgccatagc agatctgcgc agctggggct ctagggggta tccccacgcg   2340
   ccctgtagcg gcgcattaag cgcggcgggt gtggtggtta cgcgcagcgt gaccgctaca   2400
   cttgccagcg ccctagcgcc cgctcctttc gctttcttcc cttcctttct cgccacgttc   2460
   gccggctttc cccgtcaagc tctaaatcgg ggcatccctt tagggttccg atttagtgct   2520
   ttacggcacc tcgaccccaa aaaacttgat tagggtgatg gttcacgtag tgggccatcg   2580
   ccctgataga cggtttttcg ccctttgacg ttggagtcca cgttctttaa tagtggactc   2640
   ttgttccaaa ctggaacaac actcaaccct atctcggtct attcttttga tttataaggg   2700
   attttgggga tttcggccta ttggttaaaa aatgagctga tttaacaaaa atttaacgcg   2760
   aattaattct gtggaatgtg tgtcagttag ggtgtggaaa gtccccaggc tccccagcag   2820
   gcagaagtat gcaaagcatg catctcaatt agtcagcaac caggtgtgga aagtccccag   2880
   gctccccagc aggcagaagt atgcaaagca tgcatctcaa ttagtcagca accatagtcc   2940
   cgcccctaac tccgcccatc ccgcccctaa ctccgcccag ttccgcccat tctccgcccc   3000
   atggctgact aatttttttt atttatgcag aggccgaggc cgcctctgcc tctgagctat   3060
   tccagaagta gtgaggaggc ttttttggag gcctaggctt ttgcaaaaag ctcccgggag   3120
   cttgtatatc cattttcgga tctgatcagc acgtgttgac aattaatcat cggcatagta   3180
   tatcggcata gtataatacg acaaggtgag gaactaaacc atggccaagc ctttgtctca   3240
   agaagaatcc accctcattg aaagagcaac ggctacaatc aacagcatcc ccatctctga   3300
   agactacagc gtcgccagcg cagctctctc tagcgacggc cgcatcttca ctggtgtcaa   3360
   tgtatatcat ttactggggg gaccttgtgc agaactcgtg gtgctgggca ctgctgctgc   3420
   tgcggcagct ggcaacctga cttgtatcgt cgcgatcgga aatgagaaca ggggcatctt   3480
   gagcccctgc ggacggtgcc gacaggtgct tctcgatctg catcctggga tcaaagccat   3540
   agtgaaggac agtgatggac agccgacggc agttgggatt cgtgaattgc tgccctctgg   3600
   ttatgtgtgg gagggctaag cacttcgtgg ccgaggagca ggactgacac gtgctacgag   3660
   atttcgattc caccgccgcc ttctatgaaa ggttgggctt cggaatcgtt ttccgggacg   3720
   ccggctggat gatcctccag cgcggggatc tcatgctgga gttcttcgcc caccccaact   3780
   tgtttattgc agcttataat ggttacaaat aaagcaatag catcacaaat ttcacaaata   3840
   aagcattttt ttcactgcat tctagttgtg gtttgtccaa actcatcaat gtatcttatc   3900
   atgtctgtat accgtcgacc tctagctaga gcttggcgta atcatggtca tagctgtttc   3960
   ctgtgtgaaa ttgttatccg ctcacaattc cacacaacat acgagccgga agcataaagt   4020
   gtaaagcctg gggtgcctaa tgagtgagct aactcacatt aattgcgttg cgctcactgc   4080
   ccgctttcca gtcgggaaac ctgtcgtgcc agctgcatta atgaatcggc caacgcgcgg   4140
   ggagaggcgg tttgcgtatt gggcgctctt ccgcttcctc gctcactgac tcgctgcgct   4200
   cggtcgttcg gctgcggcga gcggtatcag ctcactcaaa ggcggtaata cggttatcca   4260
   cagaatcagg ggataacgca ggaaagaaca tgtgagcaaa aggccagcaa aaggccagga   4320
   accgtaaaaa ggccgcgttg ctggcgtttt tccataggct ccgcccccct gacgagcatc   4380
   acaaaaatcg acgctcaagt cagaggtggc gaaacccgac aggactataa agataccagg   4440
   cgtttccccc tggaagctcc ctcgtgcgct ctcctgttcc gaccctgccg cttaccggat   4500
   acctgtccgc ctttctccct tcgggaagcg tggcgctttc tcatagctca cgctgtaggt   4560
   atctcagttc ggtgtaggtc gttcgctcca agctgggctg tgtgcacgaa ccccccgttc   4620
   agcccgaccg ctgcgcctta tccggtaact atcgtcttga gtccaacccg gtaagacacg   4680
   acttatcgcc actggcagca gccactggta acaggattag cagagcgagg tatgtaggcg   4740
   gtgctacaga gttcttgaag tggtggccta actacggcta cactagaaga acagtatttg   4800
   gtatctgcgc tctgctgaag ccagttacct tcggaaaaag agttggtagc tcttgatccg   4860
   gcaaacaaac caccgctggt agcggttttt ttgtttgcaa gcagcagatt acgcgcagaa   4920
   aaaaaggatc tcaagaagat cctttgatct tttctacggg gtctgacgct cagtggaacg   4980
   aaaactcacg ttaagggatt ttggtcatga gattatcaaa aaggatcttc acctagatcc   5040
   ttttaaatta aaaatgaagt tttaaatcaa tctaaagtat atatgagtaa acttggtctg   5100
   acagttacca atgcttaatc agtgaggcac ctatctcagc gatctgtcta tttcgttcat   5160
   ccatagttgc ctgactcccc gtcgtgtaga taactacgat acgggagggc ttaccatctg   5220
   gccccagtgc tgcaatgata ccgcgagacc cacgctcacc ggctccagat ttatcagcaa   5280
   taaaccagcc agccggaagg gccgagcgca gaagtggtcc tgcaacttta tccgcctcca   5340
   tccagtctat taattgttgc cgggaagcta gagtaagtag ttcgccagtt aatagtttgc   5400
   gcaacgttgt tgccattgct acaggcatcg tggtgtcacg ctcgtcgttt ggtatggctt   5460
   cattcagctc cggttcccaa cgatcaaggc gagttacatg atcccccatg ttgtgcaaaa   5520
   aagcggttag ctccttcggt cctccgatcg ttgtcagaag taagttggcc gcagtgttat   5580
   cactcatggt tatggcagca ctgcataatt ctcttactgt catgccatcc gtaagatgct   5640
   tttctgtgac tggtgagtac tcaaccaagt cattctgaga atagtgtatg cggcgaccga   5700
   gttgctcttg cccggcgtca atacgggata ataccgcgcc acatagcaga actttaaaag   5760
   tgctcatcat tggaaaacgt tcttcggggc gaaaactctc aaggatctta ccgctgttga   5820
   gatccagttc gatgtaaccc actcgtgcac ccaactgatc ttcagcatct tttactttca   5880
   ccagcgtttc tgggtgagca aaaacaggaa ggcaaaatgc cgcaaaaaag ggaataaggg   5940
   cgacacggaa atgttgaata ctcatactct tcctttttca atattattga agcatttatc   6000
   agggttattg tctcatgagc ggatacatat ttgaatgtat ttagaaaaat aaacaaatag   6060
   gggttccgcg cacatttccc cgaaaagtgc cacctgacgt c                       6101
<210> 154
   <211> 5885
   <212> NUCLEIC
   <213> Artificial sequence
<220>
   <223> pCLS7673
<400> 154
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggag tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca 840
   ctgcttactg gcttatcgaa atgaattcga ctcactgttg ggagacccaa gctggctagt 900
   taagctatca caagtttgta caaaaaagca ggctggcgcg cctacacagc ggccttgcca 960
   ccatgggttc cgaggcaccc cgggccgaga cctttgtctt cctggacctg gaagccactg 1020
   ggctccccag tgtggagccc gagattgccg agctgtccct ctttgctgtc caccgctcct 1080
   ccctggagaa cccggagcac gacgagtctg gtgccctagt attgccccgg gtcctggaca 1140
   agctcacgct gtgcatgtgc ccggagcgcc ccttcactgc caaggccagc gagatcaccg 1200
   gcctgagcag tgagggcctg gcgcgatgcc ggaaggctgg ctttgatggc gccgtggtgc 1260
   ggacgctgca ggccttcctg agccgccagg cagggcccat ctgccttgtg gcccacaatg 1320
   gctttgatta tgatttcccc ctgctgtgtg ccgagctgcg gcgcctgggt gcccgcctgc 1380
   cccgggacac tgtctgcctg gacacgctgc cggccctgcg gggcctggac cgcgcccaca 1440
   gccacggcac ccgggcccgg ggccgccagg gttacagcct cggcagcctc ttccaccgct 1500
   acttccgggc agagccaagc gcagcccact cagccgaggg cgacgtgcac accctgctcc 1560
   tgatcttcct gcaccgcgcc gcagagctgc tcgcctgggc cgatgagcag gcccgtgggt 1620
   gggcccacat cgagcccatg tacttgccgc ctgatgaccc cagcctggag gcggccgact 1680
   gactcgagcg ctagcaccca gctttcttgt acaaagtggt gatctagagg gcccgcggtt 1740
   cgaaggtaag cctatcccta accctctcct cggtctcgat tctacgcgta ccggttagta 1800
   atgagtttaa acgggggagg ctaactgaaa cacggaagga gacaataccg gaaggaaccc 1860
   gcgctatgac ggcaataaaa agacagaata aaacgcacgg gtgttgggtc gtttgttcat 1920
   aaacgcgggg ttcggtccca gggctggcac tctgtcgata ccccaccgag accccattgg 1980
   ggccaatacg cccgcgtttc ttccttttcc ccaccccacc ccccaagttc gggtgaaggc 2040
   ccagggctcg cagccaacgt cggggcggca ggccctgcca tagcagatct gcgcagctgg 2100
   ggctctaggg ggtatcccca cgcgccctgt agcggcgcat taagcgcggc gggtgtggtg 2160
   gttacgcgca gcgtgaccgc tacacttgcc agcgccctag cgcccgctcc tttcgctttc 2220
   ttcccttcct ttctcgccac gttcgccggc tttccccgtc aagctctaaa tcggggcatc 2280
   cctttagggt tccgatttag tgctttacgg cacctcgacc ccaaaaaact tgattagggt 2340
   gatggttcac gtagtgggcc atcgccctga tagacggttt ttcgcccttt gacgttggag 2400
   tccacgttct ttaatagtgg actcttgttc caaactggaa caacactcaa ccctatctcg 2460
   gtctattctt ttgatttata agggattttg gggatttcgg cctattggtt aaaaaatgag 2520
   ctgatttaac aaaaatttaa cgcgaattaa ttctgtggaa tgtgtgtcag ttagggtgtg 2580
   gaaagtcccc aggctcccca gcaggcagaa gtatgcaaag catgcatctc aattagtcag 2640
   caaccaggtg tggaaagtcc ccaggctccc cagcaggcag aagtatgcaa agcatgcatc 2700
   tcaattagtc agcaaccata gtcccgcccc taactccgcc catcccgccc ctaactccgc 2760
   ccagttccgc ccattctccg ccccatggct gactaatttt ttttatttat gcagaggccg 2820
   aggccgcctc tgcctctgag ctattccaga agtagtgagg aggctttttt ggaggcctag 2880
   gcttttgcaa aaagctcccg ggagcttgta tatccatttt cggatctgat cagcacgtgt 2940
   tgacaattaa tcatcggcat agtatatcgg catagtataa tacgacaagg tgaggaacta 3000
   aaccatggcc aagcctttgt ctcaagaaga atccaccctc attgaaagag caacggctac 3060
   aatcaacagc atccccatct ctgaagacta cagcgtcgcc agcgcagctc tctctagcga 3120
   cggccgcatc ttcactggtg tcaatgtata tcattttact gggggacctt gtgcagaact 3180
   cgtggtgctg ggcactgctg ctgctgcggc agctggcaac ctgacttgta tcgtcgcgat 3240
   cggaaatgag aacaggggca tcttgagccc ctgcggacgg tgccgacagg tgcttctcga 3300
   tctgcatcct gggatcaaag ccatagtgaa ggacagtgat ggacagccga cggcagttgg 3360
   gattcgtgaa ttgctgccct ctggttatgt gtgggagggc taagcacttc gtggccgagg 3420
   agcaggactg acacgtgcta cgagatttcg attccaccgc cgccttctat gaaaggttgg 3480
   gcttcggaat cgttttccgg gacgccggct ggatgatcct ccagcgcggg gatctcatgc 3540
   tggagttctt cgcccacccc aacttgttta ttgcagctta taatggttac aaataaagca 3600
   atagcatcac aaatttcaca aataaagcat ttttttcact gcattctagt tgtggtttgt 3660
   ccaaactcat caatgtatct tatcatgtct gtataccgtc gacctctagc tagagcttgg 3720
   cgtaatcatg gtcatagctg tttcctgtgt gaaattgtta tccgctcaca attccacaca 3780
   acatacgagc cggaagcata aagtgtaaag cctggggtgc ctaatgagtg agctaactca 3840
   cattaattgc gttgcgctca ctgcccgctt tccagtcggg aaacctgtcg tgccagctgc 3900
   attaatgaat cggccaacgc gcggggagag gcggtttgcg tattgggcgc tcttccgctt 3960
   cctcgctcac tgactcgctg cgctcggtcg ttcggctgcg gcgagcggta tcagctcact 4020
   caaaggcggt aatacggtta tccacagaat caggggataa cgcaggaaag aacatgtgag 4080
   caaaaggcca gcaaaaggcc aggaaccgta aaaaggccgc gttgctggcg tttttccata 4140
   ggctccgccc ccctgacgag catcacaaaa atcgacgctc aagtcagagg tggcgaaacc 4200
   cgacaggact ataaagatac caggcgtttc cccctggaag ctccctcgtg cgctctcctg 4260
   ttccgaccct gccgcttacc ggatacctgt ccgcctttct cccttcggga agcgtggcgc 4320
   tttctcatag ctcacgctgt aggtatctca gttcggtgta ggtcgttcgc tccaagctgg 4380
   gctgtgtgca cgaacccccc gttcagcccg accgctgcgc cttatccggt aactatcgtc 4440
   ttgagtccaa cccggtaaga cacgacttat cgccactggc agcagccact ggtaacagga 4500
   ttagcagagc gaggtatgta ggcggtgcta cagagttctt gaagtggtgg cctaactacg 4560
   gctacactag aagaacagta tttggtatct gcgctctgct gaagccagtt accttcggaa   4620
   aaagagttgg tagctcttga tccggcaaac aaaccaccgc tggtagcggt ttttttgttt   4680
   gcaagcagca gattacgcgc agaaaaaaag gatctcaaga agatcctttg atcttttcta   4740
   cggggtctga cgctcagtgg aacgaaaact cacgttaagg gattttggtc atgagattat   4800
   caaaaaggat cttcacctag atccttttaa attaaaaatg aagttttaaa tcaatctaaa   4860
   gtatatatga gtaaacttgg tctgacagtt accaatgctt aatcagtgag gcacctatct   4920
   cagcgatctg tctatttcgt tcatccatag ttgcctgact ccccgtcgtg tagataacta   4980
   cgatacggga gggcttacca tctggcccca gtgctgcaat gataccgcga gacccacgct   5040
   caccggctcc agatttatca gcaataaacc agccagccgg aagggccgag cgcagaagtg   5100
   gtcctgcaac tttatccgcc tccatccagt ctattaattg ttgccgggaa gctagagtaa   5160
   gtagttcgcc agttaatagt ttgcgcaacg ttgttgccat tgctacaggc atcgtggtgt   5220
   cacgctcgtc gtttggtatg gcttcattca gctccggttc ccaacgatca aggcgagtta   5280
   catgatcccc catgttgtgc aaaaaagcgg ttagctcctt cggtcctccg atcgttgtca   5340
   gaagtaagtt ggccgcagtg ttatcactca tggttatggc agcactgcat aattctctta   5400
   ctgtcatgcc atccgtaaga tgcttttctg tgactggtga gtactcaacc aagtcattct   5460
   gagaatagtg tatgcggcga ccgagttgct cttgcccggc gtcaatacgg gataataccg   5520
   cgccacatag cagaacttta aaagtgctca tcattggaaa acgttcttcg gggcgaaaac   5580
   tctcaaggat cttaccgctg ttgagatcca gttcgatgta acccactcgt gcacccaact   5640
   gatcttcagc atcttttact ttcaccagcg tttctgggtg agcaaaaaca ggaaggcaaa   5700
   atgccgcaaa aaagggaata agggcgacac ggaaatgttg aatactcata ctcttccttt   5760
   ttcaatatta ttgaagcatt tatcagggtt attgtctcat gagcggatac atatttgaat   5820
   gtatttagaa aaataaacaa ataggggttc cgcgcacatt tccccgaaaa gtgccacctg   5880
   acgtc                                                               5885
<210> 155
   <211> 22
   <212> NUCLEIC
   <213> Artificial sequence
<220>
   <223> GS target sequence
<400> 155
   tgccccaggg tgagaaagtc ca 22
<210> 156
   <211> 6089
   <212> NUCLEIC
   <213> Artificial sequence
<220>
   <223> pCLS2222
<400> 156
   atggccaata ccaaatataa cgaagagttc ctgctgtacc tggccggctt tgtggacggt    60
   gacggtagca tcatcgctca gattaatcca aaccagtctt ctaagtttaa acatcgtcta   120
   cgtttgacct tttatgtgac tcaaaagacc cagcgccgtt ggtttctgga caaactagtg   180
   gatgaaattg gcgttggtta cgtacgtgat tctggatccg tttcccagta cgttttaagc   240
   gaaatcaagc cgctgcacaa cttcctgact caactgcagc cgtttctgga actgaaacag   300
   aaacaggcaa acctggttct gaaaattatc gaacagctgc cgtctgcaaa agaatccccg   360
   gacaaattcc tggaagtttg tacctgggtg gatcagattg cagctctgaa cgattctaag   420
   acgcgtaaaa ccacttctga aaccgttcgt gctgtgctgg acagcctgag cgggaagaag   480
   aaatcctccc cggcggccgg tggatctgat aagtataatc aggctctgtc taaatacaac   540
   caagcactgt ccaagtacaa tcaggccctg tctggtggag gcggttccaa caaaaagttc   600
   ctgctgtatc ttgctggatt tgtggattct gatggctcca tcattgctca gataaaacca   660
   cgtcaatcta acaagttcaa acaccagctc tccttgactt ttgcagtcac tcagaagaca   720
   caaagaaggt ggttcttgga caaattggtt gataggattg gtgtgggcta tgtctatgac   780
   agtggctctg tgtcagacta ccgcctgtct gaaattaagc ctcttcataa ctttctcacc   840
   caactgcaac ccttcttgaa gctcaaacag aagcaagcaa atctggtttt gaaaatcatc   900
   gagcaactgc catctgccaa ggagtcccct gacaagtttc ttgaagtgtg tacttgggtg     960
   gatcagattg ctgccttgaa tgactccaag accagaaaaa ccacctctga gactgtgagg    1020
   gcagttctgg atagcctctc tgagaagaaa aagtcctctc cttagtctag agggcccgcg    1080
   gttcgaaggt aagcctatcc ctaaccctct cctcggtctc gattctacgc gtaccggtta    1140
   gtaatgagtt taaacggggg aggctaactg aaacacggaa ggagacaata ccggaaggaa    1200
   cccgcgctat gacggcaata aaaagacaga ataaaacgca cgggtgttgg gtcgtttgtt    1260
   cataaacgcg gggttcggtc ccagggctgg cactctgtcg ataccccacc gagaccccat    1320
   tggggccaat acgcccgcgt ttcttccttt tccccacccc accccccaag ttcgggtgaa    1380
   ggcccagggc tcgcagccaa cgtcggggcg gcaggccctg ccatagcaga tctgcgcagc    1440
   tggggctcta gggggtatcc ccacgcgccc tgtagcggcg cattaagcgc ggcgggtgtg    1500
   gtggttacgc gcagcgtgac cgctacactt gccagcgccc tagcgcccgc tcctttcgct    1560
   ttcttccctt cctttctcgc cacgttcgcc ggctttcccc gtcaagctct aaatcggggc    1620
   atccctttag ggttccgatt tagtgcttta cggcacctcg accccaaaaa acttgattag    1680
   ggtgatggtt cacgtagtgg gccatcgccc tgatagacgg tttttcgccc tttgacgttg    1740
   gagtccacgt tctttaatag tggactcttg ttccaaactg gaacaacact caaccctatc    1800
   tcggtctatt cttttgattt ataagggatt ttggggattt cggcctattg gttaaaaaat    1860
   gagctgattt aacaaaaatt taacgcgaat taattctgtg gaatgtgtgt cagttagggt    1920
   gtggaaagtc cccaggctcc ccagcaggca gaagtatgca aagcatgcat ctcaattagt    1980
   cagcaaccag gtgtggaaag tccccaggct ccccagcagg cagaagtatg caaagcatgc    2040
   atctcaatta gtcagcaacc atagtcccgc ccctaactcc gcccatcccg cccctaactc    2100
   cgcccagttc cgcccattct ccgccccatg gctgactaat tttttttatt tatgcagagg    2160
   ccgaggccgc ctctgcctct gagctattcc agaagtagtg aggaggcttt tttggaggcc    2220
   taggcttttg caaaaagctc ccgggagctt gtatatccat tttcggatct gatcagcacg    2280
   tgttgacaat taatcatcgg catagtatat cggcatagta taatacgaca aggtgaggaa    2340
   ctaaaccatg gccaagcctt tgtctcaaga agaatccacc ctcattgaaa gagcaacggc    2400
   tacaatcaac agcatcccca tctctgaaga ctacagcgtc gccagcgcag ctctctctag    2460
   cgacggccgc atcttcactg gtgtcaatgt atatcatttt actgggggac cttgtgcaga    2520
   actcgtggtg ctgggcactg ctgctgctgc ggcagctggc aacctgactt gtatcgtcgc    2580
   gatcggaaat gagaacaggg gcatcttgag cccctgcgga cggtgccgac aggtgcttct    2640
   cgatctgcat cctgggatca aagccatagt gaaggacagt gatggacagc cgacggcagt    2700
   tgggattcgt gaattgctgc cctctggtta tgtgtgggag ggctaagcac ttcgtggccg    2760
   aggagcagga ctgacacgtg ctacgagatt tcgattccac cgccgccttc tatgaaaggt    2820
   tgggcttcgg aatcgttttc cgggacgccg gctggatgat cctccagcgc ggggatctca    2880
   tgctggagtt cttcgcccac cccaacttgt ttattgcagc ttataatggt tacaaataaa    2940
   gcaatagcat cacaaatttc acaaataaag catttttttc actgcattct agttgtggtt    3000
   tgtccaaact catcaatgta tcttatcatg tctgtatacc gtcgacctct agctagagct    3060
   tggcgtaatc atggtcatag ctgtttcctg tgtgaaattg ttatccgctc acaattccac    3120
   acaacatacg agccggaagc ataaagtgta aagcctgggg tgcctaatga gtgagctaac    3180
   tcacattaat tgcgttgcgc tcactgcccg ctttccagtc gggaaacctg tcgtgccagc    3240
   tgcattaatg aatcggccaa cgcgcgggga gaggcggttt gcgtattggg cgctcttccg    3300
   cttcctcgct cactgactcg ctgcgctcgg tcgttcggct gcggcgagcg gtatcagctc    3360
   actcaaaggc ggtaatacgg ttatccacag aatcagggga taacgcagga aagaacatgt    3420
   gagcaaaagg ccagcaaaag gccaggaacc gtaaaaaggc cgcgttgctg gcgtttttcc    3480
   ataggctccg cccccctgac gagcatcaca aaaatcgacg ctcaagtcag aggtggcgaa    3540
   acccgacagg actataaaga taccaggcgt ttccccctgg aagctccctc gtgcgctctc    3600
   ctgttccgac cctgccgctt accggatacc tgtccgcctt tctcccttcg ggaagcgtgg    3660
   cgctttctca tagctcacgc tgtaggtatc tcagttcggt gtaggtcgtt cgctccaagc    3720
   tgggctgtgt gcacgaaccc cccgttcagc ccgaccgctg cgccttatcc ggtaactatc    3780
   gtcttgagtc caacccggta agacacgact tatcgccact ggcagcagcc actggtaaca    3840
   ggattagcag agcgaggtat gtaggcggtg ctacagagtt cttgaagtgg tggcctaact    3900
   acggctacac tagaagaaca gtatttggta tctgcgctct gctgaagcca gttaccttcg    3960
   gaaaaagagt tggtagctct tgatccggca aacaaaccac cgctggtagc ggtttttttg    4020
   tttgcaagca gcagattacg cgcagaaaaa aaggatctca agaagatcct ttgatctttt    4080
   ctacggggtc tgacgctcag tggaacgaaa actcacgtta agggattttg gtcatgagat    4140
   tatcaaaaag gatcttcacc tagatccttt taaattaaaa atgaagtttt aaatcaatct    4200
   aaagtatata tgagtaaact tggtctgaca gttaccaatg cttaatcagt gaggcaccta    4260
   tctcagcgat ctgtctattt cgttcatcca tagttgcctg actccccgtc gtgtagataa    4320
   ctacgatacg ggagggctta ccatctggcc ccagtgctgc aatgataccg cgagacccac    4380
   gctcaccggc tccagattta tcagcaataa accagccagc cggaagggcc gagcgcagaa    4440
   gtggtcctgc aactttatcc gcctccatcc agtctattaa ttgttgccgg gaagctagag    4500
   taagtagttc gccagttaat agtttgcgca acgttgttgc cattgctaca ggcatcgtgg    4560
   tgtcacgctc gtcgtttggt atggcttcat tcagctccgg ttcccaacga tcaaggcgag    4620
   ttacatgatc ccccatgttg tgcaaaaaag cggttagctc cttcggtcct ccgatcgttg    4680
   tcagaagtaa gttggccgca gtgttatcac tcatggttat ggcagcactg cataattctc   4740
   ttactgtcat gccatccgta agatgctttt ctgtgactgg tgagtactca accaagtcat   4800
   tctgagaata gtgtatgcgg cgaccgagtt gctcttgccc ggcgtcaata cgggataata   4860
   ccgcgccaca tagcagaact ttaaaagtgc tcatcattgg aaaacgttct tcggggcgaa   4920
   aactctcaag gatcttaccg ctgttgagat ccagttcgat gtaacccact cgtgcaccca   4980
   actgatcttc agcatctttt actttcacca gcgtttctgg gtgagcaaaa acaggaaggc   5040
   aaaatgccgc aaaaaaggga ataagggcga cacggaaatg ttgaatactc atactcttcc   5100
   tttttcaata ttattgaagc atttatcagg gttattgtct catgagcgga tacatatttg   5160
   aatgtattta gaaaaataaa caaatagggg ttccgcgcac atttccccga aaagtgccac   5220
   ctgacgtcga cggatcggga gatctcccga tcccctatgg tgcactctca gtacaatctg   5280
   ctctgatgcc gcatagttaa gccagtatct gctccctgct tgtgtgttgg aggtcgctga   5340
   gtagtgcgcg agcaaaattt aagctacaac aaggcaaggc ttgaccgaca attgcatgaa   5400
   gaatctgctt agggttaggc gttttgcgct gcttcgcgat gtacgggcca gatatacgcg   5460
   ttgacattga ttattgacta gttattaata gtaatcaatt acggggtcat tagttcatag   5520
   cccatatatg gagttccgcg ttacataact tacggtaaat ggcccgcctg gctgaccgcc   5580
   caacgacccc cgcccattga cgtcaataat gacgtatgtt cccatagtaa cgccaatagg   5640
   gactttccat tgacgtcaat gggtggagta tttacggtaa actgcccact tggcagtaca   5700
   tcaagtgtat catatgccaa gtacgccccc tattgacgtc aatgacggta aatggcccgc   5760
   ctggcattat gcccagtaca tgaccttatg ggactttcct acttggcagt acatctacgt   5820
   attagtcatc gctattacca tggtgatgcg gttttggcag tacatcaatg ggcgtggata   5880
   gcggtttgac tcacggggat ttccaagtct ccaccccatt gacgtcaatg ggagtttgtt   5940
   ttggcaccaa aatcaacggg actttccaaa atgtcgtaac aactccgccc cattgacgca   6000
   aatgggcggt aggcgtgtac ggtgggaggt ctatataagc agagctctct ggctaactag   6060
   agaacccact gcttactggc ttatcgacc                                      6089
<210> 157
   <211> 6220
   <212> NUCLEIC
   <213> Artificial sequence
<220>
   <223> pCLS2510
<400> 157
   tcgagcgcta gcacccagct ttcttgtaca aagtggtgat ctagagggcc cgcggttcga     60
   aggtaagcct atccctaacc ctctcctcgg tctcgattct acgcgtaccg gttagtaatg    120
   agtttaaacg ggggaggcta actgaaacac ggaaggagac aataccggaa ggaacccgcg    180
   ctatgacggc aataaaaaga cagaataaaa cgcacgggtg ttgggtcgtt tgttcataaa    240
   cgcggggttc ggtcccaggg ctggcactct gtcgataccc caccgagacc ccattggggc    300
   caatacgccc gcgtttcttc cttttcccca ccccaccccc caagttcggg tgaaggccca    360
   gggctcgcag ccaacgtcgg ggcggcaggc cctgccatag cagatctgcg cagctggggc    420
   tctagggggt atccccacgc gccctgtagc ggcgcattaa gcgcggcggg tgtggtggtt    480
   acgcgcagcg tgaccgctac acttgccagc gccctagcgc ccgctccttt cgctttcttc    540
   ccttcctttc tcgccacgtt cgccggcttt ccccgtcaag ctctaaatcg ggggctccct     600
   ttagggttcc gatttagtgc tttacggcac ctcgacccca aaaaacttga ttagggtgat    660
   ggttcacgta gtgggccatc gccctgatag acggtttttc gccctttgac gttggagtcc    720
   acgttcttta atagtggact cttgttccaa actggaacaa cactcaaccc tatctcggtc    780
   tattcttttg atttataagg gattttgggg atttcggcct attggttaaa aaatgagctg    840
   atttaacaaa aatttaacgc gaattaattc tgtggaatgt gtgtcagtta gggtgtggaa    900
   agtccccagg ctccccagca ggcagaagta tgcaaagcat gcatctcaat tagtcagcaa    960
   ccaggtgtgg aaagtcccca ggctccccag caggcagaag tatgcaaagc atgcatctca   1020
   attagtcagc aaccatagtc ccgcccctaa ctccgcccat cccgccccta actccgccca   1080
   gttccgccca ttctccgccc catggctgac taattttttt tatttatgca gaggccgagg   1140
   ccgcctctgc ctctgagcta ttccagaagt agtgaggagg cttttttgga ggcctaggct   1200
   tttgcaaaaa gctcccggga gcttgtatat ccattttcgg atctgatcag cacgtgttga    1260
   caattaatca tcggcatagt atatcggcat agtataatac gacaaggtga ggaactaaac   1320
   catggccaag cctttgtctc aagaagaatc caccctcatt gaaagagcaa cggctacaat   1380
   caacagcatc cccatctctg aagactacag cgtcgccagc gcagctctct ctagcgacgg   1440
   ccgcatcttc actggtgtca atgtatatca ttttactggg ggaccttgtg cagaactcgt   1500
   ggtgctgggc actgctgctg ctgcggcagc tggcaacctg acttgtatcg tcgcgatcgg   1560
   aaatgagaac aggggcatct tgagcccctg cggacggtgc cgacaggtgc ttctcgatct   1620
   gcatcctggg atcaaagcca tagtgaagga cagtgatgga cagccgacgg cagttgggat 1680
   tcgtgaattg ctgccctctg gttatgtgtg ggagggctaa gcacttcgtg gccgaggagc 1740
   aggactgaca cgtgctacga gatttcgatt ccaccgccgc cttctatgaa aggttgggct 1800
   tcggaatcgt tttccgggac gccggctgga tgatcctcca gcgcggggat ctcatgctgg 1860
   agttcttcgc ccaccccaac ttgtttattg cagcttataa tggttacaaa taaagcaata 1920
   gcatcacaaa tttcacaaat aaagcatttt tttcactgca ttctagttgt ggtttgtcca 1980
   aactcatcaa tgtatcttat catgtctgta taccgtcgac ctctagctag agcttggcgt 2040
   aatcatggtc atagctgttt cctgtgtgaa attgttatcc gctcacaatt ccacacaaca 2100
   tacgagccgg aagcataaag tgtaaagcct ggggtgccta atgagtgagc taactcacat 2160
   taattgcgtt gcgctcactg cccgctttcc agtcgggaaa cctgtcgtgc cagctgcatt 2220
   aatgaatcgg ccaacgcgcg gggagaggcg gtttgcgtat tgggcgctct tccgcttcct 2280
   cgctcactga ctcgctgcgc tcggtcgttc ggctgcggcg agcggtatca gctcactcaa 2340
   aggcggtaat acggttatcc acagaatcag gggataacgc aggaaagaac atgtgagcaa 2400
   aaggccagca aaaggccagg aaccgtaaaa aggccgcgtt gctggcgttt ttccataggc 2460
   tccgcccccc tgacgagcat cacaaaaatc gacgctcaag tcagaggtgg cgaaacccga 2520
   caggactata aagataccag gcgtttcccc ctggaagctc cctcgtgcgc tctcctgttc 2580
   cgaccctgcc gcttaccgga tacctgtccg cctttctccc ttcgggaagc gtggcgcttt 2640
   ctcatagctc acgctgtagg tatctcagtt cggtgtaggt cgttcgctcc aagctgggct 2700
   gtgtgcacga accccccgtt cagcccgacc gctgcgcctt atccggtaac tatcgtcttg 2760
   agtccaaccc ggtaagacac gacttatcgc cactggcagc agccactggt aacaggatta 2820
   gcagagcgag gtatgtaggc ggtgctacag agttcttgaa gtggtggcct aactacggct 2880
   acactagaag aacagtattt ggtatctgcg ctctgctgaa gccagttacc ttcggaaaaa 2940
   gagttggtag ctcttgatcc ggcaaacaaa ccaccgctgg tagcggtttt tttgtttgca 3000
   agcagcagat tacgcgcaga aaaaaaggat ctcaagaaga tcctttgatc ttttctacgg 3060
   ggtctgacgc tcagtggaac gaaaactcac gttaagggat tttggtcatg agattatcaa 3120
   aaaggatctt cacctagatc cttttaaatt aaaaatgaag ttttaaatca atctaaagta 3180
   tatatgagta aacttggtct gacagttacc aatgcttaat cagtgaggca cctatctcag 3240
   cgatctgtct atttcgttca tccatagttg cctgactccc cgtcgtgtag ataactacga 3300
   tacgggaggg cttaccatct ggccccagtg ctgcaatgat accgcgagac ccacgctcac 3360
   cggctccaga tttatcagca ataaaccagc cagccggaag ggccgagcgc agaagtggtc 3420
   ctgcaacttt atccgcctcc atccagtcta ttaattgttg ccgggaagct agagtaagta 3480
   gttcgccagt taatagtttg cgcaacgttg ttgccattgc tacaggcatc gtggtgtcac 3540
   gctcgtcgtt tggtatggct tcattcagct ccggttccca acgatcaagg cgagttacat 3600
   gatcccccat gttgtgcaaa aaagcggtta gctccttcgg tcctccgatc gttgtcagaa 3660
   gtaagttggc cgcagtgtta tcactcatgg ttatggcagc actgcataat tctcttactg 3720
   tcatgccatc cgtaagatgc ttttctgtga ctggtgagta ctcaaccaag tcattctgag 3780
   aatagtgtat gcggcgaccg agttgctctt gcccggcgtc aatacgggat aataccgcgc 3840
   cacatagcag aactttaaaa gtgctcatca ttggaaaacg ttcttcgggg cgaaaactct 3900
   caaggatctt accgctgttg agatccagtt cgatgtaacc cactcgtgca cccaactgat 3960
   cttcagcatc ttttactttc accagcgttt ctgggtgagc aaaaacagga aggcaaaatg 4020
   ccgcaaaaaa gggaataagg gcgacacgga aatgttgaat actcatactc ttcctttttc 4080
   aatattattg aagcatttat cagggttatt gtctcatgag cggatacata tttgaatgta 4140
   tttagaaaaa taaacaaata ggggttccgc gcacatttcc ccgaaaagtg ccacctgacg 4200
   tcgacggatc gggagatctc ccgatcccct atggtgcact ctcagtacaa tctgctctga 4260
   tgccgcatag ttaagccagt atctgctccc tgcttgtgtg ttggaggtcg ctgagtagtg 4320
   cgcgagcaaa atttaagcta caacaaggca aggcttgacc gacaattgca tgaagaatct 4380
   gcttagggtt aggcgttttg cgctgcttcg cgatgtacgg gccagatata cgcgttgaca 4440
   ttgattattg actagttatt aatagtaatc aattacgggg tcattagttc atagcccata 4500
   tatggagttc cgcgttacat aacttacggt aaatggcccg cctggctgac cgcccaacga 4560
   cccccgccca ttgacgtcaa taatgacgta tgttcccata gtaacgccaa tagggacttt 4620
   ccattgacgt caatgggtgg agtatttacg gtaaactgcc cacttggcag tacatcaagt 4680
   gtatcatatg ccaagtacgc cccctattga cgtcaatgac ggtaaatggc ccgcctggca 4740
   ttatgcccag tacatgacct tatgggactt tcctacttgg cagtacatct acgtattagt 4800
   catcgctatt accatggtga tgcggttttg gcagtacatc aatgggcgtg gatagcggtt 4860
   tgactcacgg ggatttccaa gtctccaccc cattgacgtc aatgggagtt tgttttggca 4920
   ccaaaatcaa cgggactttc caaaatgtcg taacaactcc gccccattga cgcaaatggg 4980
   cggtaggcgt gtacggtggg aggtctatat aagcagagct ctctggctaa ctagagaacc 5040
   cactgcttac tggcttatcg aaatgaattc gactcactgt tgggagaccc aagctggcta 5100
   gttaagctat cacaagtttg tacaaaaaag caggctggcg cgccgaattc atggccaata 5160
   ccaaatataa cgaagagttc ctgctgtacc tggccggctt tgtggacggt gacggtagca 5220
   tcatcgctca gattaaacca aatcagtctc ataagtttaa acatgctcta cagttgacct 5280
   ttaaggtgac tcaaaagacc cagcgccgtt ggtttctgga caaactagtg gatgaaattg 5340
   gcgttggtta cgtacaggat agtggatccg tttccaacta catcttaagc gaaatcaagc 5400
   cgctgcacaa cttcctgact caactgcagc cgtttctgga actgaaacag aaacaggcaa   5460
   acctggccct gaaaattatc gaacagctgc cgtctgcaaa agaatccccg gacaaattcc   5520
   tggaagtttg tacctgggtg gatcaggttg cagctctgaa cgattctaag acgcgtaaaa   5580
   ccacttctga aaccgttcgt gctgtgctgg acagcctgag cgagaagaag aaatcctccc   5640
   cggcggccgg tggatctgat aagtataatc aggctctgtc taaatacaac caagcactgt   5700
   ccaagtacaa tcaggccctg tctggtggag gcggttccaa caaaaagttc ctgctgtatc   5760
   ttgctggatt tgtggattct gatggctcca tcattgctca gataaaacca aatcaatctc   5820
   acaagttcaa acaccagctc tccttggcct ttcaagtcac tcagaagaca caaagaaggt   5880
   ggttcttgga caaattggtt gataggattg gtgtgggcta tgtcagagac agaggctctg   5940
   tgtcagacta catcctgtct aaaattaagc ctcttcataa ctttctcacc caactgcaac   6000
   ccttcttgaa gctcaaacag aagcaagcaa atctggtttt gaaaatcatc gagcaactgc   6060
   catctgccaa ggagtcccct gacaagtttc ttgaagtgtg tacttgggtg gatcaggttg   6120
   ctgccttgaa tgactccaag accagaaaaa ccacctctga gactgtgagg gcagttctgg   6180
   atagcctctc tgagaagaaa aagtcctctc cttagagatc                         6220
<210> 158
   <211> 6233
   <212> NUCLEIC
   <213> Artificial sequence
<220>
   <223> pCLS6163
<400> 158
   taactcgagc gctagcaccc agctttcttg tacaaagtgg tgatctagag ggcccgcggt      60
   tcgaaggtaa gcctatccct aaccctctcc tcggtctcga ttctacgcgt accggttagt     120
   aatgagttta aacgggggag gctaactgaa acacggaagg agacaatacc ggaaggaacc     180
   cgcgctatga cggcaataaa aagacagaat aaaacgcacg ggtgttgggt cgtttgttca     240
   taaacgcggg gttcggtccc agggctggca ctctgtcgat accccaccga gaccccattg     300
   gggccaatac gcccgcgttt cttccttttc cccaccccac cccccaagtt cgggtgaagg     360
   cccagggctc gcagccaacg tcggggcggc aggccctgcc atagcagatc tgcgcagctg     420
   gggctctagg gggtatcccc acgcgccctg tagcggcgca ttaagcgcgg cgggtgtggt     480
   ggttacgcgc agcgtgaccg ctacacttgc cagcgcccta gcgcccgctc ctttcgcttt     540
   cttcccttcc tttctcgcca cgttcgccgg ctttccccgt caagctctaa atcgggggat    600
   ccctttaggg ttccgattta gtgctttacg gcacctcgac cccaaaaaac ttgattaggg     660
   tgatggttca cgtagtgggc catcgccctg atagacggtt tttcgccctt tgacgttgga     720
   gtccacgttc tttaatagtg gactcttgtt ccaaactgga acaacactca accctatctc     780
   ggtctattct tttgatttat aagggatttt ggggatttcg gcctattggt taaaaaatga     840
   gctgatttaa caaaaattta acgcgaatta attctgtgga atgtgtgtca gttagggtgt     900
   ggaaagtccc caggctcccc agcaggcaga agtatgcaaa gcatgcatct caattagtca     960
   gcaaccaggt gtggaaagtc cccaggctcc ccagcaggca gaagtatgca aagcatgcat    1020
   ctcaattagt cagcaaccat agtcccgccc ctaactccgc ccatcccgcc cctaactccg    1080
   cccagttccg cccattctcc gccccatggc tgactaattt tttttattta tgcagaggcc    1140
   gaggccgcct ctgcctctga gctattccag aagtagtgag gaggcttttt tggaggccta    1200
   ggcttttgca aaaagctccc gggagcttgt atatccattt tcggatctga tcagcacgtg    1260
   ttgacaatta atcatcggca tagtatatcg gcatagtata atacgacaag gtgaggaact    1320
   aaaccatggc caagcctttg tctcaagaag aatccaccct cattgaaaga gcaacggcta    1380
   caatcaacag catccccatc tctgaagact acagcgtcgc cagcgcagct ctctctagcg    1440
   acggccgcat cttcactggt gtcaatgtat atcattttac tgggggacct tgtgcagaac    1500
   tcgtggtgct gggcactgct gctgctgcgg cagctggcaa cctgacttgt atcgtcgcga    1560
   tcggaaatga gaacaggggc atcttgagcc cctgcggacg gtgccgacag gtgcttctcg    1620
   atctgcatcc tgggatcaaa gccatagtga aggacagtga tggacagccg acggcagttg    1680
   ggattcgtga attgctgccc tctggttatg tgtgggaggg ctaagcactt cgtggccgag    1740
   gagcaggact gacacgtgct acgagatttc gattccaccg ccgccttcta tgaaaggttg    1800
   ggcttcggaa tcgttttccg ggacgccggc tggatgatcc tccagcgcgg ggatctcatg    1860
   ctggagttct tcgcccaccc caacttgttt attgcagctt ataatggtta caaataaagc    1920
   aatagcatca caaatttcac aaataaagca tttttttcac tgcattctag ttgtggtttg    1980
   tccaaactca tcaatgtatc ttatcatgtc tgtataccgt cgacctctag ctagagcttg    2040
   gcgtaatcat ggtcatagct gtttcctgtg tgaaattgtt atccgctcac aattccacac    2100
   aacatacgag ccggaagcat aaagtgtaaa gcctggggtg cctaatgagt gagctaactc    2160
   acattaattg cgttgcgctc actgcccgct ttccagtcgg gaaacctgtc gtgccagctg    2220
   cattaatgaa tcggccaacg cgcggggaga ggcggtttgc gtattgggcg ctcttccgct   2280
   tcctcgctca ctgactcgct gcgctcggtc gttcggctgc ggcgagcggt atcagctcac   2340
   tcaaaggcgg taatacggtt atccacagaa tcaggggata acgcaggaaa gaacatgtga   2400
   gcaaaaggcc agcaaaaggc caggaaccgt aaaaaggccg cgttgctggc gtttttccat   2460
   aggctccgcc cccctgacga gcatcacaaa aatcgacgct caagtcagag gtggcgaaac   2520
   ccgacaggac tataaagata ccaggcgttt ccccctggaa gctccctcgt gcgctctcct   2580
   gttccgaccc tgccgcttac cggatacctg tccgcctttc tcccttcggg aagcgtggcg   2640
   ctttctcata gctcacgctg taggtatctc agttcggtgt aggtcgttcg ctccaagctg   2700
   ggctgtgtgc acgaaccccc cgttcagccc gaccgctgcg ccttatccgg taactatcgt   2760
   cttgagtcca acccggtaag acacgactta tcgccactgg cagcagccac tggtaacagg   2820
   attagcagag cgaggtatgt aggcggtgct acagagttct tgaagtggtg gcctaactac   2880
   ggctacacta gaagaacagt atttggtatc tgcgctctgc tgaagccagt taccttcgga   2940
   aaaagagttg gtagctcttg atccggcaaa caaaccaccg ctggtagcgg tttttttgtt   3000
   tgcaagcagc agattacgcg cagaaaaaaa ggatctcaag aagatccttt gatcttttct   3060
   acggggtctg acgctcagtg gaacgaaaac tcacgttaag ggattttggt catgagatta   3120
   tcaaaaagga tcttcaccta gatcctttta aattaaaaat gaagttttaa atcaatctaa   3180
   agtatatatg agtaaacttg gtctgacagt taccaatgct taatcagtga ggcacctatc   3240
   tcagcgatct gtctatttcg ttcatccata gttgcctgac tccccgtcgt gtagataact   3300
   acgatacggg agggcttacc atctggcccc agtgctgcaa tgataccgcg agacccacgc   3360
   tcaccggctc cagatttatc agcaataaac cagccagccg gaagggccga gcgcagaagt   3420
   ggtcctgcaa ctttatccgc ctccatccag tctattaatt gttgccggga agctagagta   3480
   agtagttcgc cagttaatag tttgcgcaac gttgttgcca ttgctacagg catcgtggtg   3540
   tcacgctcgt cgtttggtat ggcttcattc agctccggtt cccaacgatc aaggcgagtt   3600
   acatgatccc ccatgttgtg caaaaaagcg gttagctcct tcggtcctcc gatcgttgtc   3660
   agaagtaagt tggccgcagt gttatcactc atggttatgg cagcactgca taattctctt   3720
   actgtcatgc catccgtaag atgcttttct gtgactggtg agtactcaac caagtcattc   3780
   tgagaatagt gtatgcggcg accgagttgc tcttgcccgg cgtcaatacg ggataatacc   3840
   gcgccacata gcagaacttt aaaagtgctc atcattggaa aacgttcttc ggggcgaaaa   3900
   ctctcaagga tcttaccgct gttgagatcc agttcgatgt aacccactcg tgcacccaac   3960
   tgatcttcag catcttttac tttcaccagc gtttctgggt gagcaaaaac aggaaggcaa   4020
   aatgccgcaa aaaagggaat aagggcgaca cggaaatgtt gaatactcat actcttcctt   4080
   tttcaatatt attgaagcat ttatcagggt tattgtctca tgagcggata catatttgaa   4140
   tgtatttaga aaaataaaca aataggggtt ccgcgcacat ttccccgaaa agtgccacct   4200
   gacgtcgacg gatcgggaga tctcccgatc ccctatggtg cactctcagt acaatctgct   4260
   ctgatgccgc atagttaagc cagtatctgc tccctgcttg tgtgttggag gtcgctgagt   4320
   agtgcgcgag caaaatttaa gctacaacaa ggcaaggctt gaccgacaat tgcatgaaga   4380
   atctgcttag ggttaggcgt tttgcgctgc ttcgcgatgt acgggccaga tatacgcgtt   4440
   gacattgatt attgactagt tattaatagt aatcaattac ggggtcatta gttcatagcc   4500
   catatatgga gttccgcgtt acataactta cggtaaatgg cccgcctggc tgaccgccca   4560
   acgacccccg cccattgacg tcaataatga cgtatgttcc catagtaacg ccaataggga   4620
   ctttccattg acgtcaatgg gtggagtatt tacggtaaac tgcccacttg gcagtacatc   4680
   aagtgtatca tatgccaagt acgcccccta ttgacgtcaa tgacggtaaa tggcccgcct   4740
   ggcattatgc ccagtacatg accttatggg actttcctac ttggcagtac atctacgtat   4800
   tagtcatcgc tattaccatg gtgatgcggt tttggcagta catcaatggg cgtggatagc   4860
   ggtttgactc acggggattt ccaagtctcc accccattga cgtcaatggg agtttgtttt   4920
   ggcaccaaaa tcaacgggac tttccaaaat gtcgtaacaa ctccgcccca ttgacgcaaa   4980
   tgggcggtag gcgtgtacgg tgggaggtct atataagcag agctctctgg ctaactagag   5040
   aacccactgc ttactggctt atcgaaatga attcgactca ctgttgggag acccaagctg   5100
   gctagttaag ctatcacaag tttgtacaaa aaagcaggct ggcgcgccta cacagcggcc   5160
   ttgccaccat ggccaatacc aaatataacg aagagttcct gctgtacctg gccggctttg   5220
   tggacggtga cggtagcatc gttgctcaga ttaaaccaaa ccagcgtgct aagtttaaac   5280
   atcagctaag cttgaccttt caggtgactc aaaagaccca gcgccgttgg ctgctggaca   5340
   aactagtgga tgaaattggc gttggttacg tacaggattc tggtagcgtt tccaactacc   5400
   gtttaagcga aatcaagccg ctgcacaact tcctgactca actgcagccg tttctggaac   5460
   tgaaacagaa acaggcaaac ctggttctga aaattatcga acagctgccg tctgcaaaag   5520
   aatccccgga caaattcctg gaagtttgta cctgggctga tcagattgca gctctgaacg   5580
   attctaagac gcgtaaaacc acttctgaaa ccgttcgtgc tgtgctggac agcctgagcg   5640
   agaagaagaa accgtccccg gcggccggtg gatctgataa gtataatcag gctctgtcta   5700
   aatacaacca agcactgtcc aagtacaatc aggccctgtc tggtggaggc ggttccaaca   5760
   aaaaattcct gctgtatctt gctggatttg tggattctga tggctccatc attgctcaga   5820
   taaaaccacg tcaatcttac aagttcaaac accagctccg tttgaccttt tacgtcactc   5880
   agaagacaca aagaaggtgg ttcttggaca aattggttga tcgtattggt gtgggctatg   5940
   tcgaagactc tggctctgtg tcacgttacg ttctgtctga aattaagcct cttcataact   6000
   ttctcaccca actgcaaccc ttcttgaagc tcaaacagaa gcaagcaaat ctggttttga   6060
   aaatcatcga gcaactgcca tctgccaagg agtcccctga caagtttctt gaagtgtgta   6120
   cttgggtgga tcaggttgct gccttgaatg actccaagac cagaaaaacc acctctgaga   6180
   ctgtgagggc agttctggat agcctctctg agaagaaaaa gtcctctcct tag          6233
<210> 159
   <211> 11446
   <212> NUCLEIC
   <213> Artificial sequence
<220>
   <223> pCLS6810
<400> 159
   tcatggtcat agctgtttcc tgtgtgaaat tgttatccgc tcacaattcc acacaacata     60
   caggatccac tagcgatgta cgggccagat atacgcgttg acattgatta ttgactagtt    120
   attaatagta atcaattacg gggtcattag ttcatagccc atatatggag ttccgcgtta    180
   cataacttac ggtaaatggc ccgcctggct gaccgcccaa cgacccccgc ccattgacgt    240
   caataatgac gtatgttccc atagtaacgc caatagggac tttccattga cgtcaatggg    300
   tggagtattt acggtaaact gcccacttgg cagtacatca agtgtatcat atgccaagta    360
   cgccccctat tgacgtcaat gacggtaaat ggcccgcctg gcattatgcc cagtacatga    420
   ccttatggga ctttcctact tggcagtaca tctacgtatt agtcatcgct attaccatgg    480
   tgatgcggtt ttggcagtac atcaatgggc gtggatagcg gtttgactca cggggatttc    540
   caagtctcca ccccattgac gtcaatggga gtttgttttg gcaccaaaat caacgggact    600
   ttccaaaatg tcgtaacaac tccgccccat tgacgcaaat gggcggtagg cgtgtacggt    660
   gggaggtcta tataagcaga gctctctggc taactagaga acccactgct tactggctta    720
   tcgaaattaa tacgactcac tatagggaga cccaagctgg ctagccttag gcgcgcctcg    780
   cgagtttaaa ccgccaccat ggccgcttat ccttatgacg ttcctgatta cgctggattt    840
   atagctgccc cagggtgaga aagtccaagg aggctccgga tccggcggtt ctggatccgg    900
   cggttctggt tccgccgcta gcgggggcga ggagctgttc gccggcatcg tgcccgtgct    960
   gatcgagctg gacggcgacg tgcacggcca caagttcagc gtgcgcggcg agggcgaggg   1020
   cgacgccgac tacggcaagc tggagatcaa gttcatctgc accaccggca agctgcccgt   1080
   gccctggccc accctggtga ccaccctctg ctacggcatc cagtgcttcg cccgctaccc   1140
   cgagcacatg aagatgaacg acttcttcaa gagcgccatg cccgagggct acatccagga   1200
   gcgcaccatc cagttccagg acgacggcaa gtacaagacc cgcggcgagg tgaagttcga   1260
   gggcgacacc ctggtgaacc gcatcgagct gaagggcaag gacttcaagg aggacggcaa   1320
   catcctgggc cacaagctgg agtacagctt caacagccac aacgtgtaca tccgccccga   1380
   caaggccaac aacggcctgg aggctaactt caagacccgc cacaacatcg agggcggcgg   1440
   cgtgcagctg gccgaccact accagaccaa cgtgcccctg ggcgacggcc ccgtgctgat   1500
   ccccatcaac cactacctga gcactcagac caagatcagc aaggaccgca acgaggcccg   1560
   cgaccacatg gtgctcctgg agtccttcag cgcctgctgc cacacccacg gcatggacga   1620
   gctgtacagg taacccgggg agcggccgct cgagtctaga gggcccgttt aaacccgctg   1680
   atcagcctcg actgtgcctt ctagttgcca gccatctgtt gtttgcccct cccccgtgcc   1740
   ttccttgacc ctggaaggtg ccactcccac tgtcctttcc taataaaatg aggaaattgc   1800
   atcgcattgt ctgagtaggt gtcattctat tctggggggt ggggtggggc aggacagcaa   1860
   gggggaggat tgggaagaca atagcaggca tgctggggat gcggtgggct ctatggcttc   1920
   tgaggcggaa agaacggatc cgcagcctct ttcccaccca ccttgggact cagttctgcc   1980
   ccagatgaaa ttcagcaccc acatattaaa ttttcagaat ggaaatttaa gctgttccgg   2040
   gtgagatcct ttgaaaagac acctgaagaa gctcaaaagg aaaagaagga ttcctttgag   2100
   gggaaaccct ctctggagca atctccagca gtcctggaca aggctgatgg tcagaagcca   2160
   gtcccaactc agccattgtt aaaagcccac cctaagtttt cgaagaaatt tcacgacaac   2220
   gagaaagcaa gaggcaaagc gatccatcaa gccaaccttc gacatctctg ccgcatctgt   2280
   gggaattctt ttagagctga tgagcacaac aggagatatc cagtccatgg tcctgtggat   2340
   ggtaaaaccc taggcctttt acgaaagaag gaaaagagag ctacttcctg gccggacctc   2400
   attgccaagg ttttccggat cgatgtgaag gcagatgttg actcgatcca ccccactgag   2460
   ttctgccata actgctggag catcatgcac aggaagttta gcagtgcccc atgtgaggtt   2520
   tacttcccga ggaacgtgac catggagtgg cacccccaca caccatcctg tgacatctgc   2580
   aacactgccc gtcggggact caagaggaag agtcttcagc caaacttgca gctcagcaaa   2640
   aaactcaaaa ctgtgcttga ccaagcaaga caagcccgtc agcacaagag aagagctcag   2700
   gcaaggatca gcagcaagga tgtcatgaag aagatcgcca actgcagtaa gatacatctt   2760
   agtaccaagc tccttgcagt ggacttccca gagcactttg tgaaatccat ctcctgccag   2820
   atctgtgaac acattctggc tgaccctgtg gagaccaact gtaagcatgt cttttgccgg 2880
   gtctgcattc tcagatgcct caaagtcatg ggcagctatt gtccctcttg ccgatatcca 2940
   tgcttcccta ctgacctgga gagtccagtg aagtcctttc tgagcgtctt gaattccctg 3000
   atggtgaaat gtccagcaaa agagtgcaat gaggaggtca gtttggaaaa atataatcac 3060
   cacatctcaa gtcacaagga atcaaaagag atttttgtgc acattaataa agggggtcga 3120
   gtaacgcgtg caggcatgca agctggccgc aataaaatat ctttattttc attacatctg 3180
   tgtgttggtt ttttgtgtga atcgtaacta acatacgctc tccatcaaaa caaaacgaaa 3240
   caaaacaaac tagcaaaata ggctgtcccc agtgcaagtg caggtgccag aacatttctc 3300
   tatcgaagga tctgcgatcg ctccggtgcc cgtcagtggg cagagcgcac atcgcccaca 3360
   gtccccgaga agttgggggg aggggtcggc aattgaaccg gtgcctagag aaggtggcgc 3420
   ggggtaaact gggaaagtga tgtcgtgtac tggctccgcc tttttcccga gggtggggga 3480
   gaaccgtata taagtgcagt agtcgccgtg aacgttcttt ttcgcaacgg gtttgccgcc 3540
   agaacacagc tgaagcttcg aggggctcgc atctctcctt cacgcgcccg ccgccctacc 3600
   tgaggccgcc atccacgccg gttgagtcgc gttctgccgc ctcccgcctg tggtgcctcc 3660
   tgaactgcgt ccgccgtcta ggtaagttta aagctcaggt cgagaccggg cctttgtccg 3720
   gcgctccctt ggagcctacc tagactcagc cggctctcca cgctttgcct gaccctgctt 3780
   gctcaactct acgtctttgt ttcgttttct gttctgcgcc gttacagatc caagctgtga 3840
   ccggcgccta cgtaagtgat atctactaga tttatcaaaa agagtgttga cttgtgagcg 3900
   ctcacaattg atacttagat tcatcgagag ggacacgtcg actactaacc ttcttctctt 3960
   tcctacagct gagatcaccg gcgaaggagg gccaccatgg cttcttaccc tggacaccag 4020
   catgcttctg cctttgacca ggctgccaga tccaggggcc actccaacag gagaactgcc 4080
   ctaagaccca gaagacagca ggaagccact gaggtgaggc ctgagcagaa gatgccaacc 4140
   ctgctgaggg tgtacattga tggacctcat ggcatgggca agaccaccac cactcaactg 4200
   ctggtggcac tgggctccag ggatgacatt gtgtatgtgc ctgagccaat gacctactgg 4260
   agagtgctag gagcctctga gaccattgcc aacatctaca ccacccagca caggctggac 4320
   cagggagaaa tctctgctgg agatgctgct gtggtgatga cctctgccca gatcacaatg 4380
   ggaatgccct atgctgtgac tgatgctgtt ctggctcctc acattggagg agaggctggc 4440
   tcttctcatg cccctccacc tgccctgacc ctgatctttg acagacaccc cattgcagcc 4500
   ctgctgtgct acccagcagc aaggtacctc atgggctcca tgaccccaca ggctgtgctg 4560
   gcttttgtgg ccctgatccc tccaaccctc cctggcacca acattgttct gggagcactg 4620
   cctgaagaca gacacattga caggctggca aagaggcaga gacctggaga gagactggac 4680
   ctggccatgc tggctgcaat cagaagggtg tatggactgc tggcaaacac tgtgagatac 4740
   ctccagtgtg gaggctcttg gagagaggac tggggacagc tctctggaac agcagtgccc 4800
   cctcaaggag ctgagcccca gtccaatgct ggtccaagac cccacattgg ggacaccctg 4860
   ttcaccctgt tcagagcccc tgagctgctg gctcccaatg gagacctgta caatgtgttt 4920
   gcctgggctc tggatgttct agccaagagg ctgaggtcca tgcatgtgtt catcctggac 4980
   tatgaccagt cccctgctgg atgcagagat gctctgctgc aactaacctc tggcatggtg 5040
   cagacccatg tgaccacccc tggcagcatc cccaccatct gtgacctagc cagaaccttt 5100
   gccagggaga tgggagaggc caactaaacc tgagctagct cgacatgata agatacattg 5160
   atgagtttgg acaaaccaca actagaatgc agtgaaaaaa atgctttatt tgtgaaattt 5220
   gtgatgctat tgctttattt gtgaaatttg tgatgctatt gctttatttg taaccattat 5280
   aagctgcaat aaacaagtta acaacaacaa ttgcattcat tttatgtttc aggttcaggg 5340
   ggaggtgtgg gaggtttttt aaagcaagta aaacctctac aaatgtggta gatccatttt 5400
   tggcgtaatc atggtcatag ctgtttcctg tgtgaaattg ttatccgctc acaattccac 5460
   acaacatacg agccggaagc ataaagtgta aagcctgggg tgcctaatga gtgagctaac 5520
   tcacattaat tgcgttgcgc tcactgcccg ctttccagtc gggaaacctg tcgtgccagc 5580
   tgcattaatg aatcggccaa cgcgcgggga gaggcggttt gcgtattggg cgctcttccg 5640
   cttcctcgct cactgactcg ctgcgctcgg tcgttcggct gcggcgagcg gtatcagctc 5700
   actcaaaggc ggtaatacgg ttatccacag aatcagggga taacgcagga aagaacatgt 5760
   gagcaaaagg ccagcaaaag gccaggaacc gtaaaaaggc cgcgttgctg gcgtttttcc 5820
   ataggctccg cccccctgac gagcatcaca aaaatcgacg ctcaagtcag aggtggcgaa 5880
   acccgacagg actataaaga taccaggcgt ttccccctgg aagctccctc gtgcgctctc 5940
   ctgttccgac cctgccgctt accggatacc tgtccgcctt tctcccttcg ggaagcgtgg 6000
   cgctttctca tagctcacgc tgtaggtatc tcagttcggt gtaggtcgtt cgctccaagc 6060
   tgggctgtgt gcacgacccc cccgttcagc ccgaccgctg cgccttatcc ggtaactatc 6120
   gtcttgagtc caacccggta agacacgact tatcgccact ggcagcagcc actggtaaca 6180
   ggattagcag agcgaggtat gtaggcggtg ctacagagtt cttgaagtgg tggcctaact 6240
   acggctacac tagaagaaca gtatttggta tctgcgctct gctgaagcca gttaccttcg 6300
   gaaaaagagt tggtagctct tgatccggca aacaaaccac cgctggtagc ggtggttttt 6360
   ttgtttgcaa gcagcagatt acgcgcagaa aaaaaggatc tcaagaagat cctttgatct 6420
   tttctacggg gtctgacgct cagtggaacg aaaactcacg ttaagggatt ttggtcatga 6480
   gattatcaaa aaggatcttc acctagatcc ttttaaatta aaaatgaagt tttaaatcaa 6540
   tctaaagtat atatgagtaa acttggtctg acagttacca atgcttaatc agtgaggcac 6600
   ctatctcagc gatctgtcta tttcgttcat ccatagttgc ctgactcccc gtcgtgtaga 6660
   taactacgat acgggagggc ttaccatctg gccccagtgc tgcaatgata ccgcgagacc 6720
   cacgctcacc ggctccagat ttatcagcaa taaaccagcc agccggaagg gccgagcgca 6780
   gaagtggtcc tgcaacttta tccgcctcca tccagtctat taattgttgc cgggaagcta 6840
   gagtaagtag ttcgccagtt aatagtttgc gcaacgttgt tgccattgct acaggcatcg 6900
   tggtgtcacg ctcgtcgttt ggtatggctt cattcagctc cggttcccaa cgatcaaggc 6960
   gagttacatg atcccccatg ttgtgcaaaa aagcggttag ctccttcggt cctccgatcg 7020
   ttgtcagaag taagttggcc gcagtgttat cactcatggt tatggcagca ctgcataatt 7080
   ctcttactgt catgccatcc gtaagatgct tttctgtgac tggtgagtac tcaaccaagt 7140
   cattctgaga atagtgtatg cggcgaccga gttgctcttg cccggcgtca atacgggata 7200
   ataccgcgcc acatagcaga actttaaaag tgctcatcat tggaaaacgt tcttcggggc 7260
   gaaaactctc aaggatctta ccgctgttga gatccagttc gatgtaaccc actcgtgcac 7320
   ccaactgatc ttcagcatct tttactttca ccagcgtttc tgggtgagca aaaacaggaa 7380
   ggcaaaatgc cgcaaaaaag ggaataaggg cgacacggaa atgttgaata ctcatactct 7440
   tcctttttca atattattga agcatttatc agggttattg tctcatgagc ggatacatat 7500
   ttgaatgtat ttagaaaaat aaacaaatag gggttccgcg cacatttccc cgaaaagtgc 7560
   cacctgacgt ctaagaaacc attattatca tgacattaac ctataaaaat aggcgtatca 7620
   cgaggccctt tcgtctcgcg cgtttcggtg atgacggtga aaacctctga cacatgcagc 7680
   tcccggagac ggtcacagct tgtctgtaag cggatgccgg gagcagacaa gcccgtcagg 7740
   gcgcgtcagc gggtgttggc gggtgtcggg gctggcttaa ctatgcggca tcagagcaga 7800
   ttgtactgag agtgcaccat atgcggtgtg aaataccgca cagatgcgta aggagaaaat 7860
   accgcatcag gcgccaatat taaacttgat gagctctaga gatggtcatg cattttaaaa 7920
   agaattactc aaaatattgt cttggaatac cagagagcaa gtgctttaag tataggctgg 7980
   gaagtaaaat gctaaaggaa tgagaaggca tttggggttg agttcaacct aagaggcagg 8040
   ggagccacag ggaaagacct agcacctgcc acagaagaga attaggaagc agaattgaac 8100
   tataagcaat tttgaggtgt tcgttgggct gcagttgaaa tattttttga ggttaatgag 8160
   acatttgaaa tggccgtgta ttgtttaact cttgcatagt cctgcatagg gaacaatcta 8220
   ataggatttc tctgtgaatc aagtcttaga aatttgcttt taatttttat gaaaaacgcc 8280
   catttctttg tttttgagac agagtcctgc tctgtcatcc aggctgggtt gcagtggcgt 8340
   gatcttggcc cactgcaatc tctgcctcct gggttcaggc aattttcctg tctcagcctc 8400
   ccgagtagct gggatttcaa gtgcctgcca ccatgcccgg ctaaattttt ttgtattttt 8460
   ggtacagatg gagtatcacc atgttggcca ggctggtctc gaactcctga cctcaagtga 8520
   ttcaccagcc ttgacctccc aaagtgttgg gatcacaggc atgagccact gtgcctgtgc 8580
   cccaaaacac caatttctga tgtgtgatgc atgtaagata gaacaaactt cagtaaagcg 8640
   gggacttgaa aagaggcttt ggtaacagct gtcagcatta acccttgccc ctccgtacct 8700
   cctaatccca cccctgctca aagtatgttc atctgagaat ttgtctccat aactatgtga 8760
   ctataaaaat tctcatcgat tttgttagtt gatcaattga gggaaaaaca tatgttactt 8820
   gatataactg gtgggtcaaa agaattaacc caggcaaatt tgagataggt ggatgggatg 8880
   atggattgaa aatacagctg ctctctttcc aatcatgtac taagtaattt gggaaagatt 8940
   gatctaattg ggtctagaga gtacacttca catggcattg tttgactttt tttctgcatc 9000
   gctagcgatc tgtgcattac aactcaaatc agtcgggttt cctggcatat gtaattgcca 9060
   atgtttttta ccagaagaga aacattactc ccacctcttc ttattatgtt acaaactata 9120
   gtgctaatga ccatcgacca acagtgactt tcaggatgac ctgtgtgagt tttatctgaa 9180
   accatgtgaa tttttcatct taaaagtccc ttagaatctc agtctatgta cactcaggtt 9240
   tgttgcaggt ttagagttcc gtgttttttg tttctaatgt agacacagcc ttataattta 9300
   caacagcatt cactaattaa aattgtaagc ataattacta tccacgatac ttattattag 9360
   tttgcattca taaagctcaa aattcacttc atcctttcaa gtagtgaata attagtttct 9420
   ttgggtttgc agctttatca tccttttatg acccatttgg aagaaataaa caaccaaccc 9480
   cctggaagac tgctttaaaa agctggaaat acattgtcca gctagtacaa tgaggctaat 9540
   acaatgtgga aaatattact tttctttgat tttagtagcc tgtttatctt tacatttact 9600
   gaacaaataa ctattgagca cctaatgtat actgggaccc ttggggaggc aaagatgaat 9660
   caaagattct gtccttaaag accttaagac gcgttgacat tgattattga ctagttatta 9720
   atagtaatca attacggggt cattagttca tagcccatat atggagttcc gcgttacata 9780
   acttacggta aatggcccgc ctggctgacc gcccaacgac ccccgcccat tgacgtcaat 9840
   aatgacgtat gttcccatag taacgccaat agggactttc cattgacgtc aatgggtgga 9900
   gtatttacgg taaactgccc acttggcagt acatcaagtg tatcatatgc caagtacgcc 9960
   ccctattgac gtcaatgacg gtaaatggcc cgcctggcat tatgcccagt acatgacctt 10020
   atgggacttt cctacttggc agtacatcta cgtattagtc atcgctatta ccatggtgat 10080
   gcggttttgg cagtacatca atgggcgtgg atagcggttt gactcacggg gatttccaag 10140
   tctccacccc attgacgtca atgggagttt gttttggcac caaaatcaac gggactttcc 10200
   aaaatgtcgt aacaactccg ccccattgac gcaaatgggc ggtaggcgtg tacggtggga 10260
   ggtctatata agcagagctc cccgggagct tgtatatcca ttttcggatc tgatcaagag 10320
   acaggatgag gatcgtttcg catgattgaa caagatggat tgcacgcagg ttctccggcc 10380
   gcttgggtgg agaggctatt cggctatgac tgggcacaac agacaatcgg ctgctctgat  10440
   gccgccgtgt tccggctgtc agcgcagggg cgcccggttc tttttgtcaa gaccgacctg  10500
   tccggtgccc tgaatgaact gcaggacgag gcagcgcggc tatcgtggct ggccacgacg  10560
   ggcgttcctt gcgcagctgt gctcgacgtt gtcactgaag cgggaaggga ctggctgcta  10620
   ttgggcgaag tgccggggca ggatctcctg tcatctcacc ttgctcctgc cgagaaagta  10680
   tccatcatgg ctgatgcaat gcggcggctg catacgcttg atccggctac ctgcccattc  10740
   gaccaccaag cgaaacatcg catcgagcga gcacgtactc ggatggaagc cggtcttgtc  10800
   gatcaggatg atctggacga agagcatcag gggctcgcgc cagccgaact gttcgccagg  10860
   ctcaaggcgc gcatgcccga cggcgaggat ctcgtcgtga cccatggcga tgcctgcttg  10920
   ccgaatatca tggtggaaaa tggccgcttt tctggattca tcgactgtgg ccggctgggt  10980
   gtggcggacc gctatcagga catagcgttg gctacccgtg atattgctga agagcttggc  11040
   ggcgaatggg ctgaccgctt cctcgtgctt tacggtatcg ccgctcccga ttcgcagcgc  11100
   atcgccttct atcgccttct tgacgagttc ttctgattaa ttaacaggac tgaccgtgct  11160
   acgagatttc gattccaccg ccgccttcta tgaaaggttg ggcttcggaa tcgttttccg  11220
   ggacgccggc tggatgatcc tccagcgcgg ggatctcatg ctggagttct tcgcccaccc  11280
   caacttgttt attgcagctt ataatggtta caaataaagc aatagcatca caaatttcac  11340
   aaataaagca tttttttcac tgcattctag ttgtggtttg tccaaactca tcaatgtatc  11400
   ttatcatgtc tgtataccgt cgacctctag ctagagcttg gcgtaa                 11446
<210> 160
   <211> 64
   <212> NUCLEIC
   <213> Artificial sequence
<220>
   <223> GS Locus specific forward primer
<220>
   <221> misc_feature
   <222> (31); (32); (33); (34); (35); (36); (37); (38); (39); (40)
   <223> n is a or c or t or g
<400> 160
   ccatctcatc cctgcgtgtc tccgactcag nnnnnnnnnn gctctctggc taactagaga  60
   accc                                                               64
<210> 161
   <211> 53
   <212> NUCLEIC
   <213> Artificial sequence
<220>
   <223> GS Locus specific reverse primer
<400> 161
   cctatcccct gtgtgccttg gcagtctcag tcgatcagca cgggcacgat gcc 53
<210> 162
   <211> 67
   <212> NUCLEIC
   <213> Artificial sequence
<220>
   <223> RAG1 Locus specific forward primer
<220>
   <221> misc_feature
   <222> (31); (32); (33); (34); (35); (36); (37); (38); (39); (40)
   <223> n is a or c or t or g
<400> 162
   ccatctcatc cctgcgtgtc tccgactcag nnnnnnnnnn ggcaaagatg aatcaaagat 60
   tctgtcc 67
<210> 163
   <211> 62
   <212> NUCLEIC
   <213> Artificial sequence
<220>
   <223> XPC4 Locus specific forward primer
<220>
   <221> misc_feature
   <222> (31); (32); (33); (34); (35); (36); (37); (38); (39); (40)
   <223> n is a or c or t or g
<400> 163
   ccatctcatc cctgcgtgtc tccgactcag nnnnnnnnnn aagaggcaag aaaatgtgca 60
   gc 62
<210> 164
   <211> 60
   <212> NUCLEIC
   <213> Artificial sequence
<220>
   <223> CAPNS1 Locus specific forward primer
<220>
   <221> misc_feature
   <222> (31); (32); (33); (34); (35); (36); (37); (38); (39); (40)
   <223> n is a or c or t or g
<400> 164
   ccatctcatc cctgcgtgtc tccgactcag nnnnnnnnnn cgagtcaggg cgggattaag 60
<210> 165
   <211> 57
   <212> NUCLEIC
   <213> Artificial sequence
<220>
   <223> RAG1 Locus specific reverse primer
<400> 165
   cctatcccct gtgtgccttg gcagtctcag gatctcaccc ggaacagctt aaatttc 57
<210> 166
   <211> 54
   <212> NUCLEIC
   <213> Artificial sequence
<220>
   <223> XPC4 Locus specific reverse primer
<400> 166
   cctatcccct gtgtgccttg gcagtctcag gctgggcata tataaggtgc tcaa 54
<210> 167
   <211> 50
   <212> NUCLEIC
   <213> Artificial sequence
<220>
   <223> CAPNS1 Locus specific reverse primer
<400> 167
   cctatcccct gtgtgccttg gcagtctcag cgagacttca cggtttcgcc 50
<210> 168
<400> 168
   000
<210> 169
   <211> 5
   <212> PRT
   <213> Artificial sequence
<220>
   <223> GS stretch 1
<400> 169
   Gly Gly Gly Gly Ser
   1               5
<210> 170
   <211> 10
   <212> PRT
   <213> Artificial sequence
<220>
   <223> GS stretch 2
<400> 170
   Gly Gly Gly Gly Ser Gly Gly Gly Gly Ser
   1               5                   10
<210> 171
   <211> 595
   <212> PRT
   <213> Artificial sequence
<220>
   <223> SC_GS-5-Trex
<400> 171
   Met Ala Asn Thr Lys Tyr Asn Glu Glu Phe Leu Leu Tyr Leu Ala Gly
   1               5                   10                  15
   Phe Val Asp Ala Asp Gly Ser Ile Ile Ala Gln Ile Lys Pro Arg Gln
               20                  25                  30
   Ser Arg Lys Phe Lys His Glu Leu Ser Leu Thr Phe Asp Val Thr Gln
           35                  40                  45
   Lys Thr Gln Arg Arg Trp Phe Leu Asp Lys Leu Val Asp Glu Ile Gly
       50                  55                  60
   Val Gly Tyr Val Tyr Asp Ser Gly Ser Val Ser Tyr Tyr Gln Leu Ser
   65                  70                  75                  80
   Glu Ile Lys Pro Leu His Asn Phe Leu Thr Gln Leu Gln Pro Phe Leu
                   85                  90                  95
   Glu Leu Lys Gln Lys Gln Ala Asn Leu Val Leu Lys Ile Ile Glu Gln
               100                 105                 110
   Leu Pro Ser Ala Lys Glu Ser Pro Ala Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Gly Gly Ser Asp Lys Tyr Asn Gln Ala Leu
                   165                 170                 175
   Ser Lys Tyr Asn Gln Ala Leu Ser Lys Tyr Asn Gln Ala Leu Ser Gly
               180                 185                 190
   Gly Gly Gly Ser Asn Lys Lys Phe Leu Leu Tyr Leu Ala Gly Phe Val
           195                 200                 205
   Asp Gly Asp Gly Ser Ile Ile Ala Gln Ile Lys Pro Arg Gln Gly Tyr
       210                 215                 220
   Lys Phe Lys His Gln Leu Ser Leu Thr Phe Gln Val Thr Gln Lys Thr
   225                 230                 235                 240
   Gln Arg Arg Trp Phe Leu Asp Lys Leu Val Asp Arg Ile Gly Val Gly
                   245                 250                 255
   Tyr Val Ala Asp Arg Gly Ser Val Ser Asp Tyr Arg Leu Ser Glu Ile
               260                 265                 270
   Lys Pro Leu His Asn Phe Leu Thr Gln Leu Gln Pro Phe Leu Lys Leu
           275                 280                 285
   Lys Gln Lys Gln Ala Asn Leu Val Leu Lys Ile Ile Glu Gln Leu Pro
       290                 295                 300
   Ser Ala Lys Glu Ser Leu Asp Lys Phe Leu Glu Val Cys Thr Trp Val
   305                 310                 315                 320
   Asp Gln Ile Ala Ala Leu Asn Asp Ser Lys Thr Arg Lys Thr Thr Ser
                   325                 330                 335
   Glu Thr Val Arg Ala Val Leu Asp Ser Leu Ser Glu Lys Lys Lys Ser
               340                 345                 350
   Ser Pro Gly Gly Gly Gly Ser Glu Ala Pro Arg Ala Glu Thr Phe Val
           355                 360                 365
   Phe Leu Asp Leu Glu Ala Thr Gly Leu Pro Ser Val Glu Pro Glu Ile
       370                 375                 380
   Ala Glu Leu Ser Leu Phe Ala Val His Arg Ser Ser Leu Glu Asn Pro
   385                 390                 395                 400
   Glu His Asp Glu Ser Gly Ala Leu Val Leu Pro Arg Val Leu Asp Lys
                   405                 410                 415
   Leu Thr Leu Cys Met Cys Pro Glu Arg Pro Phe Thr Ala Lys Ala Ser
               420                 425                 430
   Glu Ile Thr Gly Leu Ser Ser Glu Gly Leu Ala Arg Cys Arg Lys Ala
           435                 440                 445
   Gly Phe Asp Gly Ala Val Val Arg Thr Leu Gln Ala Phe Leu Ser Arg
       450                 455                 460
   Gln Ala Gly Pro Ile Cys Leu Val Ala His Asn Gly Phe Asp Tyr Asp
   465                 470                 475                 480
   Phe Pro Leu Leu Cys Ala Glu Leu Arg Arg Leu Gly Ala Arg Leu Pro
                   485                 490                 495
   Arg Asp Thr Val Cys Leu Asp Thr Leu Pro Ala Leu Arg Gly Leu Asp
               500                 505                 510
   Arg Ala His Ser His Gly Thr Arg Ala Arg Gly Arg Gln Gly Tyr Ser
   515 520 525
   Leu Gly Ser Leu Phe His Arg Tyr Phe Arg Ala Glu Pro Ser Ala Ala
   530 535 540
   His Ser Ala Glu Gly Asp Val His Thr Leu Leu Leu Ile Phe Leu His
   545 550 555 560
   Arg Ala Ala Glu Leu Leu Ala Trp Ala Asp Glu Gln Ala Arg Gly Trp
   565 570 575
   Ala His Ile Glu Pro Met Tyr Leu Pro Pro Asp Asp Pro Ser Leu Glu
   580 585 590
   Ala Ala Asp
   595
<210> 172
   <211> 600
   <212> PRT
   <213> Artificial sequence
<220>
   <223> SC_GS-10-Trex
<400> 172
<210> 173
   <211> 594
   <212> PRT
   <213> Artificial sequence
<220>
   <223> Trex-5-SC_GS
<400> 173
   Met Gly Ser Glu Ala Pro Arg Ala Glu Thr Phe Val Phe Leu Asp Leu
   1               5                   10                  15
   Glu Ala Thr Gly Leu Pro Ser Val Glu Pro Glu Ile Ala Glu Leu Ser
               20                  25                  30
   Leu Phe Ala Val His Arg Ser Ser Leu Glu Asn Pro Glu His Asp Glu
           35                  40                  45
   Ser Gly Ala Leu Val Leu Pro Arg Val Leu Asp Lys Leu Thr Leu Cys
       50                  55                  60
   Met Cys Pro Glu Arg Pro Phe Thr Ala Lys Ala Ser Glu Ile Thr Gly
   65                  70                  75                  80
   Leu Ser Ser Glu Gly Leu Ala Arg Cys Arg Lys Ala Gly Phe Asp Gly
                   85                  90                  95
   Ala Val Val Arg Thr Leu Gln Ala Phe Leu Ser Arg Gln Ala Gly Pro
               100                 105                 110
   Ile Cys Leu Val Ala His Asn Gly Phe Asp Tyr Asp Phe Pro Leu Leu
           115                 120                 125
   Cys Ala Glu Leu Arg Arg Leu Gly Ala Arg Leu Pro Arg Asp Thr Val
       130                 135                 140
   Cys Leu Asp Thr Leu Pro Ala Leu Arg Gly Leu Asp Arg Ala His Ser
   145                 150                 155                 160
   His Gly Thr Arg Ala Arg Gly Arg Gln Gly Tyr Ser Leu Gly Ser Leu
                   165                 170                 175
   Phe His Arg Tyr Phe Arg Ala Glu Pro Ser Ala Ala His Ser Ala Glu
               180                 185                 190
   Gly Asp Val His Thr Leu Leu Leu Ile Phe Leu His Arg Ala Ala Glu
           195                 200                 205
   Leu Leu Ala Trp Ala Asp Glu Gln Ala Arg Gly Trp Ala His Ile Glu
       210                 215                 220
   Pro Met Tyr Leu Pro Pro Asp Asp Pro Ser Leu Glu Ala Gly Gly Gly
   225                 230                 235                 240
   Gly Ser Asn Thr Lys Tyr Asn Glu Glu Phe Leu Leu Tyr Leu Ala Gly
                   245                 250                 255
   Phe Val Asp Ala Asp Gly Ser Ile Ile Ala Gln Ile Lys Pro Arg Gln
               260                 265                 270
   Ser Arg Lys Phe Lys His Glu Leu Ser Leu Thr Phe Asp Val Thr Gln
           275                 280                 285
   Lys Thr Gln Arg Arg Trp Phe Leu Asp Lys Leu Val Asp Glu Ile Gly
       290                 295                 300
   Val Gly Tyr Val Tyr Asp Ser Gly Ser Val Ser Tyr Tyr Gln Leu Ser
   305                 310                 315                 320
   Glu Ile Lys Pro Leu His Asn Phe Leu Thr Gln Leu Gln Pro Phe Leu
                   325                 330                 335
   Glu Leu Lys Gln Lys Gln Ala Asn Leu Val Leu Lys Ile Ile Glu Gln
               340                 345                 350
   Leu Pro Ser Ala Lys Glu Ser Pro Ala Lys Phe Leu Glu Val Cys Thr
           355                 360                 365
   Trp Val Asp Gln Ile Ala Ala Leu Asn Asp Ser Lys Thr Arg Lys Thr
       370                 375                 380
   Thr Ser Glu Thr Val Arg Ala Val Leu Asp Ser Leu Ser Glu Lys Lys
   385                 390                 395                 400
   Lys Ser Ser Pro Ala Ala Gly Gly Ser Asp Lys Tyr Asn Gln Ala Leu
                   405                 410                 415
   Ser Lys Tyr Asn Gln Ala Leu Ser Lys Tyr Asn Gln Ala Leu Ser Gly
               420                 425                 430
   Gly Gly Gly Ser Asn Lys Lys Phe Leu Leu Tyr Leu Ala Gly Phe Val
           435                 440                 445
   Asp Gly Asp Gly Ser Ile Ile Ala Gln Ile Lys Pro Arg Gln Gly Tyr
       450                 455                 460
   Lys Phe Lys His Gln Leu Ser Leu Thr Phe Gln Val Thr Gln Lys Thr
   465 470 475 480
   Gln Arg Arg Trp Phe Leu Asp Lys Leu Val Asp Arg Ile Gly Val Gly
   485 490 495
   Tyr Val Ala Asp Arg Gly Ser Val Ser Asp Tyr Arg Leu Ser Glu Ile
   500 505 510
   Lys Pro Leu His Asn Phe Leu Thr Gln Leu Gln Pro Phe Leu Lys Leu
   515 520 525
   Lys Gln Lys Gln Ala Asn Leu Val Leu Lys Ile Ile Glu Gln Leu Pro
   530 535 540
   Ser Ala Lys Glu Ser Leu Asp Lys Phe Leu Glu Val Cys Thr Trp Val
   545 550 555 560
   Asp Gln Ile Ala Ala Leu Asn Asp Ser Lys Thr Arg Lys Thr Thr Ser
   565 570 575
   Glu Thr Val Arg Ala Val Leu Asp Ser Leu Ser Glu Lys Lys Lys Ser
   580 585 590
   Ser Pro
<210> 174
   <211> 599
   <212> PRT
   <213> Artificial sequence
<220>
   <223> Trex-10-SC_GS
<400> 174
   Met Gly Ser Glu Ala Pro Arg Ala Glu Thr Phe Val Phe Leu Asp Leu
   1 5 10 15
   Glu Ala Thr Gly Leu Pro Ser Val Glu Pro Glu Ile Ala Glu Leu Ser
   20 25 30
   Leu Phe Ala Val His Arg Ser Ser Leu Glu Asn Pro Glu His Asp Glu
   35 40 45
   Ser Gly Ala Leu Val Leu Pro Arg Val Leu Asp Lys Leu Thr Leu Cys
   50 55 60
   Met Cys Pro Glu Arg Pro Phe Thr Ala Lys Ala Ser Glu Ile Thr Gly
   65 70 75 80
   Leu Ser Ser Glu Gly Leu Ala Arg Cys Arg Lys Ala Gly Phe Asp Gly
   85 90 95
   Ala Val Val Arg Thr Leu Gln Ala Phe Leu Ser Arg Gln Ala Gly Pro
   100 105 110
   Ile Cys Leu Val Ala His Asn Gly Phe Asp Tyr Asp Phe Pro Leu Leu
   115 120 125
   Cys Ala Glu Leu Arg Arg Leu Gly Ala Arg Leu Pro Arg Asp Thr Val
   130 135 140
   Cys Leu Asp Thr Leu Pro Ala Leu Arg Gly Leu Asp Arg Ala His Ser
   145 150 155 160
   His Gly Thr Arg Ala Arg Gly Arg Gln Gly Tyr Ser Leu Gly Ser Leu
   165 170 175
   Phe His Arg Tyr Phe Arg Ala Glu Pro Ser Ala Ala His Ser Ala Glu
   180 185 190
   Gly Asp Val His Thr Leu Leu Leu Ile Phe Leu His Arg Ala Ala Glu
   195 200 205
   Leu Leu Ala Trp Ala Asp Glu Gln Ala Arg Gly Trp Ala His Ile Glu
   210 215 220
   Pro Met Tyr Leu Pro Pro Asp Asp Pro Ser Leu Glu Ala Gly Gly Gly
   225 230 235 240
   Gly Ser Gly Gly Gly Gly Ser Asn Thr Lys Tyr Asn Glu Glu Phe Leu
   245 250 255
   Leu Tyr Leu Ala Gly Phe Val Asp Ala Asp Gly Ser Ile Ile Ala Gln
   260 265 270
   Ile Lys Pro Arg Gln Ser Arg Lys Phe Lys His Glu Leu Ser Leu Thr
   275 280 285
   Phe Asp Val Thr Gln Lys Thr Gln Arg Arg Trp Phe Leu Asp Lys Leu
   290 295 300
   Val Asp Glu Ile Gly Val Gly Tyr Val Tyr Asp Ser Gly Ser Val Ser
   305 310 315 320
   Tyr Tyr Gln Leu Ser Glu Ile Lys Pro Leu His Asn Phe Leu Thr Gln
   325 330 335
   Leu Gln Pro Phe Leu Glu Leu Lys Gln Lys Gln Ala Asn Leu Val Leu
   340 345 350
   Lys Ile Ile Glu Gln Leu Pro Ser Ala Lys Glu Ser Pro Ala Lys Phe
   355 360 365
   Leu Glu Val Cys Thr Trp Val Asp Gln Ile Ala Ala Leu Asn Asp Ser
   370 375 380
   Lys Thr Arg Lys Thr Thr Ser Glu Thr Val Arg Ala Val Leu Asp Ser
   385 390 395 400
   Leu Ser Glu Lys Lys Lys Ser Ser Pro Ala Ala Gly Gly Ser Asp Lys
   405 410 415
   Tyr Asn Gln Ala Leu Ser Lys Tyr Asn Gln Ala Leu Ser Lys Tyr Asn
   420 425 430
   Gln Ala Leu Ser Gly Gly Gly Gly Ser Asn Lys Lys Phe Leu Leu Tyr
   435 440 445
   Leu Ala Gly Phe Val Asp Gly Asp Gly Ser Ile Ile Ala Gln Ile Lys
   450 455 460
   Pro Arg Gln Gly Tyr Lys Phe Lys His Gln Leu Ser Leu Thr Phe Gln
   465 470 475 480
   Val Thr Gln Lys Thr Gln Arg Arg Trp Phe Leu Asp Lys Leu Val Asp
   485 490 495
   Arg Ile Gly Val Gly Tyr Val Ala Asp Arg Gly Ser Val Ser Asp Tyr
   500 505 510
   Arg Leu Ser Glu Ile Lys Pro Leu His Asn Phe Leu Thr Gln Leu Gln
   515 520 525
   Pro Phe Leu Lys Leu Lys Gln Lys Gln Ala Asn Leu Val Leu Lys Ile
   530 535 540
   Ile Glu Gln Leu Pro Ser Ala Lys Glu Ser Leu Asp Lys Phe Leu Glu
   545 550 555 560
   Val Cys Thr Trp Val Asp Gln Ile Ala Ala Leu Asn Asp Ser Lys Thr
   565 570 575
   Arg Lys Thr Thr Ser Glu Thr Val Arg Ala Val Leu Asp Ser Leu Ser
               580                 585                 590
   Glu Lys Lys Lys Ser Ser Pro
           595
<210> 175
   <211> 5672
   <212> NUCLEIC
   <213> Artificial sequence
<220>
   <223> pCLS1853
<400> 175
   gacggatcgg gagatctccc gatcccctat ggtgcactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggag tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg    660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa atgaattcga ctcactgttg ggagacccaa gctggctagt    900
   taagctatca caagtttgta caaaaaagca ggctggcgcg cctacacagc ggccttgcca    960
   ccatggccaa taccaaatat aacaaagagt tcctgctgta cctggccggc tttgtggacg   1020
   gtgacggtag catcatcgct cagattaaac caaaccagtc ttataagttt aaacatcagc   1080
   taagcttgac ctttcaggtg actcaaaaga cccagcgccg ttggtttctg gacaaactag   1140
   tggatgaaat tggcgttggt tacgtacgtg atcgcggatc cgtttccaac tacatcttaa   1200
   gcgaaatcaa gccgctgcac aacttcctga ctcaactgca gccgtttctg aaactgaaac   1260
   agaaacaggc aaacctggtt ctgaaaatta tcgaacagct gccgtctgca aaagaatccc   1320
   cggacaaatt cctggaagtt tgtacctggg tggatcagat tgcagctctg aacgattcta   1380
   agacgcgtaa aaccacttct gaaaccgttc gtgctgtgct ggacagcctg agcgagaaga   1440
   agaaatcctc cccggcggcc gactgataac tcgagcgcta gcacccagct ttcttgtaca   1500
   aagtggtgat ctagagggcc cgcggttcga aggtaagcct atccctaacc ctctcctcgg   1560
   tctcgattct acgcgtaccg gttagtaatg agtttaaacg ggggaggcta actgaaacac    1620
   ggaaggagac aataccggaa ggaacccgcg ctatgacggc aataaaaaga cagaataaaa   1680
   cgcacgggtg ttgggtcgtt tgttcataaa cgcggggttc ggtcccaggg ctggcactct   1740
   gtcgataccc caccgagacc ccattggggc caatacgccc gcgtttcttc cttttcccca   1800
   ccccaccccc caagttcggg tgaaggccca gggctcgcag ccaacgtcgg ggcggcaggc   1860
   cctgccatag cagatctgcg cagctggggc tctagggggt atccccacgc gccctgtagc   1920
   ggcgcattaa gcgcggcggg tgtggtggtt acgcgcagcg tgaccgctac acttgccagc   1980
   gccctagcgc ccgctccttt cgctttcttc ccttcctttc tcgccacgtt cgccggcttt   2040
   ccccgtcaag ctctaaatcg gggcatccct ttagggttcc gatttagtgc tttacggcac    2100
   ctcgacccca aaaaacttga ttagggtgat ggttcacgta gtgggccatc gccctgatag   2160
   acggtttttc gccctttgac gttggagtcc acgttcttta atagtggact cttgttccaa   2220
   actggaacaa cactcaaccc tatctcggtc tattcttttg atttataagg gattttgggg   2280
   atttcggcct attggttaaa aaatgagctg atttaacaaa aatttaacgc gaattaattc   2340
   tgtggaatgt gtgtcagtta gggtgtggaa agtccccagg ctccccagca ggcagaagta   2400
   tgcaaagcat gcatctcaat tagtcagcaa ccaggtgtgg aaagtcccca ggctccccag   2460
   caggcagaag tatgcaaagc atgcatctca attagtcagc aaccatagtc ccgcccctaa   2520
   ctccgcccat cccgccccta actccgccca gttccgccca ttctccgccc catggctgac   2580
   taattttttt tatttatgca gaggccgagg ccgcctctgc ctctgagcta ttccagaagt   2640
   agtgaggagg cttttttgga ggcctaggct tttgcaaaaa gctcccggga gcttgtatat   2700
   ccattttcgg atctgatcag cacgtgttga caattaatca tcggcatagt atatcggcat   2760
   agtataatac gacaaggtga ggaactaaac catggccaag cctttgtctc aagaagaatc   2820
   caccctcatt gaaagagcaa cggctacaat caacagcatc cccatctctg aagactacag   2880
   cgtcgccagc gcagctctct ctagcgacgg ccgcatcttc actggtgtca atgtatatca   2940
   ttttactggg ggaccttgtg cagaactcgt ggtgctgggc actgctgctg ctgcggcagc   3000
   tggcaacctg acttgtatcg tcgcgatcgg aaatgagaac aggggcatct tgagcccctg   3060
   cggacggtgc cgacaggtgc ttctcgatct gcatcctggg atcaaagcca tagtgaagga   3120
   cagtgatgga cagccgacgg cagttgggat tcgtgaattg ctgccctctg gttatgtgtg   3180
   ggagggctaa gcacttcgtg gccgaggagc aggactgaca cgtgctacga gatttcgatt   3240
   ccaccgccgc cttctatgaa aggttgggct tcggaatcgt tttccgggac gccggctgga   3300
   tgatcctcca gcgcggggat ctcatgctgg agttcttcgc ccaccccaac ttgtttattg   3360
   cagcttataa tggttacaaa taaagcaata gcatcacaaa tttcacaaat aaagcatttt   3420
   tttcactgca ttctagttgt ggtttgtcca aactcatcaa tgtatcttat catgtctgta   3480
   taccgtcgac ctctagctag agcttggcgt aatcatggtc atagctgttt cctgtgtgaa   3540
   attgttatcc gctcacaatt ccacacaaca tacgagccgg aagcataaag tgtaaagcct   3600
   ggggtgccta atgagtgagc taactcacat taattgcgtt gcgctcactg cccgctttcc   3660
   agtcgggaaa cctgtcgtgc cagctgcatt aatgaatcgg ccaacgcgcg gggagaggcg   3720
   gtttgcgtat tgggcgctct tccgcttcct cgctcactga ctcgctgcgc tcggtcgttc   3780
   ggctgcggcg agcggtatca gctcactcaa aggcggtaat acggttatcc acagaatcag   3840
   gggataacgc aggaaagaac atgtgagcaa aaggccagca aaaggccagg aaccgtaaaa   3900
   aggccgcgtt gctggcgttt ttccataggc tccgcccccc tgacgagcat cacaaaaatc   3960
   gacgctcaag tcagaggtgg cgaaacccga caggactata aagataccag gcgtttcccc   4020
   ctggaagctc cctcgtgcgc tctcctgttc cgaccctgcc gcttaccgga tacctgtccg   4080
   cctttctccc ttcgggaagc gtggcgcttt ctcatagctc acgctgtagg tatctcagtt   4140
   cggtgtaggt cgttcgctcc aagctgggct gtgtgcacga accccccgtt cagcccgacc   4200
   gctgcgcctt atccggtaac tatcgtcttg agtccaaccc ggtaagacac gacttatcgc   4260
   cactggcagc agccactggt aacaggatta gcagagcgag gtatgtaggc ggtgctacag   4320
   agttcttgaa gtggtggcct aactacggct acactagaag aacagtattt ggtatctgcg   4380
   ctctgctgaa gccagttacc ttcggaaaaa gagttggtag ctcttgatcc ggcaaacaaa   4440
   ccaccgctgg tagcggtttt tttgtttgca agcagcagat tacgcgcaga aaaaaaggat   4500
   ctcaagaaga tcctttgatc ttttctacgg ggtctgacgc tcagtggaac gaaaactcac   4560
   gttaagggat tttggtcatg agattatcaa aaaggatctt cacctagatc cttttaaatt   4620
   aaaaatgaag ttttaaatca atctaaagta tatatgagta aacttggtct gacagttacc   4680
   aatgcttaat cagtgaggca cctatctcag cgatctgtct atttcgttca tccatagttg   4740
   cctgactccc cgtcgtgtag ataactacga tacgggaggg cttaccatct ggccccagtg   4800
   ctgcaatgat accgcgagac ccacgctcac cggctccaga tttatcagca ataaaccagc   4860
   cagccggaag ggccgagcgc agaagtggtc ctgcaacttt atccgcctcc atccagtcta   4920
   ttaattgttg ccgggaagct agagtaagta gttcgccagt taatagtttg cgcaacgttg   4980
   ttgccattgc tacaggcatc gtggtgtcac gctcgtcgtt tggtatggct tcattcagct   5040
   ccggttccca acgatcaagg cgagttacat gatcccccat gttgtgcaaa aaagcggtta   5100
   gctccttcgg tcctccgatc gttgtcagaa gtaagttggc cgcagtgtta tcactcatgg   5160
   ttatggcagc actgcataat tctcttactg tcatgccatc cgtaagatgc ttttctgtga   5220
   ctggtgagta ctcaaccaag tcattctgag aatagtgtat gcggcgaccg agttgctctt   5280
   gcccggcgtc aatacgggat aataccgcgc cacatagcag aactttaaaa gtgctcatca   5340
   ttggaaaacg ttcttcgggg cgaaaactct caaggatctt accgctgttg agatccagtt   5400
   cgatgtaacc cactcgtgca cccaactgat cttcagcatc ttttactttc accagcgttt   5460
   ctgggtgagc aaaaacagga aggcaaaatg ccgcaaaaaa gggaataagg gcgacacgga   5520
   aatgttgaat actcatactc ttcctttttc aatattattg aagcatttat cagggttatt   5580
   gtctcatgag cggatacata tttgaatgta tttagaaaaa taaacaaata ggggttccgc   5640
   gcacatttcc ccgaaaagtg ccacctgacg tc                                  5672
<210> 176
   <211> 20
   <212> NUCLEIC
   <213> Artificial sequence
<220>
   <223> CMV forward primer
<400> 176
   cgcaaatggg cggtaggcgt 20
<210> 177
   <211> 24
   <212> NUCLEIC
   <213> Artificial sequence
<220>
   <223> V5 reverse primer
<400> 177
   cgtagaatcg agaccgagga gagg 24
<210> 178
   <211> 33
   <212> NUCLEIC
   <213> Artificial sequence
<220>
   <223> Link5TrexFor primer
<400> 178
   ggaggtggag gttccgaggc accccgggcc gag 33
<210> 179
   <211> 45
   <212> NUCLEIC
   <213> Artificial sequence
<220>
   <223> Link5GSRev primer
<400> 179
   tgcctcggaa cctccacctc caggagagga ctttttcttc tcaga 45
<210> 180
   <211> 42
   <212> NUCLEIC
   <213> Artificial sequence
<220>
   <223> LinklOTrexFor primer
<400> 180
   ggcggatctg gaggtggagg ttccgaggca ccccgggccg ag 42
<210> 181
   <211> 51
   <212> NUCLEIC
   <213> Artificial sequence
<220>
   <223> Link10GSRev primer
<400> 181
   acctccacct ccagatccgc cacctccagg agaggacttt ttcttctcag a 51
<210> 182
   <211> 39
   <212> NUCLEIC
   <213> Artificial sequence
<220>
   <223> Link5GSFor primer
<400> 182
   ggaggtggag gttccaatac caaatataac gaagagttc 39
<210> 183
   <211> 45
   <212> NUCLEIC
   <213> Artificial sequence
<220>
   <223> Link5TrexRev primer
<400> 183
   ggtattggaa cctccacctc ccgcctccag gctggggtca tcagg 45
<210> 184
   <211> 48
   <212> NUCLEIC
   <213> Artificial sequence
<220>
   <223> Link10GSFor primer
<400> 184
   ggaggttctg gaggtggagg ttccaatacc aaatataacg aagagttc 48
<210> 185
   <211> 51
   <212> NUCLEIC
   <213> Artificial sequence
<220>
   <223> LinklOTrexRev primer
<400> 185
   acctccacct ccagaacctc cacctcccgc ctccaggctg gggtcatcag g 51
<210> 186
   <211> 6867
   <212> NUCLEIC
   <213> Artificial sequence
<220>
   <223> pCLS8082
<400> 186
   ctcgagcgct agcacccagc tttcttgtac aaagtggtga tctagagggc ccgcggttcg     60
   aaggtaagcc tatccctaac cctctcctcg gtctcgattc tacgcgtacc ggttagtaat    120
   gagtttaaac gggggaggct aactgaaaca cggaaggaga caataccgga aggaacccgc    180
   gctatgacgg caataaaaag acagaataaa acgcacgggt gttgggtcgt ttgttcataa    240
   acgcggggtt cggtcccagg gctggcactc tgtcgatacc ccaccgagac cccattgggg    300
   ccaatacgcc cgcgtttctt ccttttcccc accccacccc ccaagttcgg gtgaaggccc    360
   agggctcgca gccaacgtcg gggcggcagg ccctgccata gcagatctgc gcagctgggg    420
   ctctaggggg tatccccacg cgccctgtag cggcgcatta agcgcggcgg gtgtggtggt    480
   tacgcgcagc gtgaccgcta cacttgccag cgccctagcg cccgctcctt tcgctttctt    540
   cccttccttt ctcgccacgt tcgccggctt tccccgtcaa gctctaaatc ggggcatccc    600
   tttagggttc cgatttagtg ctttacggca cctcgacccc aaaaaacttg attagggtga    660
   tggttcacgt agtgggccat cgccctgata gacggttttt cgccctttga cgttggagtc    720
   cacgttcttt aatagtggac tcttgttcca aactggaaca acactcaacc ctatctcggt    780
   ctattctttt gatttataag ggattttggg gatttcggcc tattggttaa aaaatgagct    840
   gatttaacaa aaatttaacg cgaattaatt ctgtggaatg tgtgtcagtt agggtgtgga    900
   aagtccccag gctccccagc aggcagaagt atgcaaagca tgcatctcaa ttagtcagca    960
   accaggtgtg gaaagtcccc aggctcccca gcaggcagaa gtatgcaaag catgcatctc   1020
   aattagtcag caaccatagt cccgccccta actccgccca tcccgcccct aactccgccc   1080
   agttccgccc attctccgcc ccatggctga ctaatttttt ttatttatgc agaggccgag   1140
   gccgcctctg cctctgagct attccagaag tagtgaggag gcttttttgg aggcctaggc   1200
   ttttgcaaaa agctcccggg agcttgtata tccattttcg gatctgatca gcacgtgttg   1260
   acaattaatc atcggcatag tatatcggca tagtataata cgacaaggtg aggaactaaa   1320
   ccatggccaa gcctttgtct caagaagaat ccaccctcat tgaaagagca acggctacaa   1380
   tcaacagcat ccccatctct gaagactaca gcgtcgccag cgcagctctc tctagcgacg   1440
   gccgcatctt cactggtgtc aatgtatatc attttactgg gggaccttgt gcagaactcg   1500
   tggtgctggg cactgctgct gctgcggcag ctggcaacct gacttgtatc gtcgcgatcg   1560
   gaaatgagaa caggggcatc ttgagcccct gcggacggtg ccgacaggtg cttctcgatc   1620
   tgcatcctgg gatcaaagcc atagtgaagg acagtgatgg acagccgacg gcagttggga   1680
   ttcgtgaatt gctgccctct ggttatgtgt gggagggcta agcacttcgt ggccgaggag   1740
   caggactgac acgtgctacg agatttcgat tccaccgccg ccttctatga aaggttgggc   1800
   ttcggaatcg ttttccggga cgccggctgg atgatcctcc agcgcgggga tctcatgctg   1860
   gagttcttcg cccaccccaa cttgtttatt gcagcttata atggttacaa ataaagcaat   1920
   agcatcacaa atttcacaaa taaagcattt ttttcactgc attctagttg tggtttgtcc   1980
   aaactcatca atgtatctta tcatgtctgt ataccgtcga cctctagcta gagcttggcg   2040
   taatcatggt catagctgtt tcctgtgtga aattgttatc cgctcacaat tccacacaac   2100
   atacgagccg gaagcataaa gtgtaaagcc tggggtgcct aatgagtgag ctaactcaca   2160
   ttaattgcgt tgcgctcact gcccgctttc cagtcgggaa acctgtcgtg ccagctgcat   2220
   taatgaatcg gccaacgcgc ggggagaggc ggtttgcgta ttgggcgctc ttccgcttcc   2280
   tcgctcactg actcgctgcg ctcggtcgtt cggctgcggc gagcggtatc agctcactca   2340
   aaggcggtaa tacggttatc cacagaatca ggggataacg caggaaagaa catgtgagca   2400
   aaaggccagc aaaaggccag gaaccgtaaa aaggccgcgt tgctggcgtt tttccatagg   2460
   ctccgccccc ctgacgagca tcacaaaaat cgacgctcaa gtcagaggtg gcgaaacccg   2520
   acaggactat aaagatacca ggcgtttccc cctggaagct ccctcgtgcg ctctcctgtt   2580
   ccgaccctgc cgcttaccgg atacctgtcc gcctttctcc cttcgggaag cgtggcgctt   2640
   tctcatagct cacgctgtag gtatctcagt tcggtgtagg tcgttcgctc caagctgggc   2700
   tgtgtgcacg aaccccccgt tcagcccgac cgctgcgcct tatccggtaa ctatcgtctt   2760
   gagtccaacc cggtaagaca cgacttatcg ccactggcag cagccactgg taacaggatt   2820
   agcagagcga ggtatgtagg cggtgctaca gagttcttga agtggtggcc taactacggc   2880
   tacactagaa gaacagtatt tggtatctgc gctctgctga agccagttac cttcggaaaa   2940
   agagttggta gctcttgatc cggcaaacaa accaccgctg gtagcggttt ttttgtttgc   3000
   aagcagcaga ttacgcgcag aaaaaaagga tctcaagaag atcctttgat cttttctacg   3060
   gggtctgacg ctcagtggaa cgaaaactca cgttaaggga ttttggtcat gagattatca   3120
   aaaaggatct tcacctagat ccttttaaat taaaaatgaa gttttaaatc aatctaaagt   3180
   atatatgagt aaacttggtc tgacagttac caatgcttaa tcagtgaggc acctatctca   3240
   gcgatctgtc tatttcgttc atccatagtt gcctgactcc ccgtcgtgta gataactacg   3300
   atacgggagg gcttaccatc tggccccagt gctgcaatga taccgcgaga cccacgctca   3360
   ccggctccag atttatcagc aataaaccag ccagccggaa gggccgagcg cagaagtggt   3420
   cctgcaactt tatccgcctc catccagtct attaattgtt gccgggaagc tagagtaagt   3480
   agttcgccag ttaatagttt gcgcaacgtt gttgccattg ctacaggcat cgtggtgtca   3540
   cgctcgtcgt ttggtatggc ttcattcagc tccggttccc aacgatcaag gcgagttaca   3600
   tgatccccca tgttgtgcaa aaaagcggtt agctccttcg gtcctccgat cgttgtcaga   3660
   agtaagttgg ccgcagtgtt atcactcatg gttatggcag cactgcataa ttctcttact   3720
   gtcatgccat ccgtaagatg cttttctgtg actggtgagt actcaaccaa gtcattctga   3780
   gaatagtgta tgcggcgacc gagttgctct tgcccggcgt caatacggga taataccgcg   3840
   ccacatagca gaactttaaa agtgctcatc attggaaaac gttcttcggg gcgaaaactc   3900
   tcaaggatct taccgctgtt gagatccagt tcgatgtaac ccactcgtgc acccaactga   3960
   tcttcagcat cttttacttt caccagcgtt tctgggtgag caaaaacagg aaggcaaaat   4020
   gccgcaaaaa agggaataag ggcgacacgg aaatgttgaa tactcatact cttccttttt   4080
   caatattatt gaagcattta tcagggttat tgtctcatga gcggatacat atttgaatgt   4140
   atttagaaaa ataaacaaat aggggttccg cgcacatttc cccgaaaagt gccacctgac   4200
   gtcgacggat cgggagatct cccgatcccc tatggtgcac tctcagtaca atctgctctg   4260
   atgccgcata gttaagccag tatctgctcc ctgcttgtgt gttggaggtc gctgagtagt   4320
   gcgcgagcaa aatttaagct acaacaaggc aaggcttgac cgacaattgc atgaagaatc   4380
   tgcttagggt taggcgtttt gcgctgcttc gcgatgtacg ggccagatat acgcgttgac   4440
   attgattatt gactagttat taatagtaat caattacggg gtcattagtt catagcccat   4500
   atatggagtt ccgcgttaca taacttacgg taaatggccc gcctggctga ccgcccaacg   4560
   acccccgccc attgacgtca ataatgacgt atgttcccat agtaacgcca atagggactt   4620
   tccattgacg tcaatgggtg gagtatttac ggtaaactgc ccacttggca gtacatcaag   4680
   tgtatcatat gccaagtacg ccccctattg acgtcaatga cggtaaatgg cccgcctggc   4740
   attatgccca gtacatgacc ttatgggact ttcctacttg gcagtacatc tacgtattag   4800
   tcatcgctat taccatggtg atgcggtttt ggcagtacat caatgggcgt ggatagcggt   4860
   ttgactcacg gggatttcca agtctccacc ccattgacgt caatgggagt ttgttttggc   4920
   accaaaatca acgggacttt ccaaaatgtc gtaacaactc cgccccattg acgcaaatgg   4980
   gcggtaggcg tgtacggtgg gaggtctata taagcagagc tctctggcta actagagaac   5040
   ccactgctta ctggcttatc gaaatgaatt ccgtcgacca tggccaatac caaatataac   5100
   gaagagttcc tgctgtacct ggccggcttt gtggacgctg acggtagcat catcgctcag   5160
   attaaaccaa gacagtctcg gaagtttaaa catgagctaa gcttgacctt tgatgtgact   5220
   caaaagaccc agcgccgttg gtttctggac aagctagtgg atgaaattgg cgttggttac   5280
   gtatatgatt ctggatccgt ttcctattac cagttaagcg aaatcaagcc gctgcacaac   5340
   ttcctgactc aactgcagcc gtttctggaa ctgaaacaga aacaggcaaa cctggttctg   5400
   aaaattatcg aacagctgcc gtctgcaaaa gaatccccgg ccaaattcct ggaagtttgt   5460
   acctgggtgg atcagattgc agctctgaac gattctaaga cgcgtaaaac cacttctgaa   5520
   accgttcgtg ctgtgctgga tagcctgagc gagaagaaga aatcctcccc ggcggccggt   5580
   ggatctgata agtataatca ggctctgtct aaatacaacc aagcactgtc caagtacaat   5640
   caggccctgt ctggtggagg cggttccaac aaaaagttcc tgctgtatct tgctggattt   5700
   gtggatggtg atggctccat cattgctcag ataaaaccac gtcaagggta taagttcaaa   5760
   caccagctct ccttgacttt tcaggtcact cagaagacac aaagaaggtg gttcttggac   5820
   aaattggttg atcgtattgg tgtgggctat gtcgctgacc gtggctctgt gtcagactac   5880
   cgcctgtctg aaattaagcc tcttcataac tttctcaccc aactgcaacc cttcttgaag   5940
   ctcaaacaga agcaagcaaa tctggttttg aaaatcatcg agcaactgcc atctgccaag   6000
   gagtccctgg acaagtttct tgaagtgtgt acttgggtgg atcagattgc tgccttgaat   6060
   gactccaaga ccagaaaaac cacctctgag actgtgaggg cagttctgga tagcctctct   6120
   gagaagaaaa agtcctctcc tggaggtgga ggttccgagg caccccgggc cgagaccttt   6180
   gtcttcctgg acctggaagc cactgggctc cccagtgtgg agcccgagat tgccgagctg   6240
   tccctctttg ctgtccaccg ctcctccctg gagaacccgg agcacgacga gtctggtgcc   6300
   ctagtattgc cccgggtcct ggacaagctc acgctgtgca tgtgcccgga gcgccccttc   6360
   actgccaagg ccagcgagat caccggcctg agcagtgagg gcctggcgcg atgccggaag   6420
   gctggctttg atggcgccgt ggtgcggacg ctgcaggcct tcctgagccg ccaggcaggg   6480
   cccatctgcc ttgtggccca caatggcttt gattatgatt tccccctgct gtgtgccgag   6540
   ctgcggcgcc tgggtgcccg cctgccccgg gacactgtct gcctggacac gctgccggcc   6600
   ctgcggggcc tggaccgcgc ccacagccac ggcacccggg cccggggccg ccagggttac   6660
   agcctcggca gcctcttcca ccgctacttc cgggcagagc caagcgcagc ccactcagcc   6720
   gagggcgacg tgcacaccct gctcctgatc ttcctgcacc gcgccgcaga gctgctcgcc   6780
   tgggccgatg agcaggcccg tgggtgggcc cacatcgagc ccatgtactt gccgcctgat   6840
   gaccccagcc tggaggcggc cgactga                                       6867
<210> 187
   <211> 6882
   <212> NUCLEIC
   <213> Artificial sequence
<220>
   <223> pCLS8052
<400> 187
   ctcgagcgct agcacccagc tttcttgtac aaagtggtga tctagagggc ccgcggttcg     60
   aaggtaagcc tatccctaac cctctcctcg gtctcgattc tacgcgtacc ggttagtaat    120
   gagtttaaac gggggaggct aactgaaaca cggaaggaga caataccgga aggaacccgc    180
   gctatgacgg caataaaaag acagaataaa acgcacgggt gttgggtcgt ttgttcataa    240
   acgcggggtt cggtcccagg gctggcactc tgtcgatacc ccaccgagac cccattgggg    300
   ccaatacgcc cgcgtttctt ccttttcccc accccacccc ccaagttcgg gtgaaggccc    360
   agggctcgca gccaacgtcg gggcggcagg ccctgccata gcagatctgc gcagctgggg    420
   ctctaggggg tatccccacg cgccctgtag cggcgcatta agcgcggcgg gtgtggtggt    480
   tacgcgcagc gtgaccgcta cacttgccag cgccctagcg cccgctcctt tcgctttctt    540
   cccttccttt ctcgccacgt tcgccggctt tccccgtcaa gctctaaatc ggggcatccc    600
   tttagggttc cgatttagtg ctttacggca cctcgacccc aaaaaacttg attagggtga    660
   tggttcacgt agtgggccat cgccctgata gacggttttt cgccctttga cgttggagtc    720
   cacgttcttt aatagtggac tcttgttcca aactggaaca acactcaacc ctatctcggt    780
   ctattctttt gatttataag ggattttggg gatttcggcc tattggttaa aaaatgagct    840
   gatttaacaa aaatttaacg cgaattaatt ctgtggaatg tgtgtcagtt agggtgtgga    900
   aagtccccag gctccccagc aggcagaagt atgcaaagca tgcatctcaa ttagtcagca    960
   accaggtgtg gaaagtcccc aggctcccca gcaggcagaa gtatgcaaag catgcatctc   1020
   aattagtcag caaccatagt cccgccccta actccgccca tcccgcccct aactccgccc   1080
   agttccgccc attctccgcc ccatggctga ctaatttttt ttatttatgc agaggccgag   1140
   gccgcctctg cctctgagct attccagaag tagtgaggag gcttttttgg aggcctaggc   1200
   ttttgcaaaa agctcccggg agcttgtata tccattttcg gatctgatca gcacgtgttg   1260
   acaattaatc atcggcatag tatatcggca tagtataata cgacaaggtg aggaactaaa   1320
   ccatggccaa gcctttgtct caagaagaat ccaccctcat tgaaagagca acggctacaa   1380
   tcaacagcat ccccatctct gaagactaca gcgtcgccag cgcagctctc tctagcgacg   1440
   gccgcatctt cactggtgtc aatgtatatc attttactgg gggaccttgt gcagaactcg   1500
   tggtgctggg cactgctgct gctgcggcag ctggcaacct gacttgtatc gtcgcgatcg   1560
   gaaatgagaa caggggcatc ttgagcccct gcggacggtg ccgacaggtg cttctcgatc   1620
   tgcatcctgg gatcaaagcc atagtgaagg acagtgatgg acagccgacg gcagttggga   1680
   ttcgtgaatt gctgccctct ggttatgtgt gggagggcta agcacttcgt ggccgaggag   1740
   caggactgac acgtgctacg agatttcgat tccaccgccg ccttctatga aaggttgggc   1800
   ttcggaatcg ttttccggga cgccggctgg atgatcctcc agcgcgggga tctcatgctg   1860
   gagttcttcg cccaccccaa cttgtttatt gcagcttata atggttacaa ataaagcaat   1920
   agcatcacaa atttcacaaa taaagcattt ttttcactgc attctagttg tggtttgtcc   1980
   aaactcatca atgtatctta tcatgtctgt ataccgtcga cctctagcta gagcttggcg   2040
   taatcatggt catagctgtt tcctgtgtga aattgttatc cgctcacaat tccacacaac   2100
   atacgagccg gaagcataaa gtgtaaagcc tggggtgcct aatgagtgag ctaactcaca   2160
   ttaattgcgt tgcgctcact gcccgctttc cagtcgggaa acctgtcgtg ccagctgcat   2220
   taatgaatcg gccaacgcgc ggggagaggc ggtttgcgta ttgggcgctc ttccgcttcc   2280
   tcgctcactg actcgctgcg ctcggtcgtt cggctgcggc gagcggtatc agctcactca   2340
   aaggcggtaa tacggttatc cacagaatca ggggataacg caggaaagaa catgtgagca   2400
   aaaggccagc aaaaggccag gaaccgtaaa aaggccgcgt tgctggcgtt tttccatagg   2460
   ctccgccccc ctgacgagca tcacaaaaat cgacgctcaa gtcagaggtg gcgaaacccg   2520
   acaggactat aaagatacca ggcgtttccc cctggaagct ccctcgtgcg ctctcctgtt   2580
   ccgaccctgc cgcttaccgg atacctgtcc gcctttctcc cttcgggaag cgtggcgctt   2640
   tctcatagct cacgctgtag gtatctcagt tcggtgtagg tcgttcgctc caagctgggc   2700
   tgtgtgcacg aaccccccgt tcagcccgac cgctgcgcct tatccggtaa ctatcgtctt   2760
   gagtccaacc cggtaagaca cgacttatcg ccactggcag cagccactgg taacaggatt   2820
   agcagagcga ggtatgtagg cggtgctaca gagttcttga agtggtggcc taactacggc   2880
   tacactagaa gaacagtatt tggtatctgc gctctgctga agccagttac cttcggaaaa   2940
   agagttggta gctcttgatc cggcaaacaa accaccgctg gtagcggttt ttttgtttgc   3000
   aagcagcaga ttacgcgcag aaaaaaagga tctcaagaag atcctttgat cttttctacg   3060
   gggtctgacg ctcagtggaa cgaaaactca cgttaaggga ttttggtcat gagattatca   3120
   aaaaggatct tcacctagat ccttttaaat taaaaatgaa gttttaaatc aatctaaagt   3180
   atatatgagt aaacttggtc tgacagttac caatgcttaa tcagtgaggc acctatctca   3240
   gcgatctgtc tatttcgttc atccatagtt gcctgactcc ccgtcgtgta gataactacg   3300
   atacgggagg gcttaccatc tggccccagt gctgcaatga taccgcgaga cccacgctca   3360
   ccggctccag atttatcagc aataaaccag ccagccggaa gggccgagcg cagaagtggt   3420
   cctgcaactt tatccgcctc catccagtct attaattgtt gccgggaagc tagagtaagt   3480
   agttcgccag ttaatagttt gcgcaacgtt gttgccattg ctacaggcat cgtggtgtca   3540
   cgctcgtcgt ttggtatggc ttcattcagc tccggttccc aacgatcaag gcgagttaca   3600
   tgatccccca tgttgtgcaa aaaagcggtt agctccttcg gtcctccgat cgttgtcaga   3660
   agtaagttgg ccgcagtgtt atcactcatg gttatggcag cactgcataa ttctcttact   3720
   gtcatgccat ccgtaagatg cttttctgtg actggtgagt actcaaccaa gtcattctga   3780
   gaatagtgta tgcggcgacc gagttgctct tgcccggcgt caatacggga taataccgcg   3840
   ccacatagca gaactttaaa agtgctcatc attggaaaac gttcttcggg gcgaaaactc   3900
   tcaaggatct taccgctgtt gagatccagt tcgatgtaac ccactcgtgc acccaactga   3960
   tcttcagcat cttttacttt caccagcgtt tctgggtgag caaaaacagg aaggcaaaat   4020
   gccgcaaaaa agggaataag ggcgacacgg aaatgttgaa tactcatact cttccttttt   4080
   caatattatt gaagcattta tcagggttat tgtctcatga gcggatacat atttgaatgt   4140
   atttagaaaa ataaacaaat aggggttccg cgcacatttc cccgaaaagt gccacctgac   4200
   gtcgacggat cgggagatct cccgatcccc tatggtgcac tctcagtaca atctgctctg   4260
   atgccgcata gttaagccag tatctgctcc ctgcttgtgt gttggaggtc gctgagtagt   4320
   gcgcgagcaa aatttaagct acaacaaggc aaggcttgac cgacaattgc atgaagaatc   4380
   tgcttagggt taggcgtttt gcgctgcttc gcgatgtacg ggccagatat acgcgttgac   4440
   attgattatt gactagttat taatagtaat caattacggg gtcattagtt catagcccat   4500
   atatggagtt ccgcgttaca taacttacgg taaatggccc gcctggctga ccgcccaacg   4560
   acccccgccc attgacgtca ataatgacgt atgttcccat agtaacgcca atagggactt   4620
   tccattgacg tcaatgggtg gagtatttac ggtaaactgc ccacttggca gtacatcaag   4680
   tgtatcatat gccaagtacg ccccctattg acgtcaatga cggtaaatgg cccgcctggc   4740
   attatgccca gtacatgacc ttatgggact ttcctacttg gcagtacatc tacgtattag   4800
   tcatcgctat taccatggtg atgcggtttt ggcagtacat caatgggcgt ggatagcggt   4860
   ttgactcacg gggatttcca agtctccacc ccattgacgt caatgggagt ttgttttggc   4920
   accaaaatca acgggacttt ccaaaatgtc gtaacaactc cgccccattg acgcaaatgg   4980
   gcggtaggcg tgtacggtgg gaggtctata taagcagagc tctctggcta actagagaac   5040
   ccactgctta ctggcttatc gaaatgaatt ccgtcgacca tggccaatac caaatataac   5100
   gaagagttcc tgctgtacct ggccggcttt gtggacgctg acggtagcat catcgctcag   5160
   attaaaccaa gacagtctcg gaagtttaaa catgagctaa gcttgacctt tgatgtgact   5220
   caaaagaccc agcgccgttg gtttctggac aagctagtgg atgaaattgg cgttggttac   5280
   gtatatgatt ctggatccgt ttcctattac cagttaagcg aaatcaagcc gctgcacaac   5340
   ttcctgactc aactgcagcc gtttctggaa ctgaaacaga aacaggcaaa cctggttctg   5400
   aaaattatcg aacagctgcc gtctgcaaaa gaatccccgg ccaaattcct ggaagtttgt   5460
   acctgggtgg atcagattgc agctctgaac gattctaaga cgcgtaaaac cacttctgaa   5520
   accgttcgtg ctgtgctgga tagcctgagc gagaagaaga aatcctcccc ggcggccggt   5580
   ggatctgata agtataatca ggctctgtct aaatacaacc aagcactgtc caagtacaat   5640
   caggccctgt ctggtggagg cggttccaac aaaaagttcc tgctgtatct tgctggattt   5700
   gtggatggtg atggctccat cattgctcag ataaaaccac gtcaagggta taagttcaaa   5760
   caccagctct ccttgacttt tcaggtcact cagaagacac aaagaaggtg gttcttggac   5820
   aaattggttg atcgtattgg tgtgggctat gtcgctgacc gtggctctgt gtcagactac   5880
   cgcctgtctg aaattaagcc tcttcataac tttctcaccc aactgcaacc cttcttgaag   5940
   ctcaaacaga agcaagcaaa tctggttttg aaaatcatcg agcaactgcc atctgccaag   6000
   gagtccctgg acaagtttct tgaagtgtgt acttgggtgg atcagattgc tgccttgaat   6060
   gactccaaga ccagaaaaac cacctctgag actgtgaggg cagttctgga tagcctctct   6120
   gagaagaaaa agtcctctcc tggaggtggc ggatctggag gtggaggttc cgaggcaccc   6180
   cgggccgaga cctttgtctt cctggacctg gaagccactg ggctccccag tgtggagccc   6240
   gagattgccg agctgtccct ctttgctgtc caccgctcct ccctggagaa cccggagcac   6300
   gacgagtctg gtgccctagt attgccccgg gtcctggaca agctcacgct gtgcatgtgc   6360
   ccggagcgcc ccttcactgc caaggccagc gagatcaccg gcctgagcag tgagggcctg   6420
   gcgcgatgcc ggaaggctgg ctttgatggc gccgtggtgc ggacgctgca ggccttcctg   6480
   agccgccagg cagggcccat ctgccttgtg gcccacaatg gctttgatta tgatttcccc   6540
   ctgctgtgtg ccgagctgcg gcgcctgggt gcccgcctgc cccgggacac tgtctgcctg   6600
   gacacgctgc cggccctgcg gggcctggac cgcgcccaca gccacggcac ccgggcccgg   6660
   ggccgccagg gttacagcct cggcagcctc ttccaccgct acttccgggc agagccaagc   6720
   gcagcccact cagccgaggg cgacgtgcac accctgctcc tgatcttcct gcaccgcgcc   6780
   gcagagctgc tcgcctgggc cgatgagcag gcccgtgggt gggcccacat cgagcccatg   6840
   tacttgccgc ctgatgaccc cagcctggag gcggccgact ga                      6882
<210> 188
   <211> 6907
   <212> NUCLEIC
   <213> Artificial sequence
<220>
   <223> pCLS8053
<400> 188
   gacggatcgg gagatctccc gatcccctat ggtgcactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggag tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg    660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa atgaattcga ctcactgttg ggagacccaa gctggctagt    900
   taagctatca caagtttgta caaaaaagca ggctggcgcg cctacacagc ggccttgcca    960
   ccatgggttc cgaggcaccc cgggccgaga cctttgtctt cctggacctg gaagccactg   1020
   ggctccccag tgtggagccc gagattgccg agctgtccct ctttgctgtc caccgctcct   1080
   ccctggagaa cccggagcac gacgagtctg gtgccctagt attgccccgg gtcctggaca   1140
   agctcacgct gtgcatgtgc ccggagcgcc ccttcactgc caaggccagc gagatcaccg   1200
   gcctgagcag tgagggcctg gcgcgatgcc ggaaggctgg ctttgatggc gccgtggtgc   1260
   ggacgctgca ggccttcctg agccgccagg cagggcccat ctgccttgtg gcccacaatg   1320
   gctttgatta tgatttcccc ctgctgtgtg ccgagctgcg gcgcctgggt gcccgcctgc   1380
   cccgggacac tgtctgcctg gacacgctgc cggccctgcg gggcctggac cgcgcccaca    1440
   gccacggcac ccgggcccgg ggccgccagg gttacagcct cggcagcctc ttccaccgct   1500
   acttccgggc agagccaagc gcagcccact cagccgaggg cgacgtgcac accctgctcc   1560
   tgatcttcct gcaccgcgcc gcagagctgc tcgcctgggc cgatgagcag gcccgtgggt   1620
   gggcccacat cgagcccatg tacttgccgc ctgatgaccc cagcctggag gcgggaggtg   1680
   gaggttccaa taccaaatat aacgaagagt tcctgctgta cctggccggc tttgtggacg   1740
   ctgacggtag catcatcgct cagattaaac caagacagtc tcggaagttt aaacatgagc   1800
   taagcttgac ctttgatgtg actcaaaaga cccagcgccg ttggtttctg gacaagctag   1860
   tggatgaaat tggcgttggt tacgtatatg attctggatc cgtttcctat taccagttaa   1920
   gcgaaatcaa gccgctgcac aacttcctga ctcaactgca gccgtttctg gaactgaaac   1980
   agaaacaggc aaacctggtt ctgaaaatta tcgaacagct gccgtctgca aaagaatccc   2040
   cggccaaatt cctggaagtt tgtacctggg tggatcagat tgcagctctg aacgattcta   2100
   agacgcgtaa aaccacttct gaaaccgttc gtgctgtgct ggatagcctg agcgagaaga   2160
   agaaatcctc cccggcggcc ggtggatctg ataagtataa tcaggctctg tctaaataca   2220
   accaagcact gtccaagtac aatcaggccc tgtctggtgg aggcggttcc aacaaaaagt   2280
   tcctgctgta tcttgctgga tttgtggatg gtgatggctc catcattgct cagataaaac   2340
   cacgtcaagg gtataagttc aaacaccagc tctccttgac ttttcaggtc actcagaaga   2400
   cacaaagaag gtggttcttg gacaaattgg ttgatcgtat tggtgtgggc tatgtcgctg   2460
   accgtggctc tgtgtcagac taccgcctgt ctgaaattaa gcctcttcat aactttctca   2520
   cccaactgca acccttcttg aagctcaaac agaagcaagc aaatctggtt ttgaaaatca   2580
   tcgagcaact gccatctgcc aaggagtccc tggacaagtt tcttgaagtg tgtacttggg   2640
   tggatcagat tgctgccttg aatgactcca agaccagaaa aaccacctct gagactgtga   2700
   gggcagttct ggatagcctc tctgagaaga aaaagtcctc tccttagcca tggcccgcgg   2760
   ttcgaaggta agcctatccc taaccctctc ctcggtctcg attctacgcg taccggttag   2820
   taatgagttt aaacggggga ggctaactga aacacggaag gagacaatac cggaaggaac   2880
   ccgcgctatg acggcaataa aaagacagaa taaaacgcac gggtgttggg tcgtttgttc   2940
   ataaacgcgg ggttcggtcc cagggctggc actctgtcga taccccaccg agaccccatt   3000
   ggggccaata cgcccgcgtt tcttcctttt ccccacccca ccccccaagt tcgggtgaag   3060
   gcccagggct cgcagccaac gtcggggcgg caggccctgc catagcagat ctgcgcagct   3120
   ggggctctag gggtatcccc cacgcgccct gtagcggcgc attaagcgcg gcgggtgtgg   3180
   tggttacgcg cagcgtgacc gctacacttg ccagcgccct agcgcccgct cctttcgctt   3240
   tcttcccttc ctttctcgcc acgttcgccg gctttccccg tcaagctcta aatcggggca   3300
   tccctttagg gttccgattt agtgctttac ggcacctcga ccccaaaaaa cttgattagg   3360
   gtgatggttc acgtagtggg ccatcgccct gatagacggt ttttcgccct ttgacgttgg   3420
   agtccacgtt ctttaatagt ggactcttgt tccaaactgg aacaacactc aaccctatct   3480
   cggtctattc ttttgattta taagggattt tggggatttc ggcctattgg ttaaaaaatg   3540
   agctgattta acaaaaattt aacgcgaatt aattctgtgg aatgtgtgtc agttagggtg   3600
   tggaaagtcc ccaggctccc cagcaggcag aagtatgcaa agcatgcatc tcaattagtc   3660
   agcaaccagg tgtggaaagt ccccaggctc cccagcaggc agaagtatgc aaagcatgca   3720
   tctcaattag tcagcaacca tagtcccgcc cctaactccg cccatcccgc ccctaactcc   3780
   gcccagttcc gcccattctc cgccccatgg ctgactaatt ttttttattt atgcagaggc   3840
   cgaggccgcc tctgcctctg agctattcca gaagtagtga ggaggctttt ttggaggcct   3900
   aggcttttgc aaaaagctcc cgggagcttg tatatccatt ttcggatctg atcagcacgt   3960
   gttgacaatt aatcatcggc atagtatatc ggcatagtat aatacgacaa ggtgaggaac   4020
   taaaccatgg ccaagccttt gtctcaagaa gaatccaccc tcattgaaag agcaacggct   4080
   acaatcaaca gcatccccat ctctgaagac tacagcgtcg ccagcgcagc tctctctagc   4140
   gacggccgca tcttcactgg tgtcaatgta tatcatttta ctgggggacc ttgtgcagaa   4200
   ctcgtggtgc tgggcactgc tgctgctgcg gcagctggca acctgacttg tatcgtcgcg   4260
   atcggaaatg agaacagggg catcttgagc ccctgcggac ggtgccgaca ggtgcttctc   4320
   gatctgcatc ctgggatcaa agccatagtg aaggacagtg atggacagcc gacggcagtt   4380
   gggattcgtg aattgctgcc ctctggttat gtgtgggagg gctaagcact tcgtggccga   4440
   ggagcaggac tgacacgtgc tacgagattt cgattccacc gccgccttct atgaaaggtt   4500
   gggcttcgga atcgttttcc gggacgccgg ctggatgatc ctccagcgcg gggatctcat   4560
   gctggagttc ttcgcccacc ccaacttgtt tattgcagct tataatggtt acaaataaag   4620
   caatagcatc acaaatttca caaataaagc atttttttca ctgcattcta gttgtggttt   4680
   gtccaaactc atcaatgtat cttatcatgt ctgtataccg tcgacctcta gctagagctt   4740
   ggcgtaatca tggtcatagc tgtttcctgt gtgaaattgt tatccgctca caattccaca   4800
   caacatacga gccggaagca taaagtgtaa agcctggggt gcctaatgag tgagctaact   4860
   cacattaatt gcgttgcgct cactgcccgc tttccagtcg ggaaacctgt cgtgccagct   4920
   gcattaatga atcggccaac gcgcggggag aggcggtttg cgtattgggc gctcttccgc   4980
   ttcctcgctc actgactcgc tgcgctcggt cgttcggctg cggcgagcgg tatcagctca   5040
   ctcaaaggcg gtaatacggt tatccacaga atcaggggat aacgcaggaa agaacatgtg   5100
   agcaaaaggc cagcaaaagg ccaggaaccg taaaaaggcc gcgttgctgg cgtttttcca   5160
   taggctccgc ccccctgacg agcatcacaa aaatcgacgc tcaagtcaga ggtggcgaaa   5220
   cccgacagga ctataaagat accaggcgtt tccccctgga agctccctcg tgcgctctcc   5280
   tgttccgacc ctgccgctta ccggatacct gtccgccttt ctcccttcgg gaagcgtggc   5340
   gctttctcat agctcacgct gtaggtatct cagttcggtg taggtcgttc gctccaagct   5400
   gggctgtgtg cacgaacccc ccgttcagcc cgaccgctgc gccttatccg gtaactatcg   5460
   tcttgagtcc aacccggtaa gacacgactt atcgccactg gcagcagcca ctggtaacag   5520
   gattagcaga gcgaggtatg taggcggtgc tacagagttc ttgaagtggt ggcctaacta   5580
   cggctacact agaagaacag tatttggtat ctgcgctctg ctgaagccag ttaccttcgg   5640
   aaaaagagtt ggtagctctt gatccggcaa acaaaccacc gctggtagcg gtttttttgt   5700
   ttgcaagcag cagattacgc gcagaaaaaa aggatctcaa gaagatcctt tgatcttttc   5760
   tacggggtct gacgctcagt ggaacgaaaa ctcacgttaa gggattttgg tcatgagatt   5820
   atcaaaaagg atcttcacct agatcctttt aaattaaaaa tgaagtttta aatcaatcta   5880
   aagtatatat gagtaaactt ggtctgacag ttaccaatgc ttaatcagtg aggcacctat   5940
   ctcagcgatc tgtctatttc gttcatccat agttgcctga ctccccgtcg tgtagataac   6000
   tacgatacgg gagggcttac catctggccc cagtgctgca atgataccgc gagacccacg   6060
   ctcaccggct ccagatttat cagcaataaa ccagccagcc ggaagggccg agcgcagaag   6120
   tggtcctgca actttatccg cctccatcca gtctattaat tgttgccggg aagctagagt   6180
   aagtagttcg ccagttaata gtttgcgcaa cgttgttgcc attgctacag gcatcgtggt   6240
   gtcacgctcg tcgtttggta tggcttcatt cagctccggt tcccaacgat caaggcgagt   6300
   tacatgatcc cccatgttgt gcaaaaaagc ggttagctcc ttcggtcctc cgatcgttgt   6360
   cagaagtaag ttggccgcag tgttatcact catggttatg gcagcactgc ataattctct   6420
   tactgtcatg ccatccgtaa gatgcttttc tgtgactggt gagtactcaa ccaagtcatt   6480
   ctgagaatag tgtatgcggc gaccgagttg ctcttgcccg gcgtcaatac gggataatac   6540
   cgcgccacat agcagaactt taaaagtgct catcattgga aaacgttctt cggggcgaaa   6600
   actctcaagg atcttaccgc tgttgagatc cagttcgatg taacccactc gtgcacccaa   6660
   ctgatcttca gcatctttta ctttcaccag cgtttctggg tgagcaaaaa caggaaggca   6720
   aaatgccgca aaaaagggaa taagggcgac acggaaatgt tgaatactca tactcttcct   6780
   ttttcaatat tattgaagca tttatcaggg ttattgtctc atgagcggat acatatttga   6840
   atgtatttag aaaaataaac aaataggggt tccgcgcaca tttccccgaa aagtgccacc   6900
   tgacgtc                                                              6907
<210> 189
   <211> 6922
   <212> NUCLEIC
   <213> Artificial sequence
<220>
   <223> pCLS8054
<400> 189
   gacggatcgg gagatctccc gatcccctat ggtgcactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggag tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa atgaattcga ctcactgttg ggagacccaa gctggctagt 900
   taagctatca caagtttgta caaaaaagca ggctggcgcg cctacacagc ggccttgcca 960
   ccatgggttc cgaggcaccc cgggccgaga cctttgtctt cctggacctg gaagccactg 1020
   ggctccccag tgtggagccc gagattgccg agctgtccct ctttgctgtc caccgctcct 1080
   ccctggagaa cccggagcac gacgagtctg gtgccctagt attgccccgg gtcctggaca 1140
   agctcacgct gtgcatgtgc ccggagcgcc ccttcactgc caaggccagc gagatcaccg 1200
   gcctgagcag tgagggcctg gcgcgatgcc ggaaggctgg ctttgatggc gccgtggtgc 1260
   ggacgctgca ggccttcctg agccgccagg cagggcccat ctgccttgtg gcccacaatg 1320
   gctttgatta tgatttcccc ctgctgtgtg ccgagctgcg gcgcctgggt gcccgcctgc 1380
   cccgggacac tgtctgcctg gacacgctgc cggccctgcg gggcctggac cgcgcccaca 1440
   gccacggcac ccgggcccgg ggccgccagg gttacagcct cggcagcctc ttccaccgct 1500
   acttccgggc agagccaagc gcagcccact cagccgaggg cgacgtgcac accctgctcc 1560
   tgatcttcct gcaccgcgcc gcagagctgc tcgcctgggc cgatgagcag gcccgtgggt 1620
   gggcccacat cgagcccatg tacttgccgc ctgatgaccc cagcctggag gcgggaggtg 1680
   gaggttctgg aggtggaggt tccaatacca aatataacga agagttcctg ctgtacctgg 1740
   ccggctttgt ggacgctgac ggtagcatca tcgctcagat taaaccaaga cagtctcgga 1800
   agtttaaaca tgagctaagc ttgacttttg atgtgactca aaagacccag cgccgttggt 1860
   ttctggacaa gctagtggat gaaattggcg ttggttacgt atatgattct ggatccgttt 1920
   cctattacca gttaagcgaa atcaagccgc tgcacaactt cctgactcaa ctgcagccgt 1980
   ttctggaact gaaacagaaa caggcaaacc tggttctgaa aattatcgaa cagctgccgt 2040
   ctgcaaaaga atccccggcc aaattcctgg aagtttgtac ctgggtggat cagattgcag 2100
   ctctgaacga ttctaagacg cgtaaaacca cttctgaaac cgttcgtgct gtgctggata 2160
   gcctgagcga gaagaagaaa tcctccccgg cggccggtgg atctgataag tataatcagg 2220
   ctctgtctaa atacaaccaa gcactgtcca agtacaatca ggccctgtct ggtggaggcg 2280
   gttccaacaa aaagttcctg ctgtatcttg ctggatttgt ggatggtgat ggctccatca 2340
   ttgctcagat aaaaccacgt caagggtata agttcaaaca ccagctctcc ttgacttttc 2400
   aggtcactca gaagacacaa agaaggtggt tcttggacaa attggttgat cgtattggtg 2460
   tgggctatgt cgctgaccgt ggctctgtgt cagactaccg cctgtctgaa attaagcctc 2520
   ttcataactt tctcacccaa ctgcaaccct tcttgaagct caaacagaag caagcaaatc 2580
   tggttttgaa aatcatcgag caactgccat ctgccaagga gtccctggac aagtttcttg 2640
   aagtgtgtac ttgggtggat cagattgctg ccttgaatga ctccaagacc agaaaaacca 2700
   cctctgagac tgtgagggca gttctggata gcctctctga gaagaaaaag tcctctcctt 2760
   agccatggcc cgcggttcga aggtaagcct atccctaacc ctctcctcgg tctcgattct 2820
   acgcgtaccg gttagtaatg agtttaaacg gggaggctaa actgaaacac ggaaggagac 2880
   aataccggaa ggaacccgcg ctatgacggc aataaaaaga cagaataaaa cgcacgggtg 2940
   ttgggtcgtt tgttcataaa cgcggggttc ggtcccaggg ctggcactct gtcgataccc 3000
   caccgagacc ccattggggc caatacgccc gcgtttcttc cttttcccca ccccaccccc 3060
   caagttcggg tgaaggccca gggctcgcag ccaacgtcgg ggcggcaggc cctgccatag 3120
   cagatctgcg cagctggggc tctagggggt atccccacgc gccctgtagc ggcgcattaa 3180
   gcgcggcggg tgtggtggtt acgcgcagcg tgaccgctac acttgccagc gccctagcgc 3240
   ccgctccttt cgctttcttc ccttcctttc tcgccacgtt cgccggcttt ccccgtcaag 3300
   ctctaaatcg ggcatccctt ttagggttcc gatttagtgc tttacggcac ctcgacccca 3360
   aaaaacttga ttagggtgat ggttcacgta gtgggccatc gccctgatag acggtttttc 3420
   gccctttgac gttggagtcc acgttcttta atagtggact cttgttccaa actggaacaa 3480
   cactcaaccc tatctcggtc tattcttttg atttataagg gattttgggg atttcggcct 3540
   attggttaaa aaatgagctg atttaacaaa aatttaacgc gaattaattc tgtggaatgt 3600
   gtgtcagtta gggtgtggaa agtccccagg ctccccagca ggcagaagta tgcaaagcat 3660
   gcatctcaat tagtcagcaa ccaggtgtgg aaagtcccca ggctccccag caggcagaag 3720
   tatgcaaagc atgcatctca attagtcagc aaccatagtc ccgcccctaa ctccgcccat 3780
   cccgccccta actccgccca gttccgccca ttctccgccc catggctgac taattttttt 3840
   tatttatgca gaggccgagg ccgcctctgc ctctgagcta ttccagaagt agtgaggagg 3900
   cttttttgga ggcctaggct tttgcaaaaa gctcccggga gcttgtatat ccattttcgg 3960
   atctgatcag cacgtgttga caattaatca tcggcatagt atatcggcat agtataatac 4020
   gacaaggtga ggaactaaac catggccaag cctttgtctc aagaagaatc caccctcatt 4080
   gaaagagcaa cggctacaat caacagcatc cccatctctg aagactacag cgtcgccagc 4140
   gcagctctct ctagcgacgg ccgcatcttc actggtgtca atgtatatca ttttactggg 4200
   ggaccttgtg cagaactcgt ggtgctgggc actgctgctg ctgcggcagc tggcaacctg 4260
   acttgtatcg tcgcgatcgg aaatgagaac aggggcatct tgagcccctg cggacggtgc 4320
   cgacaggtgc ttctcgatct gcatcctggg atcaaagcca tagtgaagga cagtgatgga 4380
   cagccgacgg cagttgggat tcgtgaattg ctgccctctg gttatgtgtg ggagggctaa 4440
   gcacttcgtg gccgaggagc aggactgaca cgtgctacga gatttcgatt ccaccgccgc 4500
   cttctatgaa aggttgggct tcggaatcgt tttccgggac gccggctgga tgatcctcca 4560
   gcgcggggat ctcatgctgg agttcttcgc ccaccccaac ttgtttattg cagcttataa 4620
   tggttacaaa taaagcaata gcatcacaaa tttcacaaat aaagcatttt tttcactgca 4680
   ttctagttgt ggtttgtcca aactcatcaa tgtatcttat catgtctgta taccgtcgac 4740
   ctctagctag agcttggcgt aatcatggtc atagctgttt cctgtgtgaa attgttatcc 4800
   gctcacaatt ccacacaaca tacgagccgg aagcataaag tgtaaagcct ggggtgccta 4860
   atgagtgagc taactcacat taattgcgtt gcgctcactg cccgctttcc agtcgggaaa 4920
   cctgtcgtgc cagctgcatt aatgaatcgg ccaacgcgcg gggagaggcg gtttgcgtat 4980
   tgggcgctct tccgcttcct cgctcactga ctcgctgcgc tcggtcgttc ggctgcggcg 5040
   agcggtatca gctcactcaa aggcggtaat acggttatcc acagaatcag gggataacgc 5100
   aggaaagaac atgtgagcaa aaggccagca aaaggccagg aaccgtaaaa aggccgcgtt 5160
   gctggcgttt ttccataggc tccgcccccc tgacgagcat cacaaaaatc gacgctcaag 5220
   tcagaggtgg cgaaacccga caggactata aagataccag gcgtttcccc ctggaagctc 5280
   cctcgtgcgc tctcctgttc cgaccctgcc gcttaccgga tacctgtccg cctttctccc 5340
   ttcgggaagc gtggcgcttt ctcatagctc acgctgtagg tatctcagtt cggtgtaggt 5400
   cgttcgctcc aagctgggct gtgtgcacga accccccgtt cagcccgacc gctgcgcctt 5460
   atccggtaac tatcgtcttg agtccaaccc ggtaagacac gacttatcgc cactggcagc 5520
   agccactggt aacaggatta gcagagcgag gtatgtaggc ggtgctacag agttcttgaa 5580
   gtggtggcct aactacggct acactagaag aacagtattt ggtatctgcg ctctgctgaa 5640
   gccagttacc ttcggaaaaa gagttggtag ctcttgatcc ggcaaacaaa ccaccgctgg 5700
   tagcggtttt tttgtttgca agcagcagat tacgcgcaga aaaaaaggat ctcaagaaga 5760
   tcctttgatc ttttctacgg ggtctgacgc tcagtggaac gaaaactcac gttaagggat 5820
   tttggtcatg agattatcaa aaaggatctt cacctagatc cttttaaatt aaaaatgaag 5880
   ttttaaatca atctaaagta tatatgagta aacttggtct gacagttacc aatgcttaat 5940
   cagtgaggca cctatctcag cgatctgtct atttcgttca tccatagttg cctgactccc 6000
   cgtcgtgtag ataactacga tacgggaggg cttaccatct ggccccagtg ctgcaatgat 6060
   accgcgagac ccacgctcac cggctccaga tttatcagca ataaaccagc cagccggaag 6120
   ggccgagcgc agaagtggtc ctgcaacttt atccgcctcc atccagtcta ttaattgttg 6180
   ccgggaagct agagtaagta gttcgccagt taatagtttg cgcaacgttg ttgccattgc 6240
   tacaggcatc gtggtgtcac gctcgtcgtt tggtatggct tcattcagct ccggttccca 6300
   acgatcaagg cgagttacat gatcccccat gttgtgcaaa aaagcggtta gctccttcgg 6360
   tcctccgatc gttgtcagaa gtaagttggc cgcagtgtta tcactcatgg ttatggcagc 6420
   actgcataat tctcttactg tcatgccatc cgtaagatgc ttttctgtga ctggtgagta 6480
   ctcaaccaag tcattctgag aatagtgtat gcggcgaccg agttgctctt gcccggcgtc 6540
   aatacgggat aataccgcgc cacatagcag aactttaaaa gtgctcatca ttggaaaacg 6600
   ttcttcgggg cgaaaactct caaggatctt accgctgttg agatccagtt cgatgtaacc 6660
   cactcgtgca cccaactgat cttcagcatc ttttactttc accagcgttt ctgggtgagc 6720
   aaaaacagga aggcaaaatg ccgcaaaaaa gggaataagg gcgacacgga aatgttgaat 6780
   actcatactc ttcctttttc aatattattg aagcatttat cagggttatt gtctcatgag 6840
   cggatacata tttgaatgta tttagaaaaa taaacaaata ggggttccgc gcacatttcc 6900
   ccgaaaagtg ccacctgacg tc 6922
<210> 190
   <211> 354
   <212> PRT
   <213> Artificial sequence
<220>
   <223> SC_XPC4 protein
<400> 190
   Met Ala Asn Thr Lys Tyr Asn Glu Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser His Lys Phe Lys His Ala Leu Gln Leu Thr Phe Lys Val Thr Gln
           35                  40                  45
   Lys Thr Gln Arg Arg Trp Phe Leu Asp Lys Leu Val Asp Glu Ile Gly
       50                  55                  60
   Val Gly Tyr Val Gln Asp Ser Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Glu Leu Lys Gln Lys Gln Ala Asn Leu Ala Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Val Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Gly Gly Ser Asp Lys Tyr Asn Gln Ala Leu
                   165                 170                 175
   Ser Lys Tyr Asn Gln Ala Leu Ser Lys Tyr Asn Gln Ala Leu Ser Gly
               180                 185                 190
   Gly Gly Gly Ser Asn Lys Lys Phe Leu Leu Tyr Leu Ala Gly Phe Val
           195                 200                 205
   Asp Ser Asp Gly Ser Ile Ile Ala Gln Ile Lys Pro Asn Gln Ser His
       210                 215                 220
   Lys Phe Lys His Gln Leu Ser Leu Ala Phe Gln Val Thr Gln Lys Thr
   225                 230                 235                 240
   Gln Arg Arg Trp Phe Leu Asp Lys Leu Val Asp Arg Ile Gly Val Gly
                   245                 250                 255
   Tyr Val Arg Asp Arg Gly Ser Val Ser Asp Tyr Ile Leu Ser Lys Ile
               260                 265                 270
   Lys Pro Leu His Asn Phe Leu Thr Gln Leu Gln Pro Phe Leu Lys Leu
           275                 280                 285
   Lys Gln Lys Gln Ala Asn Leu Val Leu Lys Ile Ile Glu Gln Leu Pro
       290                 295                 300
   Ser Ala Lys Glu Ser Pro Asp Lys Phe Leu Glu Val Cys Thr Trp Val
   305                 310                 315                 320
   Asp Gln Val Ala Ala Leu Asn Asp Ser Lys Thr Arg Lys Thr Thr Ser
                   325                 330                 335
   Glu Thr Val Arg Ala Val Leu Asp Ser Leu Ser Glu Lys Lys Lys Ser
               340                 345                 350
   Ser Pro
<210> 191
   <211> 2686
   <212> NUCLEIC
   <213> Artificial sequence
<220>
   <223> pCLS0002
<400> 191
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgaatt cgagctcggt acccggggat    420
   cctctagagt cgacctgcag gcatgcaagc ttggcgtaat catggtcata gctgtttcct    480
   gtgtgaaatt gttatccgct cacaattcca cacaacatac gagccggaag cataaagtgt    540
   aaagcctggg gtgcctaatg agtgagctaa ctcacattaa ttgcgttgcg ctcactgccc    600
   gctttccagt cgggaaacct gtcgtgccag ctgcattaat gaatcggcca acgcgcgggg    660
   agaggcggtt tgcgtattgg gcgctcttcc gcttcctcgc tcactgactc gctgcgctcg    720
   gtcgttcggc tgcggcgagc ggtatcagct cactcaaagg cggtaatacg gttatccaca    780
   gaatcagggg ataacgcagg aaagaacatg tgagcaaaag gccagcaaaa ggccaggaac    840
   cgtaaaaagg ccgcgttgct ggcgtttttc cataggctcc gcccccctga cgagcatcac    900
   aaaaatcgac gctcaagtca gaggtggcga aacccgacag gactataaag ataccaggcg    960
   tttccccctg gaagctccct cgtgcgctct cctgttccga ccctgccgct taccggatac   1020
   ctgtccgcct ttctcccttc gggaagcgtg gcgctttctc atagctcacg ctgtaggtat   1080
   ctcagttcgg tgtaggtcgt tcgctccaag ctgggctgtg tgcacgaacc ccccgttcag   1140
   cccgaccgct gcgccttatc cggtaactat cgtcttgagt ccaacccggt aagacacgac   1200
   ttatcgccac tggcagcagc cactggtaac aggattagca gagcgaggta tgtaggcggt   1260
   gctacagagt tcttgaagtg gtggcctaac tacggctaca ctagaaggac agtatttggt   1320
   atctgcgctc tgctgaagcc agttaccttc ggaaaaagag ttggtagctc ttgatccggc   1380
   aaacaaacca ccgctggtag cggtggtttt tttgtttgca agcagcagat tacgcgcaga   1440
   aaaaaaggat ctcaagaaga tcctttgatc ttttctacgg ggtctgacgc tcagtggaac   1500
   gaaaactcac gttaagggat tttggtcatg agattatcaa aaaggatctt cacctagatc   1560
   cttttaaatt aaaaatgaag ttttaaatca atctaaagta tatatgagta aacttggtct   1620
   gacagttacc aatgcttaat cagtgaggca cctatctcag cgatctgtct atttcgttca   1680
   tccatagttg cctgactccc cgtcgtgtag ataactacga tacgggaggg cttaccatct   1740
   ggccccagtg ctgcaatgat accgcgagac ccacgctcac cggctccaga tttatcagca   1800
   ataaaccagc cagccggaag ggccgagcgc agaagtggtc ctgcaacttt atccgcctcc   1860
   atccagtcta ttaattgttg ccgggaagct agagtaagta gttcgccagt taatagtttg   1920
   cgcaacgttg ttgccattgc tacaggcatc gtggtgtcac gctcgtcgtt tggtatggct   1980
   tcattcagct ccggttccca acgatcaagg cgagttacat gatcccccat gttgtgcaaa   2040
   aaagcggtta gctccttcgg tcctccgatc gttgtcagaa gtaagttggc cgcagtgtta   2100
   tcactcatgg ttatggcagc actgcataat tctcttactg tcatgccatc cgtaagatgc   2160
   ttttctgtga ctggtgagta ctcaaccaag tcattctgag aatagtgtat gcggcgaccg   2220
   agttgctctt gcccggcgtc aatacgggat aataccgcgc cacatagcag aactttaaaa   2280
   gtgctcatca ttggaaaacg ttcttcgggg cgaaaactct caaggatctt accgctgttg   2340
   agatccagtt cgatgtaacc cactcgtgca cccaactgat cttcagcatc ttttactttc   2400
   accagcgttt ctgggtgagc aaaaacagga aggcaaaatg ccgcaaaaaa gggaataagg   2460
   gcgacacgga aatgttgaat actcatactc ttcctttttc aatattattg aagcatttat   2520
   cagggttatt gtctcatgag cggatacata tttgaatgta tttagaaaaa taaacaaata   2580
   ggggttccgc gcacatttcc ccgaaaagtg ccacctgacg tctaagaaac cattattatc   2640
   atgacattaa cctataaaaa taggcgtatc acgaggccct ttcgtc                  2686
<210> 192
   <211> 354
   <212> PRT
   <213> Artificial sequence
<220>
   <223> SC_CAPNS1 protein
<400> 192
   Met Ala Asn Thr Lys Tyr Asn Glu Glu Phe Leu Leu Tyr Leu Ala Gly
   1               5                   10                  15
   Phe Val Asp Gly Asp Gly Ser Ile Val Ala Gln Ile Lys Pro Asn Gln
               20                  25                  30
   Arg Ala Lys Phe Lys His Gln Leu Ser Leu Thr Phe Gln Val Thr Gln
           35                  40                  45
   Lys Thr Gln Arg Arg Trp Leu Leu Asp Lys Leu Val Asp Glu Ile Gly
       50                  55                  60
   Val Gly Tyr Val Gln Asp Ser Gly Ser Val Ser Asn Tyr Arg Leu Ser
   65                  70                  75                  80
   Glu Ile Lys Pro Leu His Asn Phe Leu Thr Gln Leu Gln Pro Phe Leu
   85 90 95
   Glu Leu Lys Gln Lys Gln Ala Asn Leu Val Leu Lys Ile Ile Glu Gln
   100 105 110
   Leu Pro Ser Ala Lys Glu Ser Pro Asp Lys Phe Leu Glu Val Cys Thr
   115 120 125
   Trp Ala Asp Gln Ile Ala Ala Leu Asn Asp Ser Lys Thr Arg Lys Thr
   130 135 140
   Thr Ser Glu Thr Val Arg Ala Val Leu Asp Ser Leu Ser Glu Lys Lys
   145 150 155 160
   Lys Pro Ser Pro Ala Ala Gly Gly Ser Asp Lys Tyr Asn Gln Ala Leu
   165 170 175
   Ser Lys Tyr Asn Gln Ala Leu Ser Lys Tyr Asn Gln Ala Leu Ser Gly
   180 185 190
   Gly Gly Gly Ser Asn Lys Lys Phe Leu Leu Tyr Leu Ala Gly Phe Val
   195 200 205
   Asp Ser Asp Gly Ser Ile Ile Ala Gln Ile Lys Pro Arg Gln Ser Tyr
   210 215 220
   Lys Phe Lys His Gln Leu Arg Leu Thr Phe Tyr Val Thr Gln Lys Thr
   225 230 235 240
   Gln Arg Arg Trp Phe Leu Asp Lys Leu Val Asp Arg Ile Gly Val Gly
   245 250 255
   Tyr Val Glu Asp Ser Gly Ser Val Ser Arg Tyr Val Leu Ser Glu Ile
   260 265 270
   Lys Pro Leu His Asn Phe Leu Thr Gln Leu Gln Pro Phe Leu Lys Leu
   275 280 285
   Lys Gln Lys Gln Ala Asn Leu Val Leu Lys Ile Ile Glu Gln Leu Pro
   290 295 300
   Ser Ala Lys Glu Ser Pro Asp Lys Phe Leu Glu Val Cys Thr Trp Val
   305 310 315 320
   Asp Gln Val Ala Ala Leu Asn Asp Ser Lys Thr Arg Lys Thr Thr Ser
   325 330 335
   Glu Thr Val Arg Ala Val Leu Asp Ser Leu Ser Glu Lys Lys Lys Ser
   340 345 350
   Ser Pro
<210> 193
   <211> 354
   <212> PRT
   <213> Artificial sequence
<220>
   <223> SC_GS protein
<400> 193
   Met Ala Asn Thr Lys Tyr Asn Glu Glu Phe Leu Leu Tyr Leu Ala Gly
   1               5                   10                  15
   Phe Val Asp Ala Asp Gly Ser Ile Ile Ala Gln Ile Lys Pro Arg Gln
               20                  25                  30
   Ser Arg Lys Phe Lys His Glu Leu Ser Leu Thr Phe Asp Val Thr Gln
           35                  40                  45
   Lys Thr Gln Arg Arg Trp Phe Leu Asp Lys Leu Val Asp Glu Ile Gly
       50                  55                  60
   Val Gly Tyr Val Tyr Asp Ser Gly Ser Val Ser Tyr Tyr Gln Leu Ser
   65                  70                  75                  80
   Glu Ile Lys Pro Leu His Asn Phe Leu Thr Gln Leu Gln Pro Phe Leu
                   85                  90                  95
   Glu Leu Lys Gln Lys Gln Ala Asn Leu Val Leu Lys Ile Ile Glu Gln
               100                 105                 110
   Leu Pro Ser Ala Lys Glu Ser Pro Ala Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Gly Gly Ser Asp Lys Tyr Asn Gln Ala Leu
                   165                 170                 175
   Ser Lys Tyr Asn Gln Ala Leu Ser Lys Tyr Asn Gln Ala Leu Ser Gly
               180                 185                 190
   Gly Gly Gly Ser Asn Lys Lys Phe Leu Leu Tyr Leu Ala Gly Phe Val
           195                 200                 205
   Asp Gly Asp Gly Ser Ile Ile Ala Gln Ile Lys Pro Arg Gln Gly Tyr
       210                 215                 220
   Lys Phe Lys His Gln Leu Ser Leu Thr Phe Gln Val Thr Gln Lys Thr
   225                 230                 235                 240
   Gln Arg Arg Trp Phe Leu Asp Lys Leu Val Asp Arg Ile Gly Val Gly
                   245                 250                 255
   Tyr Val Ala Asp Arg Gly Ser Val Ser Asp Tyr Arg Leu Ser Glu Ile
               260                 265                 270
   Lys Pro Leu His Asn Phe Leu Thr Gln Leu Gln Pro Phe Leu Lys Leu
           275                 280                 285
   Lys Gln Lys Gln Ala Asn Leu Val Leu Lys Ile Ile Glu Gln Leu Pro
       290                 295                 300
   Ser Ala Lys Glu Ser Leu Asp Lys Phe Leu Glu Val Cys Thr Trp Val
   305                 310                 315                 320
   Asp Gln Ile Ala Ala Leu Asn Asp Ser Lys Thr Arg Lys Thr Thr Ser
                   325                 330                 335
   Glu Thr Val Arg Ala Val Leu Asp Ser Leu Ser Glu Lys Lys Lys Ser
               340                 345                 350
   Ser Pro
<210> 194
   <211> 236
   <212> PRT
   <213> Artificial sequence
<220>
   <223> Trex2 (236 aa)
<400> 194
   Met Ser Glu Ala Pro Arg Ala Glu Thr Phe Val Phe Leu Asp Leu Glu
   1               5                   10                  15
   Ala Thr Gly Leu Pro Ser Val Glu Pro Glu Ile Ala Glu Leu Ser Leu
               20                  25                  30
   Phe Ala Val His Arg Ser Ser Leu Glu Asn Pro Glu His Asp Glu Ser
           35                  40                  45
   Gly Ala Leu Val Leu Pro Arg Val Leu Asp Lys Leu Thr Leu Cys Met
       50                  55                  60
   Cys Pro Glu Arg Pro Phe Thr Ala Lys Ala Ser Glu Ile Thr Gly Leu
   65                  70                  75                  80
   Ser Ser Glu Gly Leu Ala Arg Cys Arg Lys Ala Gly Phe Asp Gly Ala
                   85                  90                  95
   Val Val Arg Thr Leu Gln Ala Phe Leu Ser Arg Gln Ala Gly Pro Ile
               100                 105                 110
   Cys Leu Val Ala His Asn Gly Phe Asp Tyr Asp Phe Pro Leu Leu Cys
           115                 120                 125
   Ala Glu Leu Arg Arg Leu Gly Ala Arg Leu Pro Arg Asp Thr Val Cys
       130                 135                 140
   Leu Asp Thr Leu Pro Ala Leu Arg Gly Leu Asp Arg Ala His Ser His
   145                 150                 155                 160
   Gly Thr Arg Ala Arg Gly Arg Gln Gly Tyr Ser Leu Gly Ser Leu Phe
                   165                 170                 175
   His Arg Tyr Phe Arg Ala Glu Pro Ser Ala Ala His Ser Ala Glu Gly
               180                 185                 190
   Asp Val His Thr Leu Leu Leu Ile Phe Leu His Arg Ala Ala Glu Leu
           195                 200                 205
   Leu Ala Trp Ala Asp Glu Gln Ala Arg Gly Trp Ala His Ile Glu Pro
       210                 215                 220
   Met Tyr Leu Pro Pro Asp Asp Pro Ser Leu Glu Ala
   225                 230                 235
<210> 195
   <211> 167
   <212> PRT
   <213> artificial sequence
<220>
   <223> I-CreI
<400> 195
   Met Ala Asn Thr Lys Tyr Asn Lys Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Arg Asp Arg Gly Ser Val Ser Asp Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Asp
                   165
<210> 196
   <211> 6969
   <212> NUCLEIC
   <213> Artificial sequence
<220>
   <223> pCLS8518
<400> 196
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggag tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa atgaattcga ctcactgttg ggagacccaa gctggctagt     900
   taagctatca acaagtttgt acaaaaaagc aggctggcgc gcctacacag cggccttgcc     960
   accatgggtt ccgaggcacc ccgggccgag acctttgtct tcctggacct ggaagccact    1020
   gggctcccca gtgtggagcc cgagattgcc gagctgtccc tctttgctgt ccaccgctcc    1080
   tccctggaga acccggagca cgacgagtct ggtgccctag tattgccccg ggtcctggac    1140
   aagctcacgc tgtgcatgtg cccggagcgc cccttcactg ccaaggccag cgagatcacc    1200
   ggcctgagca gtgagggcct ggcgcgatgc cggaaggctg gctttgatgg cgccgtggtg    1260
   cggacgctgc aggccttcct gagccgccag gcagggccca tctgccttgt ggcccacaat    1320
   ggctttgatt atgatttccc cctgctgtgt gccgagctgc ggcgcctggg tgcccgcctg    1380
   ccccgggaca ctgtctgcct ggacacgctg ccggccctgc ggggcctgga ccgcgcccac    1440
   agccacggca cccgggcccg gggccgccag ggttacagcc tcggcagcct cttccaccgc    1500
   tacttccggg cagagccaag cgcagcccac tcagccgagg gcgacgtgca caccctgctc    1560
   ctgatcttcc tgcaccgcgc cgcagagctg ctcgcctggg ccgatgagca ggcccgtggg    1620
   tgggcccaca tcgagcccat gtacttgccg cctgatgacc ccagcctgga ggcgggaggt    1680
   ggaggttctg gaggtggagg ttccaatacc aaatataacg aagagttcct gctgtacctg    1740
   gccggctttg tggacggtga cggtagcatc gttgctcaga ttaaaccaaa ccagcgtgct    1800
   aagtttaaac atcagctaag cttgaccttt caggtgactc aaaagaccca gcgccgttgg    1860
   ctgctggaca aactagtgga tgaaattggc gttggttacg tacaggattc tggtagcgtt    1920
   tccaactacc gtttaagcga aatcaagccg ctgcacaact tcctgactca actgcagccg    1980
   tttctggaac tgaaacagaa acaggcaaac ctggttctga aaattatcga acagctgccg    2040
   tctgcaaaag aatccccgga caaattcctg gaagtttgta cctgggctga tcagattgca    2100
   gctctgaacg attctaagac gcgtaaaacc acttctgaaa ccgttcgtgc tgtgctggac    2160
   agcctgagcg agaagaagaa accgtccccg gcggccggtg gatctgataa gtataatcag    2220
   gctctgtcta aatacaacca agcactgtcc aagtacaatc aggccctgtc tggtggaggc    2280
   ggttccaaca aaaaattcct gctgtatctt gctggatttg tggattctga tggctccatc    2340
   attgctcaga taaaaccacg tcaatcttac aagttcaaac accagctccg tttgaccttt    2400
   tacgtcactc agaagacaca aagaaggtgg ttcttggaca aattggttga tcgtattggt    2460
   gtgggctatg tcgaagactc tggctctgtg tcacgttacg ttctgtctga aattaagcct    2520
   cttcataact ttctcaccca actgcaaccc ttcttgaagc tcaaacagaa gcaagcaaat    2580
   ctggttttga aaatcatcga gcaactgcca tctgccaagg agtcccctga caagtttctt    2640
   gaagtgtgta cttgggtgga tcaggttgct gccttgaatg actccaagac cagaaaaacc    2700
   acctctgaga ctgtgagggc agttctggat agcctctctg agaagaaaaa gtcctctcct    2760
   tagtaactcg agcgctagca cccagctttc ttgtacaaag tggtgatcta gagggcccgc    2820
   ggttcgaagg taagcctatc cctaaccctc tcctcggtct cgattctacg cgtaccggtt    2880
   agtaatgagt ttaaacgggg gaggctaact gaaacacgga aggagacaat accggaagga    2940
   acccgcgcta tgacggcaat aaaaagacag aataaaacgc acgggtgttg ggtcgtttgt    3000
   tcataaacgc ggggttcggt cccagggctg gcactctgtc gataccccac cgagacccca    3060
   ttggggccaa tacgcccgcg tttcttcctt ttccccaccc caccccccaa gttcgggtga    3120
   aggcccaggg ctcgcagcca acgtcggggc ggcaggccct gccatagcag atctgcgcag    3180
   ctggggctct agggggtatc cccacgcgcc ctgtagcggc gcattaagcg cggcgggtgt    3240
   ggtggttacg cgcagcgtga ccgctacact tgccagcgcc ctagcgcccg ctcctttcgc    3300
   tttcttccct tcctttctcg ccacgttcgc cggctttccc cgtcaagctc taaatcgggg    3360
   catcccttta gggttccgat ttagtgcttt acggcacctc gaccccaaaa aacttgatta    3420
   gggtgatggt tcacgtagtg ggccatcgcc ctgatagacg gtttttcgcc ctttgacgtt    3480
   ggagtccacg ttctttaata gtggactctt gttccaaact ggaacaacac tcaaccctat    3540
   ctcggtctat tcttttgatt tataagggat tttggggatt tcggcctatt ggttaaaaaa    3600
   tgagctgatt taacaaaaat ttaacgcgaa ttaattctgt ggaatgtgtg tcagttaggg    3660
   tgtggaaagt ccccaggctc cccagcaggc agaagtatgc aaagcatgca tctcaattag    3720
   tcagcaacca ggtgtggaaa gtccccaggc tccccagcag gcagaagtat gcaaagcatg    3780
   catctcaatt agtcagcaac catagtcccg cccctaactc cgcccatccc gcccctaact    3840
   ccgcccagtt ccgcccattc tccgccccat ggctgactaa ttttttttat ttatgcagag    3900
   gccgaggccg cctctgcctc tgagctattc cagaagtagt gaggaggctt ttttggaggc    3960
   ctaggctttt gcaaaaagct cccgggagct tgtatatcca ttttcggatc tgatcagcac    4020
   gtgttgacaa ttaatcatcg gcatagtata tcggcatagt ataatacgac aaggtgagga    4080
   actaaaccat ggccaagcct ttgtctcaag aagaatccac cctcattgaa agagcaacgg    4140
   ctacaatcaa cagcatcccc atctctgaag actacagcgt cgccagcgca gctctctcta    4200
   gcgacggccg catcttcact ggtgtcaatg tatatcattt tactggggga ccttgtgcag    4260
   aactcgtggt gctgggcact gctgctgctg cggcagctgg caacctgact tgtatcgtcg   4320
   cgatcggaaa tgagaacagg ggcatcttga gcccctgcgg acggtgccga caggtgcttc   4380
   tcgatctgca tcctgggatc aaagccatag tgaaggacag tgatggacag ccgacggcag   4440
   ttgggattcg tgaattgctg ccctctggtt atgtgtggga gggctaagca cttcgtggcc   4500
   gaggagcagg actgacacgt gctacgagat ttcgattcca ccgccgcctt ctatgaaagg   4560
   ttgggcttcg gaatcgtttt ccgggacgcc ggctggatga tcctccagcg cggggatctc   4620
   atgctggagt tcttcgccca ccccaacttg tttattgcag cttataatgg ttacaaataa   4680
   agcaatagca tcacaaattt cacaaataaa gcattttttt cactgcattc tagttgtggt   4740
   ttgtccaaac tcatcaatgt atcttatcat gtctgtatac cgtcgacctc tagctagagc   4800
   ttggcgtaat catggtcata gctgtttcct gtgtgaaatt gttatccgct cacaattcca   4860
   cacaacatac gagccggaag cataaagtgt aaagcctggg gtgcctaatg agtgagctaa   4920
   ctcacattaa ttgcgttgcg ctcactgccc gctttccagt cgggaaacct gtcgtgccag   4980
   ctgcattaat gaatcggcca acgcgcgggg agaggcggtt tgcgtattgg gcgctcttcc   5040
   gcttcctcgc tcactgactc gctgcgctcg gtcgttcggc tgcggcgagc ggtatcagct   5100
   cactcaaagg cggtaatacg gttatccaca gaatcagggg ataacgcagg aaagaacatg   5160
   tgagcaaaag gccagcaaaa ggccaggaac cgtaaaaagg ccgcgttgct ggcgtttttc   5220
   cataggctcc gcccccctga cgagcatcac aaaaatcgac gctcaagtca gaggtggcga   5280
   aacccgacag gactataaag ataccaggcg tttccccctg gaagctccct cgtgcgctct   5340
   cctgttccga ccctgccgct taccggatac ctgtccgcct ttctcccttc gggaagcgtg   5400
   gcgctttctc atagctcacg ctgtaggtat ctcagttcgg tgtaggtcgt tcgctccaag   5460
   ctgggctgtg tgcacgaacc ccccgttcag cccgaccgct gcgccttatc cggtaactat   5520
   cgtcttgagt ccaacccggt aagacacgac ttatcgccac tggcagcagc cactggtaac   5580
   aggattagca gagcgaggta tgtaggcggt gctacagagt tcttgaagtg gtggcctaac   5640
   tacggctaca ctagaagaac agtatttggt atctgcgctc tgctgaagcc agttaccttc   5700
   ggaaaaagag ttggtagctc ttgatccggc aaacaaacca ccgctggtag cggttttttt   5760
   gtttgcaagc agcagattac gcgcagaaaa aaaggatctc aagaagatcc tttgatcttt   5820
   tctacggggt ctgacgctca gtggaacgaa aactcacgtt aagggatttt ggtcatgaga   5880
   ttatcaaaaa ggatcttcac ctagatcctt ttaaattaaa aatgaagttt taaatcaatc   5940
   taaagtatat atgagtaaac ttggtctgac agttaccaat gcttaatcag tgaggcacct   6000
   atctcagcga tctgtctatt tcgttcatcc atagttgcct gactccccgt cgtgtagata   6060
   actacgatac gggagggctt accatctggc cccagtgctg caatgatacc gcgagaccca   6120
   cgctcaccgg ctccagattt atcagcaata aaccagccag ccggaagggc cgagcgcaga   6180
   agtggtcctg caactttatc cgcctccatc cagtctatta attgttgccg ggaagctaga   6240
   gtaagtagtt cgccagttaa tagtttgcgc aacgttgttg ccattgctac aggcatcgtg   6300
   gtgtcacgct cgtcgtttgg tatggcttca ttcagctccg gttcccaacg atcaaggcga   6360
   gttacatgat cccccatgtt gtgcaaaaaa gcggttagct ccttcggtcc tccgatcgtt   6420
   gtcagaagta agttggccgc agtgttatca ctcatggtta tggcagcact gcataattct   6480
   cttactgtca tgccatccgt aagatgcttt tctgtgactg gtgagtactc aaccaagtca   6540
   ttctgagaat agtgtatgcg gcgaccgagt tgctcttgcc cggcgtcaat acgggataat   6600
   accgcgccac atagcagaac tttaaaagtg ctcatcattg gaaaacgttc ttcggggcga   6660
   aaactctcaa ggatcttacc gctgttgaga tccagttcga tgtaacccac tcgtgcaccc   6720
   aactgatctt cagcatcttt tactttcacc agcgtttctg ggtgagcaaa aacaggaagg   6780
   caaaatgccg caaaaaaggg aataagggcg acacggaaat gttgaatact catactcttc   6840
   ctttttcaat attattgaag catttatcag ggttattgtc tcatgagcgg atacatattt   6900
   gaatgtattt agaaaaataa acaaataggg gttccgcgca catttccccg aaaagtgcca   6960
   cctgacgtc                                                           6969
<210> 197
   <211> 599
   <212> PRT
   <213> Artificial sequence
<220>
   <223> Trex-SC_CAPNS1 protein
<400> 197
   Met Gly Ser Glu Ala Pro Arg Ala Glu Thr Phe Val Phe Leu Asp Leu
   1               5                   10                  15
   Glu Ala Thr Gly Leu Pro Ser Val Glu Pro Glu Ile Ala Glu Leu Ser
               20                  25                  30
   Leu Phe Ala Val His Arg Ser Ser Leu Glu Asn Pro Glu His Asp Glu
           35                  40                  45
   Ser Gly Ala Leu Val Leu Pro Arg Val Leu Asp Lys Leu Thr Leu Cys
       50                  55                  60
   Met Cys Pro Glu Arg Pro Phe Thr Ala Lys Ala Ser Glu Ile Thr Gly
   65                  70                  75                  80
   Leu Ser Ser Glu Gly Leu Ala Arg Cys Arg Lys Ala Gly Phe Asp Gly
                   85                  90                  95
   Ala Val Val Arg Thr Leu Gln Ala Phe Leu Ser Arg Gln Ala Gly Pro
               100                 105                 110
   Ile Cys Leu Val Ala His Asn Gly Phe Asp Tyr Asp Phe Pro Leu Leu
           115                 120                 125
   Cys Ala Glu Leu Arg Arg Leu Gly Ala Arg Leu Pro Arg Asp Thr Val
       130                 135                 140
   Cys Leu Asp Thr Leu Pro Ala Leu Arg Gly Leu Asp Arg Ala His Ser
   145                 150                 155                 160
   His Gly Thr Arg Ala Arg Gly Arg Gln Gly Tyr Ser Leu Gly Ser Leu
                   165                 170                 175
   Phe His Arg Tyr Phe Arg Ala Glu Pro Ser Ala Ala His Ser Ala Glu
               180                 185                 190
   Gly Asp Val His Thr Leu Leu Leu Ile Phe Leu His Arg Ala Ala Glu
           195                 200                 205
   Leu Leu Ala Trp Ala Asp Glu Gln Ala Arg Gly Trp Ala His Ile Glu
       210                 215                 220
   Pro Met Tyr Leu Pro Pro Asp Asp Pro Ser Leu Glu Ala Gly Gly Gly
   225                 230                 235                 240
   Gly Ser Gly Gly Gly Gly Ser Asn Thr Lys Tyr Asn Glu Glu Phe Leu
                   245                 250                 255
   Leu Tyr Leu Ala Gly Phe Val Asp Gly Asp Gly Ser Ile Val Ala Gln
               260                 265                 270
   Ile Lys Pro Asn Gln Arg Ala Lys Phe Lys His Gln Leu Ser Leu Thr
           275                 280                 285
   Phe Gln Val Thr Gln Lys Thr Gln Arg Arg Trp Leu Leu Asp Lys Leu
       290                 295                 300
   Val Asp Glu Ile Gly Val Gly Tyr Val Gln Asp Ser Gly Ser Val Ser
   305                 310                 315                 320
   Asn Tyr Arg Leu Ser Glu Ile Lys Pro Leu His Asn Phe Leu Thr Gln
                   325                 330                 335
   Leu Gln Pro Phe Leu Glu Leu Lys Gln Lys Gln Ala Asn Leu Val Leu
               340                 345                 350
   Lys Ile Ile Glu Gln Leu Pro Ser Ala Lys Glu Ser Pro Asp Lys Phe
           355                 360                 365
   Leu Glu Val Cys Thr Trp Ala Asp Gln Ile Ala Ala Leu Asn Asp Ser
       370                 375                 380
   Lys Thr Arg Lys Thr Thr Ser Glu Thr Val Arg Ala Val Leu Asp Ser
   385                 390                 395                 400
   Leu Ser Glu Lys Lys Lys Pro Ser Pro Ala Ala Gly Gly Ser Asp Lys
                   405                 410                 415
   Tyr Asn Gln Ala Leu Ser Lys Tyr Asn Gln Ala Leu Ser Lys Tyr Asn
               420                 425                 430
   Gln Ala Leu Ser Gly Gly Gly Gly Ser Asn Lys Lys Phe Leu Leu Tyr
           435                 440                 445
   Leu Ala Gly Phe Val Asp Ser Asp Gly Ser Ile Ile Ala Gln Ile Lys
       450                 455                 460
   Pro Arg Gln Ser Tyr Lys Phe Lys His Gln Leu Arg Leu Thr Phe Tyr
   465                 470                 475                 480
   Val Thr Gln Lys Thr Gln Arg Arg Trp Phe Leu Asp Lys Leu Val Asp
                   485                 490                 495
   Arg Ile Gly Val Gly Tyr Val Glu Asp Ser Gly Ser Val Ser Arg Tyr
               500                 505                 510
   Val Leu Ser Glu Ile Lys Pro Leu His Asn Phe Leu Thr Gln Leu Gln
           515                 520                 525
   Pro Phe Leu Lys Leu Lys Gln Lys Gln Ala Asn Leu Val Leu Lys Ile
   530 535 540
   Ile Glu Gln Leu Pro Ser Ala Lys Glu Ser Pro Asp Lys Phe Leu Glu
   545 550 555 560
   Val Cys Thr Trp Val Asp Gln Val Ala Ala Leu Asn Asp Ser Lys Thr
   565 570 575
   Arg Lys Thr Thr Ser Glu Thr Val Arg Ala Val Leu Asp Ser Leu Ser
   580 585 590
   Glu Lys Lys Lys Ser Ser Pro
   595
<210> 198
<400> 198
   000
<210> 199
   <211> 56
   <212> NUCLEIC
   <213> Artificial sequence
<220>
   <223> CAPNS1 locus specific forward primer
<220>
   <221> misc_feature
   <222> (27); (28); (29); (30); (31); (32); (33); (34); (35); (36)
   <223> n is a or c or t or g
<400> 199
   ccatctcatc cctgcgtgtc tccgacnnnn nnnnnncgag tcagggcggg attaag 56
<210> 200
   <211> 50
   <212> NUCLEIC
   <213> Artificial sequence
<220>
   <223> CAPNS1 locus specific reverse primer
<400> 200
   cctatcccct gtgtgccttg gcagtctcag cgagacttca cggtttcgcc 50
<210> 201
   <211> 508
   <212> PRT
   <213> Homo sapiens
<220>
   <223> Human Tdt protein
<400> 201
   Met Asp Pro Pro Arg Ala Ser His Leu Ser Pro Arg Lys Lys Arg Pro
   1               5                   10                  15
   Arg Gln Thr Gly Ala Leu Met Ala Ser Ser Pro Gln Asp Ile Lys Phe
               20                  25                  30
   Gln Asp Leu Val Val Phe Ile Leu Glu Lys Lys Met Gly Thr Thr Arg
           35                  40                  45
   Arg Ala Phe Leu Met Glu Leu Ala Arg Arg Lys Gly Phe Arg Val Glu
       50                  55                  60
   Asn Glu Leu Ser Asp Ser Val Thr His Ile Val Ala Glu Asn Asn Ser
   65                  70                  75                  80
   Gly Ser Asp Val Leu Glu Trp Leu Gln Ala Gln Lys Val Gln Val Ser
                   85                  90                  95
   Ser Gln Pro Glu Leu Leu Asp Val Ser Trp Leu Ile Glu Cys Ile Arg
               100                 105                 110
   Ala Gly Lys Pro Val Glu Met Thr Gly Lys His Gln Leu Val Val Arg
           115                 120                 125
   Arg Asp Tyr Ser Asp Ser Thr Asn Pro Gly Pro Pro Lys Thr Pro Pro
       130                 135                 140
   Ile Ala Val Gln Lys Ile Ser Gln Tyr Ala Cys Gln Arg Arg Thr Thr
   145                 150                 155                 160
   Leu Asn Asn Cys Asn Gln Ile Phe Thr Asp Ala Phe Asp Ile Leu Ala
                   165                 170                 175
   Glu Asn Cys Glu Phe Arg Glu Asn Glu Asp Ser Cys Val Thr Phe Met
               180                 185                 190
   Arg Ala Ala Ser Val Leu Lys Ser Leu Pro Phe Thr Ile Ile Ser Met
           195                 200                 205
   Lys Asp Thr Glu Gly Ile Pro Cys Leu Gly Ser Lys Val Lys Gly Ile
       210                 215                 220
   Ile Glu Glu Ile Ile Glu Asp Gly Glu Ser Ser Glu Val Lys Ala Val
   225 230 235 240
   Leu Asn Asp Glu Arg Tyr Gln Ser Phe Lys Leu Phe Thr Ser Val Phe
   245 250 255
   Gly Val Gly Leu Lys Thr Ser Glu Lys Trp Phe Arg Met Gly Phe Arg
   260 265 270
   Thr Leu Ser Lys Val Arg Ser Asp Lys Ser Leu Lys Phe Thr Arg Met
   275 280 285
   Gln Lys Ala Gly Phe Leu Tyr Tyr Glu Asp Leu Val Ser Cys Val Thr
   290 295 300
   Arg Ala Glu Ala Glu Ala Val Ser Val Leu Val Lys Glu Ala Val Trp
   305 310 315 320
   Ala Phe Leu Pro Asp Ala Phe Val Thr Met Thr Gly Gly Phe Arg Arg
   325 330 335
   Gly Lys Lys Met Gly His Asp Val Asp Phe Leu Ile Thr Ser Pro Gly
   340 345 350
   Ser Thr Glu Asp Glu Glu Gln Leu Leu Gln Lys Val Met Asn Leu Trp
   355 360 365
   Glu Lys Lys Gly Leu Leu Leu Tyr Tyr Asp Leu Val Glu Ser Thr Phe
   370 375 380
   Glu Lys Leu Arg Leu Pro Ser Arg Lys Val Asp Ala Leu Asp His Phe
   385 390 395 400
   Gln Lys Cys Phe Leu Ile Phe Lys Leu Pro Arg Gln Arg Val Asp Ser
   405 410 415
   Asp Gln Ser Ser Trp Gln Glu Gly Lys Thr Trp Lys Ala Ile Arg Val
   420 425 430
   Asp Leu Val Leu Cys Pro Tyr Glu Arg Arg Ala Phe Ala Leu Leu Gly
   435 440 445
   Trp Thr Gly Ser Arg Phe Glu Arg Asp Leu Arg Arg Tyr Ala Thr His
   450 455 460
   Glu Arg Lys Met Ile Leu Asp Asn His Ala Leu Tyr Asp Lys Thr Lys
   465 470 475 480
   Arg Ile Phe Leu Lys Ala Glu Ser Glu Glu Glu Ile Phe Ala His Leu
                   485                 490                 495
   Gly Leu Asp Tyr Ile Glu Pro Trp Glu Arg Asn Ala
               500                 505
<210> 202
   <211> 6438
   <212> NUCLEIC
   <213> Artificial sequence
<220>
   <223> pCLS3841
<400> 202
   aacaaaatat taacgcttac aatttccatt cgccattcag gctgcgcaac tgttgggaag     60
   ggcgatcggt gcgggcctct tcgctattac gccagctggc gaaaggggga tgtgctgcaa    120
   ggcgattaag ttgggtaacg ccagggtttt cccagtcacg acgttgtaaa acgacggcca    180
   gtgccaagct gatctataca ttgaatcaat attggcaatt agccatatta gtcattggtt    240
   atatagcata aatcaatatt ggctattggc cattgcatac gttgtatcta tatcataata    300
   tgtacattta tattggctca tgtccaatat gaccgccatg ttgacattga ttattgacta    360
   gttattaata gtaatcaatt acggggtcat tagttcatag cccatatatg gagttccgcg    420
   ttacataact tacggtaaat ggcccgcctg gctgaccgcc caacgacccc cgcccattga    480
   cgtcaataat gacgtatgtt cccatagtaa cgccaatagg gactttccat tgacgtcaat    540
   gggtggagta tttacggtaa actgcccact tggcagtaca tcaagtgtat catatgccaa    600
   gtccgccccc tattgacgtc aatgacggta aatggcccgc ctggcattat gcccagtaca    660
   tgaccttacg ggactttcct acttggcagt acatctacgt attagtcatc gctattacca    720
   tggtgatgcg gttttggcag tacaccaatg ggcgtggata gcggtttgac tcacggggat    780
   ttccaagtct ccaccccatt gacgtcaatg ggagtttgtt ttggcaccaa aatcaacggg    840
   actttccaaa atgtcgtaat aaccccgccc cgttgacgca aatgggcggt aggcgtgtac    900
   ggtgggaggt ctatataagc agagctcgtt tagtgaaccg tcagaatttt gtaatacgac    960
   tcactatagg gcggccgcga attcagatct ggtaccggtc cggaattccc gggatatcgt   1020
   cgacccacgc gtccgcacca ccagatgggc cagccagagg cagcagcagc ctcttcccat   1080
   ggatccacca cgagcgtccc acttgagccc tcggaagaag agaccccggc agacgggtgc   1140
   cttgatggcc tcctctcctc aagacatcaa atttcaagat ttggtcgtct tcattttgga   1200
   gaagaaaatg ggaaccaccc gcagagcgtt cctcatggag ctggcccgca ggaaagggtt   1260
   cagggttgaa aatgagctca gtgattctgt cacccacatt gtagcagaga acaactcggg   1320
   ttcggatgtt ctggagtggc ttcaagcaca gaaagtacaa gtcagctcac aaccagagct   1380
   cctcgatgtc tcctggctga tcgaatgcat aggagcaggg aaaccggtgg aaatgacagg   1440
   aaaacaccag cttgttgtga gaagagacta ttcagatagc accaacccag gccccccgaa   1500
   gactccacca attgctgtac aaaagatctc ccagtatgcg tgtcagagaa gaaccacttt   1560
   aaacaactgt aaccagatat tcacggatgc ctttgatata ctggctgaaa actgtgagtt   1620
   tagagaaaat gaagactcct gtgtgacatt tatgagagca gcttctgtat tgaaatctct   1680
   gccattcaca atcatcagta tgaaggacac agaaggaatt ccctgcctgg ggtccaaggt   1740
   gaagggtatc atagaggaga ttattgaaga tggagaaagt tctgaagtta aagctgtgtt   1800
   aaatgatgaa cgatatcaat ccttcaaact ctttacttct gtatttggag tggggctgaa   1860
   gacttctgag aagtggttca ggatgggttt cagaactctg agtaaagtaa ggtcggacaa   1920
   aagcctgaaa tttacacgaa tgcagaaagc aggatttctg tattatgaag accttgtcag   1980
   ctgtgtgacc agggcagaag cagaggccgt cagtgtgctg gttaaagagg ctgtctgggc   2040
   atttcttccg gatgctttcg tcaccatgac aggagggttc cggaggggta agaagatggg   2100
   gcatgatgta gattttttaa ttaccagccc aggatcaaca gaggatgaag agcaactttt   2160
   acagaaagtg atgaacttat gggaaaagaa gggattactt ttatattatg accttgtgga   2220
   gtcaacattt gaaaagctca ggttgcctag caggaaggtt gatgctttgg atcattttca   2280
   aaagtgcttt ctgattttca aattgcctcg tcaaagagtg gacagtgacc agtccagctg   2340
   gcaggaagga aagacctgga aggccatccg tgtggattta gttctgtgcc cctacgagcg   2400
   tcgtgccttt gccctgttgg gatggactgg ctcccggcag tttgagagag acctccggcg   2460
   ctatgccaca catgagcgga agatgattct ggataaccat gctttatatg acaagaccaa   2520
   gaggatattc ctcaaagcag aaagtgaaga agaaattttt gcgcatctgg gattggatta   2580
   tattgaaccg tgggaaagaa atgcctagga aagtgttgtc aacatttttt tcctattctt   2640
   ttcaagttaa ataaattatg cttcatatta gtaaaagatg ccataggaga gtttggggtt   2700
   atttaggtct tattgaaatg cagattgcta ctagaaataa ataactttgg aaacatggga   2760
   aggtgccact ggtaatgggt aaggttctaa taggccatgt ttatgactgt tgcatagaat   2820
   tcacaatgca tttttcaaga gaaatgatgt tgtcactggt ggctcattca gggaagctca   2880
   tcaaagccca ctttgttcgc agtgtagctg aaatactgtc tatctctaat aaaaacagga   2940
   ggaaacaaaa aaaaaaaaaa aaaaaaaaaa aaaaaaaaag ggcggccgcg gtcatagctg   3000
   tttcctgaac agatcccggg tggcatccct gtgacccctc cccagtgcct ctcctggccc   3060
   tggaagttgc cactccagtg cccaccagcc ttgtcctaat aaaattaagt tgcatcattt   3120
   tgtctgacta ggtgtccttc tataatatta tggggtggag gggggtggta tggagcaagg   3180
   ggcaagttgg gaagacaacc tgtagggcct gcggggtcta ttgggaacca agctggagtg   3240
   cagtggcaca atcttggctc actgcaatct ccgcctcctg ggttcaagcg attctcctgc   3300
   ctcagcctcc cgagttgttg ggattccagg catgcatgac caggctcagc taatttttgt   3360
   ttttttggta gagacggggt ttcaccatat tggccaggct ggtctccaac tcctaatctc   3420
   aggtgatcta cccaccttgg cctcccaaat tgctgggatt acaggcgtga accactgctc   3480
   ccttccctgt ccttctgatt ttaaaataac tataccagca ggaggacgtc cagacacagc   3540
   ataggctacc tggccatgcc caaccggtgg gacatttgag ttgcttgctt ggcactgtcc   3600
   tctcatgcgt tgggtccact cagtagatgc ctgttgaatt gggtacgcgg ccagcttggc   3660
   tgtggaatgt gtgtcagtta gggtgtggaa agtccccagg ctccccagca ggcagaagta   3720
   tgcaaagcat gcatctcaat tagtcagcaa ccaggtgtgg aaagtcccca ggctccccag   3780
   caggcagaag tatgcaaagc atgcatctca attagtcagc aaccatagtc ccgcccctaa   3840
   ctccgcccat cccgccccta actccgccca gttccgccca ttctccgccc catggctgac   3900
   taattttttt tatttatgca gaggccgagg ccgcctcggc ctctgagcta ttccagaagt   3960
   agtgaggagg cttttttgga ggcctaggct tttgcaaaaa gctcctcgac tgcattaatg   4020
   aatcggccaa cgcgcgggga gaggcggttt gcgtattggg cgctcttccg cttcctcgct   4080
   cactgactcg ctgcgctcgg tcgttcggct gcggcgagcg gtatcagctc actcaaaggc   4140
   ggtaatacgg ttatccacag aatcagggga taacgcagga aagaacatgt gagcaaaagg   4200
   ccagcaaaag gccaggaacc gtaaaaaggc cgcgttgctg gcgtttttcc ataggctccg   4260
   cccccctgac gagcatcaca aaaatcgacg ctcaagtcag aggtggcgaa acccgacagg   4320
   actataaaga taccaggcgt ttccccctgg aagctccctc gtgcgctctc ctgttccgac   4380
   cctgccgctt accggatacc tgtccgcctt tctcccttcg ggaagcgtgg cgctttctca   4440
   tagctcacgc tgtaggtatc tcagttcggt gtaggtcgtt cgctccaagc tgggctgtgt   4500
   gcacgaaccc cccgttcagc ccgaccgctg cgccttatcc ggtaactatc gtcttgagtc   4560
   caacccggta agacacgact tatcgccact ggcagcagcc actggtaaca ggattagcag   4620
   agcgaggtat gtaggcggtg ctacagagtt cttgaagtgg tggcctaact acggctacac   4680
   tagaagaaca gtatttggta tctgcgctct gctgaagcca gttaccttcg gaaaaagagt   4740
   tggtagctct tgatccggca aacaaaccac cgctggtagc ggtggttttt ttgtttgcaa   4800
   gcagcagatt acgcgcagaa aaaaaggatc tcaagaagat cctttgatct tttctacggg   4860
   gtctgacgct cagtggaacg aaaactcacg ttaagggatt ttggtcatga gattatcaaa   4920
   aaggatcttc acctagatcc ttttaaatta aaaatgaagt tttaaatcaa tctaaagtat   4980
   atatgagtaa acttggtctg acagttacca atgcttaatc agtgaggcac ctatctcagc   5040
   gatctgtcta tttcgttcat ccatagttgc ctgactcccc gtcgtgtaga taactacgat   5100
   acgggagggc ttaccatctg gccccagtgc tgcaatgata ccgcgagacc cacgctcacc   5160
   ggctccagat ttatcagcaa taaaccagcc agccggaagg gccgagcgca gaagtggtcc   5220
   tgcaacttta tccgcctcca tccagtctat taattgttgc cgggaagcta gagtaagtag   5280
   ttcgccagtt aatagtttgc gcaacgttgt tgccattgct acaggcatcg tggtgtcacg   5340
   ctcgtcgttt ggtatggctt cattcagctc cggttcccaa cgatcaaggc gagttacatg   5400
   atcccccatg ttgtgcaaaa aagcggttag ctccttcggt cctccgatcg ttgtcagaag   5460
   taagttggcc gcagtgttat cactcatggt tatggcagca ctgcataatt ctcttactgt   5520
   catgccatcc gtaagatgct tttctgtgac tggtgagtac tcaaccaagt cattctgaga   5580
   atagtgtatg cggcgaccga gttgctcttg cccggcgtca atacgggata ataccgcgcc   5640
   acatagcaga actttaaaag tgctcatcat tggaaaacgt tcttcggggc gaaaactctc   5700
   aaggatctta ccgctgttga gatccagttc gatgtaaccc actcgtgcac ccaactgatc   5760
   ttcagcatct tttactttca ccagcgtttc tgggtgagca aaaacaggaa ggcaaaatgc   5820
   cgcaaaaaag ggaataaggg cgacacggaa atgttgaata ctcatactct tcctttttca   5880
   atattattga agcatttatc agggttattg tctcatgagc ggatacatat ttgaatgtat   5940
   ttagaaaaat aaacaaatag gggttccgcg cacatttccc cgaaaagtgc cacctgacgc   6000
   gccctgtagc ggcgcattaa gcgcggcggg tgtggtggtt acgcgcagcg tgaccgctac   6060
   acttgccagc gccctagcgc ccgctccttt cgctttcttc ccttcctttc tcgccacgtt   6120
   cgccggcttt ccccgtcaag ctctaaatcg ggggctccct ttagggttcc gatttagtgc   6180
   tttacggcac ctcgacccca aaaaacttga ttagggtgat ggttcacgta gtgggccatc   6240
   gccctgatag acggtttttc gccctttgac gttggagtcc acgttcttta atagtggact   6300
   cttgttccaa actggaacaa cactcaaccc tatctcggtc tattcttttg atttataagg   6360
   gattttgccg atttcggcct attggttaaa aaatgagctg atttaacaaa aatttaacgc   6420
   gaattttaac aaaatatt                                                 6438
<210> 203
   <211> 48
   <212> NUCLEIC
   <213> Artificial sequence
<220>
   <223> LinkTDTFor primer
<400> 203
   ggcggatctg gaggtggagg ttccgatcca ccacgagcgt cccacttg 48
<210> 204
   <211> 29
   <212> NUCLEIC
   <213> Artificial sequence
<220>
   <223> TDTRev
<400> 204
   ggctcgagct aggcatttct ttcccacgg 29
<210> 205
   <211> 30
   <212> NUCLEIC
   <213> Artificial sequence
<220>
   <223> TDTFor
<400> 205
   ggcgcgccat ggatccacca cgagcgtccc 30
<210> 206
   <211> 48
   <212> NUCLEIC
   <213> Artificial sequence
<220>
   <223> Link10TDTRev
<400> 206
   acctccacct ccagaacctc cacctccggc atttctttcc cacggttc 48
<210> 207
   <211> 24
   <212> NUCLEIC
   <213> Artificial sequence
<220>
   <223> N1 meganuclease target sequence
<400> 207
   ttgttctcag gtacctcagc cagc 24
<210> 208
   <211> 49
   <212> NUCLEIC
   <213> Artificial sequence
<220>
   <223> N2 TALEN target sequence
<400> 208
   tatatttaag cacttatatg tgtgtaacag gtataagtaa ccataaaca 49
<210> 209
   <211> 1065
   <212> PRT
   <213> Artificial sequence
<220>
   <223> N2 TALEN monomer 1 protein
<400> 209
   Met Ala Asp Pro Ile Arg Ser Arg Thr Pro Ser Pro Ala Arg Glu Leu
   1               5                   10                  15
   Leu Pro Gly Pro Gln Pro Asp Gly Val Gln Pro Thr Ala Asp Arg Gly
               20                  25                  30
   Val Ser Pro Pro Ala Gly Gly Pro Leu Asp Gly Leu Pro Ala Arg Arg
           35                  40                  45
   Thr Met Ser Arg Thr Arg Leu Pro Ser Pro Pro Ala Pro Ser Pro Ala
       50                  55                  60
   Phe Ser Ala Gly Ser Phe Ser Asp Leu Leu Arg Gln Phe Asp Pro Ser
   65                  70                  75                  80
   Leu Phe Asn Thr Ser Leu Phe Asp Ser Leu Pro Pro Phe Gly Ala His
                   85                  90                  95
   His Thr Glu Ala Ala Thr Gly Glu Trp Asp Glu Val Gln Ser Gly Leu
               100                 105                 110
   Arg Ala Ala Asp Ala Pro Pro Pro Thr Met Arg Val Ala Val Thr Ala
           115                 120                 125
   Ala Arg Pro Pro Arg Ala Lys Pro Ala Pro Arg Arg Arg Ala Ala Gln
       130                 135                 140
   Pro Ser Asp Ala Ser Pro Ala Ala Gln Val Asp Leu Arg Thr Leu Gly
   145                 150                 155                 160
   Tyr Ser Gln Gln Gln Gln Glu Lys Ile Lys Pro Lys Val Arg Ser Thr
                   165                 170                 175
   Val Ala Gln His His Glu Ala Leu Val Gly His Gly Phe Thr His Ala
               180                 185                 190
   His Ile Val Ala Leu Ser Gln His Pro Ala Ala Leu Gly Thr Val Ala
           195                 200                 205
   Val Lys Tyr Gln Asp Met Ile Ala Ala Leu Pro Glu Ala Thr His Glu
       210                 215                 220
   Ala Ile Val Gly Val Gly Lys Gln Trp Ser Gly Ala Arg Ala Leu Glu
   225                 230                 235                 240
   Ala Leu Leu Thr Val Ala Gly Glu Leu Arg Gly Pro Pro Leu Gln Leu
                   245                 250                 255
   Asp Thr Gly Gln Leu Leu Lys Ile Ala Lys Arg Gly Gly Val Thr Ala
               260                 265                 270
   Val Glu Ala Val His Ala Trp Arg Asn Ala Leu Thr Gly Ala Pro Leu
           275                 280                 285
   Asn Leu Thr Pro Gln Gln Val Val Ala Ile Ala Ser Asn Asn Gly Gly
       290                 295                 300
   Lys Gln Ala Leu Glu Thr Val Gln Arg Leu Leu Pro Val Leu Cys Gln
   305                 310                 315                 320
   Ala His Gly Leu Thr Pro Gln Gln Val Val Ala Ile Ala Ser Asn Gly
                   325                 330                 335
   Gly Gly Lys Gln Ala Leu Glu Thr Val Gln Arg Leu Leu Pro Val Leu
               340                 345                 350
   Cys Gln Ala His Gly Leu Thr Pro Gln Gln Val Val Ala Ile Ala Ser
           355                 360                 365
   Asn Gly Gly Gly Lys Gln Ala Leu Glu Thr Val Gln Arg Leu Leu Pro
       370                 375                 380
   Val Leu Cys Gln Ala His Gly Leu Thr Pro Gln Gln Val Val Ala Ile
   385                 390                 395                 400
   Ala Ser Asn Gly Gly Gly Lys Gln Ala Leu Glu Thr Val Gln Arg Leu
                   405                 410                 415
   Leu Pro Val Leu Cys Gln Ala His Gly Leu Thr Pro Glu Gln Val Val
               420                 425                 430
   Ala Ile Ala Ser Asn Ile Gly Gly Lys Gln Ala Leu Glu Thr Val Gln
           435                 440                 445
   Ala Leu Leu Pro Val Leu Cys Gln Ala His Gly Leu Thr Pro Gln Gln
       450                 455                 460
   Val Val Ala Ile Ala Ser Asn Gly Gly Gly Lys Gln Ala Leu Glu Thr
   465                 470                 475                 480
   Val Gln Arg Leu Leu Pro Val Leu Cys Gln Ala His Gly Leu Thr Pro
                   485                 490                 495
   Gln Gln Val Val Ala Ile Ala Ser Asn Asn Gly Gly Lys Gln Ala Leu
               500                 505                 510
   Glu Thr Val Gln Arg Leu Leu Pro Val Leu Cys Gln Ala His Gly Leu
           515                 520                 525
   Thr Pro Gln Gln Val Val Ala Ile Ala Ser Asn Asn Gly Gly Lys Gln
       530                 535                 540
   Ala Leu Glu Thr Val Gln Arg Leu Leu Pro Val Leu Cys Gln Ala His
   545                 550                 555                 560
   Gly Leu Thr Pro Gln Gln Val Val Ala Ile Ala Ser Asn Gly Gly Gly
                   565                 570                 575
   Lys Gln Ala Leu Glu Thr Val Gln Arg Leu Leu Pro Val Leu Cys Gln
               580                 585                 590
   Ala His Gly Leu Thr Pro Gln Gln Val Val Ala Ile Ala Ser Asn Gly
           595                 600                 605
   Gly Gly Lys Gln Ala Leu Glu Thr Val Gln Arg Leu Leu Pro Val Leu
       610                 615                 620
   Cys Gln Ala His Gly Leu Thr Pro Glu Gln Val Val Ala Ile Ala Ser
   625                 630                 635                 640
   Asn Ile Gly Gly Lys Gln Ala Leu Glu Thr Val Gln Ala Leu Leu Pro
                   645                 650                 655
   Val Leu Cys Gln Ala His Gly Leu Thr Pro Glu Gln Val Val Ala Ile
               660                 665                 670
   Ala Ser His Asp Gly Gly Lys Gln Ala Leu Glu Thr Val Gln Arg Leu
           675                 680                 685
   Leu Pro Val Leu Cys Gln Ala His Gly Leu Thr Pro Gln Gln Val Val
       690                 695                 700
   Ala Ile Ala Ser Asn Gly Gly Gly Lys Gln Ala Leu Glu Thr Val Gln
   705                 710                 715                 720
   Arg Leu Leu Pro Val Leu Cys Gln Ala His Gly Leu Thr Pro Gln Gln
                   725                 730                 735
   Val Val Ala Ile Ala Ser Asn Gly Gly Gly Lys Gln Ala Leu Glu Thr
               740                 745                 750
   Val Gln Arg Leu Leu Pro Val Leu Cys Gln Ala His Gly Leu Thr Pro
           755                 760                 765
   Glu Gln Val Val Ala Ile Ala Ser Asn Ile Gly Gly Lys Gln Ala Leu
       770                 775                 780
   Glu Thr Val Gln Ala Leu Leu Pro Val Leu Cys Gln Ala His Gly Leu
   785                 790                 795                 800
   Thr Pro Gln Gln Val Val Ala Ile Ala Ser Asn Gly Gly Gly Arg Pro
                   805                 810                 815
   Ala Leu Glu Ser Ile Val Ala Gln Leu Ser Arg Pro Asp Pro Ala Leu
               820                 825                 830
   Ala Ala Leu Thr Asn Asp His Leu Val Ala Leu Ala Cys Leu Gly Gly
           835                 840                 845
   Arg Pro Ala Leu Asp Ala Val Lys Lys Gly Leu Gly Asp Pro Ile Ser
       850                 855                 860
   Arg Ser Gln Leu Val Lys Ser Glu Leu Glu Glu Lys Lys Ser Glu Leu
   865                 870                 875                 880
   Arg His Lys Leu Lys Tyr Val Pro His Glu Tyr Ile Glu Leu Ile Glu
                   885                 890                 895
   Ile Ala Arg Asn Ser Thr Gln Asp Arg Ile Leu Glu Met Lys Val Met
   900 905 910
   Glu Phe Phe Met Lys Val Tyr Gly Tyr Arg Gly Lys His Leu Gly Gly
   915 920 925
   Ser Arg Lys Pro Asp Gly Ala Ile Tyr Thr Val Gly Ser Pro Ile Asp
   930 935 940
   Tyr Gly Val Ile Val Asp Thr Lys Ala Tyr Ser Gly Gly Tyr Asn Leu
   945 950 955 960
   Pro Ile Gly Gln Ala Asp Glu Met Gln Arg Tyr Val Glu Glu Asn Gln
   965 970 975
   Thr Arg Asn Lys His Ile Asn Pro Asn Glu Trp Trp Lys Val Tyr Pro
   980 985 990
   Ser Ser Val Thr Glu Phe Lys Phe Leu Phe Val Ser Gly His Phe Lys
   995 1000 1005
   Gly Asn Tyr Lys Ala Gln Leu Thr Arg Leu Asn His Ile Thr Asn Cys
   1010 1015 1020
   Asn Gly Ala Val Leu Ser Val Glu Glu Leu Leu Ile Gly Gly Glu Met
   1025 1030 1035 1040
   Ile Lys Ala Gly Thr Leu Thr Leu Glu Glu Val Arg Arg Lys Phe Asn
   1045 1050 1055
   Asn Gly Glu Ile Asn Phe Ala Ala Asp
   1060 1065
<210> 210
   <211> 1065
   <212> PRT
   <213> Artificial sequence
<220>
   <223> N2 TALEN monomer 2 protein
<400> 210
   Met Ala Asp Pro Ile Arg Ser Arg Thr Pro Ser Pro Ala Arg Glu Leu
   1               5                   10                  15
   Leu Pro Gly Pro Gln Pro Asp Gly Val Gln Pro Thr Ala Asp Arg Gly
               20                  25                  30
   Val Ser Pro Pro Ala Gly Gly Pro Leu Asp Gly Leu Pro Ala Arg Arg
           35                  40                  45
   Thr Met Ser Arg Thr Arg Leu Pro Ser Pro Pro Ala Pro Ser Pro Ala
       50                  55                  60
   Phe Ser Ala Gly Ser Phe Ser Asp Leu Leu Arg Gln Phe Asp Pro Ser
   65                  70                  75                  80
   Leu Phe Asn Thr Ser Leu Phe Asp Ser Leu Pro Pro Phe Gly Ala His
                   85                  90                  95
   His Thr Glu Ala Ala Thr Gly Glu Trp Asp Glu Val Gln Ser Gly Leu
               100                 105                 110
   Arg Ala Ala Asp Ala Pro Pro Pro Thr Met Arg Val Ala Val Thr Ala
           115                 120                 125
   Ala Arg Pro Pro Arg Ala Lys Pro Ala Pro Arg Arg Arg Ala Ala Gln
       130                 135                 140
   Pro Ser Asp Ala Ser Pro Ala Ala Gln Val Asp Leu Arg Thr Leu Gly
   145                 150                 155                 160
   Tyr Ser Gln Gln Gln Gln Glu Lys Ile Lys Pro Lys Val Arg Ser Thr
                   165                 170                 175
   Val Ala Gln His His Glu Ala Leu Val Gly His Gly Phe Thr His Ala
               180                 185                 190
   His Ile Val Ala Leu Ser Gln His Pro Ala Ala Leu Gly Thr Val Ala
           195                 200                 205
   Val Lys Tyr Gln Asp Met Ile Ala Ala Leu Pro Glu Ala Thr His Glu
       210                 215                 220
   Ala Ile Val Gly Val Gly Lys Gln Trp Ser Gly Ala Arg Ala Leu Glu
   225                 230                 235                 240
   Ala Leu Leu Thr Val Ala Gly Glu Leu Arg Gly Pro Pro Leu Gln Leu
                   245                 250                 255
   Asp Thr Gly Gln Leu Leu Lys Ile Ala Lys Arg Gly Gly Val Thr Ala
               260                 265                 270
   Val Glu Ala Val His Ala Trp Arg Asn Ala Leu Thr Gly Ala Pro Leu
           275                 280                 285
   Asn Leu Thr Pro Glu Gln Val Val Ala Ile Ala Ser Asn Ile Gly Gly
   290 295 300
   Lys Gln Ala Leu Glu Thr Val Gln Ala Leu Leu Pro Val Leu Cys Gln
   305 310 315 320
   Ala His Gly Leu Thr Pro Gln Gln Val Val Ala Ile Ala Ser Asn Gly
   325 330 335
   Gly Gly Lys Gln Ala Leu Glu Thr Val Gln Arg Leu Leu Pro Val Leu
   340 345 350
   Cys Gln Ala His Gly Leu Thr Pro Glu Gln Val Val Ala Ile Ala Ser
   355 360 365
   Asn Ile Gly Gly Lys Gln Ala Leu Glu Thr Val Gln Ala Leu Leu Pro
   370 375 380
   Val Leu Cys Gln Ala His Gly Leu Thr Pro Gln Gln Val Val Ala Ile
   385 390 395 400
   Ala Ser Asn Gly Gly Gly Lys Gln Ala Leu Glu Thr Val Gln Arg Leu
   405 410 415
   Leu Pro Val Leu Cys Gln Ala His Gly Leu Thr Pro Gln Gln Val Val
   420 425 430
   Ala Ile Ala Ser Asn Gly Gly Gly Lys Gln Ala Leu Glu Thr Val Gln
   435 440 445
   Arg Leu Leu Pro Val Leu Cys Gln Ala His Gly Leu Thr Pro Gln Gln
   450 455 460
   Val Val Ala Ile Ala Ser Asn Gly Gly Gly Lys Gln Ala Leu Glu Thr
   465 470 475 480
   Val Gln Arg Leu Leu Pro Val Leu Cys Gln Ala His Gly Leu Thr Pro
   485 490 495
   Glu Gln Val Val Ala Ile Ala Ser Asn Ile Gly Gly Lys Gln Ala Leu
   500 505 510
   Glu Thr Val Gln Ala Leu Leu Pro Val Leu Cys Gln Ala His Gly Leu
   515 520 525
   Thr Pro Glu Gln Val Val Ala Ile Ala Ser Asn Ile Gly Gly Lys Gln
   530 535 540
   Ala Leu Glu Thr Val Gln Ala Leu Leu Pro Val Leu Cys Gln Ala His
   545                 550                 555                 560
   Gly Leu Thr Pro Gln Gln Val Val Ala Ile Ala Ser Asn Asn Gly Gly
                   565                 570                 575
   Lys Gln Ala Leu Glu Thr Val Gln Arg Leu Leu Pro Val Leu Cys Gln
               580                 585                 590
   Ala His Gly Leu Thr Pro Glu Gln Val Val Ala Ile Ala Ser His Asp
           595                 600                 605
   Gly Gly Lys Gln Ala Leu Glu Thr Val Gln Arg Leu Leu Pro Val Leu
       610                 615                 620
   Cys Gln Ala His Gly Leu Thr Pro Glu Gln Val Val Ala Ile Ala Ser
   625                 630                 635                 640
   Asn Ile Gly Gly Lys Gln Ala Leu Glu Thr Val Gln Ala Leu Leu Pro
                   645                 650                 655
   Val Leu Cys Gln Ala His Gly Leu Thr Pro Glu Gln Val Val Ala Ile
               660                 665                 670
   Ala Ser His Asp Gly Gly Lys Gln Ala Leu Glu Thr Val Gln Arg Leu
           675                 680                 685
   Leu Pro Val Leu Cys Gln Ala His Gly Leu Thr Pro Gln Gln Val Val
       690                 695                 700
   Ala Ile Ala Ser Asn Gly Gly Gly Lys Gln Ala Leu Glu Thr Val Gln
   705                 710                 715                 720
   Arg Leu Leu Pro Val Leu Cys Gln Ala His Gly Leu Thr Pro Gln Gln
                   725                 730                 735
   Val Val Ala Ile Ala Ser Asn Gly Gly Gly Lys Gln Ala Leu Glu Thr
               740                 745                 750
   Val Gln Arg Leu Leu Pro Val Leu Cys Gln Ala His Gly Leu Thr Pro
           755                 760                 765
   Glu Gln Val Val Ala Ile Ala Ser Asn Ile Gly Gly Lys Gln Ala Leu
       770                 775                 780
   Glu Thr Val Gln Ala Leu Leu Pro Val Leu Cys Gln Ala His Gly Leu
   785                 790                 795                 800
   Thr Pro Gln Gln Val Val Ala Ile Ala Ser Asn Gly Gly Gly Arg Pro
   805 810 815
   Ala Leu Glu Ser Ile Val Ala Gln Leu Ser Arg Pro Asp Pro Ala Leu
   820 825 830
   Ala Ala Leu Thr Asn Asp His Leu Val Ala Leu Ala Cys Leu Gly Gly
   835 840 845
   Arg Pro Ala Leu Asp Ala Val Lys Lys Gly Leu Gly Asp Pro Ile Ser
   850 855 860
   Arg Ser Gln Leu Val Lys Ser Glu Leu Glu Glu Lys Lys Ser Glu Leu
   865 870 875 880
   Arg His Lys Leu Lys Tyr Val Pro His Glu Tyr Ile Glu Leu Ile Glu
   885 890 895
   Ile Ala Arg Asn Ser Thr Gln Asp Arg Ile Leu Glu Met Lys Val Met
   900 905 910
   Glu Phe Phe Met Lys Val Tyr Gly Tyr Arg Gly Lys His Leu Gly Gly
   915 920 925
   Ser Arg Lys Pro Asp Gly Ala Ile Tyr Thr Val Gly Ser Pro Ile Asp
   930 935 940
   Tyr Gly Val Ile Val Asp Thr Lys Ala Tyr Ser Gly Gly Tyr Asn Leu
   945 950 955 960
   Pro Ile Gly Gln Ala Asp Glu Met Gln Arg Tyr Val Glu Glu Asn Gln
   965 970 975
   Thr Arg Asn Lys His Ile Asn Pro Asn Glu Trp Trp Lys Val Tyr Pro
   980 985 990
   Ser Ser Val Thr Glu Phe Lys Phe Leu Phe Val Ser Gly His Phe Lys
   995 1000 1005
   Gly Asn Tyr Lys Ala Gln Leu Thr Arg Leu Asn His Ile Thr Asn Cys
   1010 1015 1020
   Asn Gly Ala Val Leu Ser Val Glu Glu Leu Leu Ile Gly Gly Glu Met
   1025 1030 1035 1040
   Ile Lys Ala Gly Thr Leu Thr Leu Glu Glu Val Arg Arg Lys Phe Asn
   1045 1050 1055
   Asn Gly Glu Ile Asn Phe Ala Ala Asp
                   1060                1065
<210> 211
   <211> 8083
   <212> NUCLEIC
   <213> Artificial sequence
<220>
   <223> pCLS8964
<400> 211
   gtttgtttaa acttggtacc ataactagtt cggcgcgcca ctagcgctgt cacgcgtctc     60
   catggccgac cccattcgtt cgcgcacacc aagtcctgcc cgcgagcttc tgcccggacc    120
   ccaacccgat ggggttcagc cgactgcaga tcgtggggtg tctccgcctg ccggcggccc    180
   cctggatggc ttgccggctc ggcggacgat gtcccggacc cggctgccat ctccccctgc    240
   cccctcacct gcgttctcgg cgggcagctt cagtgacctg ttacgtcagt tcgatccgtc    300
   actttttaat acatcgcttt ttgattcatt gcctcccttc ggcgctcacc atacagaggc    360
   tgccacaggc gagtgggatg aggtgcaatc gggtctgcgg gcagccgacg cccccccacc    420
   caccatgcgc gtggctgtca ctgccgcgcg gcccccgcgc gccaagccgg cgccgcgacg    480
   acgtgctgcg caaccctccg acgcttcgcc ggcggcgcag gtggatctac gcacgctcgg    540
   ctacagccag cagcaacagg agaagatcaa accgaaggtt cgttcgacag tggcgcagca    600
   ccacgaggca ctggtcggcc acgggtttac acacgcgcac atcgttgcgt taagccaaca    660
   cccggcagcg ttagggaccg tcgctgtcaa gtatcaggac atgatcgcag cgttgccaga    720
   ggcgacacac gaagcgatcg ttggcgtcgg caaacagtgg tccggcgcac gcgctctgga    780
   ggccttgctc acggtggcgg gagagttgag aggtccaccg ttacagttgg acacaggcca    840
   acttctcaag attgcaaaac gtggcggcgt gaccgcagtg gaggcagtgc atgcatggcg    900
   caatgcactg acgggtgccc cgctcaactt gaccccccag caggtggtgg ccatcgccag    960
   caataatggt ggcaagcagg cgctggagac ggtccagcgg ctgttgccgg tgctgtgcca   1020
   ggcccacggc ttgacccccc agcaggtggt ggccatcgcc agcaatggcg gtggcaagca   1080
   ggcgctggag acggtccagc ggctgttgcc ggtgctgtgc caggcccacg gcttgacccc   1140
   ccagcaggtg gtggccatcg ccagcaatgg cggtggcaag caggcgctgg agacggtcca   1200
   gcggctgttg ccggtgctgt gccaggccca cggcttgacc ccccagcagg tggtggccat   1260
   cgccagcaat ggcggtggca agcaggcgct ggagacggtc cagcggctgt tgccggtgct   1320
   gtgccaggcc cacggcttga ccccggagca ggtggtggcc atcgccagca atattggtgg   1380
   caagcaggcg ctggagacgg tgcaggcgct gttgccggtg ctgtgccagg cccacggctt   1440
   gaccccccag caggtggtgg ccatcgccag caatggcggt ggcaagcagg cgctggagac   1500
   ggtccagcgg ctgttgccgg tgctgtgcca ggcccacggc ttgacccccc agcaggtggt   1560
   ggccatcgcc agcaataatg gtggcaagca ggcgctggag acggtccagc ggctgttgcc   1620
   ggtgctgtgc caggcccacg gcttgacccc ccagcaggtg gtggccatcg ccagcaataa   1680
   tggtggcaag caggcgctgg agacggtcca gcggctgttg ccggtgctgt gccaggccca   1740
   cggcttgacc ccccagcagg tggtggccat cgccagcaat ggcggtggca agcaggcgct   1800
   ggagacggtc cagcggctgt tgccggtgct gtgccaggcc cacggcttga ccccccagca   1860
   ggtggtggcc atcgccagca atggcggtgg caagcaggcg ctggagacgg tccagcggct   1920
   gttgccggtg ctgtgccagg cccacggctt gaccccggag caggtggtgg ccatcgccag   1980
   caatattggt ggcaagcagg cgctggagac ggtgcaggcg ctgttgccgg tgctgtgcca   2040
   ggcccacggc ttgaccccgg agcaggtggt ggccatcgcc agccacgatg gcggcaagca   2100
   ggcgctggag acggtccagc ggctgttgcc ggtgctgtgc caggcccacg gcttgacccc   2160
   ccagcaggtg gtggccatcg ccagcaatgg cggtggcaag caggcgctgg agacggtcca   2220
   gcggctgttg ccggtgctgt gccaggccca cggcttgacc ccccagcagg tggtggccat   2280
   cgccagcaat ggcggtggca agcaggcgct ggagacggtc cagcggctgt tgccggtgct   2340
   gtgccaggcc cacggcttga ccccggagca ggtggtggcc atcgccagca atattggtgg   2400
   caagcaggcg ctggagacgg tgcaggcgct gttgccggtg ctgtgccagg cccacggctt   2460
   gacccctcag caggtggtgg ccatcgccag caatggcggc ggcaggccgg cgctggagag   2520
   cattgttgcc cagttatctc gccctgatcc ggcgttggcc gcgttgacca acgaccacct   2580
   cgtcgccttg gcctgcctcg gcgggcgtcc tgcgctggat gcagtgaaaa agggattggg   2640
   ggatcctatc agccgttccc agctggtgaa gtccgagctg gaggagaaga aatccgagtt   2700
   gaggcacaag ctgaagtacg tgccccacga gtacatcgag ctgatcgaga tcgcccggaa   2760
   cagcacccag gaccgtatcc tggagatgaa ggtgatggag ttcttcatga aggtgtacgg 2820
   ctacaggggc aagcacctgg gcggctccag gaagcccgac ggcgccatct acaccgtggg 2880
   ctcccccatc gactacggcg tgatcgtgga caccaaggcc tactccggcg gctacaacct 2940
   gcccatcggc caggccgacg aaatgcagag gtacgtggag gagaaccaga ccaggaacaa 3000
   gcacatcaac cccaacgagt ggtggaaggt gtacccctcc agcgtgaccg agttcaagtt 3060
   cctgttcgtg tccggccact tcaagggcaa ctacaaggcc cagctgacca ggctgaacca 3120
   catcaccaac tgcaacggcg ccgtgctgtc cgtggaggag ctcctgatcg gcggcgagat 3180
   gatcaaggcc ggcaccctga ccctggagga ggtgaggagg aagttcaaca acggcgagat 3240
   caacttcgcg gccgactgat aactcgagcg atcctctagg aaagcggccg cggagctcca 3300
   ggaattctgc agatcgactg tgccttctag ttgccagcca tctgttgttt gcccctcccc 3360
   cgtgccttcc ttgaccctgg aaggtgccac tcccactgtc ctttcctaat aaaatgagga 3420
   aattgcatcg cattgtctga gtaggtgtca ttctattctg gggggtgggg tggggcagga 3480
   cagcaagggg gaggattggg aagacaatag caggcatgct ggggatgcgg tgggctctat 3540
   ggatcctcta gagtcgacct gcaggcatgc aagcttggcg taatcatggt catagctgtt 3600
   tcctgtgtga aattgttatc cgctcacaat tccacacaac atacgagccg gaagcataaa 3660
   gtgtaaagcc tggggtgcct aatgagtgag ctaactcaca ttaattgcgt tgcgctcact 3720
   gcccgctttc cagtcgggaa acctgtcgtg ccagctgcat taatgaatcg gccaacgcgc 3780
   ggggagaggc ggtttgcgta ttgggcgctc ttccgcttcc tcgctcactg actcgctgcg 3840
   ctcggtcgtt cggctgcggc gagcggtatc agctcactca aaggcggtaa tacggttatc 3900
   cacagaatca ggggataacg caggaaagaa catgtgagca aaaggccagc aaaaggccag 3960
   gaaccgtaaa aaggccgcgt tgctggcgtt tttccatagg ctccgccccc ctgacgagca 4020
   tcacaaaaat cgacgctcaa gtcagaggtg gcgaaacccg acaggactat aaagatacca 4080
   ggcgtttccc cctggaagct ccctcgtgcg ctctcctgtt ccgaccctgc cgcttaccgg 4140
   atacctgtcc gcctttctcc cttcgggaag cgtggcgctt tctcatagct cacgctgtag 4200
   gtatctcagt tcggtgtagg tcgttcgctc caagctgggc tgtgtgcacg aaccccccgt 4260
   tcagcccgac cgctgcgcct tatccggtaa ctatcgtctt gagtccaacc cggtaagaca 4320
   cgacttatcg ccactggcag cagccactgg taacaggatt agcagagcga ggtatgtagg 4380
   cggtgctaca gagttcttga agtggtggcc taactacggc tacactagaa ggacagtatt 4440
   tggtatctgc gctctgctga agccagttac cttcggaaaa agagttggta gctcttgatc 4500
   cggcaaacaa accaccgctg gtagcggtgg tttttttgtt tgcaagcagc agattacgcg 4560
   cagaaaaaaa ggatctcaag aagatccttt gatcttttct acggggtctg acgctcagtg 4620
   gaacgaaaac tcacgttaag ggattttggt catgagatta tcaaaaagga tcttcaccta 4680
   gatcctttta aattaaaaat gaagttttaa atcaatctaa agtatatatg agtaaacttg 4740
   gtctgacagt taccaatgct taatcagtga ggcacctatc tcagcgatct gtctatttcg 4800
   ttcatccata gttgcctgac tccccgtcgt gtagataact acgatacggg agggcttacc 4860
   atctggcccc agtgctgcaa tgataccgcg agacccacgc tcaccggctc cagatttatc 4920
   agcaataaac cagccagccg gaagggccga gcgcagaagt ggtcctgcaa ctttatccgc 4980
   ctccatccag tctattaatt gttgccggga agctagagta agtagttcgc cagttaatag 5040
   tttgcgcaac gttgttgcca ttgctacagg catcgtggtg tcacgctcgt cgtttggtat 5100
   ggcttcattc agctccggtt cccaacgatc aaggcgagtt acatgatccc ccatgttgtg 5160
   caaaaaagcg gttagctcct tcggtcctcc gatcgttgtc agaagtaagt tggccgcagt 5220
   gttatcactc atggttatgg cagcactgca taattctctt actgtcatgc catccgtaag 5280
   atgcttttct gtgactggtg agtactcaac caagtcattc tgagaatagt gtatgcggcg 5340
   accgagttgc tcttgcccgg cgtcaatacg ggataatacc gcgccacata gcagaacttt 5400
   aaaagtgctc atcattggaa aacgttcttc ggggcgaaaa ctctcaagga tcttaccgct 5460
   gttgagatcc agttcgatgt aacccactcg tgcacccaac tgatcttcag catcttttac 5520
   tttcaccagc gtttctgggt gagcaaaaac aggaaggcaa aatgccgcaa aaaagggaat 5580
   aagggcgaca cggaaatgtt gaatactcat actcttcctt tttcaatatt attgaagcat 5640
   ttatcagggt tattgtctca tgagcggata catatttgaa tgtatttaga aaaataaaca 5700
   aataggggtt ccgcgcacat ttccccgaaa agtgccacct gacgtctaag aaaccattat 5760
   tatcatgaca ttaacctata aaaataggcg tatcacgagg ccctttcgtc tcgcgcgttt 5820
   cggtgatgac ggtgaaaacc tctgacacat gcagctcccg gagacggtca cagcttgtct 5880
   gtaagcggat gccgggagca gacaagcccg tcagggcgcg tcagcgggtg ttggcgggtg 5940
   tcggggctgg cttaactatg cggcatcaga gcagattgta ctgagagtgc accatatgcg 6000
   gtgtgaaata ccgcacagat gcgtaaggag aaaataccgc atcaggcgcc attcgccatt 6060
   caggctgcgc aactgttggg aagggcgatc ggtgcgggcc tcttcgctat tacgccagct 6120
   ggcgaaaggg ggatgtgctg caaggcgatt aagttgggta acgccagggt tttcccagtc 6180
   acgacgttgt aaaacgacgg ccagtgaatt cgcgccaaag ctaactgtag gactgagtct 6240
   attctaaact gaaagcctgg acatctggag taccaggggg agatgacgtg ttacgggctt 6300
   ccataaaagc agctggcttt gaatggaagg agccaagagg ccagcacagg agcggattcg 6360
   tcgctttcac ggccatcgag ccgaacctct cgcaagtccg tgagccgtta aggaggcccc 6420
   cagtcccgac ccttcgcccc aagcccctcg ggtccccggg gcctggtact ccttgccaca 6480
   cgggaggggc gcggaagccg gggcggagga ggagccaacc ccgggctggg ctgagacccg 6540
   cagaggaaga cgctctaggg atttgtcccg gactagcgag atggcaaggc tgaggacggg   6600
   aggctgattg agaggcgaag gtacacccta atctcaatac aacctttgga gctaagccag   6660
   caatggtaga gggaagattc tgcacgtccc ttccaggcgg cctccccgtc accacccccc   6720
   ccaacccgcc ccgaccggag ctgagagtaa ttcatacaaa aggactcgcc cctgccttgg   6780
   ggaatcccag ggaccgtcgt taaactccca ctaacgtaga acccagagat cgctgcgttc   6840
   ccgccccctc acccgcccgc tctcgtcatc actgaggtgg agaagagcat gcgtgaggct   6900
   ccggtgcccg tcagtgggca gagcgcacat cgcccacagt ccccgagaag ttggggggag   6960
   gggtcggcaa ttgaaccggt gcctagagaa ggtggcgcgg ggtaaactgg gaaagtgatg   7020
   tcgtgtactg gctccgcctt tttcccgagg gtgggggaga accgtatata agtgcagtag   7080
   tcgccgtgaa cgttcttttt cgcaacgggt ttgccgccag aacacaggta agtgccgtgt   7140
   gtggttcccg cgggcctggc ctctttacgg gttatggccc ttgcgtgcct tgaattactt   7200
   ccacgcccct ggctgcagta cgtgattctt gatcccgagc ttcgggttgg aagtgggtgg   7260
   gagagttcga ggccttgcgc ttaaggagcc ccttcgcctc gtgcttgagt tgaggcctgg   7320
   cttgggcgct ggggccgccg cgtgcgaatc tggtggcacc ttcgcgcctg tctcgctgct   7380
   ttcgataagt ctctagccat ttaaaatttt tgatgacctg ctgcgacgct ttttttctgg   7440
   caagatagtc ttgtaaatgc gggccaagat cgatctgcac actggtattt cggtttttgg   7500
   ggccgcgggc ggcgacgggg cccgtgcgtc ccagcgcaca tgttcggcga ggcggggcct   7560
   gcgagcgcgg ccaccgagaa tcggacgggg gtagtctcaa gctggccggc ctgctctggt   7620
   gcctggcctc gcgccgccgt gtatcgcccc gccctgggcg gcaaggctgg cccggtcggc   7680
   accagttgcg tgagcggaaa gatggccgct tcccggccct gctgcaggga gctcaaaatg   7740
   gaggacgcgg cgctcgggag agcgggcggg tgagtcaccc acacaaagga aaagggcctt   7800
   tccgtcctca gccgtcgctt catgtgactc cacggagtac cgggcgccgt ccaggcacct   7860
   cgattagttc tcgagctttt ggagtacgtc gtctttaggt tggggggagg ggttttatgc   7920
   gatggagttt ccccacactg agtgggtgga gactgaagtt aggccagctt ggcacttgat   7980
   gtaattctcc ttggaatttg ccctttttga gtttggatct tggttcattc tcaagcctca   8040
   gacagtggtt caaagttttt ttcttccatt tcaggtgtcg tgg                      8083
<210> 212
   <211> 8083
   <212> NUCLEIC
   <213> Artificial sequence
<220>
   <223> pCLS8965
<400> 212
   gtttgtttaa acttggtacc ataactagtt cggcgcgcca ctagcgctgt cacgcgtctc      60
   catggccgac cccattcgtt cgcgcacacc aagtcctgcc cgcgagcttc tgcccggacc     120
   ccaacccgat ggggttcagc cgactgcaga tcgtggggtg tctccgcctg ccggcggccc     180
   cctggatggc ttgccggctc ggcggacgat gtcccggacc cggctgccat ctccccctgc     240
   cccctcacct gcgttctcgg cgggcagctt cagtgacctg ttacgtcagt tcgatccgtc     300
   actttttaat acatcgcttt ttgattcatt gcctcccttc ggcgctcacc atacagaggc     360
   tgccacaggc gagtgggatg aggtgcaatc gggtctgcgg gcagccgacg cccccccacc     420
   caccatgcgc gtggctgtca ctgccgcgcg gcccccgcgc gccaagccgg cgccgcgacg     480
   acgtgctgcg caaccctccg acgcttcgcc ggcggcgcag gtggatctac gcacgctcgg     540
   ctacagccag cagcaacagg agaagatcaa accgaaggtt cgttcgacag tggcgcagca     600
   ccacgaggca ctggtcggcc acgggtttac acacgcgcac atcgttgcgt taagccaaca     660
   cccggcagcg ttagggaccg tcgctgtcaa gtatcaggac atgatcgcag cgttgccaga     720
   ggcgacacac gaagcgatcg ttggcgtcgg caaacagtgg tccggcgcac gcgctctgga     780
   ggccttgctc acggtggcgg gagagttgag aggtccaccg ttacagttgg acacaggcca     840
   acttctcaag attgcaaaac gtggcggcgt gaccgcagtg gaggcagtgc atgcatggcg     900
   caatgcactg acgggtgccc cgctcaactt gaccccggag caggtggtgg ccatcgccag     960
   caatattggt ggcaagcagg cgctggagac ggtgcaggcg ctgttgccgg tgctgtgcca    1020
   ggcccacggc ttgacccccc agcaggtggt ggccatcgcc agcaatggcg gtggcaagca    1080
   ggcgctggag acggtccagc ggctgttgcc ggtgctgtgc caggcccacg gcttgacccc    1140
   ggagcaggtg gtggccatcg ccagcaatat tggtggcaag caggcgctgg agacggtgca    1200
   ggcgctgttg ccggtgctgt gccaggccca cggcttgacc ccccagcagg tggtggccat    1260
   cgccagcaat ggcggtggca agcaggcgct ggagacggtc cagcggctgt tgccggtgct    1320
   gtgccaggcc cacggcttga ccccccagca ggtggtggcc atcgccagca atggcggtgg    1380
   caagcaggcg ctggagacgg tccagcggct gttgccggtg ctgtgccagg cccacggctt    1440
   gaccccccag caggtggtgg ccatcgccag caatggcggt ggcaagcagg cgctggagac    1500
   ggtccagcgg ctgttgccgg tgctgtgcca ggcccacggc ttgaccccgg agcaggtggt 1560
   ggccatcgcc agcaatattg gtggcaagca ggcgctggag acggtgcagg cgctgttgcc 1620
   ggtgctgtgc caggcccacg gcttgacccc ggagcaggtg gtggccatcg ccagcaatat 1680
   tggtggcaag caggcgctgg agacggtgca ggcgctgttg ccggtgctgt gccaggccca 1740
   cggcttgacc ccccagcagg tggtggccat cgccagcaat aatggtggca agcaggcgct 1800
   ggagacggtc cagcggctgt tgccggtgct gtgccaggcc cacggcttga ccccggagca 1860
   ggtggtggcc atcgccagcc acgatggcgg caagcaggcg ctggagacgg tccagcggct 1920
   gttgccggtg ctgtgccagg cccacggctt gaccccggag caggtggtgg ccatcgccag 1980
   caatattggt ggcaagcagg cgctggagac ggtgcaggcg ctgttgccgg tgctgtgcca 2040
   ggcccacggc ttgaccccgg agcaggtggt ggccatcgcc agccacgatg gcggcaagca 2100
   ggcgctggag acggtccagc ggctgttgcc ggtgctgtgc caggcccacg gcttgacccc 2160
   ccagcaggtg gtggccatcg ccagcaatgg cggtggcaag caggcgctgg agacggtcca 2220
   gcggctgttg ccggtgctgt gccaggccca cggcttgacc ccccagcagg tggtggccat 2280
   cgccagcaat ggcggtggca agcaggcgct ggagacggtc cagcggctgt tgccggtgct 2340
   gtgccaggcc cacggcttga ccccggagca ggtggtggcc atcgccagca atattggtgg 2400
   caagcaggcg ctggagacgg tgcaggcgct gttgccggtg ctgtgccagg cccacggctt 2460
   gacccctcag caggtggtgg ccatcgccag caatggcggc ggcaggccgg cgctggagag 2520
   cattgttgcc cagttatctc gccctgatcc ggcgttggcc gcgttgacca acgaccacct 2580
   cgtcgccttg gcctgcctcg gcgggcgtcc tgcgctggat gcagtgaaaa agggattggg 2640
   ggatcctatc agccgttccc agctggtgaa gtccgagctg gaggagaaga aatccgagtt 2700
   gaggcacaag ctgaagtacg tgccccacga gtacatcgag ctgatcgaga tcgcccggaa 2760
   cagcacccag gaccgtatcc tggagatgaa ggtgatggag ttcttcatga aggtgtacgg 2820
   ctacaggggc aagcacctgg gcggctccag gaagcccgac ggcgccatct acaccgtggg 2880
   ctcccccatc gactacggcg tgatcgtgga caccaaggcc tactccggcg gctacaacct 2940
   gcccatcggc caggccgacg aaatgcagag gtacgtggag gagaaccaga ccaggaacaa 3000
   gcacatcaac cccaacgagt ggtggaaggt gtacccctcc agcgtgaccg agttcaagtt 3060
   cctgttcgtg tccggccact tcaagggcaa ctacaaggcc cagctgacca ggctgaacca 3120
   catcaccaac tgcaacggcg ccgtgctgtc cgtggaggag ctcctgatcg gcggcgagat 3180
   gatcaaggcc ggcaccctga ccctggagga ggtgaggagg aagttcaaca acggcgagat 3240
   caacttcgcg gccgactgat aactcgagcg atcctctagg aaagcggccg cggagctcca 3300
   ggaattctgc agatcgactg tgccttctag ttgccagcca tctgttgttt gcccctcccc 3360
   cgtgccttcc ttgaccctgg aaggtgccac tcccactgtc ctttcctaat aaaatgagga 3420
   aattgcatcg cattgtctga gtaggtgtca ttctattctg gggggtgggg tggggcagga 3480
   cagcaagggg gaggattggg aagacaatag caggcatgct ggggatgcgg tgggctctat 3540
   ggatcctcta gagtcgacct gcaggcatgc aagcttggcg taatcatggt catagctgtt 3600
   tcctgtgtga aattgttatc cgctcacaat tccacacaac atacgagccg gaagcataaa 3660
   gtgtaaagcc tggggtgcct aatgagtgag ctaactcaca ttaattgcgt tgcgctcact 3720
   gcccgctttc cagtcgggaa acctgtcgtg ccagctgcat taatgaatcg gccaacgcgc 3780
   ggggagaggc ggtttgcgta ttgggcgctc ttccgcttcc tcgctcactg actcgctgcg 3840
   ctcggtcgtt cggctgcggc gagcggtatc agctcactca aaggcggtaa tacggttatc 3900
   cacagaatca ggggataacg caggaaagaa catgtgagca aaaggccagc aaaaggccag 3960
   gaaccgtaaa aaggccgcgt tgctggcgtt tttccatagg ctccgccccc ctgacgagca 4020
   tcacaaaaat cgacgctcaa gtcagaggtg gcgaaacccg acaggactat aaagatacca 4080
   ggcgtttccc cctggaagct ccctcgtgcg ctctcctgtt ccgaccctgc cgcttaccgg 4140
   atacctgtcc gcctttctcc cttcgggaag cgtggcgctt tctcatagct cacgctgtag 4200
   gtatctcagt tcggtgtagg tcgttcgctc caagctgggc tgtgtgcacg aaccccccgt 4260
   tcagcccgac cgctgcgcct tatccggtaa ctatcgtctt gagtccaacc cggtaagaca 4320
   cgacttatcg ccactggcag cagccactgg taacaggatt agcagagcga ggtatgtagg 4380
   cggtgctaca gagttcttga agtggtggcc taactacggc tacactagaa ggacagtatt 4440
   tggtatctgc gctctgctga agccagttac cttcggaaaa agagttggta gctcttgatc 4500
   cggcaaacaa accaccgctg gtagcggtgg tttttttgtt tgcaagcagc agattacgcg 4560
   cagaaaaaaa ggatctcaag aagatccttt gatcttttct acggggtctg acgctcagtg 4620
   gaacgaaaac tcacgttaag ggattttggt catgagatta tcaaaaagga tcttcaccta 4680
   gatcctttta aattaaaaat gaagttttaa atcaatctaa agtatatatg agtaaacttg 4740
   gtctgacagt taccaatgct taatcagtga ggcacctatc tcagcgatct gtctatttcg 4800
   ttcatccata gttgcctgac tccccgtcgt gtagataact acgatacggg agggcttacc 4860
   atctggcccc agtgctgcaa tgataccgcg agacccacgc tcaccggctc cagatttatc 4920
   agcaataaac cagccagccg gaagggccga gcgcagaagt ggtcctgcaa ctttatccgc 4980
   ctccatccag tctattaatt gttgccggga agctagagta agtagttcgc cagttaatag 5040
   tttgcgcaac gttgttgcca ttgctacagg catcgtggtg tcacgctcgt cgtttggtat 5100
   ggcttcattc agctccggtt cccaacgatc aaggcgagtt acatgatccc ccatgttgtg 5160
   caaaaaagcg gttagctcct tcggtcctcc gatcgttgtc agaagtaagt tggccgcagt 5220
   gttatcactc atggttatgg cagcactgca taattctctt actgtcatgc catccgtaag 5280
   atgcttttct gtgactggtg agtactcaac caagtcattc tgagaatagt gtatgcggcg 5340
   accgagttgc tcttgcccgg cgtcaatacg ggataatacc gcgccacata gcagaacttt 5400
   aaaagtgctc atcattggaa aacgttcttc ggggcgaaaa ctctcaagga tcttaccgct 5460
   gttgagatcc agttcgatgt aacccactcg tgcacccaac tgatcttcag catcttttac 5520
   tttcaccagc gtttctgggt gagcaaaaac aggaaggcaa aatgccgcaa aaaagggaat 5580
   aagggcgaca cggaaatgtt gaatactcat actcttcctt tttcaatatt attgaagcat 5640
   ttatcagggt tattgtctca tgagcggata catatttgaa tgtatttaga aaaataaaca 5700
   aataggggtt ccgcgcacat ttccccgaaa agtgccacct gacgtctaag aaaccattat 5760
   tatcatgaca ttaacctata aaaataggcg tatcacgagg ccctttcgtc tcgcgcgttt 5820
   cggtgatgac ggtgaaaacc tctgacacat gcagctcccg gagacggtca cagcttgtct 5880
   gtaagcggat gccgggagca gacaagcccg tcagggcgcg tcagcgggtg ttggcgggtg 5940
   tcggggctgg cttaactatg cggcatcaga gcagattgta ctgagagtgc accatatgcg 6000
   gtgtgaaata ccgcacagat gcgtaaggag aaaataccgc atcaggcgcc attcgccatt 6060
   caggctgcgc aactgttggg aagggcgatc ggtgcgggcc tcttcgctat tacgccagct 6120
   ggcgaaaggg ggatgtgctg caaggcgatt aagttgggta acgccagggt tttcccagtc 6180
   acgacgttgt aaaacgacgg ccagtgaatt cgcgccaaag ctaactgtag gactgagtct 6240
   attctaaact gaaagcctgg acatctggag taccaggggg agatgacgtg ttacgggctt 6300
   ccataaaagc agctggcttt gaatggaagg agccaagagg ccagcacagg agcggattcg 6360
   tcgctttcac ggccatcgag ccgaacctct cgcaagtccg tgagccgtta aggaggcccc 6420
   cagtcccgac ccttcgcccc aagcccctcg ggtccccggg gcctggtact ccttgccaca 6480
   cgggaggggc gcggaagccg gggcggagga ggagccaacc ccgggctggg ctgagacccg 6540
   cagaggaaga cgctctaggg atttgtcccg gactagcgag atggcaaggc tgaggacggg 6600
   aggctgattg agaggcgaag gtacacccta atctcaatac aacctttgga gctaagccag 6660
   caatggtaga gggaagattc tgcacgtccc ttccaggcgg cctccccgtc accacccccc 6720
   ccaacccgcc ccgaccggag ctgagagtaa ttcatacaaa aggactcgcc cctgccttgg 6780
   ggaatcccag ggaccgtcgt taaactccca ctaacgtaga acccagagat cgctgcgttc 6840
   ccgccccctc acccgcccgc tctcgtcatc actgaggtgg agaagagcat gcgtgaggct 6900
   ccggtgcccg tcagtgggca gagcgcacat cgcccacagt ccccgagaag ttggggggag 6960
   gggtcggcaa ttgaaccggt gcctagagaa ggtggcgcgg ggtaaactgg gaaagtgatg 7020
   tcgtgtactg gctccgcctt tttcccgagg gtgggggaga accgtatata agtgcagtag 7080
   tcgccgtgaa cgttcttttt cgcaacgggt ttgccgccag aacacaggta agtgccgtgt 7140
   gtggttcccg cgggcctggc ctctttacgg gttatggccc ttgcgtgcct tgaattactt 7200
   ccacgcccct ggctgcagta cgtgattctt gatcccgagc ttcgggttgg aagtgggtgg 7260
   gagagttcga ggccttgcgc ttaaggagcc ccttcgcctc gtgcttgagt tgaggcctgg 7320
   cttgggcgct ggggccgccg cgtgcgaatc tggtggcacc ttcgcgcctg tctcgctgct 7380
   ttcgataagt ctctagccat ttaaaatttt tgatgacctg ctgcgacgct ttttttctgg 7440
   caagatagtc ttgtaaatgc gggccaagat cgatctgcac actggtattt cggtttttgg 7500
   ggccgcgggc ggcgacgggg cccgtgcgtc ccagcgcaca tgttcggcga ggcggggcct 7560
   gcgagcgcgg ccaccgagaa tcggacgggg gtagtctcaa gctggccggc ctgctctggt 7620
   gcctggcctc gcgccgccgt gtatcgcccc gccctgggcg gcaaggctgg cccggtcggc 7680
   accagttgcg tgagcggaaa gatggccgct tcccggccct gctgcaggga gctcaaaatg 7740
   gaggacgcgg cgctcgggag agcgggcggg tgagtcaccc acacaaagga aaagggcctt 7800
   tccgtcctca gccgtcgctt catgtgactc cacggagtac cgggcgccgt ccaggcacct 7860
   cgattagttc tcgagctttt ggagtacgtc gtctttaggt tggggggagg ggttttatgc 7920
   gatggagttt ccccacactg agtgggtgga gactgaagtt aggccagctt ggcacttgat 7980
   gtaattctcc ttggaatttg ccctttttga gtttggatct tggttcattc tcaagcctca 8040
   gacagtggtt caaagttttt ttcttccatt tcaggtgtcg tgg 8083
<210> 213
   <211> 5428
   <212> NUCLEIC
   <213> Artificial sequence
<220>
   <223> pCLS0003
<400> 213
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggag tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctagc 900
   gtttaaactt aagcttggta ccgagctcgg atccactagt ccagtgtggt ggaattctgc 960
   agatatccag cacagtggcg gccgctcgag tctagagggc ccgtttaaac ccgctgatca 1020
   gcctcgactg tgccttctag ttgccagcca tctgttgttt gcccctcccc cgtgccttcc 1080
   ttgaccctgg aaggtgccac tcccactgtc ctttcctaat aaaatgagga aattgcatcg 1140
   cattgtctga gtaggtgtca ttctattctg gggggtgggg tggggcagga cagcaagggg 1200
   gaggattggg aagacaatag caggcatgct ggggatgcgg tgggctctat ggcttctgag 1260
   gcggaaagaa ccagctgggg ctctaggggg tatccccacg cgccctgtag cggcgcatta 1320
   agcgcggcgg gtgtggtggt tacgcgcagc gtgaccgcta cacttgccag cgccctagcg 1380
   cccgctcctt tcgctttctt cccttccttt ctcgccacgt tcgccggctt tccccgtcaa 1440
   gctctaaatc gggggctccc tttagggttc cgatttagtg ctttacggca cctcgacccc 1500
   aaaaaacttg attagggtga tggttcacgt agtgggccat cgccctgata gacggttttt 1560
   cgccctttga cgttggagtc cacgttcttt aatagtggac tcttgttcca aactggaaca 1620
   acactcaacc ctatctcggt ctattctttt gatttataag ggattttgcc gatttcggcc 1680
   tattggttaa aaaatgagct gatttaacaa aaatttaacg cgaattaatt ctgtggaatg 1740
   tgtgtcagtt agggtgtgga aagtccccag gctccccagc aggcagaagt atgcaaagca 1800
   tgcatctcaa ttagtcagca accaggtgtg gaaagtcccc aggctcccca gcaggcagaa 1860
   gtatgcaaag catgcatctc aattagtcag caaccatagt cccgccccta actccgccca 1920
   tcccgcccct aactccgccc agttccgccc attctccgcc ccatggctga ctaatttttt 1980
   ttatttatgc agaggccgag gccgcctctg cctctgagct attccagaag tagtgaggag 2040
   gcttttttgg aggcctaggc ttttgcaaaa agctcccggg agcttgtata tccattttcg 2100
   gatctgatca agagacagga tgaggatcgt ttcgcatgat tgaacaagat ggattgcacg 2160
   caggttctcc ggccgcttgg gtggagaggc tattcggcta tgactgggca caacagacaa 2220
   tcggctgctc tgatgccgcc gtgttccggc tgtcagcgca ggggcgcccg gttctttttg 2280
   tcaagaccga cctgtccggt gccctgaatg aactgcagga cgaggcagcg cggctatcgt 2340
   ggctggccac gacgggcgtt ccttgcgcag ctgtgctcga cgttgtcact gaagcgggaa 2400
   gggactggct gctattgggc gaagtgccgg ggcaggatct cctgtcatct caccttgctc 2460
   ctgccgagaa agtatccatc atggctgatg caatgcggcg gctgcatacg cttgatccgg 2520
   ctacctgccc attcgaccac caagcgaaac atcgcatcga gcgagcacgt actcggatgg 2580
   aagccggtct tgtcgatcag gatgatctgg acgaagagca tcaggggctc gcgccagccg 2640
   aactgttcgc caggctcaag gcgcgcatgc ccgacggcga ggatctcgtc gtgacccatg 2700
   gcgatgcctg cttgccgaat atcatggtgg aaaatggccg cttttctgga ttcatcgact 2760
   gtggccggct gggtgtggcg gaccgctatc aggacatagc gttggctacc cgtgatattg 2820
   ctgaagagct tggcggcgaa tgggctgacc gcttcctcgt gctttacggt atcgccgctc 2880
   ccgattcgca gcgcatcgcc ttctatcgcc ttcttgacga gttcttctga gcgggactct 2940
   ggggttcgaa atgaccgacc aagcgacgcc caacctgcca tcacgagatt tcgattccac 3000
   cgccgccttc tatgaaaggt tgggcttcgg aatcgttttc cgggacgccg gctggatgat 3060
   cctccagcgc ggggatctca tgctggagtt cttcgcccac cccaacttgt ttattgcagc 3120
   ttataatggt tacaaataaa gcaatagcat cacaaatttc acaaataaag catttttttc 3180
   actgcattct agttgtggtt tgtccaaact catcaatgta tcttatcatg tctgtatacc 3240
   gtcgacctct agctagagct tggcgtaatc atggtcatag ctgtttcctg tgtgaaattg 3300
   ttatccgctc acaattccac acaacatacg agccggaagc ataaagtgta aagcctgggg 3360
   tgcctaatga gtgagctaac tcacattaat tgcgttgcgc tcactgcccg ctttccagtc 3420
   gggaaacctg tcgtgccagc tgcattaatg aatcggccaa cgcgcgggga gaggcggttt 3480
   gcgtattggg cgctcttccg cttcctcgct cactgactcg ctgcgctcgg tcgttcggct 3540
   gcggcgagcg gtatcagctc actcaaaggc ggtaatacgg ttatccacag aatcagggga 3600
   taacgcagga aagaacatgt gagcaaaagg ccagcaaaag gccaggaacc gtaaaaaggc 3660
   cgcgttgctg gcgtttttcc ataggctccg cccccctgac gagcatcaca aaaatcgacg 3720
   ctcaagtcag aggtggcgaa acccgacagg actataaaga taccaggcgt ttccccctgg 3780
   aagctccctc gtgcgctctc ctgttccgac cctgccgctt accggatacc tgtccgcctt 3840
   tctcccttcg ggaagcgtgg cgctttctca tagctcacgc tgtaggtatc tcagttcggt 3900
   gtaggtcgtt cgctccaagc tgggctgtgt gcacgaaccc cccgttcagc ccgaccgctg 3960
   cgccttatcc ggtaactatc gtcttgagtc caacccggta agacacgact tatcgccact 4020
   ggcagcagcc actggtaaca ggattagcag agcgaggtat gtaggcggtg ctacagagtt   4080
   cttgaagtgg tggcctaact acggctacac tagaagaaca gtatttggta tctgcgctct   4140
   gctgaagcca gttaccttcg gaaaaagagt tggtagctct tgatccggca aacaaaccac   4200
   cgctggtagc ggtttttttg tttgcaagca gcagattacg cgcagaaaaa aaggatctca   4260
   agaagatcct ttgatctttt ctacggggtc tgacgctcag tggaacgaaa actcacgtta   4320
   agggattttg gtcatgagat tatcaaaaag gatcttcacc tagatccttt taaattaaaa   4380
   atgaagtttt aaatcaatct aaagtatata tgagtaaact tggtctgaca gttaccaatg   4440
   cttaatcagt gaggcaccta tctcagcgat ctgtctattt cgttcatcca tagttgcctg   4500
   actccccgtc gtgtagataa ctacgatacg ggagggctta ccatctggcc ccagtgctgc   4560
   aatgataccg cgagacccac gctcaccggc tccagattta tcagcaataa accagccagc   4620
   cggaagggcc gagcgcagaa gtggtcctgc aactttatcc gcctccatcc agtctattaa   4680
   ttgttgccgg gaagctagag taagtagttc gccagttaat agtttgcgca acgttgttgc   4740
   cattgctaca ggcatcgtgg tgtcacgctc gtcgtttggt atggcttcat tcagctccgg   4800
   ttcccaacga tcaaggcgag ttacatgatc ccccatgttg tgcaaaaaag cggttagctc   4860
   cttcggtcct ccgatcgttg tcagaagtaa gttggccgca gtgttatcac tcatggttat   4920
   ggcagcactg cataattctc ttactgtcat gccatccgta agatgctttt ctgtgactgg   4980
   tgagtactca accaagtcat tctgagaata gtgtatgcgg cgaccgagtt gctcttgccc   5040
   ggcgtcaata cgggataata ccgcgccaca tagcagaact ttaaaagtgc tcatcattgg   5100
   aaaacgttct tcggggcgaa aactctcaag gatcttaccg ctgttgagat ccagttcgat   5160
   gtaacccact cgtgcaccca actgatcttc agcatctttt actttcacca gcgtttctgg   5220
   gtgagcaaaa acaggaaggc aaaatgccgc aaaaaaggga ataagggcga cacggaaatg   5280
   ttgaatactc atactcttcc tttttcaata ttattgaagc atttatcagg gttattgtct   5340
   catgagcgga tacatatttg aatgtattta gaaaaataaa caaatagggg ttccgcgcac   5400
   atttccccga aaagtgccac ctgacgtc                                      5428
<210> 214
   <211> 57
   <212> NUCLEIC
   <213> Artificial sequence
<220>
   <223> F_T2 primer
<400> 214
   ccatctcatc cctgcgtgtc tccgactcag tagctttaca tttactgaac aaataac 57
<210> 215
   <211> 57
   <212> NUCLEIC
   <213> Artificial sequence
<220>
   <223> R_T1 primer
<400> 215
   cctatcccct gtgtgccttg gcagtctcag gatctcaccc ggaacagctt aaatttc 57
<210> 216
   <211> 354
   <212> PRT
   <213> Artificial sequence
<220>
   <223> SC_RAG
<400> 216
   Met Ala Asn Thr Lys Tyr Asn Glu Glu Phe Leu Leu Tyr Leu Ala Gly
   1 5 10 15
   Phe Val Asp Gly Asp Gly Ser Ile Ile Ala Gln Ile Asn Pro Asn Gln
   20 25 30
   Ser Ser Lys Phe Lys His Arg Leu Arg Leu Thr Phe Tyr Val Thr Gln
   35 40 45
   Lys Thr Gln Arg Arg Trp Phe Leu Asp Lys Leu Val Asp Glu Ile Gly
   50 55 60
   Val Gly Tyr Val Arg Asp Ser Gly Ser Val Ser Gln Tyr Val Leu Ser
   65 70 75 80
   Glu Ile Lys Pro Leu His Asn Phe Leu Thr Gln Leu Gln Pro Phe Leu
   85 90 95
   Glu Leu Lys Gln Lys Gln Ala Asn Leu Val Leu Lys Ile Ile Glu Gln
   100 105 110
   Leu Pro Ser Ala Lys Glu Ser Pro Asp Lys Phe Leu Glu Val Cys Thr
   115 120 125
   Trp Val Asp Gln Ile Ala Ala Leu Asn Asp Ser Lys Thr Arg Lys Thr
   130 135 140
   Thr Ser Glu Thr Val Arg Ala Val Leu Asp Ser Leu Ser Gly Lys Lys
   145 150 155 160
   Lys Ser Ser Pro Ala Ala Gly Gly Ser Asp Lys Tyr Asn Gln Ala Leu
   165 170 175
   Ser Lys Tyr Asn Gln Ala Leu Ser Lys Tyr Asn Gln Ala Leu Ser Gly
   180 185 190
   Gly Gly Gly Ser Asn Lys Lys Phe Leu Leu Tyr Leu Ala Gly Phe Val
   195 200 205
   Asp Ser Asp Gly Ser Ile Ile Ala Gln Ile Lys Pro Arg Gln Ser Asn
   210 215 220
   Lys Phe Lys His Gln Leu Ser Leu Thr Phe Ala Val Thr Gln Lys Thr
   225 230 235 240
   Gln Arg Arg Trp Phe Leu Asp Lys Leu Val Asp Arg Ile Gly Val Gly
   245 250 255
   Tyr Val Tyr Asp Ser Gly Ser Val Ser Asp Tyr Arg Leu Ser Glu Ile
               260                 265                 270
   Lys Pro Leu His Asn Phe Leu Thr Gln Leu Gln Pro Phe Leu Lys Leu
           275                 280                 285
   Lys Gln Lys Gln Ala Asn Leu Val Leu Lys Ile Ile Glu Gln Leu Pro
       290                 295                 300
   Ser Ala Lys Glu Ser Pro Asp Lys Phe Leu Glu Val Cys Thr Trp Val
   305                 310                 315                 320
   Asp Gln Ile Ala Ala Leu Asn Asp Ser Lys Thr Arg Lys Thr Thr Ser
                   325                 330                 335
   Glu Thr Val Arg Ala Val Leu Asp Ser Leu Ser Glu Lys Lys Lys Ser
               340                 345                 350
   Ser Pro